# EUROPEAN PATENT SPECIFICATION

(11) **EP 4 216 933 B1**
(45) Date of publication and mention of the grant of the patent: **18.09.2024**
(21) Application number: 21799143.9
(22) Date of filing: 23.09.2021
(51) Int. Cl.: A61K 9/127, A61K 48/00, A61K 9/51

(54) **PIPERAZINE-BASED CATIONIC LIPIDS**
KATIONISCHE LIPIDE AUF PIPERAZINBASIS
LIPIDES CATIONIQUES À BASE DE PIPÉRAZINE

(30) Priority: 23.09.2020 US 202063082101 P
(43) Date of publication of application: 02.08.2023
(73) Proprietor: Translate Bio, Inc., Waltham, MA 02451 (US)
(72) Inventor: KARMAKAR, Saswata, Waltham, MA 02451 (US); DASARI, Ramesh, Waltham, MA 02451 (US); LANDIS, Ryan, Waltham, MA 02451 (US)
(74) Representative: Carpmaels & Ransford LLP
(86) International application number: PCT/US2021/051763
(87) International publication number: WO 2022/066916

(56) References cited:
- US-A1- 2015 376 144
- ISLAM R U ET AL: "Efficient nucleic acid transduction with lipoplexes containing novel piperazine- and polyamine-conjugated cholesterol derivatives", BIOORGANIC & MEDICINAL CHEMISTRY LETTERS, ELSEVIER, AMSTERDAM, NL, vol. 19, no. 1, 1 January 2009 (2009-01-01), pages 100 - 103, XP025816882, ISSN: 0960-894X, [retrieved on 20081106], DOI: 10.1016/J.BMCL.2008.11.009

## Description

### RELATED APPLICATIONS

This application claims priority to U.S. Provisional Application Serial Number 63/082,101, filed on September 23, 2020.

### BACKGROUND

Delivery of nucleic acids has been explored extensively as a potential therapeutic option for certain disease states. In particular, messenger RNA (mRNA) therapy has become an increasingly important option for treatment of various diseases, including for those associated with deficiency of one or more proteins. Patent document US 2015/0376144 discloses stereochemically enriched compositions for delivery of nucleic acids.

Efficient delivery of liposome-encapsulated nucleic acids remains an active area of research. The cationic lipid component plays an important role in facilitating effective encapsulation of the nucleic acid during the loading of liposomes. In addition, cationic lipids may play an important role in the efficient release of the nucleic acid cargo from the liposome into the cytoplasm of a target cell. Various cationic lipids suitable for *in vivo* use have been discovered. However, there remains a need to identify lipids that can be synthesized efficiently and cheaply without the formation of potentially toxic by-products.

### SUMMARY OF THE INVENTION

The present invention provides, among other things, cationic lipid compounds for *in vivo* delivery of therapeutic agents, such as nucleic acids. It is contemplated that these compounds are capable of highly effective *in vivo* delivery while maintaining a favorable toxicity profile.

The cationic lipids of the present invention can be synthesized from readily available starting reagents. The cationic lipids of the present invention also have unexpectedly high encapsulation efficiencies. The cationic lipids of the present invention also comprise cleavable groups (e.g., esters and disulphides) that are contemplated to improve biodegradability and thus contribute to their favorable toxicity profile.

In an aspect, provided herein are cationic lipids having a structure according to Formula (I'): 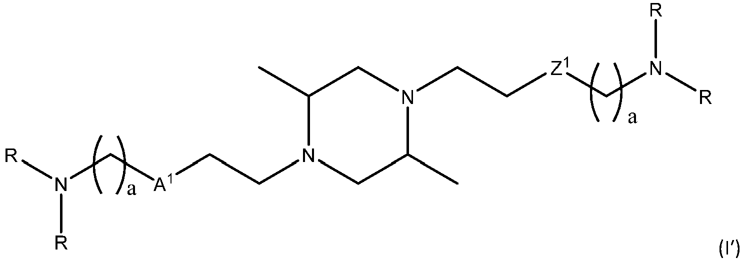 or a pharmaceutically acceptable salt thereof wherein:
**A¹** is selected from 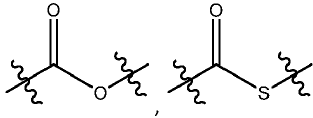 and -S-S-, wherein the left hand side of each depicted structure is bound to the -(CH₂)ₐ-;
**Z¹** is selected from 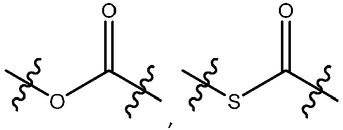 and -S-S-, wherein the right hand side of each depicted structure is bound to the -(CH₂)ₐ-;
each **R** is independently selected from:
   (i) 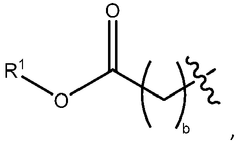 wherein each **R¹** is independently selected from optionally substituted alkyl, optionally substituted alkenyl, optionally substituted alkynyl, - optionally substituted alkyl-(C=O)-O-optionally substituted alkyl, and -optionally substituted alkyl-O-(C=O)-optionally substituted alkyl; and
   (ii) 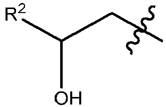 wherein each **R²** is independently selected from optionally substituted alkyl, optionally substituted alkenyl, optionally substituted alkynyl, and optionally substituted acyl;
each **a** is independently selected from 2, 3, 4, and 5; and
each **b** is independently selected from 2, 3, 4, 5, 6 and 7.

In an aspect, provided herein are cationic lipids having a structure according to Formula (I): 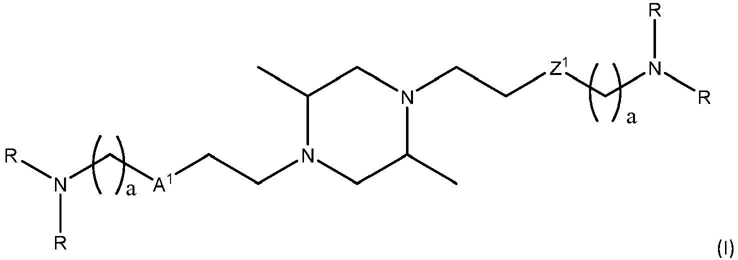 or a pharmaceutically acceptable salt thereof wherein:
**A¹** is selected from 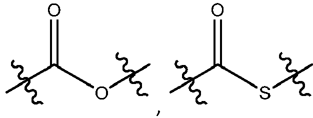 and -S-S-, wherein the left hand side of each depicted structure is bound to the -(CH₂)ₐ-;
**Z¹** is selected from 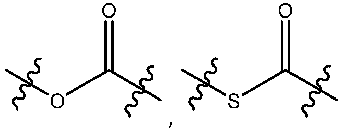 and -S-S-, wherein the right hand side of each depicted structure is bound to the -(CH₂)ₐ-;
each **R** is independently selected from:
   (iii) 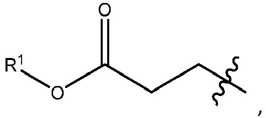 wherein each **R¹** is independently selected from optionally substituted alkyl, optionally substituted alkenyl, optionally substituted alkynyl, -optionally substituted alkyl-(C=O)-O-optionally substituted alkyl, and -optionally substituted alkyl-O-(C=O)-optionally substituted alkyl; and
   (iv) 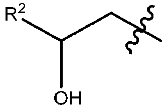 wherein each **R²** is independently selected from optionally substituted alkyl, optionally substituted alkenyl, optionally substituted alkynyl, and optionally substituted acyl; and
each **a** is independently selected from 2, 3, 4, and 5.

In an aspect, provided herein are cationic lipids that are pharmaceutically acceptable salts of Formula (I').

In an aspect, provided herein are cationic lipids that are pharmaceutically acceptable salts of Formula (I).

In an aspect, provided herein are compositions comprising the cationic lipid of the present invention, one or more non-cationic lipids, one or more cholesterol-based lipids and one or more PEG-modified lipid. In an aspect, the composition is a lipid nanoparticle, optionally a liposome.

In an aspect, the compositions comprising the cationic lipids of the present invention may be compositions for use in therapy.

### BRIEF DESCRIPTION OF DRAWINGS

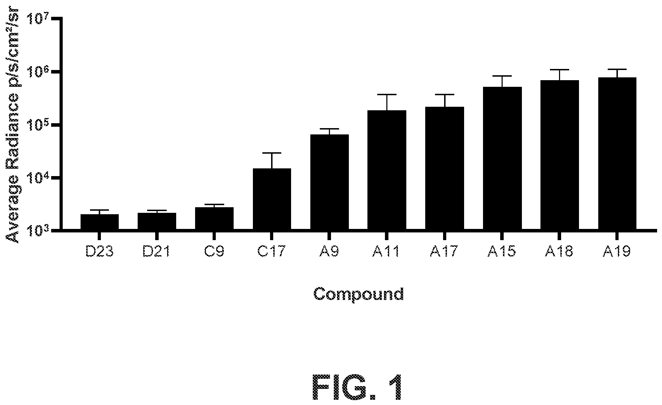

**FIG. 1** depicts *in vivo* protein production resulting from the delivery of mRNA (*i.e.*, FFL mRNA) using lipid nanoparticles comprising Compound **D23, D21, C9, C17, A9, A11, A17, A15, A18,** or **A19** as described herein. As shown in this Figure, use of these compounds can result in high levels of *in vivo* protein production (*i.e.*, FFL protein) after administration.

### DETAILED DESCRIPTION OF CERTAIN EMBODIMENTS

### Definitions

In order for the present invention to be more readily understood, certain terms are first defined below. Additional definitions for the following terms and other terms are set forth throughout the specification.

*Amino acid:* As used herein, the term "amino acid," in its broadest sense, refers to any compound and/or substance that can be incorporated into a polypeptide chain. In some embodiments, an amino acid has the general structure H₂N-C(H)(R)-COOH. In some embodiments, an amino acid is a naturally occurring amino acid. In some embodiments, an amino acid is a synthetic amino acid; in some embodiments, an amino acid is a d-amino acid; in some embodiments, an amino acid is an l-amino acid. "Standard amino acid" refers to any of the twenty standard l-amino acids commonly found in naturally occurring peptides. "Nonstandard amino acid" refers to any amino acid, other than the standard amino acids, regardless of whether it is prepared synthetically or obtained from a natural source. As used herein, "synthetic amino acid" encompasses chemically modified amino acids, including but not limited to salts, amino acid derivatives (such as amides), and/or substitutions. Amino acids, including carboxy- and/or amino-terminal amino acids in peptides, can be modified by methylation, amidation, acetylation, protecting groups, and/or substitution with other chemical groups that can change the peptide's circulating half-life without adversely affecting their activity. Amino acids may participate in a disulfide bond. Amino acids may comprise one or posttranslational modifications, such as association with one or more chemical entities (*e.g.,* methyl groups, acetate groups, acetyl groups, phosphate groups, formyl moieties, isoprenoid groups, sulfate groups, polyethylene glycol moieties, lipid moieties, carbohydrate moieties, biotin moieties, *etc*.). The term "amino acid" is used interchangeably with "amino acid residue," and may refer to a free amino acid and/or to an amino acid residue of a peptide. It will be apparent from the context in which the term is used whether it refers to a free amino acid or a residue of a peptide.

*Animal:* As used herein, the term "animal" refers to any member of the animal kingdom. In some embodiments, "animal" refers to humans, at any stage of development. In some embodiments, "animal" refers to non-human animals, at any stage of development. In certain embodiments, the non-human animal is a mammal (*e.g.,* a rodent, a mouse, a rat, a rabbit, a monkey, a dog, a cat, a sheep, a bovine, a primate, and/or a pig). In some embodiments, animals include, but are not limited to, mammals, birds, reptiles, amphibians, fish, insects, and/or worms. In some embodiments, an animal may be a transgenic animal, genetically-engineered animal, and/or a clone.

*Approximately or about:* As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

*Biologically active:* As used herein, the term "biologically active" refers to a characteristic of any agent that has activity in a biological system, and particularly in an organism. For instance, an agent that, when administered to an organism, has a biological effect on that organism, is considered to be biologically active.

*Delivery:* As used herein, the term "delivery" encompasses both local and systemic delivery. For example, delivery of mRNA encompasses situations in which an mRNA is delivered to a target tissue and the encoded protein is expressed and retained within the target tissue (also referred to as "local distribution" or "local delivery"), and situations in which an mRNA is delivered to a target tissue and the encoded protein is expressed and secreted into patient's circulation system (*e.g.,* serum) and systematically distributed and taken up by other tissues (also referred to as "systemic distribution" or "systemic delivery").

*Expression:* As used herein, "expression" of a nucleic acid sequence refers to translation of an mRNA into a polypeptide, assemble multiple polypeptides into an intact protein (*e.g.,* enzyme) and/or post-translational modification of a polypeptide or fully assembled protein (*e.g.,* enzyme). In this application, the terms "expression" and "production," and grammatical equivalents thereof, are used interchangeably.

*Functional:* As used herein, a "functional" biological molecule is a biological molecule in a form in which it exhibits a property and/or activity by which it is characterized.

*Half-life:* As used herein, the term "half-life" is the time required for a quantity such as nucleic acid or protein concentration or activity to fall to half of its value as measured at the beginning of a time period.

*Helper lipid:* The term "helper lipid" as used herein refers to any neutral or zwitterionic lipid material including cholesterol. Without wishing to be held to a particular theory, helper lipids may add stability, rigidity, and/or fluidity within lipid bilayers/nanoparticles.

*Improve, increase, or reduce:* As used herein, the terms "improve," "increase," or "reduce," or grammatical equivalents, indicate values that are relative to a baseline measurement, such as a measurement in the same individual prior to initiation of the treatment described herein, or a measurement in a control subject (or multiple control subject) in the absence of the treatment described herein. A "control subject" is a subject afflicted with the same form of disease as the subject being treated, who is about the same age as the subject being treated.

*In Vitro:* As used herein, the term *"in vitro"* refers to events that occur in an artificial environment, *e.g.,* in a test tube or reaction vessel, in cell culture, *etc.,* rather than within a multi-cellular organism.

*In Vivo:* As used herein, the term *"in vivo"* refers to events that occur within a multi-cellular organism, such as a human and a non-human animal. In the context of cell-based systems, the term may be used to refer to events that occur within a living cell (as opposed to, for example, *in vitro* systems).

*Isolated:* As used herein, the term "isolated" refers to a substance and/or entity that has been (1) separated from at least some of the components with which it was associated when initially produced (whether in nature and/or in an experimental setting), and/or (2) produced, prepared, and/or manufactured by the hand of man. isolated substances and/or entities may be separated from about 10%, about 20%, about 30%, about 40%, about 50%, about 60%, about 70%, about 80%, about 90%, about 91%, about 92%, about 93%, about 94%, about 95%, about 96%, about 97%, about 98%, about 99%, or more than about 99% of the other components with which they were initially associated. In some embodiments, isolated agents are about 80%, about 85%, about 90%, about 91%, about 92%, about 93%, about 94%, about 95%, about 96%, about 97%, about 98%, about 99%, or more than about 99% pure. As used herein, a substance is "pure" if it is substantially free of other components. As used herein, calculation of percent purity of isolated substances and/or entities should not include excipients (*e.g.,* buffer, solvent, water, *etc*.).

*Liposome:* As used herein, the term "liposome" refers to any lamellar, multilamellar, or solid nanoparticle vesicle. Typically, a liposome as used herein can be formed by mixing one or more lipids or by mixing one or more lipids and polymer(s). In some embodiments, a liposome suitable for the present invention contains a cationic lipids(s) and optionally non-cationic lipid(s), optionally cholesterol-based lipid(s), and/or optionally PEG-modified lipid(s).

*messenger RNA (mRNA):* As used herein, the term "messenger RNA (mRNA)" or "mRNA" refers to a polynucleotide that encodes at least one polypeptide. mRNA as used herein encompasses both modified and unmodified RNA. The term "modified mRNA" related to mRNA comprising at least one chemically modified nucleotide. mRNA may contain one or more coding and non-coding regions. mRNA can be purified from natural sources, produced using recombinant expression systems and optionally purified, chemically synthesized, *etc.* Where appropriate, *e.g.,* in the case of chemically synthesized molecules, mRNA can comprise nucleoside analogs such as analogs having chemically modified bases or sugars, backbone modifications, *etc.* An mRNA sequence is presented in the 5' to 3' direction unless otherwise indicated. In some embodiments, an mRNA is or comprises natural nucleosides (*e.g.,* adenosine, guanosine, cytidine, uridine); nucleoside analogs (*e.g.*, 2-aminoadenosine, 2-thiothymidine, inosine, pyrrolo-pyrimidine, 3-methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, and 2-thiocytidine); chemically modified bases; biologically modified bases (*e.g.,* methylated bases); intercalated bases; modified sugars (*e.g.,* 2'-fluororibose, ribose, 2'-deoxyribose, arabinose, and hexose); and/or modified phosphate groups (*e.g.,* phosphorothioates and 5'-*N-*phosphoramidite linkages).

*Nucleic acid:* As used herein, the term "nucleic acid," in its broadest sense, refers to any compound and/or substance that is or can be incorporated into a polynucleotide chain. In some embodiments, a nucleic acid is a compound and/or substance that is or can be incorporated into a polynucleotide chain via a phosphodiester linkage. In some embodiments, "nucleic acid" refers to individual nucleic acid residues (*e.g.,* nucleotides and/or nucleosides). In some embodiments, "nucleic acid" refers to a polynucleotide chain comprising individual nucleic acid residues. In some embodiments, "nucleic acid" encompasses RNA as well as single and/or double-stranded DNA and/or cDNA. In some embodiments, "nucleic acid" encompasses ribonucleic acids (RNA), including but not limited to any one or more of interference RNAs (RNAi), small interfering RNA (siRNA), short hairpin RNA (shRNA), antisense RNA (aRNA), messenger RNA (mRNA), modified messenger RNA (mmRNA), long non-coding RNA (lncRNA), micro-RNA (miRNA) multimeric coding nucleic acid (MCNA), polymeric coding nucleic acid (PCNA), guide RNA (gRNA) and CRISPR RNA (crRNA). In some embodiments, "nucleic acid" encompasses deoxyribonucleic acid (DNA), including but not limited to any one or more of single-stranded DNA (ssDNA), double-stranded DNA (dsDNA) and complementary DNA (cDNA). In some embodiments, "nucleic acid" encompasses both RNA and DNA. In embodiments, DNA may be in the form of antisense DNA, plasmid DNA, parts of a plasmid DNA, pre-condensed DNA, a product of a polymerase chain reaction (PCR), vectors (*e.g.,* P1, PAC, BAC, YAC, artificial chromosomes), expression cassettes, chimeric sequences, chromosomal DNA, or derivatives of these groups. In embodiments, RNA may be in the form of messenger RNA (mRNA), ribosomal RNA (rRNA), signal recognition particle RNA (7 SL RNA or SRP RNA), transfer RNA (tRNA), transfer-messenger RNA (tmRNA), small nuclear RNA (snRNA), small nucleolar RNA (snoRNA), SmY RNA, small Cajal body-specific RNA (scaRNA), guide RNA (gRNA), ribonuclease P (RNase P), Y RNA, telomerase RNA component (TERC), spliced leader RNA (SL RNA), antisense RNA (aRNA or asRNA), cis-natural antisense transcript (cis-NAT), CRISPR RNA (crRNA), long noncoding RNA (lncRNA), micro-RNA (miRNA), piwi-interacting RNA (piRNA), small interfering RNA (siRNA), transacting siRNA (tasiRNA), repeat associated siRNA (rasiRNA), 73K RNA, retrotransposons, a viral genome, a viroid, satellite RNA, or derivatives of these groups. In some embodiments, a nucleic acid is a mRNA encoding a protein such as an enzyme.

*Patient:* As used herein, the term "patient" or "subject" refers to any organism to which a provided composition may be administered, *e.g.,* for experimental, diagnostic, prophylactic, cosmetic, and/or therapeutic purposes. Typical patients include animals (*e.g.,* mammals such as mice, rats, rabbits, non-human primates, and/or humans). In some embodiments, a patient is a human. A human includes pre- and post-natal forms.

*Pharmaceutically acceptable:* The term "pharmaceutically acceptable," as used herein, refers to substances that, within the scope of sound medical judgment, are suitable for use in contact with the tissues of human beings and animals without excessive toxicity, irritation, allergic response, or other problem or complication, commensurate with a reasonable benefit/risk ratio.

*Pharmaceutically acceptable salt:* Pharmaceutically acceptable salts are well known in the art. For example, S. M. Berge et al., describes pharmaceutically acceptable salts in detail in J. Pharmaceutical Sciences (1977) 66:1-19. Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid, and perchloric acid or with organic acids such as acetic acid, oxalic acid, maleic acid, tartaric acid, citric acid, succinic acid, or malonic acid, or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium and N⁺(C₁₋₄ alkyl)₄ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, sulfonate, and aryl sulfonate. Further pharmaceutically acceptable salts include salts formed from the quarternization of an amine using an appropriate electrophile, *e.g.,* an alkyl halide, to form a quarternized alkylated amino salt.

*Systemic distribution or delivery:* As used herein, the terms "systemic distribution" or "systemic delivery," or grammatical equivalents thereof, refer to a delivery or distribution mechanism or approach that affect the entire body or an entire organism. Typically, systemic distribution or delivery is accomplished via body's circulation system, *e.g.,* blood stream. Compared to the definition of "local distribution or delivery."

*Subject:* As used herein, the term "subject" refers to a human or any non-human animal (*e.g.,* mouse, rat, rabbit, dog, cat, cattle, swine, sheep, horse or primate). A human includes pre- and post-natal forms. In many embodiments, a subject is a human being. A subject can be a patient, which refers to a human presenting to a medical provider for diagnosis or treatment of a disease. The term "subject" is used herein interchangeably with "individual" or "patient." A subject can be afflicted with or is susceptible to a disease or disorder but may or may not display symptoms of the disease or disorder.

*Substantially:* As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

*Target tissues:* As used herein, the term "target tissues" refers to any tissue that is affected by a disease to be treated. In some embodiments, target tissues include those tissues that display disease-associated pathology, symptom, or feature.

*Therapeutically effective amount:* As used herein, the term "therapeutically effective amount" of a therapeutic agent means an amount that is sufficient, when administered to a subject suffering from or susceptible to a disease, disorder, and/or condition, to treat, diagnose, prevent, and/or delay the onset of the symptom(s) of the disease, disorder, and/or condition. It will be appreciated by those of ordinary skill in the art that a therapeutically effective amount is typically administered via a dosing regimen comprising at least one unit dose.

*Treating:* As used herein, the term "treat," "treatment," or "treating" refers to any method used to partially or completely alleviate, ameliorate, relieve, inhibit, prevent, delay onset of, reduce severity of and/or reduce incidence of one or more symptoms or features of a particular disease, disorder, and/or condition. Treatment may be administered to a subject who does not exhibit signs of a disease and/or exhibits only early signs of the disease for the purpose of decreasing the risk of developing pathology associated with the disease.

### Chemical definitions

*Acyl:* As used herein, the term "acyl" refers to R^{Z}-(C=O)-, wherein R^{Z} is, for example, any alkyl, alkenyl, alkynyl, heteroalkyl or heteroalkylene.

*Aliphatic:* As used herein, the term aliphatic refers to C₁-C₅₀ hydrocarbons and includes both saturated and unsaturated hydrocarbons. An aliphatic may be linear, branched, or cyclic. For example, C₁-C₂₀ aliphatics can include C₁-C₂₀ alkyls (*e.g.,* linear or branched C₁-C₂₀ saturated alkyls), C₂-C₂₀ alkenyls (*e.g.,* linear or branched C₄-C₂₀ dienyls, linear or branched C₆-C₂₀ trienyls, and the like), and C₂-C₂₀ alkynyls (*e.g.,* linear or branched C₂-C₂₀ alkynyls). C₁-C₂₀ aliphatics can include C₃-C₂₀ cyclic aliphatics (*e.g.,* C₃-C₂₀ cycloalkyls, C₄-C₂₀ cycloalkenyls, or C₈-C₂₀ cycloalkynyls). In certain embodiments, the aliphatic may comprise one or more cyclic aliphatic and/or one or more heteroatoms such as oxygen, nitrogen, or sulfur and may optionally be substituted with one or more substituents such as alkyl, halo, alkoxyl, hydroxy, amino, aryl, ether, ester or amide. An aliphatic group is unsubstituted or substituted with one or more substituent groups as described herein. For example, an aliphatic may be substituted with one or more (*e.g.,* 1, 2, 3, 4, 5, or 6 independently selected substituents) of halogen, -COR", -CO₂H, -CO₂R", -CN, -OH, -OR", -OCOR', -OCO₂R", -NH₂, - NHR", -N(R")₂, -SR" or-SO₂R", wherein each instance of **R"** independently is C₁-C₂₀ aliphatic (*e.g.,* C₁-C₂₀ alkyl, C₁-C₁₅ alkyl, C₁-C₁₀ alkyl, or C₁-C₃ alkyl). In embodiments, **R"** independently is an unsubstituted alkyl (*e.g.,* unsubstituted C₁-C₂₀ alkyl, C₁-C₁₅ alkyl, C₁-C₁₀ alkyl, or C₁-C₃ alkyl). In embodiments, **R"** independently is unsubstituted C₁-C₃ alkyl. In embodiments, the aliphatic is unsubstituted. In embodiments, the aliphatic does not include any heteroatoms. *Alkyl:* As used herein, the term "alkyl" means acyclic linear and branched hydrocarbon groups, *e.g.* "C₁-C₃₀ alkyl" refers to alkyl groups having 1-30 carbons. An alkyl group may be linear or branched. Examples of alkyl groups include, but are not limited to, methyl, ethyl, n-propyl, isopropyl, butyl, isobutyl, sec-butyl, tert-butyl, pentyl, isopentyl tert-pentylhexyl, isohexyl, *etc.* The term "lower alkyl" means an alkyl group straight chain or branched alkyl having 1 to 6 carbon atoms. Other alkyl groups will be readily apparent to those of skill in the art given the benefit of the present disclosure. An alkyl group may be unsubstituted or substituted with one or more substituent groups as described herein. For example, an alkyl group may be substituted with one or more (*e.g.,* 1, 2, 3, 4, 5, or 6 independently selected substituents) of halogen, -COR", -CO₂H, -CO₂R", -CN, -OH, -OR", -OCOR', -OCO₂R", -NH₂, - NHR", -N(R")₂, -SR" or-SO₂R", wherein each instance of **R"** independently is C₁-C₂₀ aliphatic (*e.g.,* C₁-C₂₀ alkyl, C₁-C₁₅ alkyl, C₁-C₁₀ alkyl, or C₁-C₃ alkyl). In embodiments, **R"** independently is an unsubstituted alkyl (*e.g.,* unsubstituted C₁-C₂₀ alkyl, C₁-C₁₅ alkyl, C₁-C₁₀ alkyl, or C₁-C₃ alkyl). In embodiments, **R"** independently is unsubstituted C₁-C₃ alkyl. In embodiments, the alkyl is substituted (*e.g.,* with 1, 2, 3, 4, 5, or 6 substituent groups as described herein). In embodiments, an alkyl group is substituted with a -OH group and may also be referred to herein as a "hydroxyalkyl" group, where the prefix denotes the -OH group and "alkyl" is as described herein.

As used herein, "alkyl" also refers to a radical of a straight-chain or branched saturated hydrocarbon group having from 1 to 50 carbon atoms ("C₁-C₅₀ alkyl"). In some embodiments, an alkyl group has 1 to 40 carbon atoms ("C₁-C₄₀ alkyl"). In some embodiments, an alkyl group has 1 to 30 carbon atoms ("C₁-C₃₀ alkyl"). In some embodiments, an alkyl group has 1 to 20 carbon atoms ("C₁-C₂₀ alkyl"). In some embodiments, an alkyl group has 1 to 10 carbon atoms ("C₁-C₁₀ alkyl"). In some embodiments, an alkyl group has 1 to 9 carbon atoms ("C₁-C₉ alkyl"). In some embodiments, an alkyl group has 1 to 8 carbon atoms ("C₁-C₈ alkyl"). In some embodiments, an alkyl group has 1 to 7 carbon atoms ("C₁-C₇ alkyl"). In some embodiments, an alkyl group has 1 to 6 carbon atoms ("C₁-C₆ alkyl"). In some embodiments, an alkyl group has 1 to 5 carbon atoms ("C₁-C₅ alkyl"). In some embodiments, an alkyl group has 1 to 4 carbon atoms ("C₁-C₄ alkyl"). In some embodiments, an alkyl group has 1 to 3 carbon atoms ("C₁-C₃ alkyl"). In some embodiments, an alkyl group has 1 to 2 carbon atoms ("C₁-C₂ alkyl"). In some embodiments, an alkyl group has 1 carbon atom ("C₁ alkyl"). In some embodiments, an alkyl group has 2 to 6 carbon atoms ("C₂-C₆ alkyl"). Examples of C₁-C₆ alkyl groups include, without limitation, methyl (C₁), ethyl (C₂), n-propyl (C₃), isopropyl (C₃), n-butyl (C₄), tert-butyl (C₄), sec-butyl (C₄), iso-butyl (C₄), n-pentyl (C₅), 3-pentanyl (C₅), amyl (C₅), neopentyl (C₅), 3-methyl-2-butanyl (C₅), tertiary amyl (C₅), and n-hexyl (C₆). Additional examples of alkyl groups include n-heptyl (C₇), n-octyl (C₈) and the like. Unless otherwise specified, each instance of an alkyl group is independently unsubstituted (an "unsubstituted alkyl") or substituted (a "substituted alkyl") with one or more substituents. In certain embodiments, the alkyl group is an unsubstituted C₁-C₅₀ alkyl. In certain embodiments, the alkyl group is a substituted C₁-C₅₀ alkyl.

Affixing the suffix "-ene" to a group indicates the group is a divalent moiety, *e.g.,* arylene is the divalent moiety of aryl, and heteroarylene is the divalent moiety of heteroaryl.

*Alkylene:* The term "alkylene," as used herein, represents a saturated divalent straight or branched chain hydrocarbon group and is exemplified by methylene, ethylene, isopropylene and the like. Likewise, the term "alkenylene" as used herein represents an unsaturated divalent straight or branched chain hydrocarbon group having one or more unsaturated carbon-carbon double bonds that may occur in any stable point along the chain, and the term "alkynylene" herein represents an unsaturated divalent straight or branched chain hydrocarbon group having one or more unsaturated carbon-carbon triple bonds that may occur in any stable point along the chain. In certain embodiments, an alkylene, alkenylene, or alkynylene group may comprise one or more cyclic aliphatic and/or one or more heteroatoms such as oxygen, nitrogen, or sulfur and may optionally be substituted with one or more substituents such as alkyl, halo, alkoxyl, hydroxy, amino, aryl, ether, ester or amide. For example, an alkylene, alkenylene, or alkynylene may be substituted with one or more (*e.g.,* 1, 2, 3, 4, 5, or 6 independently selected substituents) of halogen, -COR", -CO₂H, -CO₂R", -CN, -OH, -OR", -OCOR", -OCO₂R", -NH₂, -NHR", -N(R")₂, -SR" or -SO₂R", wherein each instance of **R"** independently is C₁-C₂₀ aliphatic (*e.g.,* C₁-C₂₀ alkyl, C₁-C₁₅ alkyl, C₁-C₁₀ alkyl, or C₁-C₃ alkyl). In embodiments, **R"** independently is an unsubstituted alkyl (*e.g.,* unsubstituted C₁-C₂₀ alkyl, C₁-C₁₅ alkyl, C₁-C₁₀ alkyl, or C₁-C₃ alkyl). In embodiments, **R"** independently is unsubstituted C₁-C₃ alkyl. In certain embodiments, an alkylene, alkenylene, or alkynylene is unsubstituted. In certain embodiments, an alkylene, alkenylene, or alkynylene does not include any heteroatoms. *Alkenyl:* As used herein, "alkenyl" means any linear or branched hydrocarbon chains having one or more unsaturated carbon-carbon double bonds that may occur in any stable point along the chain, *e.g.* "C₂-C₃₀ alkenyl" refers to an alkenyl group having 2-30 carbons. For example, an alkenyl group includes prop-2-enyl, but-2-enyl, but-3-enyl, 2-methylprop-2-enyl, hex-2-enyl, hex-5-enyl, 2,3-dimethylbut-2-enyl, and the like. In embodiments, the alkenyl comprises 1, 2, or 3 carbon-carbon double bond. In embodiments, the alkenyl comprises a single carbon-carbon double bond. In embodiments, multiple double bonds (*e.g.,* 2 or 3) are conjugated. An alkenyl group may be unsubstituted or substituted with one or more substituent groups as described herein. For example, an alkenyl group may be substituted with one or more (*e.g.,* 1, 2, 3, 4, 5, or 6 independently selected substituents) of halogen, -COR", -CO₂H, -CO₂R", -CN, -OH, -OR", -OCOR", -OCO₂R", -NH₂, -NHR", - N(R")₂, -SR" or-SO₂R", wherein each instance of **R"** independently is C₁-C₂₀ aliphatic (*e.g.,* C₁-C₂₀ alkyl, C₁-C₁₅ alkyl, C₁-C₁₀ alkyl, or C₁-C₃ alkyl). In embodiments, **R"** independently is an unsubstituted alkyl (*e.g.,* unsubstituted C₁-C₂₀ alkyl, C₁-C₁₅ alkyl, C₁-C₁₀ alkyl, or C₁-C₃ alkyl). In embodiments, **R"** independently is unsubstituted C₁-C₃ alkyl. In embodiments, the alkenyl is unsubstituted. In embodiments, the alkenyl is substituted (*e.g.,* with 1, 2, 3, 4, 5, or 6 substituent groups as described herein). In embodiments, an alkenyl group is substituted with a-OH group and may also be referred to herein as a "hydroxyalkenyl" group, where the prefix denotes the -OH group and "alkenyl" is as described herein.

As used herein, "alkenyl" also refers to a radical of a straight-chain or branched hydrocarbon group having from 2 to 50 carbon atoms and one or more carbon-carbon double bonds (e.g., 1, 2, 3, or 4 double bonds) ("C₂-C₅₀ alkenyl"). In some embodiments, an alkenyl group has 2 to 40 carbon atoms ("C₂-C₄₀ alkenyl"). In some embodiments, an alkenyl group has 2 to 30 carbon atoms ("C₂-C₃₀ alkenyl"). In some embodiments, an alkenyl group has 2 to 20 carbon atoms ("C₂-C₂₀ alkenyl"). In some embodiments, an alkenyl group has 2 to 10 carbon atoms ("C₂-C₁₀ alkenyl"). In some embodiments, an alkenyl group has 2 to 9 carbon atoms ("C₂-C₉ alkenyl"). In some embodiments, an alkenyl group has 2 to 8 carbon atoms ("C₂-C₈ alkenyl"). In some embodiments, an alkenyl group has 2 to 7 carbon atoms ("C₂-C₇ alkenyl"). In some embodiments, an alkenyl group has 2 to 6 carbon atoms ("C₂-C₆ alkenyl"). In some embodiments, an alkenyl group has 2 to 5 carbon atoms ("C₂-C₅ alkenyl"). In some embodiments, an alkenyl group has 2 to 4 carbon atoms ("C₂-C₄ alkenyl"). In some embodiments, an alkenyl group has 2 to 3 carbon atoms ("C₂-C₃ alkenyl"). In some embodiments, an alkenyl group has 2 carbon atoms ("C₂ alkenyl"). The one or more carbon-carbon double bonds can be internal (such as in 2-butenyl) or terminal (such as in 1-butenyl). Examples of C₂-C₄ alkenyl groups include, without limitation, ethenyl (C₂), 1-propenyl (C₃), 2-propenyl (C₃), 1-butenyl (C₄), 2-butenyl (C₄), butadienyl (C₄), and the like. Examples of C₂-C₆ alkenyl groups include the aforementioned C₂-C₄ alkenyl groups as well as pentenyl (C₅), pentadienyl (C₅), hexenyl (C6), and the like. Additional examples of alkenyl include heptenyl (C₇), octenyl (C₈), octatrienyl (C₈), and the like. Unless otherwise specified, each instance of an alkenyl group is independently unsubstituted (an "unsubstituted alkenyl") or substituted (a "substituted alkenyl") with one or more substituents. In certain embodiments, the alkenyl group is an unsubstituted C₂-C₅₀ alkenyl. In certain embodiments, the alkenyl group is a substituted C₂-C₅₀ alkenyl.

*Alkynyl:* As used herein, "alkynyl" means any hydrocarbon chain of either linear or branched configuration, having one or more carbon-carbon triple bonds occurring in any stable point along the chain, *e.g.,* "C₂-C₃₀ alkynyl", refers to an alkynyl group having 2-30 carbons. Examples of an alkynyl group include prop-2-ynyl, but-2-ynyl, but-3-ynyl, pent-2-ynyl, 3-methylpent-4-ynyl, hex-2-ynyl, hex-5-ynyl, *etc.* In embodiments, an alkynyl comprises one carbon-carbon triple bond. An alkynyl group may be unsubstituted or substituted with one or more substituent groups as described herein. For example, an alkynyl group may be substituted with one or more (*e.g.,* 1, 2, 3, 4, 5, or 6 independently selected substituents) of halogen, -COR", -CO₂H, -CO₂R", -CN, -OH, -OR", -OCOR", - OCO₂R", -NH₂, -NHR", -N(R")₂, -SR" or-SO₂R", wherein each instance of **R"** independently is C₁-C₂₀ aliphatic (*e.g.,* C₁-C₂₀ alkyl, C₁-C₁₅ alkyl, C₁-C₁₀ alkyl, or C₁-C₃ alkyl). In embodiments, **R"** independently is an unsubstituted alkyl (*e.g.,* unsubstituted C₁-C₂₀ alkyl, C₁-C₁₅ alkyl, C₁-C₁₀ alkyl, or C₁-C₃ alkyl). In embodiments, **R"** independently is unsubstituted C₁-C₃ alkyl. In embodiments, the alkynyl is unsubstituted. In embodiments, the alkynyl is substituted (*e.g.,* with 1, 2, 3, 4, 5, or 6 substituent groups as described herein).

As used herein, "alkynyl" also refers to a radical of a straight-chain or branched hydrocarbon group having from 2 to 50 carbon atoms and one or more carbon-carbon triple bonds (e.g., 1, 2, 3, or 4 triple bonds) and optionally one or more double bonds (e.g., 1, 2, 3, or 4 double bonds) ("C₂-C₅₀ alkynyl"). An alkynyl group that has one or more triple bonds and one or more double bonds is also referred to as an "ene-yne". In some embodiments, an alkynyl group has 2 to 40 carbon atoms ("C₂-C₄₀ alkynyl"). In some embodiments, an alkynyl group has 2 to 30 carbon atoms ("C₂-C₃₀ alkynyl"). In some embodiments, an alkynyl group has 2 to 20 carbon atoms ("C₂-C₂₀ alkynyl"). In some embodiments, an alkynyl group has 2 to 10 carbon atoms ("C₂-C₁₀ alkynyl"). In some embodiments, an alkynyl group has 2 to 9 carbon atoms ("C₂-C₉ alkynyl"). In some embodiments, an alkynyl group has 2 to 8 carbon atoms ("C₂-C₈ alkynyl"). In some embodiments, an alkynyl group has 2 to 7 carbon atoms ("C₂-C₇ alkynyl"). In some embodiments, an alkynyl group has 2 to 6 carbon atoms ("C₂-C₆ alkynyl"). In some embodiments, an alkynyl group has 2 to 5 carbon atoms ("C₂-C₅ alkynyl"). In some embodiments, an alkynyl group has 2 to 4 carbon atoms ("C₂-C₄ alkynyl"). In some embodiments, an alkynyl group has 2 to 3 carbon atoms ("C₂-C₃ alkynyl"). In some embodiments, an alkynyl group has 2 carbon atoms ("C₂ alkynyl"). The one or more carbon-- triple bonds can be internal (such as in 2-butynyl) or terminal (such as in 1-butynyl). Examples of C₂-C₄ alkynyl groups include, without limitation, ethynyl (C₂), 1-propynyl (C₃), 2-propynyl (C₃), 1-butynyl (C₄), 2-butynyl (C₄), and the like. Examples of C₂-C₆ alkenyl groups include the aforementioned C₂-C₄ alkynyl groups as well as pentynyl (C₅), hexynyl (C₆), and the like. Additional examples of alkynyl include heptynyl (C₇), octynyl (C₈), and the like. Unless otherwise specified, each instance of an alkynyl group is independently unsubstituted (an "unsubstituted alkynyl") or substituted (a "substituted alkynyl") with one or more substituents. In certain embodiments, the alkynyl group is an unsubstituted C₂-C₅₀ alkynyl. In certain embodiments, the alkynyl group is a substituted C₂-C₅₀ alkynyl.

*Aryl:* The term "aryl" used alone or as part of a larger moiety as in "aralkyl," refers to a monocyclic, bicyclic, or tricyclic carbocyclic ring system having a total of six to fourteen ring members, wherein said ring system has a single point of attachment to the rest of the molecule, at least one ring in the system is aromatic and wherein each ring in the system contains 4 to 7 ring members. In embodiments, an aryl group has 6 ring carbon atoms ("C₆ aryl," *e.g.,* phenyl). In some embodiments, an aryl group has 10 ring carbon atoms ("C₁₀ aryl," *e.g.,* naphthyl such as 1-naphthyl and 2-naphthyl). In some embodiments, an aryl group has 14 ring carbon atoms ("C₁₄ aryl," *e.g.,* anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Exemplary aryls include phenyl, naphthyl, and anthracene.

As used herein, "aryl" also refers to a radical of a monocyclic or polycyclic (e.g., bicyclic or tricyclic) 4n+2 aromatic ring system (e.g., having 6, 10, or 14 π electrons shared in a cyclic array) having 6-14 ring carbon atoms and zero heteroatoms provided in the aromatic ring system ("C₆-C₁₄ aryl"). In some embodiments, an aryl group has 6 ring carbon atoms ("C₆ aryl"; e.g., phenyl). In some embodiments, an aryl group has 10 ring carbon atoms ("C₁₀ aryl"; e.g., naphthyl such as 1-naphthyl and 2-naphthyl). In some embodiments, an aryl group has 14 ring carbon atoms ("C₁₄ aryl"; e.g., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Unless otherwise specified, each instance of an aryl group is independently unsubstituted (an "unsubstituted aryl") or substituted (a "substituted aryl") with one or more substituents. In certain embodiments, the aryl group is an unsubstituted C₆-C₁₄ aryl. In certain embodiments, the aryl group is a substituted C₆-C₁₄ aryl.

*Arylene:* The term "arylene" as used herein refers to an aryl group that is divalent (that is, having two points of attachment to the molecule). Exemplary arylenes include phenylene (*e.g.,* unsubstituted phenylene or substituted phenylene).

*Carbocyclyl:* As used herein, "carbocyclyl" or "carbocyclic" refers to a radical of a non-aromatic cyclic hydrocarbon group having from 3 to 10 ring carbon atoms ("C₃-C₁₀ carbocyclyl") and zero heteroatoms in the non-aromatic ring system. In some embodiments, a carbocyclyl group has 3 to 8 ring carbon atoms ("C₃-C₈ carbocyclyl"). In some embodiments, a carbocyclyl group has 3 to 7 ring carbon atoms ("C₃-C₇ carbocyclyl"). In some embodiments, a carbocyclyl group has 3 to 6 ring carbon atoms ("C₃-C₆ carbocyclyl"). In some embodiments, a carbocyclyl group has 4 to 6 ring carbon atoms ("C₄-C₆ carbocyclyl"). In some embodiments, a carbocyclyl group has 5 to 6 ring carbon atoms ("C₅-C₆ carbocyclyl"). In some embodiments, a carbocyclyl group has 5 to 10 ring carbon atoms ("C₅-C₁₀ carbocyclyl"). Exemplary C₃-C₆ carbocyclyl groups include, without limitation, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), and the like. Exemplary C₃-C₈ carbocyclyl groups include, without limitation, the aforementioned C₃-C₆ carbocyclyl groups as well as cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptanyl (C₇), bicyclo[2.2.2]octanyl (C₈), and the like. Exemplary C₃-C₁₀ carbocyclyl groups include, without limitation, the aforementioned C₃-C₈ carbocyclyl groups as well as cyclononyl (C₉), cyclononenyl (C₉), cyclodecyl (C₁₀), cyclodecenyl (C₁₀), octahydro-1H-indenyl (C₉), decahydronaphthalenyl (C₁₀), spiro[4.5]decanyl (C₁₀), and the like. As the foregoing examples illustrate, in certain embodiments, the carbocyclyl group is either monocyclic ("monocyclic carbocyclyl") or polycyclic (e.g., containing a fused, bridged or spiro ring system such as a bicyclic system ("bicyclic carbocyclyl") or tricyclic system ("tricyclic carbocyclyl")) and can be saturated or can contain one or more carbon-carbon double or triple bonds. "Carbocyclyl" also includes ring systems wherein the carbocyclyl ring, as defined above, is fused with one or more aryl or heteroaryl groups wherein the point of attachment is on the carbocyclyl ring, and in such instances, the number of carbons continue to designate the number of carbons in the carbocyclic ring system. Unless otherwise specified, each instance of a carbocyclyl group is independently unsubstituted (an "unsubstituted carbocyclyl") or substituted (a "substituted carbocyclyl") with one or more substituents. In certain embodiments, the carbocyclyl group is an unsubstituted C₃-C₁₀ carbocyclyl. In certain embodiments, the carbocyclyl group is a substituted C₃-C₁₀ carbocyclyl.

In some embodiments, "carbocyclyl" or "carbocyclic" is referred to as a "cycloalkyl", i.e., a monocyclic, saturated carbocyclyl group having from 3 to 10 ring carbon atoms ("C₃-C₁₀ cycloalkyl"). In some embodiments, a cycloalkyl group has 3 to 8 ring carbon atoms ("C₃-C₈ cycloalkyl"). In some embodiments, a cycloalkyl group has 3 to 6 ring carbon atoms ("C₃-C₆, cycloalkyl"). In some embodiments, a cycloalkyl group has 4 to 6 ring carbon atoms ("C₄-C₆ cycloalkyl"). In some embodiments, a cycloalkyl group has 5 to 6 ring carbon atoms ("C₅-C₆ cycloalkyl"). In some embodiments, a cycloalkyl group has 5 to 10 ring carbon atoms ("C₅-C₁₀ cycloalkyl"). Examples of C₅-C₆ cycloalkyl groups include cyclopentyl (C₅) and cyclohexyl (C₅). Examples of C3-C₆ cycloalkyl groups include the aforementioned C₅-C₆ cycloalkyl groups as well as cyclopropyl (C₃) and cyclobutyl (C₄). Examples of C₃-C₈ cycloalkyl groups include the aforementioned C₃-C₆ cycloalkyl groups as well as cycloheptyl (C₇) and cyclooctyl (C₈). Unless otherwise specified, each instance of a cycloalkyl group is independently unsubstituted (an "unsubstituted cycloalkyl") or substituted (a "substituted cycloalkyl") with one or more substituents. In certain embodiments, the cycloalkyl group is an unsubstituted C₃-C₁₀ cycloalkyl. In certain embodiments, the cycloalkyl group is a substituted C₃-C₁₀ cycloalkyl.

*Halogen:* As used herein, the term "halogen" means fluorine, chlorine, bromine, or iodine.

*Heteroalkyl:* The term "heteroalkyl" is meant a branched or unbranched alkyl, alkenyl, or alkynyl group having from 1 to 14 carbon atoms in addition to 1, 2, 3 or 4 heteroatoms independently selected from the group consisting of N, O, S, and P. Heteroalkyls include tertiary amines, secondary amines, ethers, thioethers, amides, thioamides, carbamates, thiocarbamates, hydrazones, imines, phosphodiesters, phosphoramidates, sulfonamides, and disulfides. A heteroalkyl group may optionally include monocyclic, bicyclic, or tricyclic rings, in which each ring desirably has three to six members. Examples of heteroalkyls include polyethers, such as methoxymethyl and ethoxyethyl.

*Heteroalkylene:* The term "heteroalkylene," as used herein, represents a divalent form of a heteroalkyl group as described herein.

*Heteroaryl:* The term "heteroaryl," as used herein, is fully unsaturated heteroatom-containing ring wherein at least one ring atom is a heteroatom such as, but not limited to, nitrogen and oxygen.

As used herein, "heteroaryl" also refers to a radical of a 5-14 membered monocyclic or polycyclic (e.g., bicyclic or tricyclic) 4n+2 aromatic ring system (e.g., having 6, 10, or 14 π electrons shared in a cyclic array) having ring carbon atoms and 1 or more (e.g., 1, 2, 3, or 4 ring heteroatoms) ring heteroatoms provided in the aromatic ring system, wherein each heteroatom is independently selected from oxygen, sulfur, nitrogen, boron, silicon, and phosphorus ("5-14 membered heteroaryl"). In heteroaryl groups that contain one or more nitrogen atoms, the point of attachment can be a carbon or nitrogen atom, as valency permits. Heteroaryl polycyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused polycyclic (aryl/heteroaryl) ring system. Polycyclic heteroaryl groups wherein one ring does not contain a heteroatom (e.g., indolyl, quinolinyl, carbazolyl, and the like) the point of attachment can be on either ring, i.e., either the ring bearing a heteroatom (e.g., 2-indolyl) or the ring that does not contain a heteroatom (e.g., 5-indolyl).

In some embodiments, a heteroaryl group is a 5-10 membered aromatic ring system having ring carbon atoms and 1 or more (e.g., 1, 2, 3, or 4) ring heteroatoms provided in the aromatic ring system, wherein each heteroatom is independently selected from oxygen, sulfur, nitrogen, boron, silicon, and phosphorus ("5-10 membered heteroaryl"). In some embodiments, a heteroaryl group is a 5-8 membered aromatic ring system having ring carbon atoms and 1 or more (e.g., 1, 2, 3, or 4) ring heteroatoms provided in the aromatic ring system, wherein each heteroatom is independently selected from oxygen, sulfur, nitrogen, boron, silicon, and phosphorus ("5-8 membered heteroaryl"). In some embodiments, a heteroaryl group is a 5-6 membered aromatic ring system having ring carbon atoms and 1 or more (e.g., 1, 2, 3, or 4) ring heteroatoms provided in the aromatic ring system, wherein each heteroatom is independently selected from oxygen, sulfur, nitrogen, boron, silicon, and phosphorus ("5-6 membered heteroaryl"). In some embodiments, the 5-6 membered heteroaryl has 1 or more (e.g., 1, 2, or 3) ring heteroatoms selected from oxygen, sulfur, nitrogen, boron, silicon, and phosphorus. In some embodiments, the 5-6 membered heteroaryl has 1 or 2 ring heteroatoms selected from oxygen, sulfur, nitrogen, boron, silicon, and phosphorus. In some embodiments, the 5-6 membered heteroaryl has 1 ring heteroatom selected from oxygen, sulfur, nitrogen, boron, silicon, and phosphorus. Unless otherwise specified, each instance of a heteroaryl group is independently unsubstituted (an "unsubstituted heteroaryl") or substituted (a "substituted heteroaryl") with one or more substituents. In certain embodiments, the heteroaryl group is an unsubstituted 5-14 membered heteroaryl. In certain embodiments, the heteroaryl group is a substituted 5-14 membered heteroaryl.

Exemplary 5-membered heteroaryl groups containing 1 heteroatom include, without limitation, pyrrolyl, furanyl and thiophenyl. Exemplary 5-membered heteroaryl groups containing 2 heteroatoms include, without limitation, imidazolyl, pyrazolyl, oxazolyl, isoxazolyl, thiazolyl, and isothiazolyl. Exemplary 5-membered heteroaryl groups containing 3 heteroatoms include, without limitation, triazolyl, oxadiazolyl, and thiadiazolyl. Exemplary 5-membered heteroaryl groups containing 4 heteroatoms include, without limitation, tetrazolyl. Exemplary 6-membered heteroaryl groups containing 1 heteroatom include, without limitation, pyridinyl. Exemplary 6-membered heteroaryl groups containing 2 heteroatoms include, without limitation, pyridazinyl, pyrimidinyl, and pyrazinyl. Exemplary 6-membered heteroaryl groups containing 3 or 4 heteroatoms include, without limitation, triazinyl and tetrazinyl, respectively. Exemplary 7-membered heteroaryl groups containing 1 heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl. Exemplary tricyclic heteroaryl groups include, without limitation, phenanthridinyl, dibenzofuranyl, carbazolyl, acridinyl, phenothiazinyl, phenoxazinyl and phenazinyl.

As used herein, "heterocyclyl" or "heterocyclic" refers to a radical of a 3- to 14-membered non-aromatic ring system having ring carbon atoms and 1 or more (e.g., 1, 2, 3, or 4) ring heteroatoms, wherein each heteroatom is independently selected from oxygen, sulfur, nitrogen, boron, silicon, and phosphorus ("3-14 membered heterocyclyl"). In heterocyclyl groups that contain one or more nitrogen atoms, the point of attachment can be a carbon or nitrogen atom, as valency permits. A heterocyclyl group can either be monocyclic ("monocyclic heterocyclyl") or polycyclic (e.g., a fused, bridged or spiro ring system such as a bicyclic system ("bicyclic heterocyclyl") or tricyclic system ("tricyclic heterocyclyl")). and can be saturated or can contain one or more carbon-carbon double or triple bonds. Heterocyclyl polycyclic ring systems can include one or more heteroatoms in one or both rings. "Heterocyclyl" also includes ring systems wherein the heterocyclyl ring, as defined above, is fused with one or more carbocyclyl groups wherein the point of attachment is either on the carbocyclyl or heterocyclyl ring, or ring systems wherein the heterocyclyl ring, as defined above, is fused with one or more aryl or heteroaryl groups, wherein the point of attachment is on the heterocyclyl ring, and in such instances, the number of ring members continue to designate the number of ring members in the heterocyclyl ring system. Unless otherwise specified, each instance of heterocyclyl is independently unsubstituted (an "unsubstituted heterocyclyl") or substituted (a "substituted heterocyclyl") with one or more substituents. In certain embodiments, the heterocyclyl group is an unsubstituted 3-14 membered heterocyclyl. In certain embodiments, the heterocyclyl group is a substituted 3-14 membered heterocyclyl.

In some embodiments, a heterocyclyl group is a 5-10 membered non-aromatic ring system having ring carbon atoms and 1 or more (e.g., 1, 2, 3, or 4) ring heteroatoms, wherein each heteroatom is independently selected from oxygen, sulfur, nitrogen, boron, silicon, and phosphorus ("5-10 membered heterocyclyl"). In some embodiments, a heterocyclyl group is a 5-8 membered non-aromatic ring system having ring carbon atoms and 1 or more (e.g., 1, 2, 3, or 4) ring heteroatoms, wherein each heteroatom is independently selected from oxygen, sulfur, nitrogen, boron, silicon, and phosphorus ("5-8 membered heterocyclyl"). In some embodiments, a heterocyclyl group is a 5-6 membered non-aromatic ring system having ring carbon atoms and 1 or more (e.g., 1, 2, 3, or 4) ring heteroatoms, wherein each heteroatom is independently selected from oxygen, sulfur, nitrogen, boron, silicon, and phosphorus ("5-6 membered heterocyclyl"). In some embodiments, the 5-6 membered heterocyclyl has 1 or more (e.g., 1, 2, or 3) ring heteroatoms selected from oxygen, sulfur, nitrogen, boron, silicon, and phosphorus. In some embodiments, the 5-6 membered heterocyclyl has 1 or 2 ring heteroatoms selected from oxygen, sulfur, nitrogen, boron, silicon, and phosphorus. In some embodiments, the 5-6 membered heterocyclyl has 1 ring heteroatom selected from oxygen, sulfur, nitrogen, boron, silicon, and phosphorus.

Exemplary 3-membered heterocyclyl groups containing 1 heteroatom include, without limitation, azirdinyl, oxiranyl, thiorenyl. Exemplary 4-membered heterocyclyl groups containing 1 heteroatom include, without limitation, azetidinyl, oxetanyl and thietanyl. Exemplary 5-membered heterocyclyl groups containing 1 heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5- membered heterocyclyl groups containing 2 heteroatoms include, without limitation, dioxolanyl, oxathiolanyl and dithiolanyl. Exemplary 5-membered heterocyclyl groups containing 3 heteroatoms include, without limitation, triazolinyl, oxadiazolinyl, and thiadiazolinyl. Exemplary 6-membered heterocyclyl groups containing 1 heteroatom include, without limitation, piperidinyl, tetrahydropyranyl, dihydropyridinyl, and thianyl. Exemplary 6-membered heterocyclyl groups containing 2 heteroatoms include, without limitation, piperazinyl, morpholinyl, dithianyl, dioxanyl. Exemplary 6-membered heterocyclyl groups containing 2 heteroatoms include, without limitation, triazinanyl. Exemplary 7-membered heterocyclyl groups containing 1 heteroatom include, without limitation, azepanyl, oxepanyl and thiepanyl. Exemplary 8-membered heterocyclyl groups containing 1 heteroatom include, without limitation, azocanyl, oxecanyl and thiocanyl. Exemplary bicyclic heterocyclyl groups include, without limitation, indolinyl, isoindolinyl, dihydrobenzofuranyl, dihydrobenzothienyl, tetrahydrobenzothienyl, tetrahydrobenzofuranyl, tetrahydroindolyl, tetrahydroquinolinyl, tetrahydroisoquinolinyl, decahydroquinolinyl, decahydroisoquinolinyl, octahydrochromenyl, octahydroisochromenyl, decahydronaphthyridinyl, decahydro-1,8-naphthyridinyl, octahydropyrrolo[3,2-b]pyrrole, indolinyl, phthalimidyl, naphthalimidyl, chromanyl, chromenyl, 1H-benzo[e][1,4]diazepinyl, 1,4,5,7-tetrahydropyrano[3,4-b] pyrrolyl, 5,6-dihydro-4H-furo[3,2-b]pyrrolyl, 6,7-dihydro-5H-furo[3,2-b]pyranyl, 5,7-dihydro-4H-thieno[2,3-c]pyranyl, 2,3-dihydro-1H-pyrrolo[2,3-b ]pyridinyl, 2,3-dihydrofuro[2,3-b]pyridinyl, 4,5,6,7-tetrahydro-1H-pyrrolo-[2,3-b]pyridinyl, 4,5,6,7-tetrahydrofuro[3,2-c]pyridinyl, 4,5,6,7-tetrahydrothieno [3,2- b]pyridinyl, 1,2,3,4-tetrahydro-1,6-naphthyridinyl, and the like.

*Heterocycloalkyl:* The term "heterocycloalkyl," as used herein, is a non-aromatic ring wherein at least one atom is a heteroatom such as, but not limited to, nitrogen, oxygen, sulfur, or phosphorus, and the remaining atoms are carbon. The heterocycloalkyl group can be substituted or unsubstituted.

As understood from the above, alkyl, alkenyl, alkynyl, acyl, carbocyclyl, heterocyclyl, aryl, and heteroaryl groups, as defined herein, are, in certain embodiments, optionally substituted. Optionally substituted refers to a group which may be substituted or unsubstituted (e.g., "substituted" or "unsubstituted" alkyl, "substituted" or "unsubstituted" alkenyl, "substituted" or "unsubstituted" alkynyl, "substituted" or "unsubstituted" heteroalkyl, "substituted" or "unsubstituted" heteroalkenyl, "substituted" or'unsubstituted" heteroalkynyl, "substituted" or "unsubstituted" carbocyclyl, "substituted" or "unsubstituted" heterocyclyl, "substituted" or "unsubstituted" aryl or "substituted" or "unsubstituted" heteroaryl group. In general, the term "substituted" means that at least one hydrogen present on a group is replaced with a permissible substituent, e.g., a substituent which upon substitution results in a stable compound, e.g., a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds, any of the substituents described herein that results in the formation of a stable compound. The present invention contemplates any and all such combinations in order to arrive at a stable compound. For purposes of this invention, heteroatoms such as nitrogen may have hydrogen substituents and/or any suitable substituent as described herein which satisfy the valencies of the heteroatoms and results in the formation of a stable moiety.

Exemplary carbon atom substituents include, but are not limited to, halogen, -CN, - NO₂, -N₃, -SO₂, -SO₃H, -OH, -OR^{aa}, -ON(R^{bb})₂, -N(R^{bb})₂, -N(R^{bb})₃+X⁻, -N(OR^{cc})R^{bb}, -SeH, -SeR^{aa}, -SH, -SR^{aa}, -SSR^{cc}, -C(=O)R^{aa}, -CO₂H, -CHO, -C(OR^{cc})₂, -CO₂R^{aa}, -OC(=O)R^{aa}, -OCO₂R^{aa}, -C(=O)N(R^{bb})₂, - OC(=O)N(R^{bb})₂, -NR^{bb}C(=O)R^{aa}, -NR^{bb}CO₂R^{aa}, -NR^{bb}C(=O)N(R^{bb})₂, -C(=NR^{bb})R^{aa}, -C(=NR^{bb})OR^{aa}, - OC(=NR^{bb})R^{aa}, - OC(=NR^{bb})OR^{aa}, -C(=NR^{bb})N(R^{bb})₂, -OC(=NR^{bb})N(R^{bb})₂, -NR^{bb}C(=NR^{bb})N(R^{bb})₂, - C(=O)NR^{bb}SO₂R^{aa}, -NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, - Si(R^{aa})₃ -OSi(R^{aa})₃ -C(=S)N(R^{bb})₂, -C(=O)SR^{aa}, -C(=S)SR^{aa}, - SC(=S)SR^{aa}, -SC(=O)SR^{aa}, -OC(=O)SR^{aa}, - SC(=O)OR^{aa}, -SC(=O)R^{aa}, -P(=O)₂R^{aa}, -OP(=O)₂R^{aa}, -P(=O)(R^{aa})₂, -OP(=O)(R^{aa})₂, -OP(=O)(OR^{cc})₂, - P(=O)₂N(R^{bb})₂, -OP(=O)₂N(R^{bb})₂, - P(=O)(NR^{bb})₂, -OP(=O)(NR^{bb})₂, -NR^{bb}P(=O)(OR^{cc})₂, - NR^{bb}P(=O)(NR^{bb})₂, -P(R^{cc})₂, - P(R^{cc})₃, -OP(R^{cc})₂, -OP(R^{cc})₃, -B(R^{aa})₂, -B(OR^{cc})₂, -BR^{aa}(OR^{cc}), C₁-C₅₀ alkyl, C₂-C₅₀ alkenyl, C₂-C₅₀ alkynyl, C₃-C₁₄ carbocyclyl, 3-14 membered heterocyclyl, C₆-C₁₄ aryl, and 5-14 membered heteroaryl, wherein each alkyl, alkenyl, alkynyl, carbocyclyl, heterocyclyl, aryl, and heteroaryl is independently substituted with 0, 1, 2, 3, 4, or 5 R^{dd} groups; or two geminal hydrogens on a carbon atom are replaced with the group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb}, or =NOR^{cc};
each instance of R^{aa} is, independently, selected from C₁-C₅₀ alkyl, C₂-C₅₀ alkenyl, C₂-C₅₀ alkynyl, C₃-C₁₀ carbocyclyl, 3-14 membered heterocyclyl, C₆-C₁₄ aryl, and 5-14 membered heteroaryl, or two R^{aa} groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups;
each instance of R^{bb} is, independently, selected from hydrogen, -OH, -OR^{aa}, - N(R^{cc})₂, -CN, - C(=O)R^{aa}, -C(=O)N(R^{cc})₂, -CO₂R^{aa}, -SO₂R^{aa}, -C(=NR^{cc})OR^{aa}, - C(=NR^{cc})N(R^{cc})₂, -SO₂N(R^{cc})₂, -SO₂R^{cc}, - SO₂OR^{cc}, -SOR^{aa}, -C(=S)N(R^{cc})₂, -C(=O)SR^{cc}, - C(=S)SR^{cc}, -P(=O)₂R^{aa}, -P(=O)(R^{aa})₂, -P(=O)₂N(R^{cc})₂, - P(=O)(NR^{cc})₂, C₁-C₅₀ alkyl, C₂-C₅₀ alkenyl, C₂-C₅₀ alkynyl, C₃-C₁₀ carbocyclyl, 3-14 membered heterocyclyl, C₆-C₁₄ aryl, and 5-14 membered heteroaryl, or two R^{bb} groups, together with the heteroatom to which they are attached, form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups;
each instance of R^{cc} is, independently, selected from hydrogen, C₁-C₅₀ alkyl, C₂-C₅₀ alkenyl, C₂-C₅₀ alkynyl, C₃-C₁₀ carbocyclyl, 3-14 membered heterocyclyl, C₆-C₁₄ aryl, and 5-14 membered heteroaryl, or two R^{cc} groups, together with the heteroatom to which they are attached, form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups;
each instance of R^{dd} is, independently, selected from halogen, -CN, -NO₂, -N₃, - SO₂H, -SO₃H, - OH, -OR^{ee}, -ON(R^{ff})₂, -N(R^{ff})₂, -N(R^{ff})₃+X⁻, -N(OR^{ee})R^{ff}, -SH, -SR^{ee}, - SSR^{ee}, -C(=O)R^{ee}, -CO₂H, -CO₂R^{ee}, - OC(=O)R^{ee}, -OCO₂R^{ee}, -C(=O)N(R^{ff})₂, - OC(=O)N(R^{ff})₂, -NR^{ff}C(=O)R^{ee}, -NR^{ff}CO₂R^{ee}, -NR^{ff}C(=O)N(R^{ff})₂, - C(=NR^{ff})OR^{ee}, - OC(=NR^{ff})R^{ee}, -OC(=NR^{ff})OR^{ee}, -C(=NR^{ff})N(R^{ff})₂, -OC(=NR^{ff})N(R^{ff})₂, -NR^{ff}C(=NR^{ff})N(R^{ff})₂, -NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, - C(=S)N(R^{ff})₂, -C(=O)SR^{ee}, -C(=S)SR^{ee}, -SC(=S)SR^{ee}, -P(=O)₂R^{ee}, - P(=O)(R^{ee})₂, -OP(=O)(R^{ee})₂, - OP(=O)(OR^{ee})₂, C₁-C₅₀ alkyl, C₂-C₅₀ alkenyl, C₂-C₅₀ alkynyl, C₃-C₁₀ carbocyclyl, 3-10 membered heterocyclyl, C₆-C₁₀ aryl, 5-10 membered heteroaryl, wherein each alkyl, alkenyl, alkynyl, carbocyclyl, heterocyclyl, aryl, and heteroaryl is independently substituted with 0, 1, 2, 3, 4, or 5 R^{gg} groups, or two geminal R^{dd} substituents can be joined to form =O or =S;
each instance of R^{ee} is, independently, selected from C₁-C₅₀ alkyl, C₂-C₅₀ alkenyl, C₂-C₅₀ alkynyl, C₃-C₁₀ carbocyclyl, C₆-C₁₀ aryl, 3-10 membered heterocyclyl, and 3-10 membered heteroaryl, wherein each alkyl, alkenyl, alkynyl, carbocyclyl, heterocyclyl, aryl, and heteroaryl is independently substituted with 0, 1, 2, 3, 4, or 5 R^{gg} groups;
each instance of R^{ff} is, independently, selected from hydrogen, C₁-C₅₀ alkyl, C₂-C₅₀ alkenyl, C₂-C₅₀ alkynyl, C₃-C₁₀ carbocyclyl, 3-10 membered heterocyclyl, C₆-C₁₀ aryl and 5-10 membered heteroaryl, or two R^{ff} groups, together with the heteroatom to which they are attached, form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{gg} groups; and
each instance of R^{gg} is, independently, halogen, -CN, -NO₂, -N₃, -SO₂H, -SO₃H, -OH, -OC₁-C₅₀ alkyl, -ON(C₁-C₅₀ alkyl)₂, -N(C₁-C₅₀ alkyl)₂, -N(C₁-C₅₀ alkyl)₃+X⁻, -NH(C₁-C₅₀ alkyl)₂+X⁻, -NH₂(C₁-C₅₀ alkyl) +X⁻, -NH₃+X⁻, -N(OC₁-C₅₀ alkyl)(C₁-C₅₀ alkyl), -N(OH)(C₁-C₅₀ alkyl), -NH(OH), -SH, -SC₁-C₅₀ alkyl, - SS(C₁-C₅₀ alkyl), -C(=O)(C₁-C₅₀ alkyl), -CO₂H, -CO₂(C₁-C₅₀ alkyl), -OC(=O)(C₁-C₅₀ alkyl), -OCO₂(C₁-C₅₀ alkyl), -C(=O)NH₂, -C(=O)N(C₁-C₅₀ alkyl)₂, -OC(=O)NH(C₁-C₅₀ alkyl), -NHC(=O)(C₁-C₅₀ alkyl), -N(C₁-C₅₀ alkyl)C(=O)(C₁-C₅₀ alkyl), -NHCO₂(C₁-C₅₀ alkyl), -NHC(=O)N(C₁-C₅₀ alkyl)₂, -NHC(=O)NH(C₁-C₅₀ alkyl), -NHC(=O)NH₂, -C(=NH)O(C₁-C₅₀ alkyl),-OC(=NH)(C₁-C₅₀ alkyl), -OC(=NH)OC₁-C₅₀ alkyl, - C(=NH)N(C₁-C₅₀ alkyl)₂, -C(=NH)NH(C₁-C₅₀ alkyl), -C(=NH)NH₂, -OC(=NH)N(C₁-C₅₀alkyl)₂, -OC(NH)NH(C₁-C₅₀ alkyl), -OC(NH)NH₂, -NHC(NH)N(C₁-C₅₀ alkyl)₂, -NHC(=NH)NH₂, -NHSO₂(C₁-C₅₀ alkyl), -SO₂N(C₁-C₅₀ alkyl)₂, -SO₂NH(C₁-C₅₀ alkyl), - SO₂NH₂,-SO₂(C₁-C₅₀ alkyl), -SO₂O(C₁-C₅₀ alkyl), -OSO₂(C₁-C₆ alkyl), -SO(C₁-C₆ alkyl), -Si(C₁-C₅₀ alkyl)₃, -OSi(C₁-C₆ alkyl)₃, -C(=S)N(C₁-C₅₀ alkyl)₂, C(=S)NH(C₁-C₅₀ alkyl), C(=S)NH₂, - C(=O)S(C₁-C₆ alkyl), -C(=S)S(C₁-C₆ alkyl), -SC(=S)S(C₁-C₆ alkyl), -P(=O)₂(C₁-C₅₀ alkyl), -P(=O)(C₁-C₅₀ alkyl)₂, -OP(=O)(C₁-C₅₀ alkyl)₂, -OP(=O)(OC₁-C₅₀ alkyl)₂, C₁-C₅₀ alkyl, C₂-C₅₀ alkenyl, C₂-C₅₀ alkynyl, C₃-C₁₀ carbocyclyl, C₆-C₁₀ aryl, 3-10 membered heterocyclyl, 5-10 membered heteroaryl; or two geminal R^{gg} substituents can be joined to form =O or =S; wherein X⁻ is a counterion.

As used herein, the term "halo" or "halogen" refers to fluorine (fluoro, -F), chlorine (chloro, - Cl), bromine (bromo, -Br), or iodine (iodo, -I).

As used herein, a "counterion" is a negatively charged group associated with a positively charged quarternary amine in order to maintain electronic neutrality. Exemplary counterions include halide ions (e.g., F⁻, Cl⁻, Br⁻, I⁻), NO₃⁻, ClO₄⁻, OH⁻, H₂PO₄⁻, HSO₄⁻, sulfonate ions (e.g., methansulfonate, trifluoromethanesulfonate, p-toluenesulfonate, benzenesulfonate, 10-camphor sulfonate, naphthalene-2-sulfonate, naphthalene-I-sulfonic acid-5-sulfonate, ethan-1-sulfonic acid-2-sulfonate, and the like), and carboxylate ions (e.g., acetate, ethanoate, propanoate, benzoate, glycerate, lactate, tartrate, glycolate, and the like).

Nitrogen atoms can be substituted or unsubstituted as valency permits, and include primary, secondary, tertiary, and quarternary nitrogen atoms. Exemplary nitrogen atom substitutents include, but are not limited to, hydrogen, -OH, -OR^{aa}, -N(R^{cc})₂, -CN, - C(=O)R^{aa}, -C(=O)N(R^{cc})₂, -CO₂R^{aa}, -SO₂R^{aa}, - C(=NR^{bb})R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, - C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, C₁-C₅₀ alkyl, C₂-C₅₀ alkenyl, C₂-C₅₀ alkynyl, C₃-C₁₀ carbocyclyl, 3-14 membered heterocyclyl, C₆-C₁₄ aryl, and 5-14 membered heteroaryl, or two R^{cc} groups, together with the N atom to which they are attached, form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups, and wherein R^{aa}, R^{bb}, R^{cc} and R^{dd} are as defined above.

In certain embodiments, the substituent present on a nitrogen atom is a nitrogen protecting group (also referred to as an amino protecting group). Nitrogen protecting groups are well known in the art and include those described in detail in Protecting Groups in Organic Synthesis, T. W. Greene and P. G. M. Wuts, 3rd edition, John Wiley & Sons, 1999.

For example, nitrogen protecting groups such as amide groups (e.g., - C(=O)R^{aa}) include, but are not limited to, formamide, acetamide, chloroacetamide, trichloroacetamide, trifluoroacetamide, phenylacetamide, 3-phenylpropanamide, picolinamide, 3-pyridylcarboxamide, N-benzoylphenylalanyl derivative, benzamide, p-phenylbenzamide, o-nitophenylacetamide, o-nitrophenoxyacetamide, acetoacetamide, (N'-dithiobenzyloxyacylamino)acetamide, 3-(p-hydroxyphenyl)propanamide, 3-(o-nitrophenyl)propanamide, 2-methyl-2-(o-nitrophenoxy)propanamide, 2-methyl-2-(o-phenylazophenoxy)propanamide, 4-chlorobutanamide, 3-methyl-3-nitrobutanamide, o-nitrocinnamide, N-acetylmethionine derivative, o-nitrobenzamide and o-(benzoyloxymethyl)benzamide.

Nitrogen protecting groups such as carbamate groups (e.g., -C(=O)OR^{aa}) include, but are not limited to, methyl carbamate, ethyl carbamante, 9-fluorenylmethyl carbamate (Fmoc), 9-(2-sulfo)fluorenylmethyl carbamate, 9-(2,7-dibromo)fluoroenylmethyl carbamate, 2,7-di-t-butyl-[9-(10,10-dioxo-10,10,10,10-tetrahydrothioxanthyl)]methyl carbamate (DBD-Tmoc), 4-methoxyphenacyl carbamate (Phenoc), 2,2,2-trichloroethyl carbamate (Troc), 2-trimethylsilylethyl carbamate (Teoc), 2-phenylethyl carbamate (hZ), 1-(1-adamantyl)-1-methylethyl carbamate (Adpoc), 1,1-dimethyl-2-haloethyl carbamate, 1,1-dimethyl-2,2-dibromoethyl carbamate (DB-t-BOC), 1,1-dimethyl-2,2,2-trichloroethyl carbamate (TCBOC), 1-methyl-1-(4-biphenylyl)ethyl carbamate (Bpoc), 1-(3,5-di-t-butylphenyl)-1-methylethyl carbamate (t-Bumeoc), 2-(2'-and 4'-pyridyl)ethyl carbamate (Pyoc), 2-(N,N-dicyclohexylcarboxamido)ethyl carbamate, t-butyl carbamate (BOC), 1-adamantyl carbamate (Adoc), vinyl carbamate (Voc), allyl carbamate (Alloc), 1-isopropylallyl carbamate (Ipaoc), cinnamyl carbamate (Coc), 4-nitrocinnamyl carbamate (Noc), 8-quinolyl carbamate, N-hydroxypiperidinyl carbamate, alkyldithio carbamate, benzyl carbamate (Cbz), p-methoxybenzyl carbamate (Moz), p-nitobenzyl carbamate, p-bromobenzyl carbamate, p-chlorobenzyl carbamate, 2,4-dichlorobenzyl carbamate, 4-methylsulfinylbenzyl carbamate (Msz), 9-anthrylmethyl carbamate, diphenylmethyl carbamate, 2-methylthioethyl carbamate, 2-methylsulfonylethyl carbamate, 2-(p-toluenesulfonyl)ethyl carbamate, [2-(1,3-dithianyl)]methyl carbamate (Dmoc), 4- methylthiophenyl carbamate (Mtpc), 2,4-dimethylthiophenyl carbamate (Bmpc), 2-phosphonioethyl carbamate (Peoc), 2-triphenylphosphonioisopropyl carbamate (Ppoc), 1,1-dimethyl-2-cyanoethyl carbamate, m-chloro-p-acyloxybenzyl carbamate, p-(dihydroxyboryl)benzyl carbamate, 5-benzisoxazolylmethyl carbamate, 2-(trifluoromethyl)-6-chromonylmethyl carbamate (Tcroc), m-nitrophenyl carbamate, 3,5-dimethoxybenzyl carbamate, o-nitrobenzyl carbamate, 3,4-dimethoxy-6-nitrobenzyl carbamate, phenyl(o-nitrophenyl)methyl carbamate, t-amyl carbamate, S-benzyl thiocarbamate, p-cyanobenzyl carbamate, cyclobutyl carbamate, cyclohexyl carbamate, cyclopentyl carbamate, cyclopropylmethyl carbamate, p-decyloxybenzyl carbamate, 2,2-dimethoxyacylvinyl carbamate, o-(N,N-dimethylcarboxamido)benzyl carbamate, 1,1-dimethyl-3-(N,N-dimethylcarboxamido)propyl carbamate, 1,1-dimethylpropynyl carbamate, di(2-pyridyl)methyl carbamate, 2-furanylmethyl carbamate, 2-iodoethyl carbamate, isoborynl carbamate, isobutyl carbamate, isonicotinyl carbamate, p-(p'-methoxyphenylazo)benzyl carbamate, 1-methylcyclobutyl carbamate, 1-methylcyclohexyl carbamate, 1-methyl-l-cyclopropylmethyl carbamate, 1-methyl-1(3,5-dimethoxyphenyl)ethyl carbamate, 1-methyl-1-(p-phenylazophenyl)ethyl carbamate, 1-methyl-I-phenylethyl carbamate, 1- methyl-1-(4-pyridyl)ethyl carbamate, phenyl carbamate, p-(phenylazo)benzyl carbamate, 2,4,6-tri-t-butylphenyl carbamate, 4-(trimethylammonium)benzyl carbamate, and 2,4,6-trimethylbenzyl carbamate.

Nitrogen protecting groups such as sulfonamide groups (e.g., -S(=O)₂R^{aa}) include, but are not limited to, p-toluenesulfonamide (Ts), benzenesulfonamide, 2,3,6,-trimethyl-4-methoxybenzenesulfonamide (Mtr), 2,4,6-trimethoxybenzenesulfonamide (Mtb), 2,6-dimethyl-4-methoxybenzenesulfonamide (Pme), 2,3,5,6-tetramethyl-4-methoxybenzenesulfonamide (Mte), 4-methoxybenzenesulfonamide (Mbs), 2,4,6-trimethylbenzenesulfonamide (Mts), 2,6-dimethoxy-4-methylbenzenesulfonamide (iMds), 2,2,5,7,8-pentamethylchroman-6-sulfonamide (Pmc), methanesulfonamide (Ms), β-trimethylsilylethanesulfonamide (SES), 9-anthracenesulfonamide, 4-(4',8'-dimethoxynaphthylmethyl)benzenesulfonamide (DNMBS), benzylsulfonamide, trifluoromethylsulfonamide, and phenacylsulfonamide.

Other nitrogen protecting groups include, but are not limited to, phenothiazinyl-(10)-acyl derivative, N'-p-toluenesulfonylaminoacyl derivative, N' -phenylaminothioacyl derivative, N-benzoylphenylalanyl derivative, N-acetylmethionine derivative, 4,5-diphenyl-3-oxazolin-2-one, N-phthalimide, N-dithiasuccinimide (Dts), N-2,3-diphenylmaleimide, N-2,5-dimethylpyrrole, N-1,1,4,4-tetramethyldisilylazacyclopentane adduct (STABASE), 5-substituted 1,3-dimethyl-1,3,5-triazacyclohexan-2-one, 5-substituted 1,3-dibenzyl-1,3,5-triazacyclohexan-2-one, 1- substituted 3,5-dinitro-4-pyridone, N-methylamine, N-allylamine, N-[2- (trimethylsilyl)ethoxy]methylamine (SEM), N-3-acetoxypropylamine, N-(1-isopropy1-4-nitro-2-oxo-3-pyroolin-3-yl)amine, quaternary ammonium salts, N-benzylamine, N-di(4-methoxyphenyl)methylamine, N-5-dibenzosuberylamine, N-triphenylmethylamine (Tr), N-[(4-methoxyphenyl)diphenylmethyl]amine (MMTr), N-9-phenylfluorenylamine (PhF), N-2,7 -dichloro-9-fluorenylmethyleneamine, N-ferrocenylmethylamino (Fcm), N-2- picolylamino N'-oxide, N-1,1-dimethylthiomethyleneamine, N-benzylideneamine, N-p-methoxybenzylideneamine, N-diphenylmethyleneamine, N-[(2-pyridyl)mesityl]methyleneamine, N-(N' ,N'-dimethylaminomethylene)amine, N,N' -isopropylidenediamine, N-p-nitrobenzylideneamine, N-salicylideneamine, N-5- chlorosalicylideneamine, N-(5-chloro-2-hydroxyphenyl)phenylmethyleneamine, N-cyclohexylideneamine, N-(5,5-dimethyl-3-oxo-l-cyclohexenyl)amine, N-borane derivative, N-diphenylborinic acid derivative, N-[phenyl(pentaacylchromium- or tungsten)acyl]amine, N-copper chelate, N-zinc chelate, N-nitroamine, N-nitrosoamine, amine N-oxide, diphenylphosphinamide (Dpp), dimethylthiophosphinamide (Mpt), diphenylthiophosphinamide (Ppt), dialkyl phosphoramidates, dibenzyl phosphoramidate, diphenyl phosphoramidate, benzenesulfenamide, o-nitrobenzenesulfenamide (Nps), 2,4- dinitrobenzenesulfenamide, pentachlorobenzenesulfenamide, 2-nitro-4-methoxybenzenesulfenamide, triphenylmethylsulfenamide, and 3-nitropyridinesulfenamide (Npys).

In certain embodiments, the substituent present on an oxygen atom is an oxygen protecting group (also referred to as a hydroxyl protecting group). Oxygen protecting groups are well known in the art and include those described in detail in Protecting Groups in Organic Synthesis, T. W. Greene and P. G. M. Wuts, 3rd edition, John Wiley & Sons, 1999.

Exemplary oxygen protecting groups include, but are not limited to, methyl, methoxylmethyl (MOM), methylthiomethyl (MTM), t-butylthiomethyl, (phenyldimethylsilyl)methoxymethyl (SMOM), benzyloxymethyl (BOM), p-methoxybenzyloxymethyl (PMBM), (4-methoxyphenoxy)methyl (p-AOM), guaiacolmethyl (GUM), t-butoxymethyl, 4-pentenyloxymethyl (POM), siloxymethyl, 2-methoxyethoxymethyl (MEM), 2,2,2-trichloroethoxymethyl, bis(2-chloroethoxy)methyl, 2-(trimethylsilyl)ethoxymethyl (SEMOR), tetrahydropyranyl (THP), 3-bromotetrahydropyranyl, tetrahydrothiopyranyl, 1-methoxycyclohexyl, 4- methoxytetrahydropyranyl (MTHP), 4-methoxytetrahydrothiopyranyl, 4- methoxytetrahydrothiopyranyl S,S-dioxide, 1-[(2-chloro-4-methyl)phenyl]-4-methoxypiperidin-4-y1 (CTMP), 1,4-dioxan-2-yl, tetrahydrofuranyl, tetrahydrothiofuranyl, 2,3,3a,4,5,6,7,7a-octahydro-7,8,8-trimethyl-4,7-methanobenzofuran-2-yl, 1-ethoxyethyl, 1-(2-chloroethoxy)ethyl, 1-methyl-I-methoxyethyl, 1-methyl-1-benzyloxyethyl, 1-methyl-1-benzyloxy-2-fluoroethyl, 2,2,2-trichloroethyl, 2-trimethylsilylethyl, 2- (phenylselenyl)ethyl, t-butyl, allyl, p-chlorophenyl, p-methoxyphenyl, 2,4-dinitrophenyl, benzyl (Bn), p-methoxybenzyl, 3,4-dimethoxybenzyl, o-nitrobenzyl, p-nitrobenzyl, p-halobenzyl, 2,6-dichlorobenzyl, p-cyanobenzyl, p-phenylbenzyl, 2-picolyl, 4-picolyl, 3- methyl-2-picoly1 N-oxido, diphenylmethyl, p,p'-dinitrobenzhydryl, 5-dibenzosuberyl, triphenylmethyl, α-naphthyldiphenylmethyl, p-methoxyphenyldiphenylmethyl, di(p-methoxyphenyl)phenylmethyl, tri(p-methoxyphenyl)methyl, 4-(4'- bromophenacyloxyphenyl)diphenylmethyl, 4,4',4"-tris(4,5-dichlorophthalimidophenyl)methyl, 4,4',4"-tris(levulinoyloxyphenyl)methyl, 4,4',4"-tris(benzoyloxyphenyl)methyl, 3-(imidazol-1-yl)bis(4',4"-dimethoxyphenyl)methyl, 1,1-bis(4-methoxyphenyl)-1'-pyrenylmethyl, 9-anthryl, 9-(9-phenyl)xanthenyl, 9-(9-phenyl-10-oxo)anthryl, 1,3-benzodisulfuran-2-yl, benzisothiazolyl S,S-dioxido, trimethylsilyl (TMS), triethylsilyl (TES), triisopropylsilyl (TIPS), dimethylisopropylsilyl (IPDMS), diethylisopropylsilyl (DEIPS), dimethylthexylsilyl, t-butyldimethylsilyl (TBDMS), t-butyldiphenylsily1 (TBDPS), tribenzylsilyl, tri-p-xylylsilyl, triphenylsilyl, diphenylmethylsilyl (DPMS), t-butylmethoxyphenylsilyl (TBMPS), formate, benzoylformate, acetate, chloroacetate, dichloroacetate, trichloroacetate, trifluoroacetate, methoxyacetate, triphenylmethoxyacetate, phenoxyacetate, p-chlorophenoxyacetate, 3- phenylpropionate, 4-oxopentanoate (levulinate), 4,4-(ethylenedithio)pentanoate (levulinoyldithioacetal), pivaloate, adamantoate, crotonate, 4-methoxycrotonate, benzoate, p-phenylbenzoate, 2,4,6-trimethylbenzoate (mesitoate), alkyl methyl carbonate, 9- fluorenylmethyl carbonate (Fmoc), alkyl ethyl carbonate, alkyl 2,2,2-trichloroethyl carbonate (Troc), 2-(trimethylsilyl)ethyl carbonate (TMSEC), 2-(phenylsulfonyl) ethyl carbonate (Psec), 2-(triphenylphosphonio) ethyl carbonate (Peoc), alkyl isobutyl carbonate, alkyl vinyl carbonate alkyl allyl carbonate, alkyl p-nitrophenyl carbonate, alkyl benzyl carbonate, alkyl p-methoxybenzyl carbonate, alkyl 3,4-dimethoxybenzyl carbonate, alkyl o-nitrobenzyl carbonate, alkyl p-nitrobenzyl carbonate, alkyl S-benzyl thiocarbonate, 4-ethoxy-1-napththyl carbonate, methyl dithiocarbonate, 2-iodobenzoate, 4-azidobutyrate, 4-nitro-4-methylpentanoate, o-(dibromomethyl)benzoate, 2-formylbenzenesulfonate, 2-(methylthiomethoxy)ethyl, 4-(methylthiomethoxy)butyrate, 2- (methylthiomethoxymethyl)benzoate, 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, isobutyrate, monosuccinoate, (E)-2-methyl-2-butenoate, o-(methoxyacyl)benzoate, α-naphthoate, nitrate, alkyl N,N,N',N'-tetramethylphosphorodiamidate, alkyl N-phenylcarbamate, borate, dimethylphosphinothioyl, alkyl 2,4-dinitrophenylsulfenate, sulfate, methanesulfonate (mesylate), benzylsulfonate, and tosylate (Ts).

In certain embodiments, the substituent present on a sulfur atom is a sulfur protecting group (also referred to as a thiol protecting group). Sulfur protecting groups are well known in the art and include those described in detail in Protecting Groups in Organic Synthesis, T. W. Greene and P. G. M. Wuts, 3rd edition, John Wiley & Sons, 1999.

Exemplary sulfur protecting groups include, but are not limited to, alkyl, benzyl, p-methoxybenzyl, 2,4,6-trimethylbenzyl, 2,4,6-trimethoxybenzyl, o-hydroxybenzyl, p-hydroxybenzyl, o-acetoxybenzyl, p-acetoxybenzyl, p-nitrobenzyl, 4-picolyl, 2-quinolinylmethyl, 2-picolyl N-oxido, 9-anthrylmethyl, 9-fluorenylmethyl, xanthenyl, ferrocenylmethyl, diphenylmethyl, bis(4-methoxyphenyl)methyl, 5-dibenzosuberyl, triphenylmethyl, diphenyl-4-pyridylmethyl, phenyl, 2,4-dinitrophenyl, t-butyl, 1-adamantyl, methoxymethyl (MOM), isobutoxymethyl, benzyloxymethyl, 2-tetrahydropyranyl, benzylthiomethyl, phenylthiomethyl, thiazolidino, acetamidomethyl, trimethylacetamidomethyl, benzamidomethyl, allyloxycarbonylaminomethyl, phenylacetamidomethyl, phthalimidomethyl, acetylmethyl, carboxymethyl, cyanomethyl, (2-nitro-1-phenyl)ethyl, 2-(2,4-dinitrophenyl)ethyl, 2-cyanoethyl, 2-(Trimethylsilyl)ethyl, 2,2-bis(carboethoxy)ethyl, (1-m-nitrophenyl-2-benzoyl)othyl, 2-phenylsulfonylethyl, 2-(4-methylphenylsulfonyl)-2-methylprop-2-yl, acetyl, benzoyl, trifluoroacetyl, N-[[(p-biphenylyl)isopropoxy]carbonyl]-N-methyl]- γ-aminothiobutyrate, 2,2,2-trichloroethoxycarbonyl, t-butoxycarbonyl, benzyloxycarbonyl, p-methoxybenzyloxycarbonyl, N-ethyl, N-methoxymethyl, sulfonate, sulfenylthiocarbonate, 3-nitro-2-pyridinesulfenyl sulfide, oxathiolone.

### Compounds of the Invention

Liposomal-based vehicles are considered an attractive carrier for therapeutic agents and remain subject to continued development efforts. While liposomal-based vehicles that comprise certain lipid components have shown promising results with regard to encapsulation, stability and site localization, there remains a great need for improvement of liposomal-based delivery systems. For example, a significant drawback of liposomal delivery systems relates to the construction of liposomes that have sufficient cell culture or in vivo stability to reach desired target cells and/or intracellular compartments, and the ability of such liposomal delivery systems to efficiently release their encapsulated materials to such target cells.

In particular, there remains a need for improved lipids compounds that demonstrate, *e.g.,* improved pharmacokinetic properties and which are capable of delivering macromolecules, such as nucleic acids, to a wide variety cell types and tissues with enhanced efficiency. Importantly, there also remains a particular need for novel lipid compounds that are characterized as having, *e.g.,* reduced toxicity and are capable of efficiently delivering encapsulated nucleic acids and polynucleotides to targeted cells, tissues and organs.

Described herein are cationic lipid compounds for improved *in vivo* delivery of therapeutic agents, such as nucleic acids. In particular, a cationic lipid described herein may be used, optionally with other lipids, to formulate a lipid-based nanoparticle (*e.g.,* a liposome) for encapsulating therapeutic agents, such as nucleic acids (*e.g.,* DNA, siRNA, mRNA, and/or microRNA) for therapeutic use.

In embodiments, compounds of the invention as described herein can provide one or more desired characteristics or properties. That is, in certain embodiments, compounds of the invention as described herein can be characterized as having one or more properties that afford such compounds advantages relative to other similarly classified lipids. For example, compounds disclosed herein can allow for the control and tailoring of the properties of liposomal compositions (*e.g.,* lipid nanoparticles) of which they are a component. In particular, compounds disclosed herein can be characterized by enhanced transfection efficiencies and their ability to provoke specific biological outcomes. Such outcomes can include, for example enhanced cellular uptake, endosomal/lysosomal disruption capabilities and/or promoting the release of encapsulated materials (*e.g.,* polynucleotides) intracellularly. Additionally, the compounds disclosed herein have advantageous pharmacokinetic properties, biodistribution, and efficiency (*e.g.,* due to the different disassociate rates of the polymer group used).

The present application demonstrates that not only are the cationic lipids of the present invention synthetically tractable from readily available starting materials, but they also have unexpectedly high encapsulation efficiencies.

Additionally, the cationic lipids of the present invention have cleavable groups such as ester groups and disulphides. These cleavable groups (*e.g.,* esters and disulphides) are contemplated to improve biodegradability and thus contribute to their favorable toxicity profile.

Provided herein are compounds which are cationic lipids. For example, the cationic lipids of the present invention include compounds having a structure according to Formula (I'): 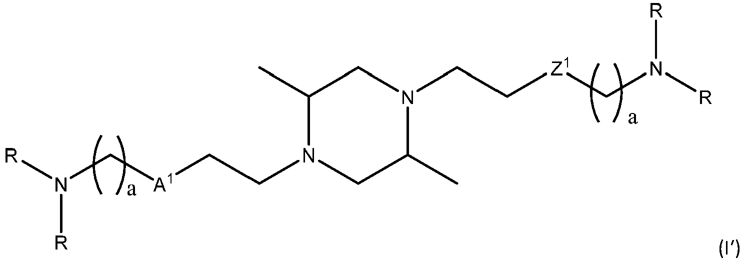 or a pharmaceutically acceptable salt thereof wherein:
**A¹** is selected from 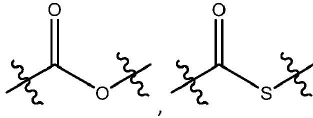 and -S-S-, wherein the left hand side of each depicted structure is bound to the -(CH₂)ₐ-;
**Z¹** is selected from 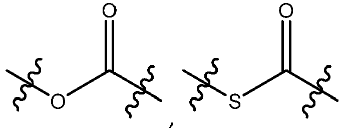 and -S-S-, wherein the right hand side of each depicted structure is bound to the -(CH₂)ₐ-;
each **R** is independently selected from:
   (i) 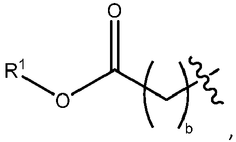 wherein each **R¹** is independently selected from optionally substituted alkyl, optionally substituted alkenyl, optionally substituted alkynyl, - optionally substituted alkyl-(C=O)-O-optionally substituted alkyl, and -optionally substituted alkyl-O-(C=O)-optionally substituted alkyl; and
   (ii) 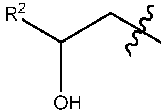 wherein each **R²** is independently selected from optionally substituted alkyl, optionally substituted alkenyl, optionally substituted alkynyl, and optionally substituted acyl;
each **a** is independently selected from 2, 3, 4, and 5; and
each **b** is independently selected from 2, 3, 4, 5, 6 and 7.

In embodiments, the cationic lipids of the present invention include compounds having a structure according to Formula (I): 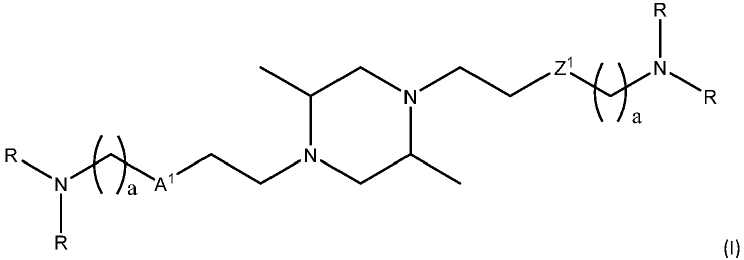 or a pharmaceutically acceptable salt thereof wherein:
**A¹** is selected from 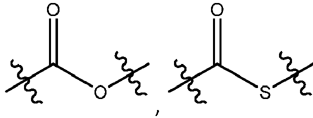 and -S-S-, wherein the left hand side of each depicted structure is bound to the -(CH₂)ₐ-;
**Z¹** is selected from 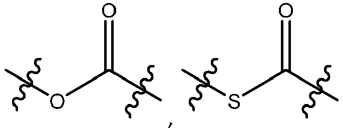 and -S-S-, wherein the right hand side of each depicted structure is bound to the -(CH₂)ₐ-;
each **R** is independently selected from:
   (iii) 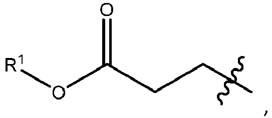 wherein each **R¹** is independently selected from optionally substituted alkyl, optionally substituted alkenyl, optionally substituted alkynyl, -optionally substituted alkyl-(C=O)-O-optionally substituted alkyl, and -optionally substituted alkyl-O-(C=O)-optionally substituted alkyl; and
   (iv) 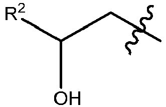 wherein each **R²** is independently selected from optionally substituted alkyl, optionally substituted alkenyl, optionally substituted alkynyl, and optionally substituted acyl; and
each **a** is independently selected from 2, 3, 4, and 5.

In embodiments, the cationic lipids of the present invention include compounds having a structure according to Formula (Ia): 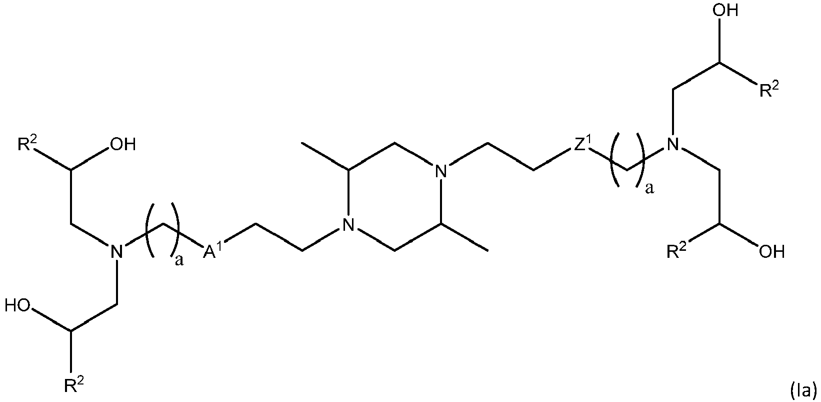 or a pharmaceutically acceptable salt thereof wherein **A¹**, **Z¹**, **R²** and **a** are as defined for Formula (I).

In embodiments, the cationic lipids of the present invention include compounds having a structure according to Formula (Ib): 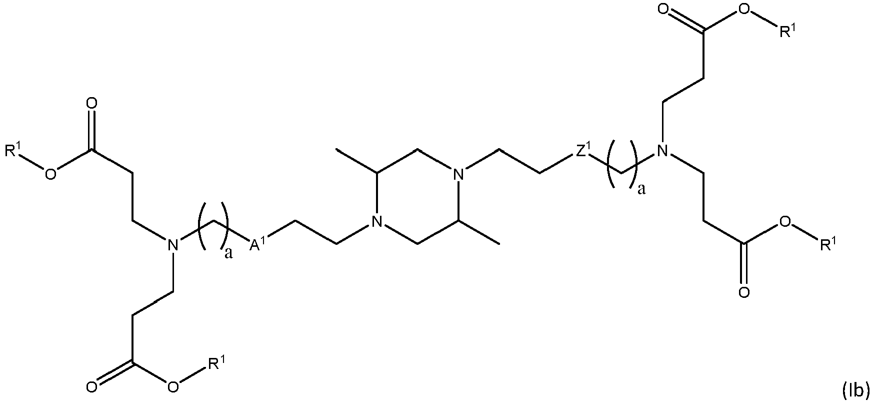 or a pharmaceutically acceptable salt thereof wherein **A¹**, **Z¹**, **R¹** and **a** are as defined for Formula (I).

In embodiments, the cationic lipids of the present invention include compounds having a structure according to Formula (Ib'): 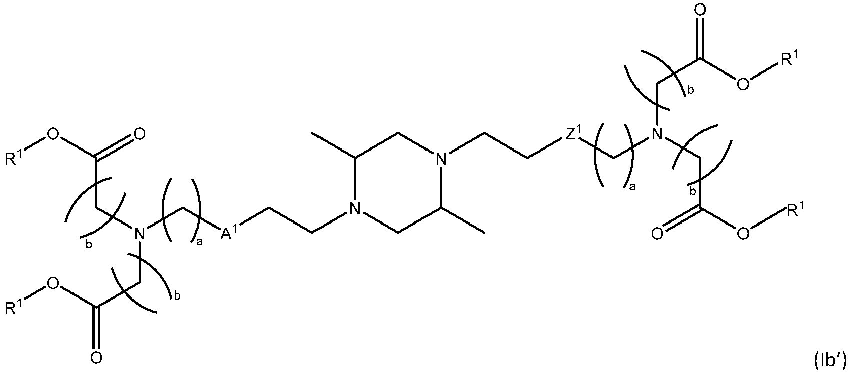 or a pharmaceutically acceptable salt thereof wherein **A¹**, **Z¹**, **R¹**, **a** and **b** are as defined for Formula (I').

In embodiments, the cationic lipids of the present invention include compounds having a structure according to Formula (Ic): 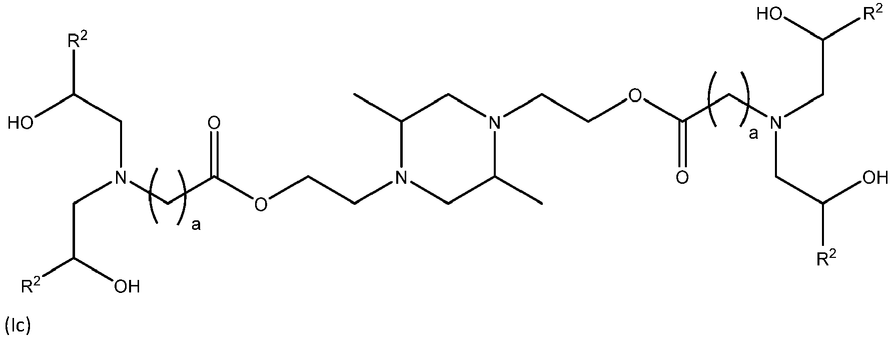 or a pharmaceutically acceptable salt thereof wherein **R²** and **a** are as defined for Formula (I).

In embodiments of the invention (e.g. of Formula (Ic), or a pharmaceutically acceptable salt thereof), each a is independently selected from 2, 3 and 4.

In embodiments of the invention (e.g. of Formula (Ic), or a pharmaceutically acceptable salt thereof), each **a** is the same.

In embodiments of the invention (e.g. of Formula (Ic), or a pharmaceutically acceptable salt thereof), each **a** is 3.

In embodiments of the invention (e.g. of Formula (Ic), or a pharmaceutically acceptable salt thereof), **R²** is independently selected from optionally substituted alkyl. In embodiments of the invention, **R²** is independently selected from optionally substituted C₅-C₅₀ alkyl. In any of the above embodiments, **R²** is independently selected from optionally substituted C₅-C₄₀ alkyl. In any of the above embodiments, **R²** is independently selected from optionally substituted C₅-C₃₀ alkyl. In any of the above embodiments, **R²** is independently selected from optionally substituted C₅-C₂₅ alkyl. In any of the above embodiments, **R²** is independently selected from optionally substituted C₅-C₂₀ alkyl.

In embodiments of the invention (e.g. of Formula (Ic), or a pharmaceutically acceptable salt thereof), **R²** is independently selected from selected from C₈H₁₇, C₁₀H₂₁, C₁₂H₂₅, C₁₄H₂₉, C₁₆H₃₃, C₁₆H₃₁, C₁₆H₂₉, and C₁₆H₂₇.

In some embodiments of the invention (e.g. of Formula (Ic), or a pharmaceutically acceptable salt thereof), **R²** is optionally substituted alkyl. In some embodiments, the optional substituted alkyl is alkyl substituted with -OC(=O)R^{aa}, wherein each R^{aa} is independently selected from optionally substituted alkyl. In some embodiments, the optional substituted alkyl is C₁₋₂₀ alkyl substituted with -OC(=O)R^{aa}, wherein each R^{aa} is independently selected from optionally substituted C₁-C₅₀ alkyl. In some embodiments, the optional substituted alkyl is C₁₋₁₀ alkyl substituted with - OC(=O)R^{aa}, wherein each R^{aa} is independently selected from optionally substituted C₁-C₅₀ alkyl. In some embodiments, the optional substituted alkyl is C₁₋₅ alkyl substituted with -OC(=O)R^{aa}, wherein each R^{aa} is independently selected from optionally substituted C₁-C₅₀ alkyl.

In any of the above embodiments, R^{aa} is independently selected from optionally substituted C₁-C₄₀ alkyl. In any of the above embodiments, R^{aa} is independently selected from optionally substituted C₁-C₃₀ alkyl. In any of the above embodiments, R^{aa} is independently selected from optionally substituted C₁-C₂₅ alkyl. In any of the above embodiments, R^{aa} is independently selected from optionally substituted C₁-C₂₀ alkyl. In any of the above embodiments, R^{aa} is independently selected from optionally substituted C₁-C₁₅ alkyl. In any of the above embodiments, R^{aa} is independently selected from optionally substituted C₁-C₁₀ alkyl. In any of the above embodiments, R^{aa} is independently selected from optionally substituted C₂-C₈ alkyl. In any of the above embodiments, R^{aa} is independently selected from optionally substituted C₃-C₇ alkyl.

In embodiments of the invention (e.g. of Formula (Ic), or a pharmaceutically acceptable salt thereof), each **R²** is the same.

In embodiments of the invention (e.g. of Formula (Ic), or a pharmaceutically acceptable salt thereof), each **R²** is C₁₀H₂₁.

In embodiments of the invention (e.g. of Formula (Ic), or a pharmaceutically acceptable salt thereof), each **R²** is C₁₋₂₀ alkyl substituted with -OC(=O)R^{aa}, wherein each R^{aa} is independently selected from optionally substituted C₁-C₂₀ alkyl.

In embodiments of the invention (e.g. of Formula (Ic), or a pharmaceutically acceptable salt thereof), each **a** is independently selected from 2, 3 and 4 and **R²** is independently selected from optionally substituted alkyl. In any of the above embodiments, **R²** is independently selected from optionally substituted C₅-C₅₀ alkyl. In any of the above embodiments, **R²** is independently selected from optionally substituted C₅-C₄₀ alkyl. In any of the above embodiments, **R²** is independently selected from optionally substituted C₅-C₃₀ alkyl. In any of the above embodiments, **R²** is independently selected from optionally substituted C₅-C₂₅ alkyl. In any of the above embodiments, **R²** is independently selected from optionally substituted C₅-C₂₀ alkyl.

In embodiments of the invention (e.g. of Formula (Ic), or a pharmaceutically acceptable salt thereof), each **a** is independently selected from 2, 3 and 4 and **R²** is independently selected from optionally substituted alkyl wherein the optionally substituted alkyl is alkyl substituted with - OC(=O)R^{aa}, wherein each R^{aa} is independently selected from optionally substituted alkyl. In some embodiments, the optional substituted alkyl is C₁₋₂₀ alkyl substituted with -OC(=O)R^{aa}, wherein each R^{aa} is independently selected from optionally substituted C₁-C₅₀ alkyl. In some embodiments, the optional substituted alkyl is C₁₋₁₀ alkyl substituted with -OC(=O)R^{aa}, wherein each R^{aa} is independently selected from optionally substituted C₁-C₅₀ alkyl. In some embodiments, the optional substituted alkyl is C₁₋₅ alkyl substituted with -OC(=O)R^{aa}, wherein each R^{aa} is independently selected from optionally substituted C₁-C₅₀ alkyl.

In any of the above embodiments, R^{aa} is independently selected from optionally substituted C₁-C₄₀ alkyl. In any of the above embodiments, R^{aa} is independently selected from optionally substituted C₁-C₃₀ alkyl. In any of the above embodiments, R^{aa} is independently selected from optionally substituted C₁-C₂₅ alkyl. In any of the above embodiments, R^{aa} is independently selected from optionally substituted C₁-C₂₀ alkyl. In any of the above embodiments, R^{aa} is independently selected from optionally substituted C₁-C₁₅ alkyl. In any of the above embodiments, R^{aa} is independently selected from optionally substituted C₁-C₁₀ alkyl. In any of the above embodiments, R^{aa} is independently selected from optionally substituted C₂-C₈ alkyl. In any of the above embodiments, R^{aa} is independently selected from optionally substituted C₃-C₇ alkyl.

In embodiments of the invention (e.g. of Formula (Ic), or a pharmaceutically acceptable salt thereof), each **a** is independently selected from 2, 3 and 4 and **R²** is independently selected from selected from C₈H₁₇, C₁₀H₂₁, C₁₂H₂₅, C₁₄H₂₉, C₁₆H₃₃, C₁₆H₃₁, C₁₆H₂₉, and C₁₆H₂₇.

In embodiments of the invention (e.g. of Formula (Ic), or a pharmaceutically acceptable salt thereof), each **a** is 3 and **R²** is independently selected from selected from C₈H₁₇, C₁₀H₂₁, C₁₂H₂₅, C₁₄H₂₉, C₁₆H₃₃, C₁₆H₃₁, C₁₆H₂₉, and C₁₆H₂₇.

In embodiments of the invention (e.g. of Formula (Ic), or a pharmaceutically acceptable salt thereof), each **a** is 3 and **R²** is independently selected from selected from optional substituted alkyl wherein the optionally substituted alkyl is alkyl substituted with -OC(=O)R^{aa}, wherein each R^{aa} is independently selected from optionally substituted alkyl. In some embodiments, the optional substituted alkyl is C₁₋₂₀alkyl substituted with -OC(=O)R^{aa}, wherein each R^{aa} is independently selected from optionally substituted C₁-C₅₀ alkyl. In some embodiments, the optional substituted alkyl is C₁₋₁₀ alkyl substituted with -OC(=O)R^{aa}, wherein each R^{aa} is independently selected from optionally substituted C₁-C₅₀ alkyl. In some embodiments, the optional substituted alkyl is C₁₋₅ alkyl substituted with -OC(=O)R^{aa}, wherein each R^{aa} is independently selected from optionally substituted C₁-C₅₀ alkyl.

In any of the above embodiments, R^{aa} is independently selected from optionally substituted C₁-C₄₀ alkyl. In any of the above embodiments, R^{aa} is independently selected from optionally substituted C₁-C₃₀ alkyl. In any of the above embodiments, R^{aa} is independently selected from optionally substituted C₁-C₂₅ alkyl. In any of the above embodiments, R^{aa} is independently selected from optionally substituted C₁-C₂₀ alkyl. In any of the above embodiments, R^{aa} is independently selected from optionally substituted C₁-C₁₅ alkyl. In any of the above embodiments, R^{aa} is independently selected from optionally substituted C₁-C₁₀ alkyl. In any of the above embodiments, R^{aa} is independently selected from optionally substituted C₂-C₈ alkyl. In any of the above embodiments, R^{aa} is independently selected from optionally substituted C₃-C₇ alkyl.

In embodiments of the invention (e.g. of Formula (Ic), or a pharmaceutically acceptable salt thereof), each **a** is the same and each **R²** is the same.

In embodiments of the invention (e.g. of Formula (Ic), or a pharmaceutically acceptable salt thereof), each **a** is 3 and each **R²** is C₁₀H₂₁.

In embodiments of the invention (e.g. of Formula (Ic), or a pharmaceutically acceptable salt thereof), each **a** is 3 and each **R²** is C₁₋₂₀ alkyl substituted with -OC(=O)R^{aa}, wherein each R^{aa} is independently selected from optionally substituted C₁-C₂₀ alkyl.

In embodiments, the cationic lipids of the present invention are compounds having the structure: 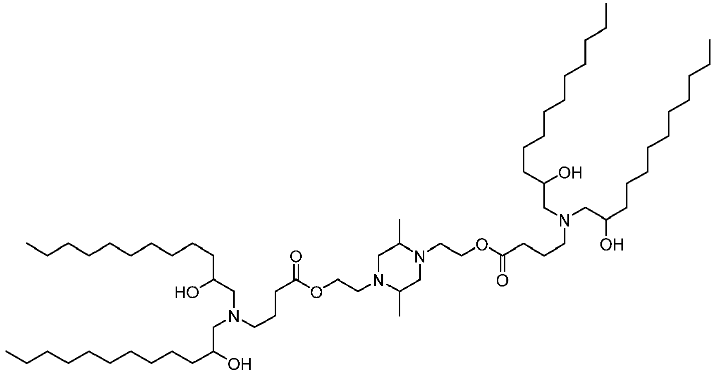 or a pharmaceutically acceptable salt thereof.

In embodiments, the cationic lipids of the present invention are compounds having the structure: 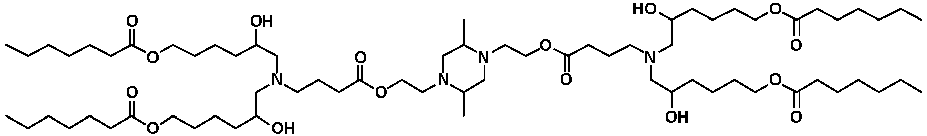 or a pharmaceutically acceptable salt thereof.

In embodiments, the cationic lipids of the present invention are compounds having the structure: 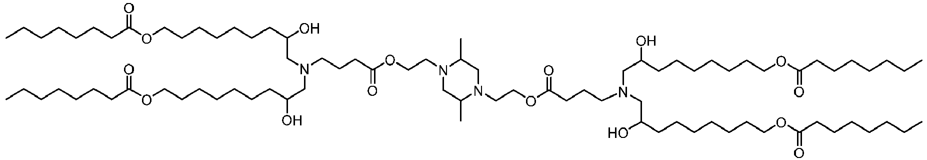 or a pharmaceutically acceptable salt thereof.

In embodiments, the cationic lipids of the present invention include compounds having a structure according to Formula (Id): 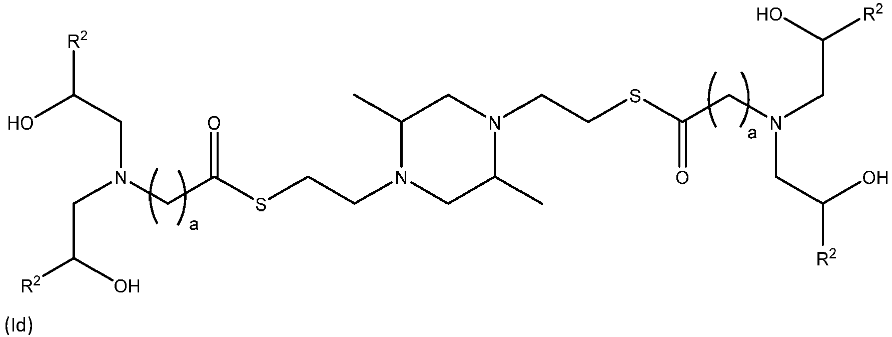 or a pharmaceutically acceptable salt thereof, wherein **R²** and **a** are as defined for Formula (I).

In embodiments, the cationic lipids of the present invention include compounds having a structure according to Formula (le): 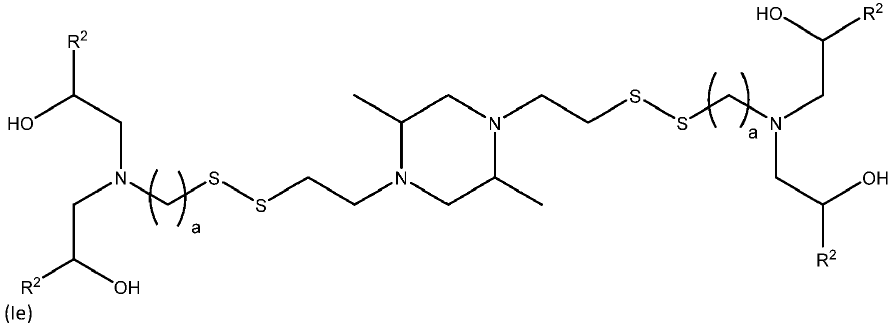 or a pharmaceutically acceptable salt thereof, wherein **R²** and **a** are as defined in Formula (I).

In embodiments, the cationic lipids of the present invention include compounds having a structure according to Formula (If): 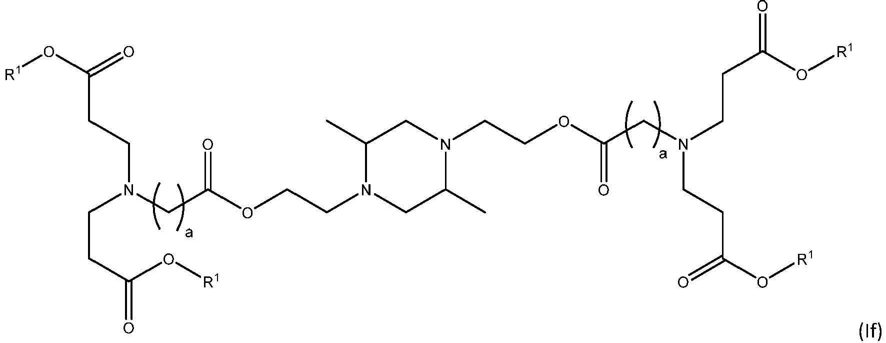 or a pharmaceutically acceptable salt thereof, wherein **R¹** and **a** are as defined in Formula (I).

In embodiments, the cationic lipids of the present invention include compounds having a structure according to Formula (If'): 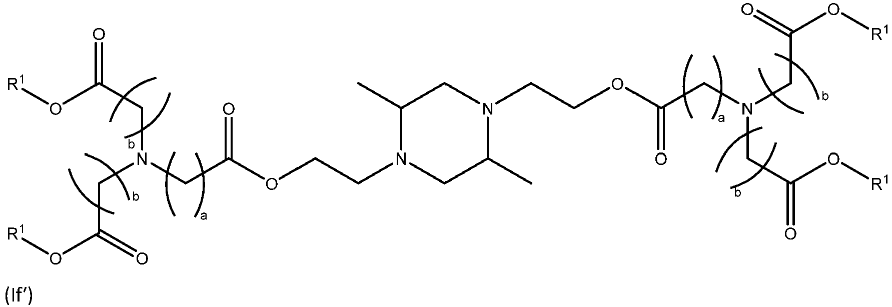 or a pharmaceutically acceptable salt thereof, wherein **R¹ a,** and **b** are as defined in Formula (I').

In embodiments of the invention (e.g. of Formula (If'), or a pharmaceutically acceptable salt thereof), each **a** is independently selected from 2, 3 and 4.

In embodiments of the invention (e.g. of Formula (If'), or a pharmaceutically acceptable salt thereof), each a is the same.

In embodiments of the invention (e.g. of Formula (If'), or a pharmaceutically acceptable salt thereof), each **a** is 3.

In embodiments of the invention (e.g. of Formula (If'), or a pharmaceutically acceptable salt thereof), each **b** is independently selected from 2, 3, 4, 5, 6, and 7.

In embodiments of the invention (e.g. of Formula (If'), or a pharmaceutically acceptable salt thereof), each **b** is the same.

In embodiments of the invention (e.g. of Formula (If'), or a pharmaceutically acceptable salt thereof), each **b** is 3.

In embodiments of the invention (e.g. of Formula (If'), or a pharmaceutically acceptable salt thereof), each **b** is 7.

In embodiments of the invention (e.g. of Formula (If'), or a pharmaceutically acceptable salt thereof), **R¹** is independently selected from optionally substituted alkyl. In any of the above embodiments, **R¹** is independently selected from optionally substituted C₅-C₅₀ alkyl. In any of the above embodiments, **R¹** is independently selected from optionally substituted C₅-C₄₀ alkyl. In any of the above embodiments, **R¹** is independently selected from optionally substituted C₅-C₃₀ alkyl. In any of the above embodiments, **R¹** is independently selected from optionally substituted C₅-C₂₅ alkyl. In any of the above embodiments, **R¹** is independently selected from optionally substituted C₅-C₂₀ alkyl.

In embodiments of the invention (e.g. of Formula (If'), or a pharmaceutically acceptable salt thereof), each **R¹** is the same.

In embodiments of the invention (e.g. of Formula (If'), or a pharmaceutically acceptable salt thereof), each **R¹** is optionally substituted C₅-C₂₀ alkyl.

In embodiments of the invention (e.g. of Formula (If'), or a pharmaceutically acceptable salt thereof), each **a** is independently selected from 2, 3 and 4, each **b** is independently selected from 2, 3, 4, 5, 6, and 7, and **R¹** is independently selected from optionally substituted alkyl. In any of the above embodiments, **R¹** is independently selected from optionally substituted C₅-C₅₀ alkyl. In any of the above embodiments, **R¹** is independently selected from optionally substituted C₅-C₄₀ alkyl. In any of the above embodiments, **R¹** is independently selected from optionally substituted C₅-C₃₀ alkyl. In any of the above embodiments, **R¹** is independently selected from optionally substituted C₅-C₂₅ alkyl. In any of the above embodiments, **R¹** is independently selected from optionally substituted C₅-C₂₀ alkyl.

In embodiments of the invention (e.g. of Formula (If'), or a pharmaceutically acceptable salt thereof), each **a** is 3, each **b** is independently selected from 2, 3, 4, 5, 6, and 7, and **R¹** is independently selected from selected from optionally substituted alkyl. In any of the above embodiments, **R¹** is independently selected from optionally substituted C₅-C₅₀ alkyl. In any of the above embodiments, **R¹** is independently selected from optionally substituted C₅-C₄₀ alkyl. In any of the above embodiments, **R¹** is independently selected from optionally substituted C₅-C₃₀ alkyl. In any of the above embodiments, **R¹** is independently selected from optionally substituted C₅-C₂₅ alkyl. In any of the above embodiments, **R¹** is independently selected from optionally substituted C₅-C₂₀ alkyl.

In embodiments of the invention (e.g. of Formula (If'), or a pharmaceutically acceptable salt thereof), each **a** is 3, each **b** is independently selected from 2, 3, 4, 5, 6, and 7, and each **R¹** is optionally substituted C₅-C₂₀ alkyl.

In embodiments of the invention (e.g. of Formula (If'), or a pharmaceutically acceptable salt thereof, each **a** is 3, each **b** is 3, and each **R¹** is optionally substituted C₅-C₂₀ alkyl.

In one embodiment, the cationic lipid of the present invention is a compound having the structure: 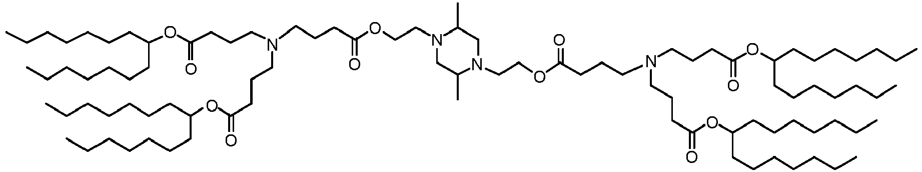 or a pharmaceutically acceptable salt thereof.

In embodiments of the invention (e.g. of Formula (If'), or a pharmaceutically acceptable salt thereof), **R¹** is independently selected from optionally substituted alkenyl. In any of the above embodiments, **R¹** is independently selected from optionally substituted C₅-C₅₀ alkenyl. In any of the above embodiments, **R¹** is independently selected from optionally substituted C₅-C₄₀ alkenyl. In any of the above embodiments, **R¹** is independently selected from optionally substituted C₅-C₃₀ alkenyl. In any of the above embodiments, **R¹** is independently selected from optionally substituted C₅-C₂₅ alkenyl. In any of the above embodiments, **R¹** is independently selected from optionally substituted C₅-C₂₀ alkenyl.

In embodiments of the invention (e.g. of Formula (If'), or a pharmaceutically acceptable salt thereof), each **R¹** is the same.

In embodiments of the invention (e.g. of Formula (If'), or a pharmaceutically acceptable salt thereof), each **R¹** is optionally substituted C₅-C₂₀ alkenyl.

In embodiments of the invention (e.g. of Formula (If'), or a pharmaceutically acceptable salt thereof), each **a** is independently selected from 2, 3 and 4, each **b** is independently selected from 2, 3, 4, 5, 6, and 7, and **R¹** is independently selected from optionally substituted alkenyl. In any of the above embodiments, **R¹** is independently selected from optionally substituted C₅-C₅₀ alkenyl. In any of the above embodiments, **R¹** is independently selected from optionally substituted C₅-C₄₀ alkenyl. In any of the above embodiments, **R¹** is independently selected from optionally substituted C₅-C₃₀ alkenyl. In any of the above embodiments, **R¹** is independently selected from optionally substituted C₅-C₂₅ alkenyl. In any of the above embodiments, **R¹** is independently selected from optionally substituted C₅-C₂₀ alkenyl.

In embodiments of the invention (e.g. of Formula (If'), or a pharmaceutically acceptable salt thereof), each **a** is 3, each **b** is independently selected from 2, 3, 4, 5, 6, and 7, and **R¹** is independently selected from selected from optionally substituted alkenyl. In any of the above embodiments, **R¹** is independently selected from optionally substituted C₅-C₅₀ alkenyl. In any of the above embodiments, **R¹** is independently selected from optionally substituted C₅-C₄₀ alkenyl. In any of the above embodiments, **R¹** is independently selected from optionally substituted C₅-C₃₀ alkenyl. In any of the above embodiments, **R¹** is independently selected from optionally substituted C₅-C₂₅ alkenyl. In any of the above embodiments, **R¹** is independently selected from optionally substituted C₅-C₂₀ alkenyl.

In embodiments of the invention (e.g. of Formula (If'), or a pharmaceutically acceptable salt thereof), each **a** is 3, each **b** is independently selected from 2, 3, 4, 5, 6, and 7, and each **R¹** is optionally substituted C₅-C₂₀ alkenyl.

In embodiments of the invention (e.g. of Formula (I**f**'), or a pharmaceutically acceptable salt thereof, each **a** is 3, each **b** is 7, and each **R¹** is optionally substituted C₅-C₂₀ alkenyl.

In one embodiment, the cationic lipid of the present invention is a compound having the structure: 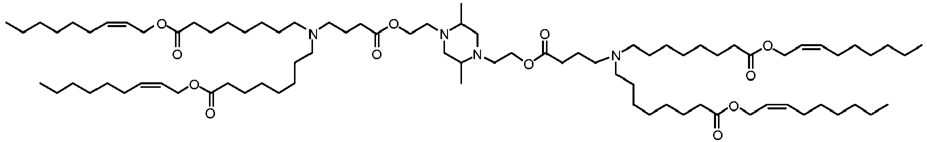 or a pharmaceutically acceptable salt thereof.

In any of the above embodiments (e.g. a compound of Formula (I'), (I), (Ia), (Ib), (Ib'), (Ic), (Id), (Ie), (If), (If') or a pharmaceutically acceptable salt thereof), each **a** is independently selected from 2, 3 and 4.

In some embodiments (e.g. a compound of Formula (I'), (I), (Ia), (lb), (lb'), (Ic), (Id), (le), (If), (If') or a pharmaceutically acceptable salt thereof), each **a** is 2. In some embodiments (e.g. a compound of Formula (I'), (I), (Ia), (lb), (lb'), (Ic), (Id), (le), (If), (I**f'**) or a pharmaceutically acceptable salt thereof), each **a** is 3. In some embodiments (e.g. a compound of Formula (I'), (I), (Ia), (Ib), (Ib'), (Ic), (Id), (le), (If), (If') or a pharmaceutically acceptable salt thereof), each **a** is 4.

In any of the above embodiments (e.g. a compound of Formula (I'), (I), (Ia), (Ib), (Ib'), (Ic), (Id), (Ie), (If), (If') or a pharmaceutically acceptable salt thereof), each **a** is the same.

In any of the above embodiments (e.g. a compound of Formula (I'), (I), (Ia), (Ib), (Ib'), (Ic), (Id), (Ie), (If), (If') or a pharmaceutically acceptable salt thereof), each **a** is different.

In any of the above embodiments (e.g. a compound of Formula (I'), (Ib'), (If') or a pharmaceutically acceptable salt thereof), each **b** is independently selected from 2, 3, 4, 5, 6 and 7.

In some embodiments (e.g. a compound of Formula (I'), (Ib'), (If') or a pharmaceutically acceptable salt thereof), each **b** is 2. In some embodiments (e.g. a compound of Formula (I'), (Ib'), (If') or a pharmaceutically acceptable salt thereof), each **b** is 3. In some embodiments (e.g. a compound of Formula (I'), (Ib'), (If') or a pharmaceutically acceptable salt thereof), each **b** is 4. In some embodiments (e.g. a compound of Formula (I'), (Ib'), (If') or a pharmaceutically acceptable salt thereof), each **b** is 5. In some embodiments (e.g. a compound of Formula (I'), (Ib'), (If') or a pharmaceutically acceptable salt thereof), each **b** is 6. In some embodiments (e.g. a compound of Formula (I'), (Ib'), (If') or a pharmaceutically acceptable salt thereof), each **b** is 7.

In any of the above embodiments (e.g. a compound of Formula (I'), (Ib'), (If') or a pharmaceutically acceptable salt thereof), each **b** is the same.

In any of the above embodiments (e.g. a compound of Formula (I'), (Ib'), (If') or a pharmaceutically acceptable salt thereof), each **b** is different.

In embodiments **A¹** is selected from 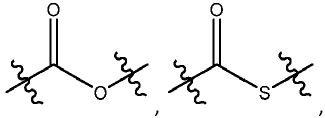 wherein the left hand side of each depicted structure is bound to the -(CH₂)ₐ-.

In embodiments **A¹** is 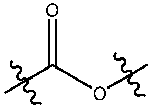 wherein the left hand side of each depicted structure is bound to the -(CH₂)ₐ-. In embodiments **A¹** is 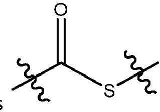 wherein the left hand side of each depicted structure is bound to the -(CH₂)ₐ-.

In embodiments **Z¹** is selected from 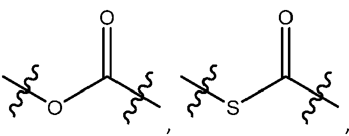 wherein the right hand side of each depicted structure is bound to the -(CH₂)ₐ-.

In embodiments **Z¹** is 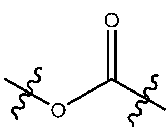 wherein the right hand side of each depicted structure is bound to the -(CH₂)ₐ-. In embodiments **Z¹** is 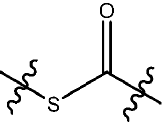 wherein the right hand side of each depicted structure is bound to the -(CH₂)ₐ-. In embodiments **Z¹** is -S-S-.

In embodiments **A¹** is 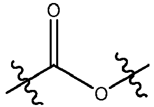 wherein the left hand side of each depicted structure is bound to the -(CH₂)ₐ- and **Z¹** is 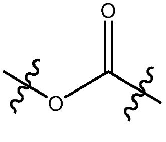 wherein the right hand side of each depicted structure is bound to the -(CH₂)ₐ-.

In embodiments, **A¹** is 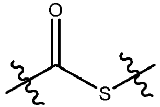 wherein the left hand side of each depicted structure is bound to the -(CH₂)ₐ- and **Z¹** is 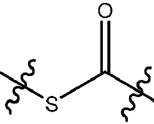 wherein the right hand side of each depicted structure is bound to the -(CH₂)ₐ-.

In embodiments, **A¹** and **Z¹** are each -S-S-.

In any of the above embodiments (e.g. a compound of Formula (I'), (I), (Ib), (Ib'), (If), (I**f**'), or a pharmaceutically acceptable salt thereof), each **R¹** is the same.

In any of the above embodiments (e.g. a compound of Formula (I'), (I), (Ib), (Ib'), (If), (I**f**'), or a pharmaceutically acceptable salt thereof), each **R¹** is different.

In any of the above embodiments (e.g. a compound of Formula (I'), (I), (Ib), (Ib'), (If), (I**f**'), or a pharmaceutically acceptable salt thereof), each **R¹** is independently selected from: 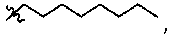 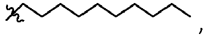 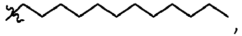 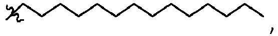 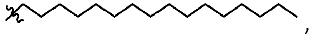 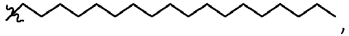 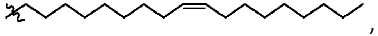 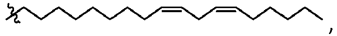 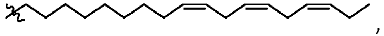 and

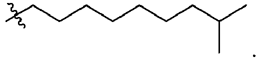

In any of the above embodiments (e.g. a compound of Formula (I'), (I), (Ib), (Ib'), (If), (I**f**'), or a pharmaceutically acceptable salt thereof), each **R¹** is independently selected from: 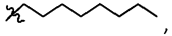 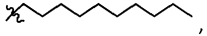 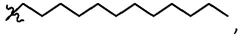 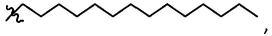 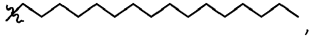 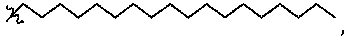 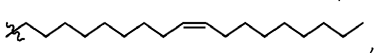 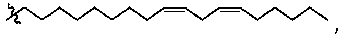 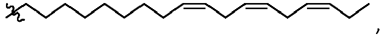 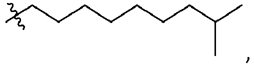 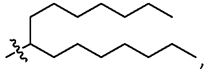 and

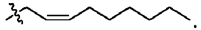

In any of the above embodiments (e.g. a compound of Formula (I'), (I), (Ib), (Ib'), (If), (I**f**'), or a pharmaceutically acceptable salt thereof), each **R¹** is independently selected from optionally substituted alkyl, optionally substituted alkenyl, -optionally substituted alkyl-(C=O)-O-optionally substituted alkyl, or -optionally substituted alkyl-O-(C=O)-optionally substituted alkyl.

In any of the above embodiments (e.g. a compound of Formula (I'), (I), (Ib), (Ib'), (If), (I**f**'), or a pharmaceutically acceptable salt thereof), each **R¹** is independently selected from optionally substituted Cs-Cso alkyl, optionally substituted C₅-C₅₀ alkenyl, optionally substituted C₅-C₅₀ alkynyl, - optionally substituted C₂-C₂₅ alkyl-(C=O)-O-optionally substituted C₂-C₂₅ alkyl, or -optionally substituted C₂-C₂₅ alkyl-O-(C=O)-optionally substituted C₂-C₂₅ alkyl.

In any of the above embodiments (e.g. a compound of Formula (I'), (I), (Ib), (Ib'), (If), (I**f**'), or a pharmaceutically acceptable salt thereof), each **R¹** is independently selected from optionally substituted C₅-C₄₀ alkyl, optionally substituted C₅-C₄₀ alkenyl, optionally substituted C₅-C₄₀ alkynyl, - optionally substituted C₂-C₂₀ alkyl-(C=O)-O-optionally substituted C₂-C₂₀ alkyl, or -optionally substituted C₂-C₂₀ alkyl-O-(C=O)-optionally substituted C₂-C₂₀ alkyl.

In any of the above embodiments (e.g. a compound of Formula (I'), (I), (Ib), (Ib'), (If), (I**f**'), or a pharmaceutically acceptable salt thereof), each **R¹** is independently selected from optionally substituted C₅-C₃₀ alkyl, optionally substituted C₅-C₃₀ alkenyl, optionally substituted C₅-C₃₀ alkynyl, - optionally substituted C₂-C₁₅ alkyl-(C=O)-O-optionally substituted C₂-C₁₅ alkyl, or -optionally substituted C₂-C₁₅ alkyl-O-(C=O)-optionally substituted C₂-C₁₅ alkyl.

In any of the above embodiments (e.g. a compound of Formula (I'), (I), (Ib), (Ib'), (If), (I**f**'), or a pharmaceutically acceptable salt thereof), each **R¹** is independently selected from optionally substituted C₅-C₂₅ alkyl, optionally substituted C₅-C₂₅ alkenyl, optionally substituted C₅-C₂₅ alkynyl, - optionally substituted C₂-C₁₅ alkyl-(C=O)-O-optionally substituted C₂-C₁₅ alkyl, or -optionally substituted C₂-C₁₅ alkyl-O-(C=O)-optionally substituted C₂-C₁₅ alkyl.

In any of the above embodiments (e.g. a compound of Formula (I'), (I), (Ib), (Ib'), (If), (I**f**'), or a pharmaceutically acceptable salt thereof), each **R¹** is independently selected from optionally substituted C₅-C₂₀ alkyl, optionally substituted C₅-C₂₀ alkenyl, optionally substituted C₅-C₂₀ alkynyl, - optionally substituted C₂-C₁₀ alkyl-(C=O)-O-optionally substituted C₂-C₁₀ alkyl, or -optionally substituted C₂-C₁₀ alkyl-O-(C=O)-optionally substituted C₂-C₁₀ alkyl.

In any of the above embodiments (e.g. a compound of Formula (I'), (I),(Ib), (Ib'), (If), (I**f**'), or a pharmaceutically acceptable salt thereof), each **R¹** is independently selected from optionally substituted alkyl. In any of the above embodiments (e.g. a compound of Formula (I'), (I), (lb), (lb'), (If), (I**f**'), or a pharmaceutically acceptable salt thereof), each **R¹** is independently selected from optionally substituted C₅-C₅₀ alkyl. In any of the above embodiments (e.g. a compound of Formula (I'), (I), (Ib), (Ib'), (If), (I**f**'), or a pharmaceutically acceptable salt thereof), each **R¹** is independently selected from optionally substituted C₅-C₄₀ alkyl. In any of the above embodiments (e.g. a compound of Formula (I'), (I), (Ib), (Ib'), (If), (I**f**'), or a pharmaceutically acceptable salt thereof), each **R¹** is independently selected from optionally substituted C₅-C₃₀ alkyl. In any of the above embodiments (e.g. a compound of Formula (I'), (I), (Ib), (Ib'), (If), (I**f**'), or a pharmaceutically acceptable salt thereof), each **R¹** is independently selected from optionally substituted C₅-C₂₅ alkyl. In any of the above embodiments (e.g. a compound of Formula (I'), (I), (Ib), (Ib'), (If), (I**f**'), or a pharmaceutically acceptable salt thereof), each **R¹** is independently selected from optionally substituted C₅-C₂₀ alkyl.

In any of the above embodiments (e.g. a compound of Formula (I'), (I), (Ib), (Ib'), (If), (I**f**'), or a pharmaceutically acceptable salt thereof), each **R¹** is independently selected from optionally substituted alkenyl. In any of the above embodiments (e.g. a compound of Formula (I'), (I), (Ib), (Ib'), (If), (I**f**'), or a pharmaceutically acceptable salt thereof), each **R¹** is independently selected from optionally substituted C₅-C₅₀ alkenyl. In any of the above embodiments (e.g. a compound of Formula (I'), (I), (Ib), (Ib'), (If), (I**f**'), or a pharmaceutically acceptable salt thereof), each **R¹** is independently selected from optionally substituted C₅-C₄₀ alkenyl. In any of the above embodiments (e.g. a compound of Formula (I'), (I), (Ib), (Ib'), (If), (I**f**'), or a pharmaceutically acceptable salt thereof), each **R¹** is independently selected from optionally substituted C₅-C₃₀ alkenyl. In any of the above embodiments (e.g. a compound of Formula (I'), (I), (Ib), (Ib'), (If), (I**f**'), or a pharmaceutically acceptable salt thereof), each **R¹** is independently selected from optionally substituted C₅-C₂₅ alkenyl. In any of the above embodiments (e.g. a compound of Formula (I'), (I), (Ib), (Ib'), (If), (I**f**'), or a pharmaceutically acceptable salt thereof), each **R¹** is independently selected from optionally substituted C₅-C₂₀ alkenyl.

In any of the above embodiments (e.g. a compound of Formula (I'), (I), (Ib), (Ib'), (If), (I**f**'), or a pharmaceutically acceptable salt thereof), each **R¹** is independently selected from optionally substituted alkynyl. In any of the above embodiments (e.g. a compound of Formula (I'), (I), (Ib), (Ib'), (If), (I**f**'), or a pharmaceutically acceptable salt thereof), each **R¹** is independently selected from optionally substituted C₅-C₅₀ alkynyl. In any of the above embodiments (e.g. a compound of Formula (I'), (I), (Ib), (Ib'), (If), (I**f**'), or a pharmaceutically acceptable salt thereof), each **R¹** is independently selected from optionally substituted C₅-C₄₀ alkynyl. In any of the above embodiments (e.g. a compound of Formula (I'), (I), (Ib), (Ib'), (If), (I**f**'), or a pharmaceutically acceptable salt thereof), each **R¹** is independently selected from optionally substituted C₅-C₃₀ alkynyl. In any of the above embodiments (e.g. a compound of Formula (I'), (I), (Ib), (Ib'), (If), (I**f**'), or a pharmaceutically acceptable salt thereof), each **R¹** is independently selected from optionally substituted C₅-C₂₅ alkynyl. In any of the above embodiments (e.g. a compound of Formula (I'), (I), (Ib), (Ib'), (If), (I**f**'), or a pharmaceutically acceptable salt thereof), each **R¹** is independently selected from optionally substituted C₅-C₂₀ alkynyl.

In any of the above embodiments (e.g. a compound of Formula (I'), (I), (Ib), (Ib'), (If), (I**f**'), or a pharmaceutically acceptable salt thereof), each **R¹** is independently selected from -optionally substituted alkyl-(C=O)-O-optionally substituted alkyl. In any of the above embodiments (e.g. a compound of Formula (I'), (I), (Ib), (Ib'), (If), (I**f**'), or a pharmaceutically acceptable salt thereof), each **R¹** is independently selected from -optionally substituted C₂-C₂₅ alkyl-(C=O)-O-optionally substituted C₂-C₂₅ alkyl. In any of the above embodiments (e.g. a compound of Formula (I'), (I), (Ib), (Ib'), (If), (I**f**'), or a pharmaceutically acceptable salt thereof), each **R¹** is independently selected from -optionally substituted C₂-C₂₀ alkyl-(C=O)-O-optionally substituted C₂-C₂₀ alkyl. In any of the above embodiments (e.g. a compound of Formula (I'), (I), (Ib), (Ib'), (If), (I**f**'), or a pharmaceutically acceptable salt thereof), each **R¹** is independently selected from -optionally substituted C₂-C₁₅ alkyl-(C=O)-O-optionally substituted C₂-C₁₅ alkyl. In any of the above embodiments (e.g. a compound of Formula (I'), (I), (Ib), (Ib'), (If), (I**f**'), or a pharmaceutically acceptable salt thereof), each **R¹** is independently selected from -optionally substituted C₂-C₁₀ alkyl-(C=O)-O-optionally substituted C₂-C₁₀ alkyl.

In any of the above embodiments (e.g. a compound of Formula (I'), (I), (Ib), (Ib'), (If), (I**f**'), or a pharmaceutically acceptable salt thereof), each **R¹** is independently selected from -optionally substituted alkyl-O-(C=O)-optionally substituted alkyl. In any of the above embodiments (e.g. a compound of Formula (I'), (I), (Ib), (Ib'), (If), (I**f**'), or a pharmaceutically acceptable salt thereof), each **R¹** is independently selected from -optionally substituted C₂-C₂₅ alkyl-O-(C=O)-optionally substituted C₂-C₂₅ alkyl. In any of the above embodiments (e.g. a compound of Formula (I'), (I), (Ib), (Ib'), (If), (I**f**'), or a pharmaceutically acceptable salt thereof), each **R¹** is independently selected from -optionally substituted C₂-C₂₀ alkyl-O-(C=O)-optionally substituted C₂-C₂₀ alkyl. In any of the above embodiments (e.g. a compound of Formula (I'), (I), (Ib), (Ib'), (If), (I**f**'), or a pharmaceutically acceptable salt thereof), each **R¹** is independently selected from -optionally substituted C₂-C₁₅ alkyl-O-(C=O)-optionally substituted C₂-C₁₅ alkyl. In any of the above embodiments (e.g. a compound of Formula (I'), (I), (Ib), (Ib'), (If), (I**f**'), or a pharmaceutically acceptable salt thereof), each **R¹** is independently selected from -optionally substituted C₂-C₁₀ alkyl-O-(C=O)-optionally substituted C₂-C₁₀ alkyl.

In any of the above embodiments (e.g. a compound of Formula (I'), (I), (Ib), (Ib'), (If), (I**f**'), or a pharmaceutically acceptable salt thereof), each **R¹** is independently selected from C₈H₁₇, C₁₀H₂₁, C₁₂H₂₅, C₁₄H₂₉, C₁₆H₃₃, C₁₈H₃₇, C₁₈H₃₅, C₁₈H₃₃, and C₁₈H₃₁.

In any of the above embodiments (e.g. a compound of Formula (I'), (I), (Ib), (Ib'), (If), (I**f**'), or a pharmaceutically acceptable salt thereof), **R¹** is C₈H₁₇.

In any of the above embodiments (e.g. a compound of Formula (I'), (I), (lb), (lb'), (If), (I**f**'), or a pharmaceutically acceptable salt thereof), **R¹** is C₁₀H₂₁.

In any of the above embodiments (e.g. a compound of Formula (I'), (I), (lb), (lb'), (If), (I**f**'), or a pharmaceutically acceptable salt thereof), **R¹** is C₁₂H₂₅.

In any of the above embodiments (e.g. a compound of Formula (I'), (I), (lb), (lb'), (If), (I**f**'), or a pharmaceutically acceptable salt thereof), **R¹** is C₁₄H₂₉.

In any of the above embodiments (e.g. a compound of Formula (I'), (I), (lb), (lb'), (If), (I**f**'), or a pharmaceutically acceptable salt thereof), **R¹** is C₁₆H₃₃.

In any of the above embodiments (e.g. a compound of Formula (I'), (I), (lb), (lb'), (If), (I**f**'), or a pharmaceutically acceptable salt thereof), **R¹** is C₁₈H₃₇.

In any of the above embodiments (e.g. a compound of Formula (I'), (I), (lb), (lb'), (If), (I**f**'), or a pharmaceutically acceptable salt thereof), **R¹** is C₁₈H₃₅.

In any of the above embodiments (e.g. a compound of Formula (I'), (I), (lb), (lb'), (If), (I**f**'), or a pharmaceutically acceptable salt thereof), **R¹** is C₁₈H₃₃.

In any of the above embodiments (e.g. a compound of Formula (I'), (I), (lb), (lb'), (If), (I**f**'), or a pharmaceutically acceptable salt thereof), **R¹** is C₁₈H₃₁.

In any of the above embodiments (e.g. a compound of Formula (I'), (I), (Ia), (Ic), (Id), (le), or a pharmaceutically acceptable salt thereof), each **R²** is the same.

In any of the above embodiments (e.g. a compound of Formula (I'), (I), (Ia), (Ic), (Id), (le), or a pharmaceutically acceptable salt thereof), each **R²** is different.

In any of the above embodiments (e.g. a compound of Formula (I'), (I), (Ia), (Ic), (Id), (le), or a pharmaceutically acceptable salt thereof), each **R²** is independently selected from: 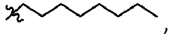 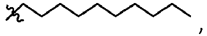 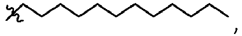 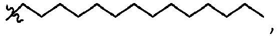 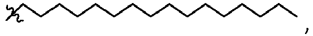 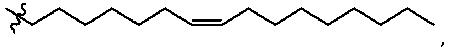 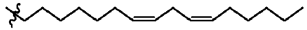 and

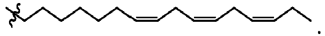

In any of the above embodiments (e.g. a compound of Formula (I'), (I), (Ia), (Ic), (Id), (le), or a pharmaceutically acceptable salt thereof), each **R²** is independently selected from: 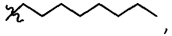 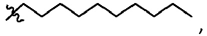 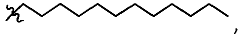 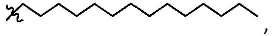 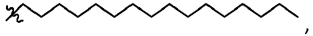 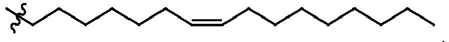 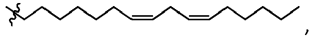 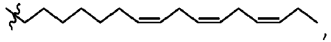 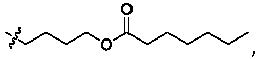 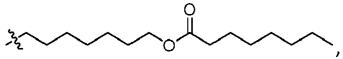 and

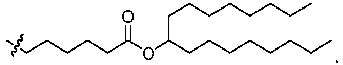

In any of the above embodiments (e.g. a compound of Formula (I'), (I), (Ia), (Ic), (Id), (Ie), or a pharmaceutically acceptable salt thereof), **R²** is independently selected from optionally substituted alkyl, optionally substituted alkenyl, optionally substituted.

In any of the above embodiments (e.g. a compound of Formula (I'), (I), (Ia), (Ic), (Id), (le), or a pharmaceutically acceptable salt thereof), **R²** is independently selected from optionally substituted alkyl. In embodiments, (e.g. a compound of Formula (I'), (I), (Ia), (Ic), (Id), (le), or a pharmaceutically acceptable salt thereof) **R²** is independently selected from optionally substituted C₅-C₅₀ alkyl. In any of the above embodiments (e.g. a compound of Formula (I'), (I), (Ia), (Ic), (Id), (le), or a pharmaceutically acceptable salt thereof), **R²** is independently selected from optionally substituted C₅-C₄₀ alkyl. In any of the above embodiments (e.g. a compound of Formula (I'), (I), (Ia), (Ic), (Id), (le), or a pharmaceutically acceptable salt thereof), **R²** is independently selected from optionally substituted C₅-C₃₀ alkyl. In any of the above embodiments (e.g. a compound of Formula (I'), (I), (Ia), (Ic), (Id), (le), or a pharmaceutically acceptable salt thereof), **R²** is independently selected from optionally substituted C₅-C₂₅ alkyl. In any of the above embodiments, **R²** is independently selected from optionally substituted C₅-C₂₀ alkyl.

In any of the above embodiments (e.g. a compound of Formula (I'), (I), (Ia), (Ic), (Id), (le), or a pharmaceutically acceptable salt thereof), **R²** is independently selected from optionally substituted alkenyl. In any of the above embodiments (e.g. a compound of Formula (I'), (I), (Ia), (Ic), (Id), (le), or a pharmaceutically acceptable salt thereof), **R²** is independently selected from optionally substituted C₅-C₅₀ alkenyl. In any of the above embodiments (e.g. a compound of Formula (I'), (I), (Ia), (Ic), (Id), (le), or a pharmaceutically acceptable salt thereof), **R²** is independently selected from optionally substituted C₅-C₄₀ alkenyl. In any of the above embodiments (e.g. a compound of Formula (I'), (I), (Ia), (Ic), (Id), (Ie), or a pharmaceutically acceptable salt thereof), **R²** is independently selected from optionally substituted C₅-C₃₀ alkenyl. In any of the above embodiments (e.g. a compound of Formula (I'), (I), (Ia), (Ic), (Id), (Ie), or a pharmaceutically acceptable salt thereof), **R²** is independently selected from optionally substituted C₅-C₂₅ alkenyl. In any of the above embodiments (e.g. a compound of Formula (I'), (I), (Ia), (Ic), (Id), (Ie), or a pharmaceutically acceptable salt thereof), **R²** is independently selected from optionally substituted C₅-C₂₀ alkenyl.

In any of the above embodiments (e.g. a compound of Formula (I'), (I), (Ia), (Ic), (Id), (Ie), or a pharmaceutically acceptable salt thereof), **R²** is independently selected from optionally substituted alkynyl. In any of the above embodiments (e.g. a compound of Formula (I'), (I), (Ia), (Ic), (Id), (Ie), or a pharmaceutically acceptable salt thereof), **R²** is independently selected from optionally substituted C₅-C₅₀ alkynyl. In any of the above embodiments (e.g. a compound of Formula (I'), (I), (Ia), (Ic), (Id), (le), or a pharmaceutically acceptable salt thereof), **R²** is independently selected from optionally substituted C₅-C₄₀ alkynyl. In any of the above embodiments (e.g. a compound of Formula (I'), (I), (Ia), (Ic), (Id), (le), or a pharmaceutically acceptable salt thereof), **R²** is independently selected from optionally substituted C₅-C₃₀ alkynyl. In any of the above embodiments (e.g. a compound of Formula (I'), (I), (Ia), (Ic), (Id), (Ie), or a pharmaceutically acceptable salt thereof), **R²** is independently selected from optionally substituted C₅-C₂₅ alkynyl. In any of the above embodiments (e.g. a compound of Formula (I'), (I), (Ia), (Ic), (Id), (Ie), or a pharmaceutically acceptable salt thereof), **R²** is independently selected from optionally substituted C₅-C₂₀ alkynyl.

In any of the above embodiments (e.g. a compound of Formula (I'), (I), (Ia), (Ic), (Id), (Ie), or a pharmaceutically acceptable salt thereof), **R²** is independently selected from optionally substituted acyl. In any of the above embodiments (e.g. a compound of Formula (I'), (I), (Ia), (Ic), (Id), (Ie), or a pharmaceutically acceptable salt thereof), **R²** is independently selected from optionally substituted C₅-C₅₀ acyl. In any of the above embodiments (e.g. a compound of Formula (I'), (I), (Ia), (Ic), (Id), (Ie), or a pharmaceutically acceptable salt thereof), **R²** is independently selected from optionally substituted C₅-C₄₀ acyl. In any of the above embodiments (e.g. a compound of Formula (I'), (I), (Ia), (Ic), (Id), (le), or a pharmaceutically acceptable salt thereof), **R²** is independently selected from optionally substituted C₅-C₃₀ acyl. In any of the above embodiments (e.g. a compound of Formula (I'), (I), (Ia), (Ic), (Id), (le), or a pharmaceutically acceptable salt thereof), **R²** is independently selected from optionally substituted C₅-C₂₅ acyl. In any of the above embodiments (e.g. a compound of Formula (I'), (I), (Ia), (Ic), (Id), (le), or a pharmaceutically acceptable salt thereof), **R²** is independently selected from optionally substituted C₅-C₂₀ acyl.

In any of the above embodiments (e.g. a compound of Formula (I'), (I), (Ia), (Ic), (Id), (le), or a pharmaceutically acceptable salt thereof), **R²** is independently selected from selected from C₈H₁₇, C₁₀H₂₁, C₁₂H₂₅, C₁₄H₂₉, C₁₆H₃₃, C₁₆H₃₁, C₁₆H₂₉, and C₁₆H₂₇.

In any of the above embodiments (e.g. a compound of Formula (I'), (I), (Ia), (Ic), (Id), (le), or a pharmaceutically acceptable salt thereof), **R²** is C₈H₁₇.

In any of the above embodiments (e.g. a compound of Formula (I'), (I), (Ia), (Ic), (Id), (le), or a pharmaceutically acceptable salt thereof), **R²** is C₁₀H₂₁.

In any of the above embodiments (e.g. a compound of Formula (I'), (I), (Ia), (Ic), (Id), (Ie), or a pharmaceutically acceptable salt thereof), **R²** is C₁₂H₂₅.

In any of the above embodiments (e.g. a compound of Formula (I'), (I), (Ia), (Ic), (Id), (le), or a pharmaceutically acceptable salt thereof), **R²** is C₁₄H₂₉.

In any of the above embodiments (e.g. a compound of Formula (I'), (I), (Ia), (Ic), (Id), (le), or a pharmaceutically acceptable salt thereof), **R²** is C₁₆H₃₃.

In any of the above embodiments (e.g. a compound of Formula (I'), (I), (Ia), (Ic), (Id), (le), or a pharmaceutically acceptable salt thereof), **R²** is C₁₆H₃₁.

In any of the above embodiments (e.g. a compound of Formula (I'), (I), (Ia), (Ic), (Id), (le), or a pharmaceutically acceptable salt thereof), **R²** is C₁₆H₂₉.

In any of the above embodiments (e.g. a compound of Formula (I'), (I), (Ia), (Ic), (Id), (le), or a pharmaceutically acceptable salt thereof), **R²** is C₁₆H₂₇.

In embodiments, the cationic lipids of the present invention include compounds selected from those depicted in Tables A-D, or a pharmaceutically acceptable salt thereof.

In embodiments, a composition comprising the cationic lipid of any one of the preceding embodiments, one or more non-cationic lipids, one or more cholesterol-based lipids and one or more PEG-modified lipid is provided. In embodiments, this composition is a lipid nanoparticle. In embodiments, the one or more cationic lipid(s) constitute(s) about 30 mol %-60 mol % of the lipid nanoparticle. In embodiments, the one or more non-cationic lipid(s) constitute(s) 10 mol%-50 mol% of the lipid nanoparticle. In embodiments, the one or more PEG-modified lipid(s) constitute(s) 1 mol%-10 mol% of the lipid nanoparticle. In embodiments, the cholesterol-based lipid constitutes 10 mol%-50 mol% of the lipid nanoparticle. In embodiments, the lipid nanoparticle encapsulates a nucleic acid, optionally an mRNA encoding a peptide or protein. In embodiments, the lipid nanoparticles have an encapsulation percentage for mRNA of at least 70%. In embodiments, the lipid nanoparticles have an encapsulation percentage for mRNA of at least 75%. In embodiments, the lipid nanoparticles have an encapsulation percentage for mRNA of at least 80%. In embodiments, the lipid nanoparticles have an encapsulation percentage for mRNA of at least 85%. In embodiments, the lipid nanoparticles have an encapsulation percentage for mRNA of at least 90%. In embodiments, the lipid nanoparticles have an encapsulation percentage for mRNA of at least 95%.

In embodiments, the composition of any one of the preceding embodiments is for use in therapy.

In embodiments, the composition of any one of the preceding embodiments is for use in a method of treating or preventing a disease amenable to treatment or prevention by the peptide or protein encoded by the mRNA, optionally wherein the disease is (a) a protein deficiency, optionally wherein the protein deficiency affects the liver, lung, brain or muscle, (b) an autoimmune disease, (c) an infectious disease, or (d) cancer.

In embodiments, the composition is administered intravenously, intrathecally or intramuscularly, or by pulmonary delivery, optionally through nebulization.

### Exemplary Compounds

Exemplary compounds include those described in Tables A-D, or a pharmaceutically acceptable salt thereof.

**Table A - Piperazine-based ester/ester cationic lipids**

| **Table A - Piperazine-based ester/ester cationic lipids** | |
|---|---|
| **A1** | 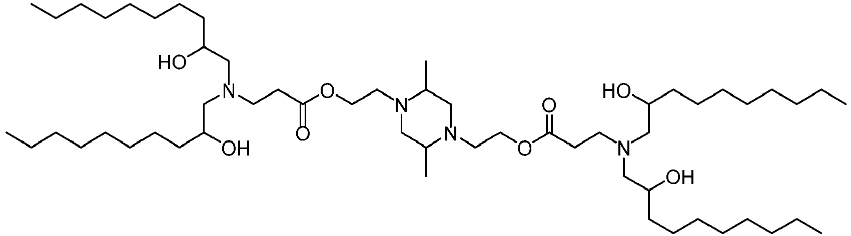 |
| **A2** | 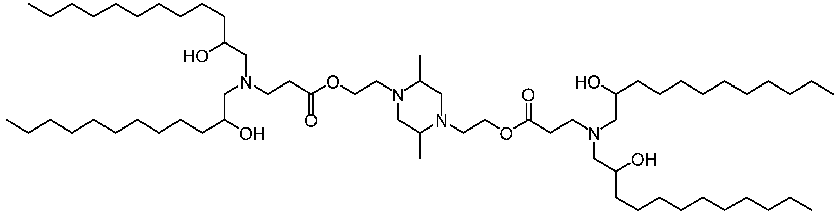 |
| **A3** | 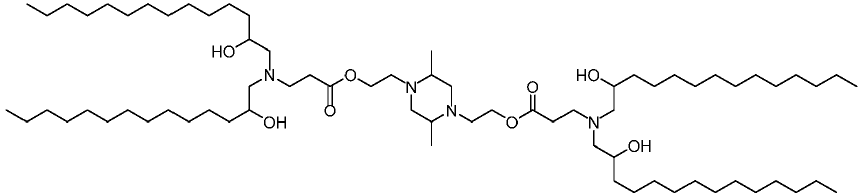 |
| **A4** | 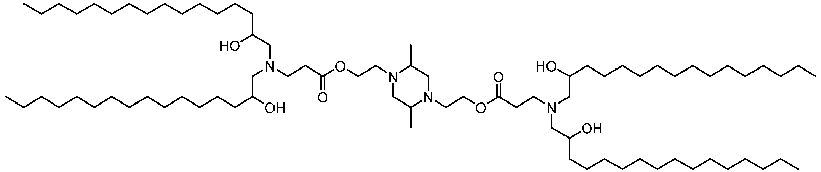 |
| **A5** | 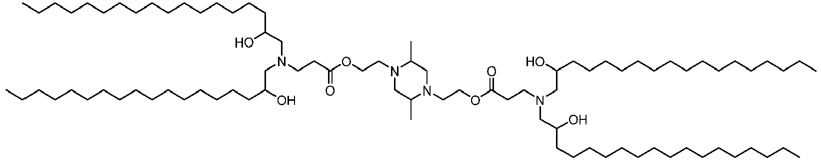 |
| **A6** | 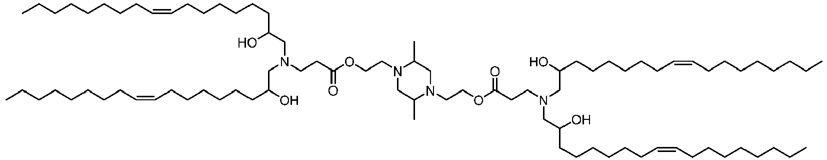 |
| **A7** | 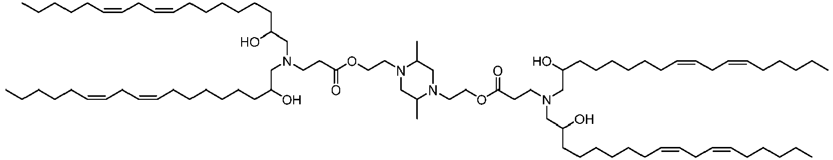 |
| **AS** | 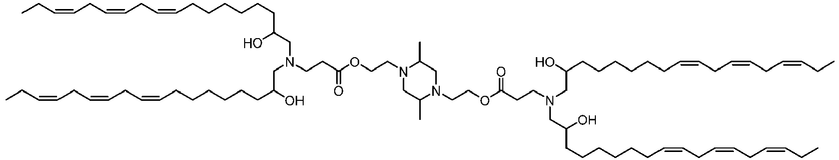 |
| **A9** | 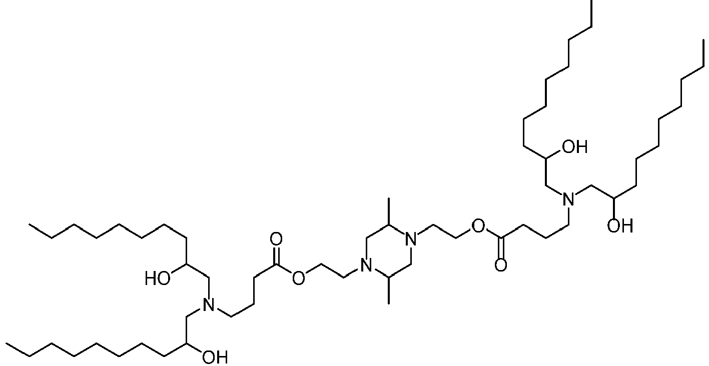 |
| **A10** | 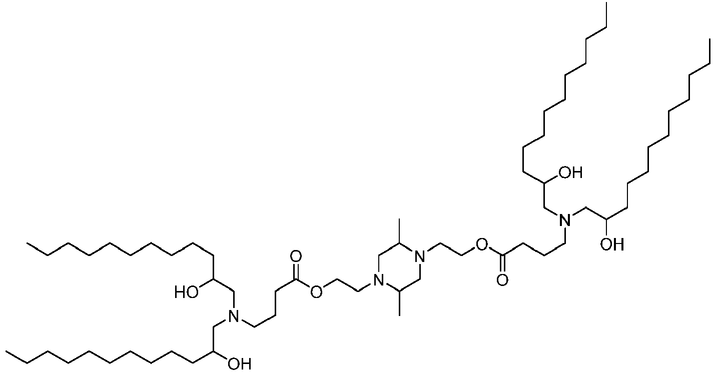 |
| **A11** | 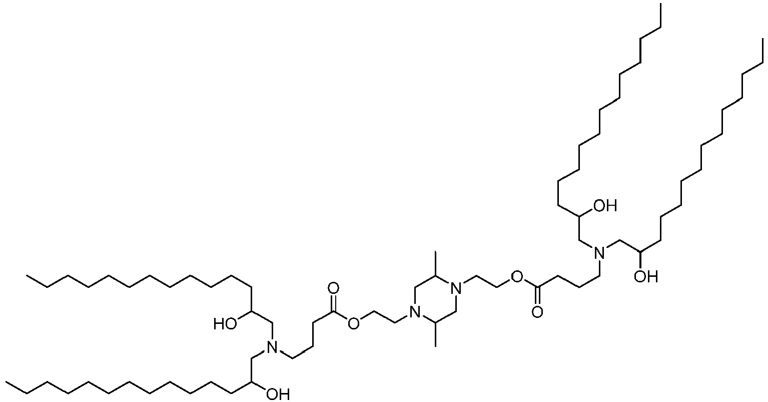 |
| **A12** | 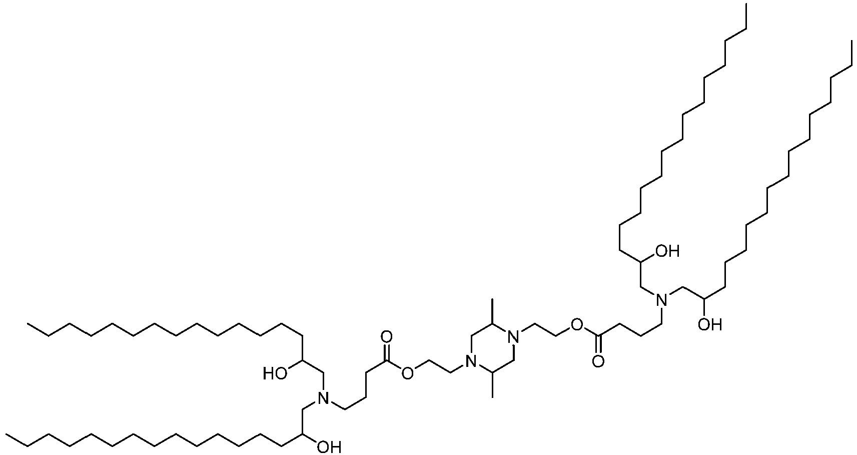 |
| **A13** | 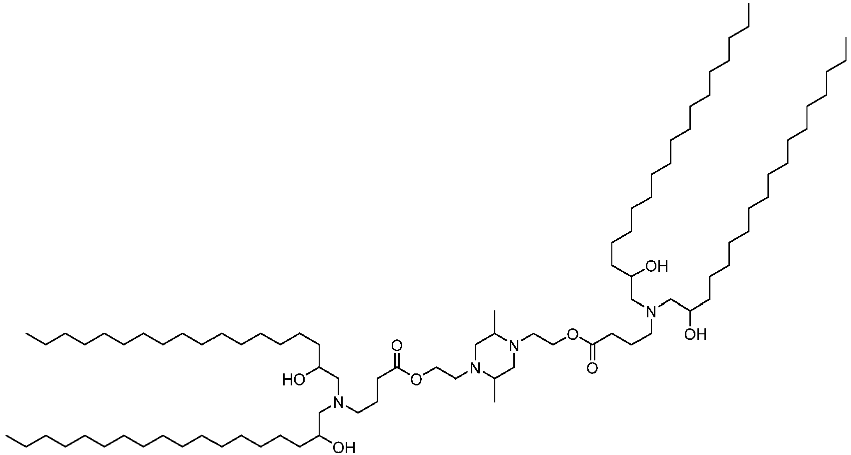 |
| **A14** | 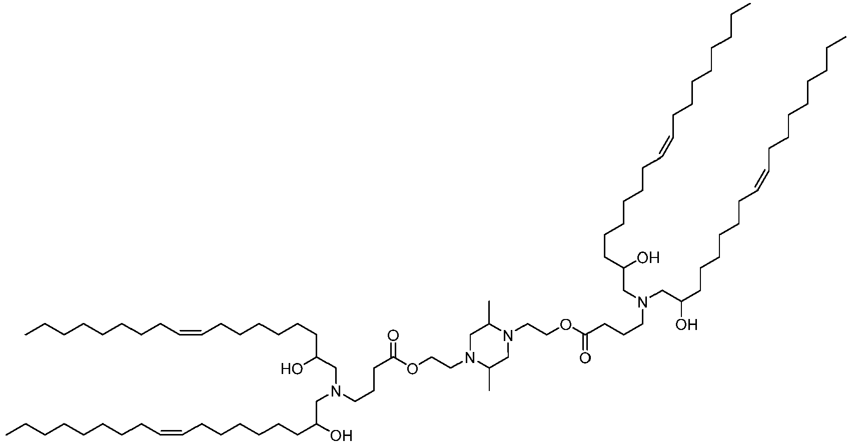 |
| **A15** | 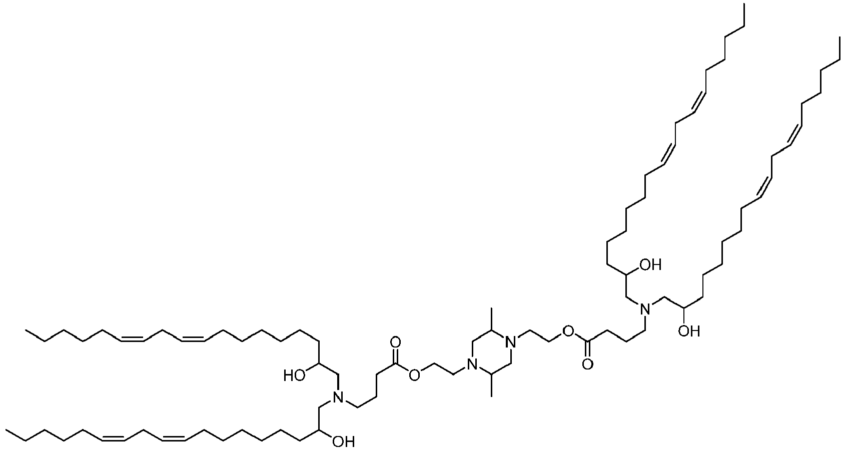 |
| **A16** | 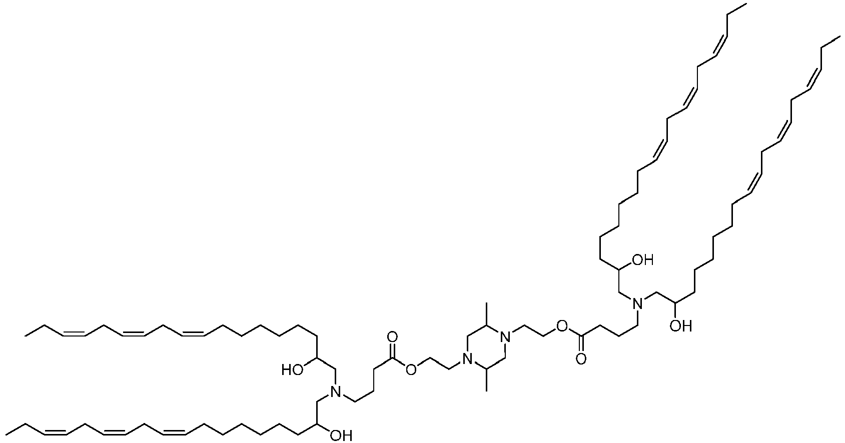 |
| **A17** | 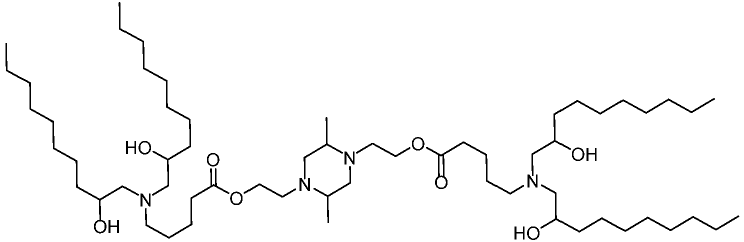 |
| **A18** | 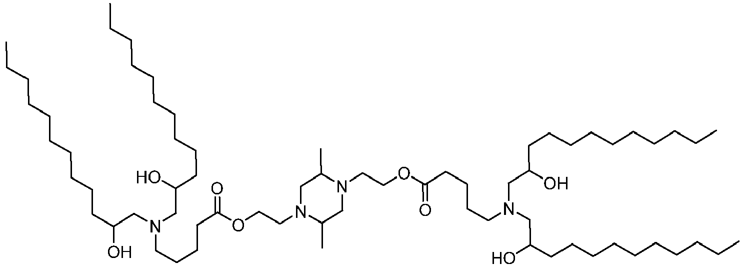 |
| **A19** | 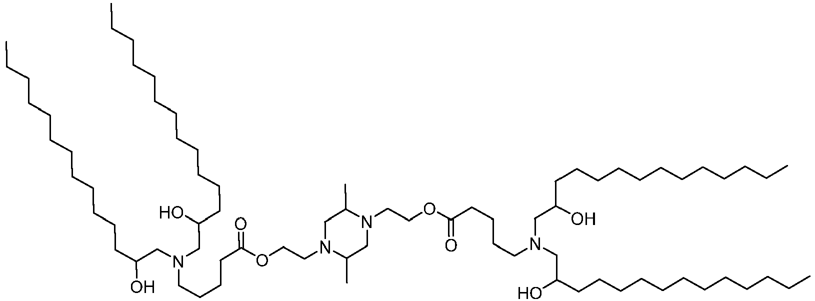 |
| **A20** | 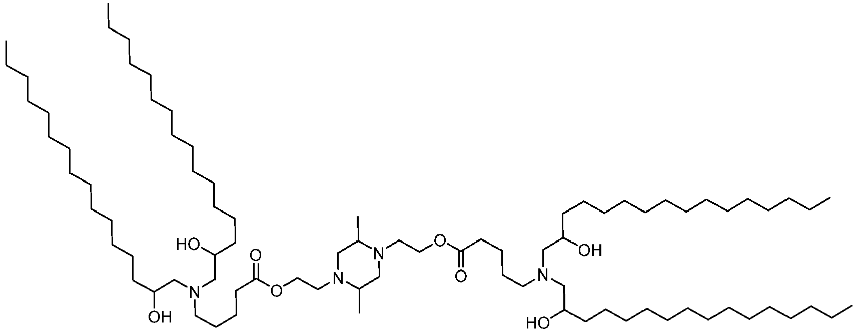 |
| **A21** | 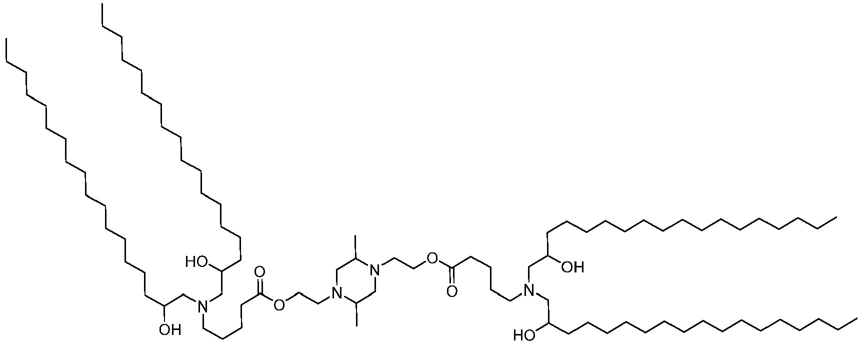 |
| **A22** | 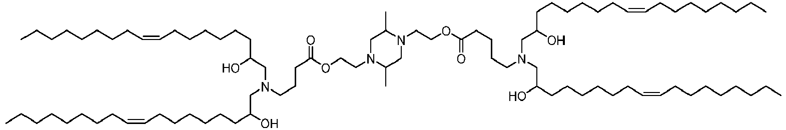 |
| **A23** | 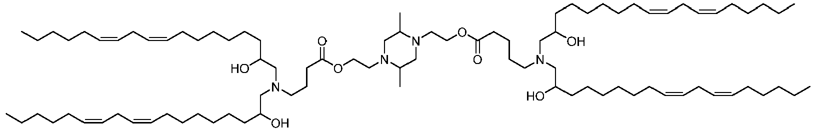 |
| **A24** | 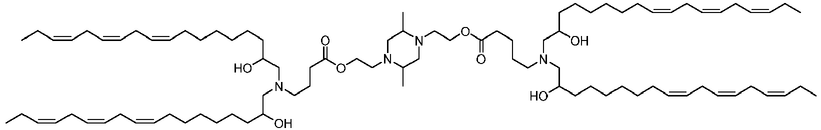 |
| **A25** | 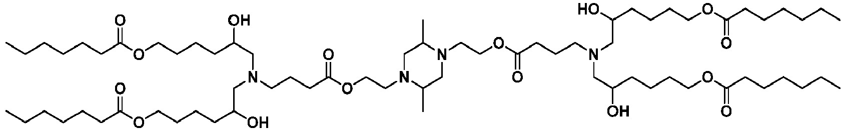 |
| **A26** | 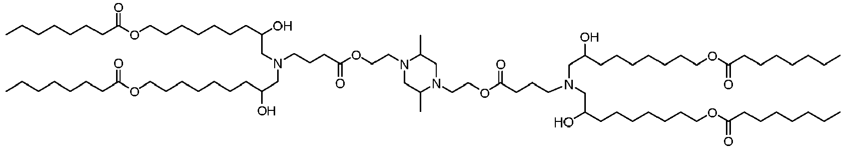 |

**Table B - TEP-based disulfide/disulfide cationic lipids**

| **Table B - TEP-based disulfide/disulfide cationic lipids** | |
|---|---|
| **B1** | 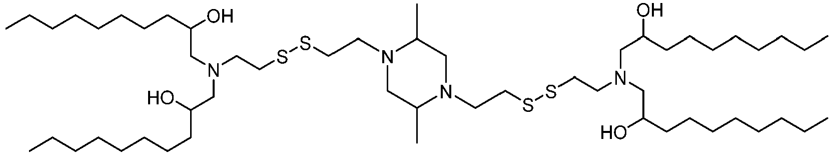 |
| **B2** | 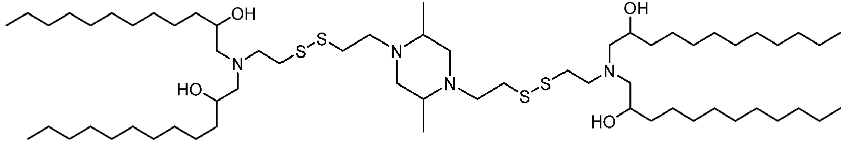 |
| **B3** | 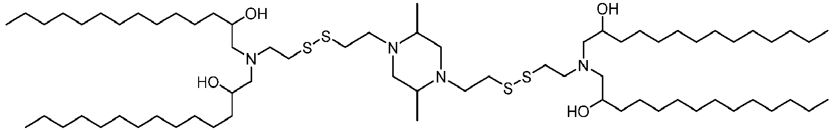 |
| **B4** | 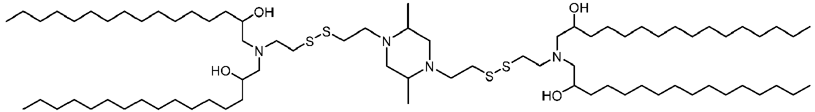 |
| **B5** | 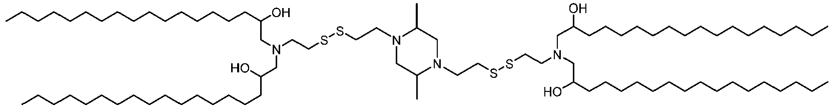 |
| **B6** | 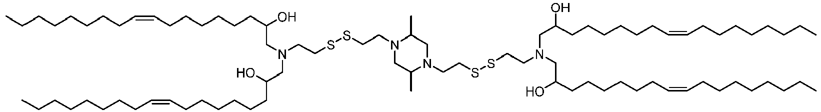 |
| **B7** | 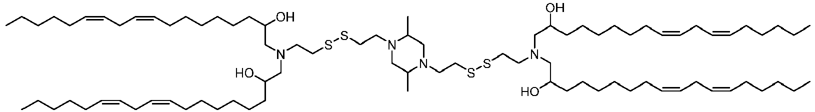 |
| **B8** | 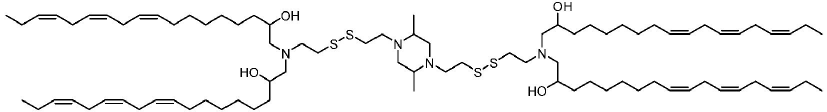 |
| **B9** | 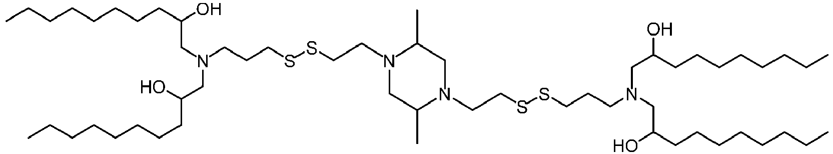 |
| **B10** | 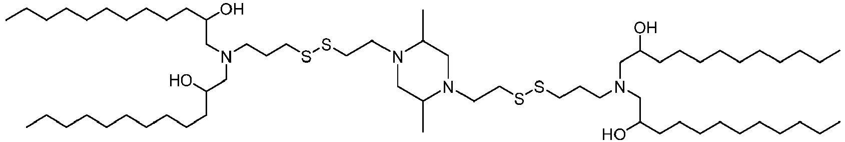 |
| **B11** | 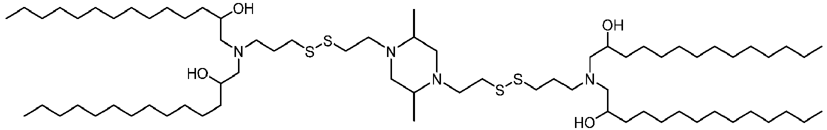 |
| **B12** | 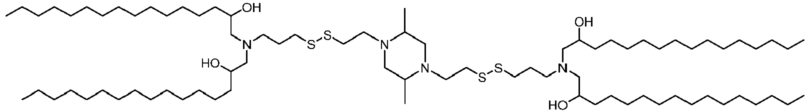 |
| **B13** | 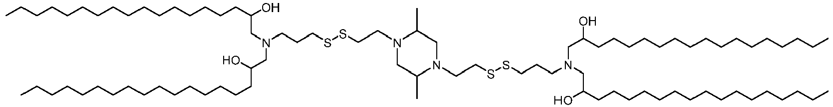 |
| **B14** | 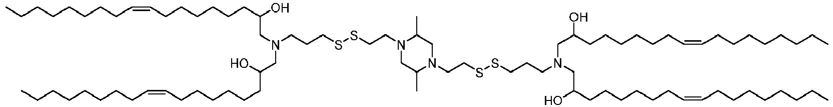 |
| **B15** | 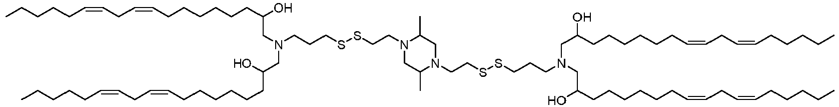 |
| **B16** | 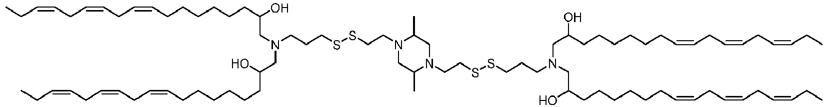 |
| **B17** | 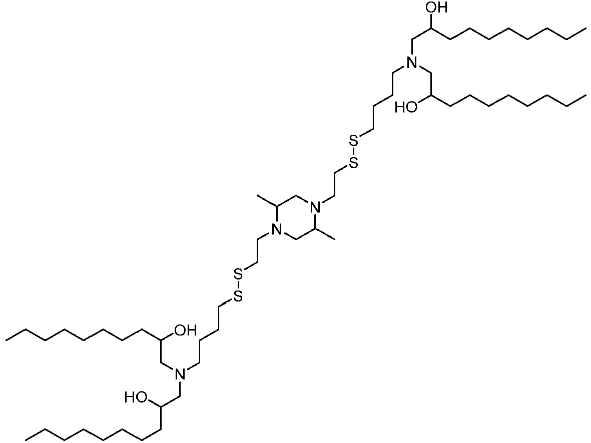 |
| **B18** | 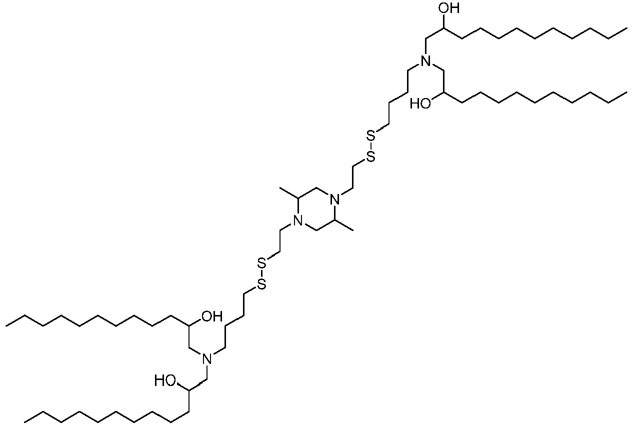 |
| **B19** | 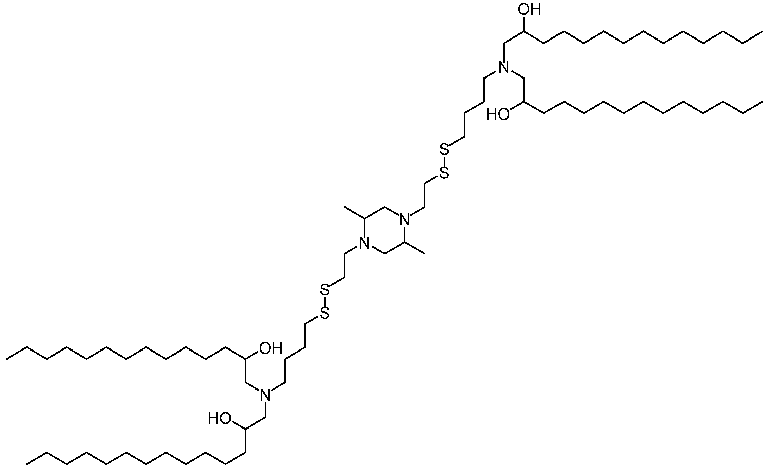 |
| **B20** | 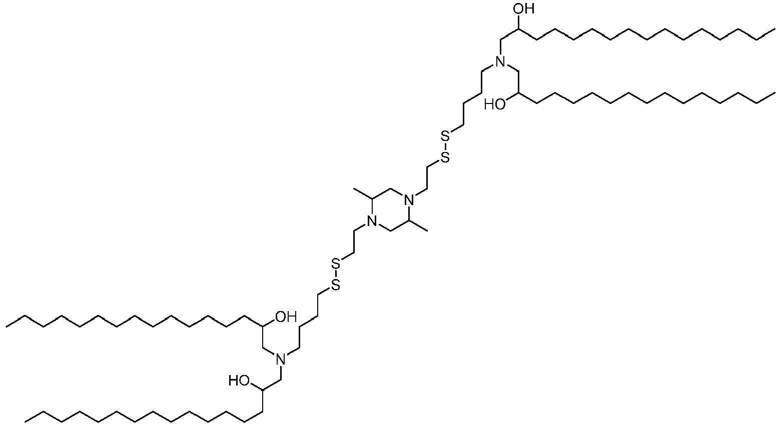 |
| **B21** | 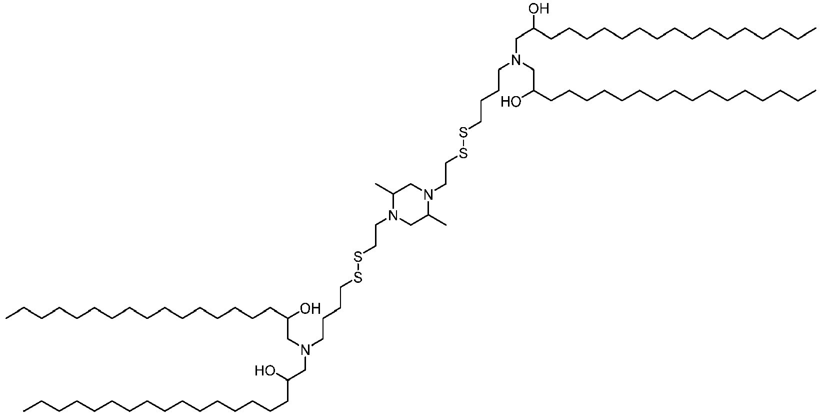 |
| **B22** | 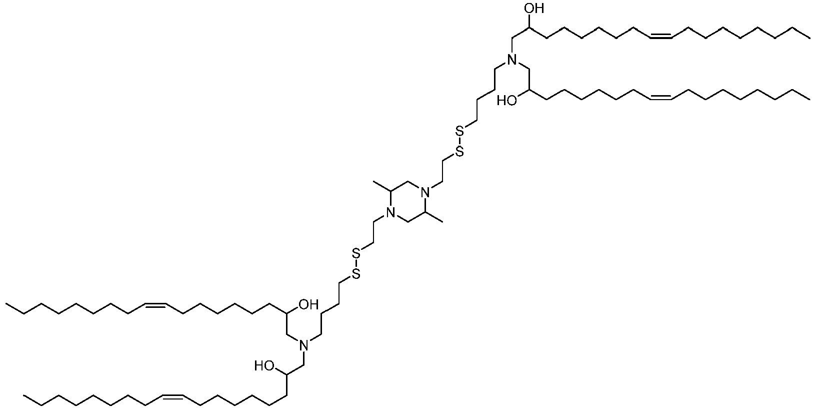 |
| **B23** | 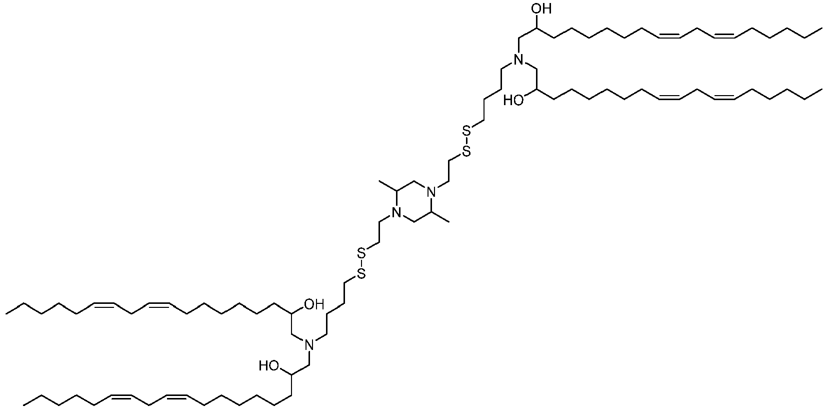 |
| **B24** | 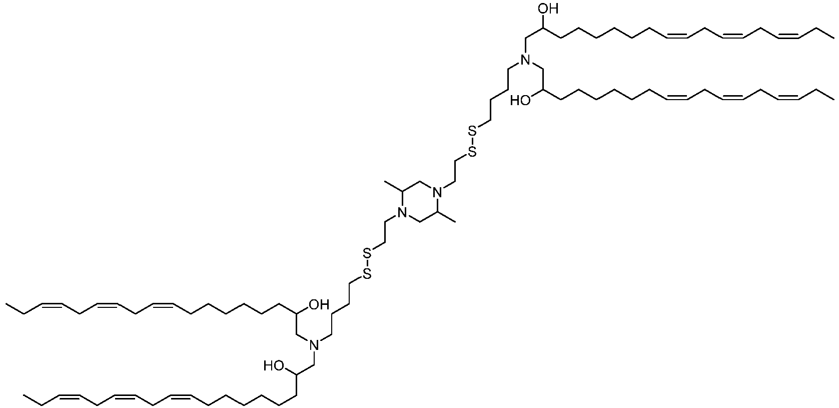 |

**Table C - TEP-based thioester/thioester cationic lipids**

| **Table C - TEP-based thioester/thioester cationic lipids** | |
|---|---|
| **C1** | 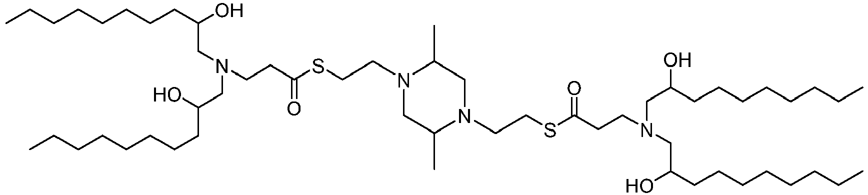 |
| **C2** | 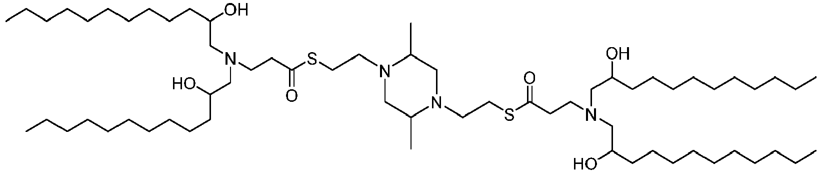 |
| **C3** | 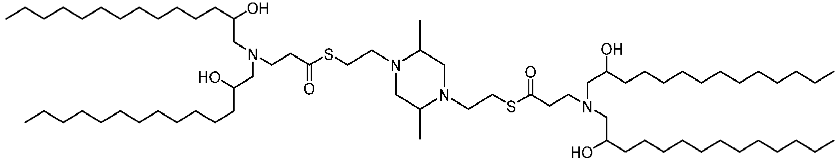 |
| **C4** | 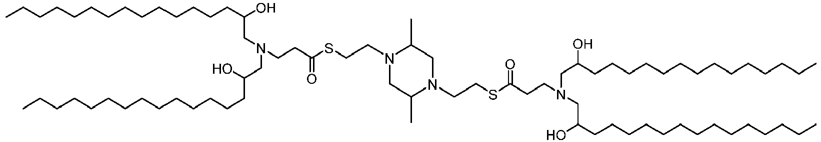 |
| **C5** | 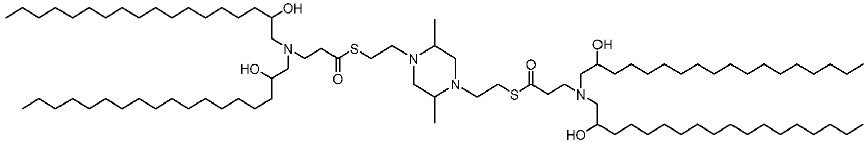 |
| **C6** | 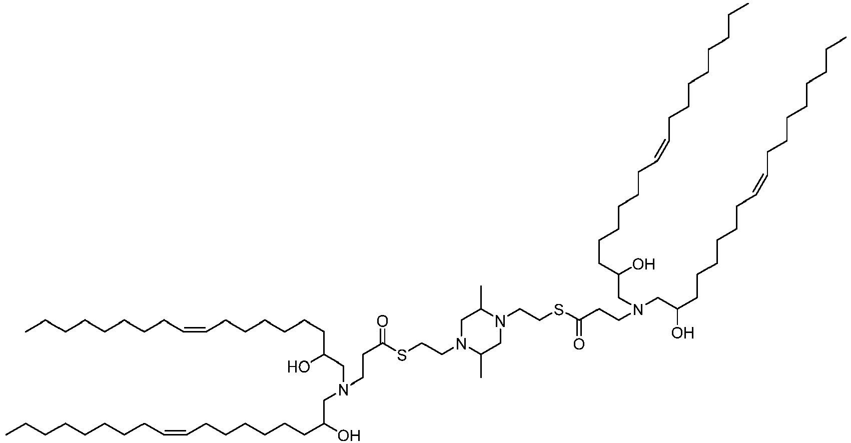 |
| **C7** | 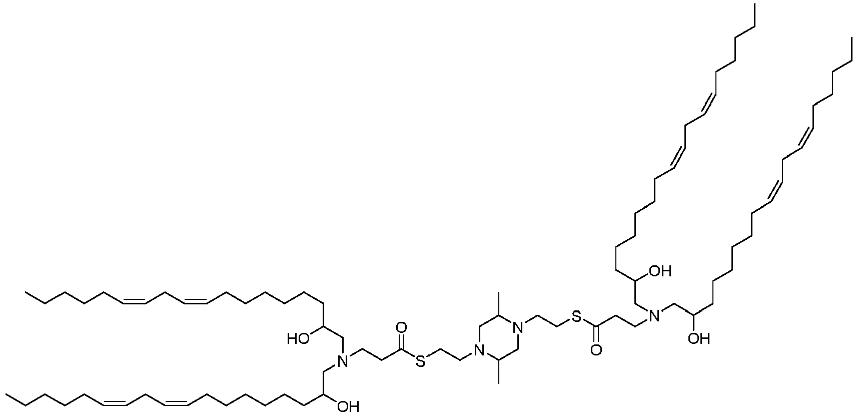 |
| **C8** | 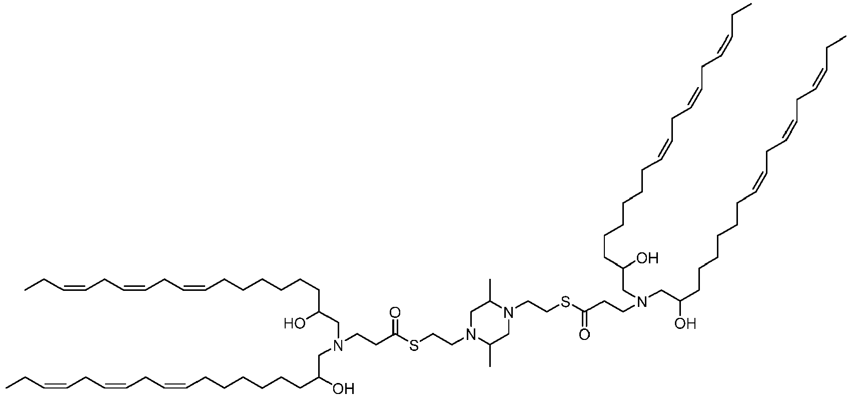 |
| **C9** | 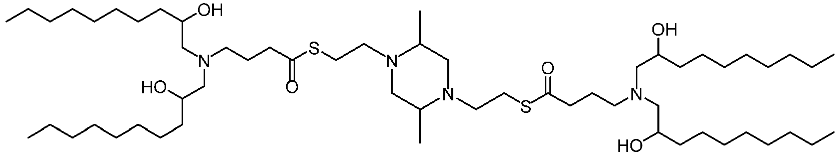 |
| **C10** | 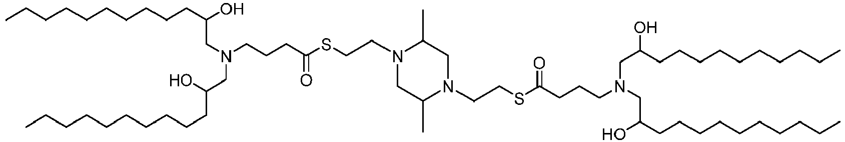 |
| **C11** | 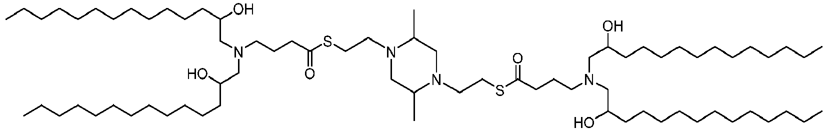 |
| **C12** | 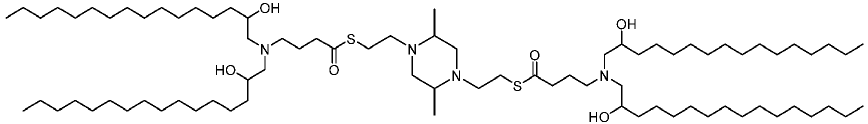 |
| **C13** | 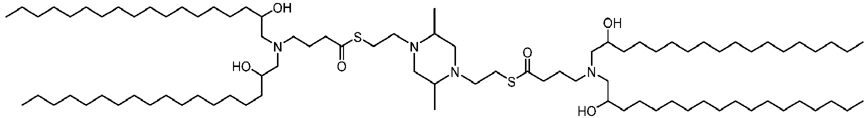 |
| **C14** | 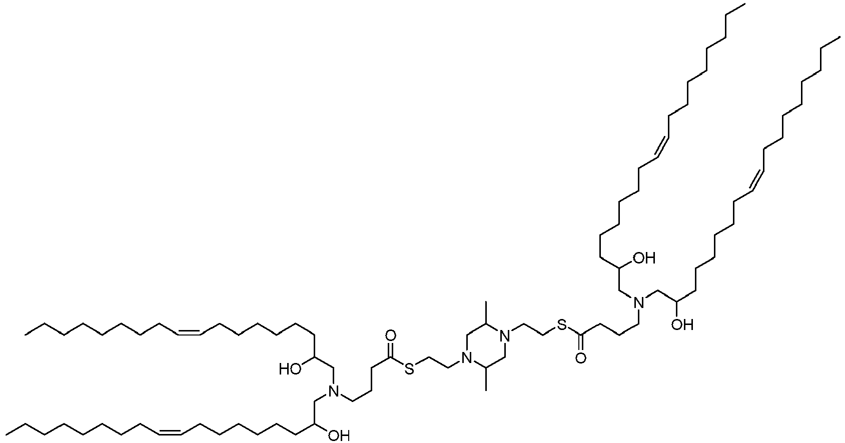 |
| **C15** | 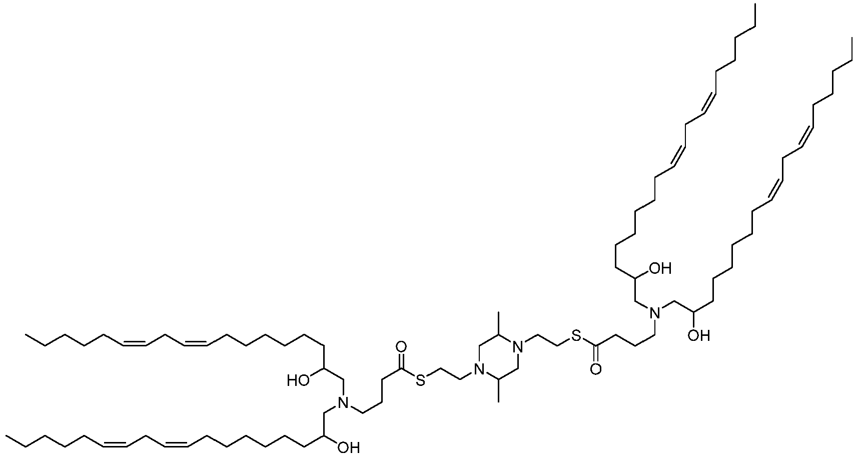 |
| **C16** | 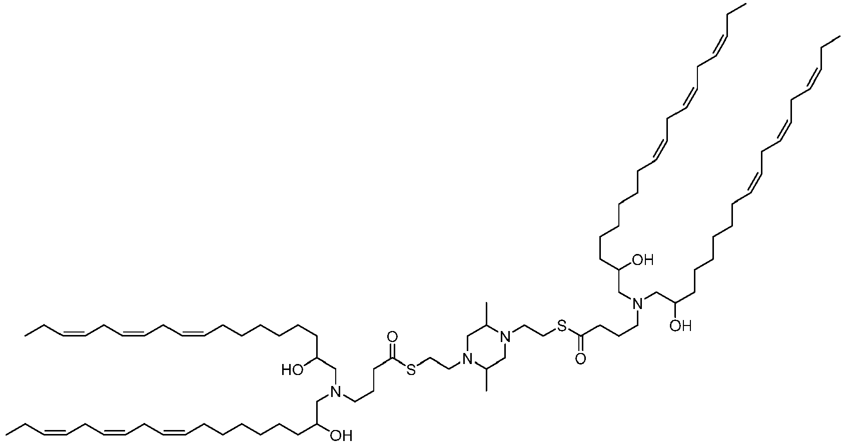 |
| **C17** | 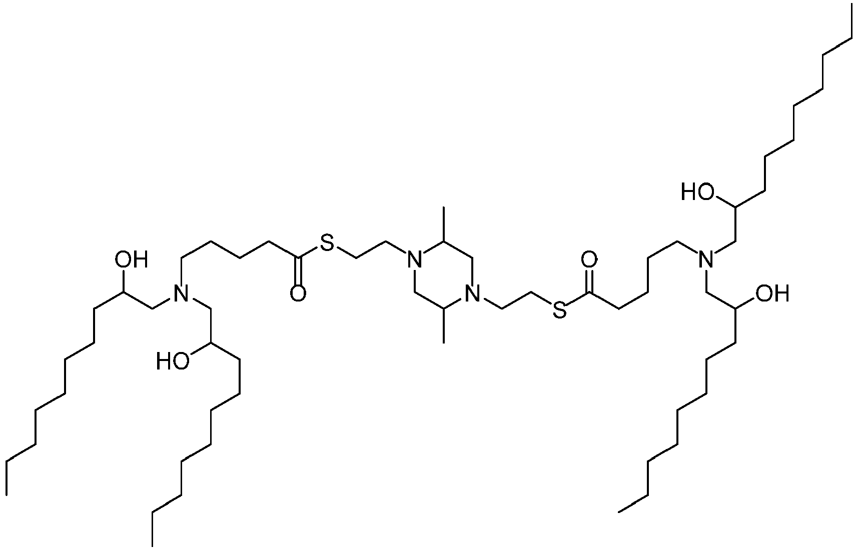 |
| **C18** | 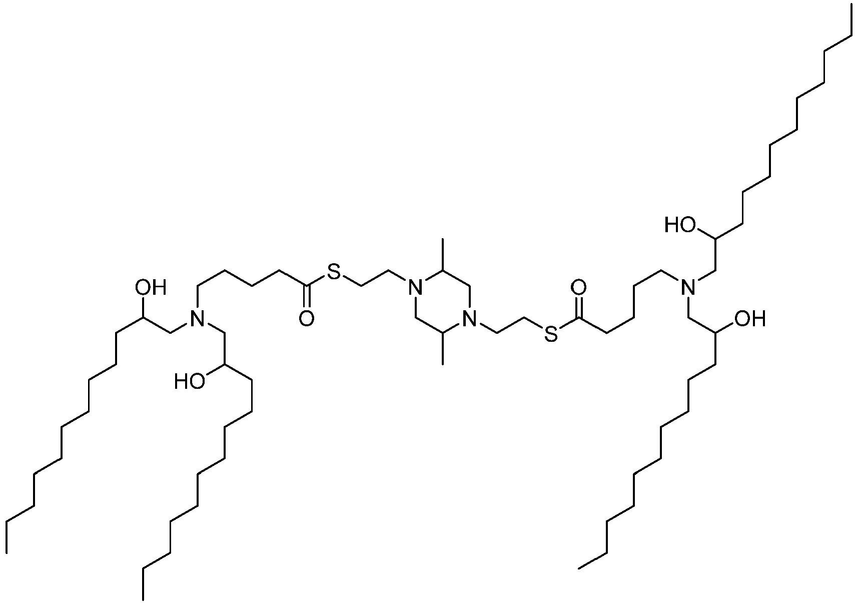 |
| **C19** | 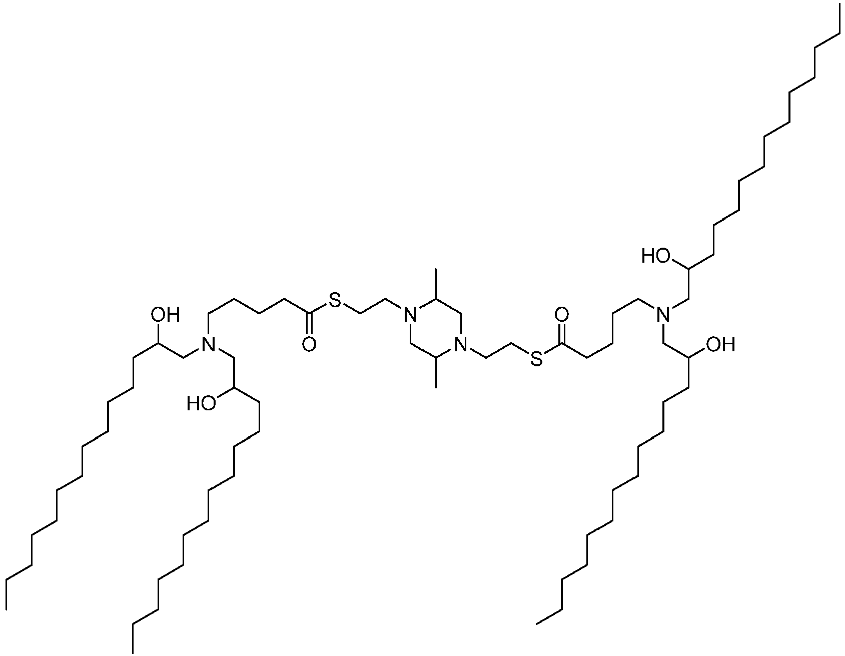 |
| **C20** | 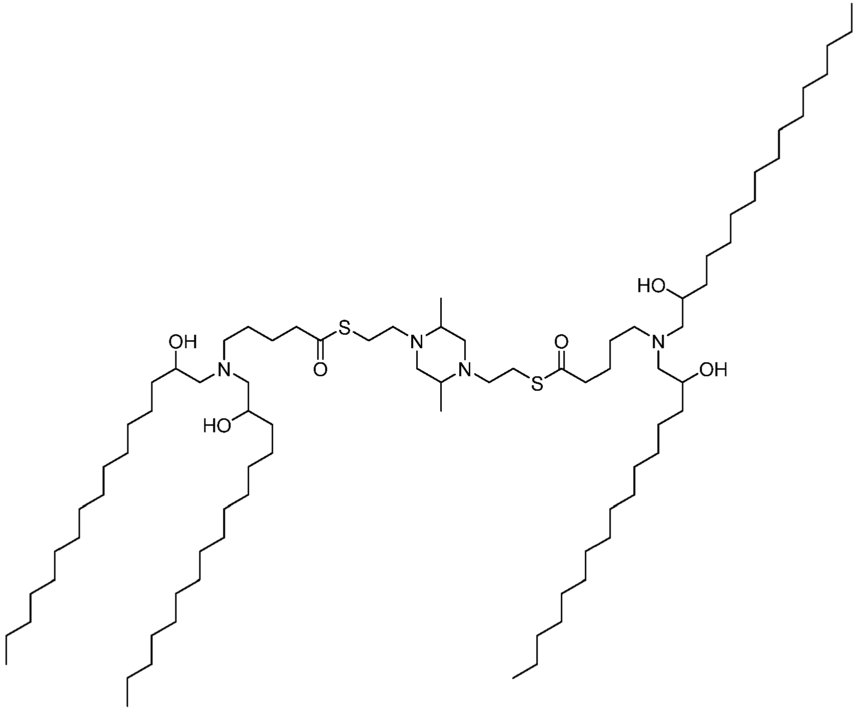 |
| **C21** | 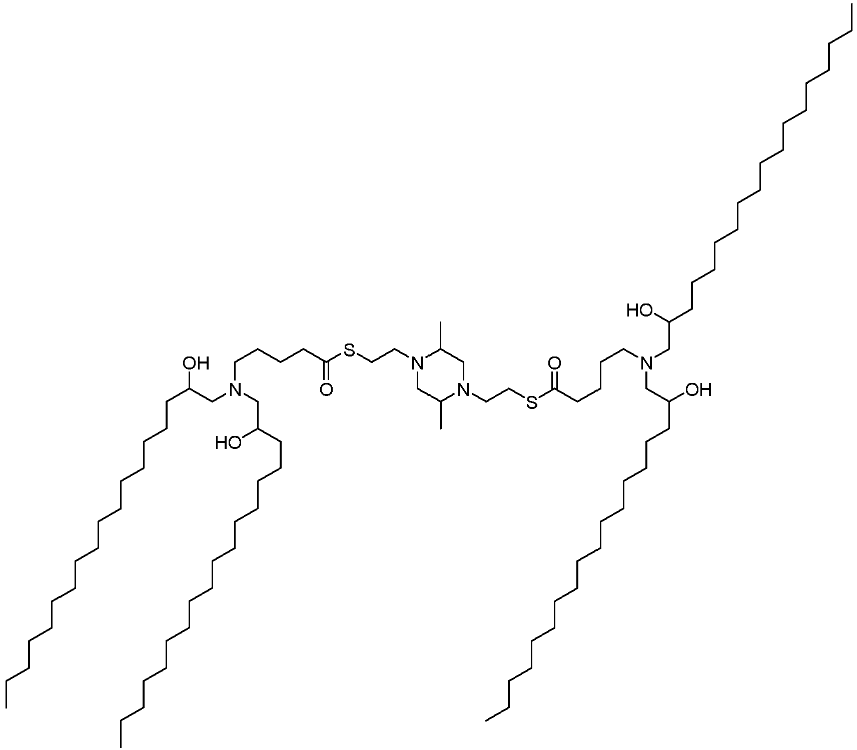 |
| **C22** | 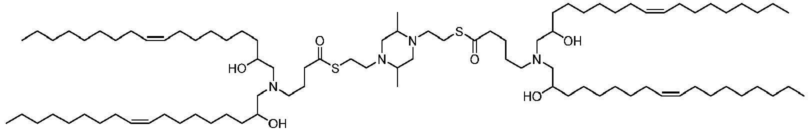 |
| **C23** | 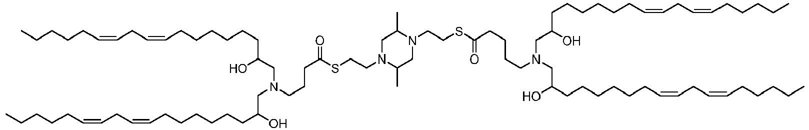 |
| **C24** | 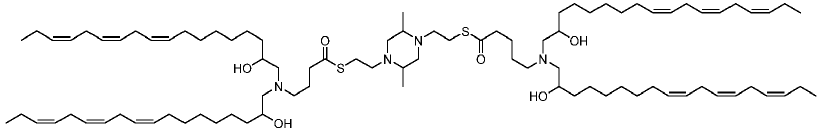 |

**Table D - HEP-based ester/ester cationic lipids**

| | **Table D - HEP-based ester/ester cationic lipids** |
|---|---|
| **D1** | 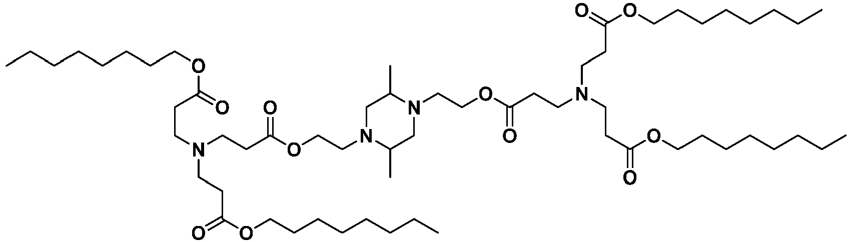 |
| **D2** | 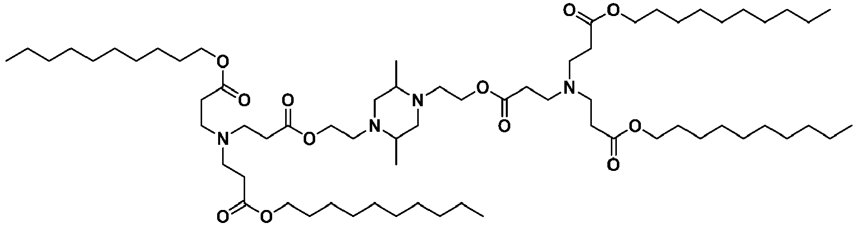 |
| **D3** | 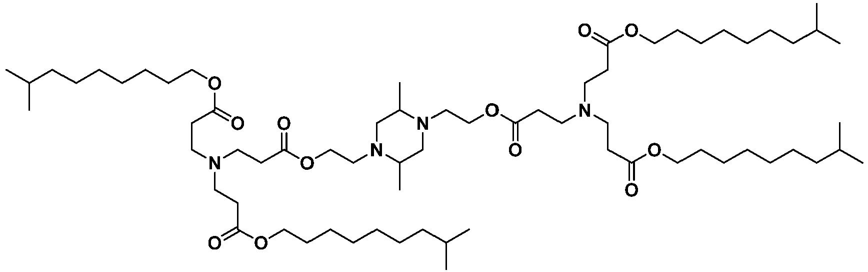 |
| **D4** | 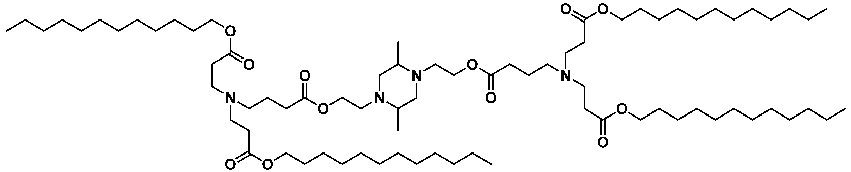 |
| **D5** | 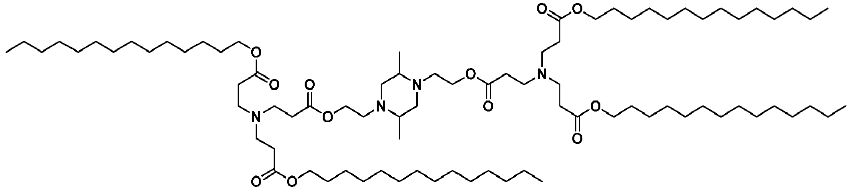 |
| **D6** | 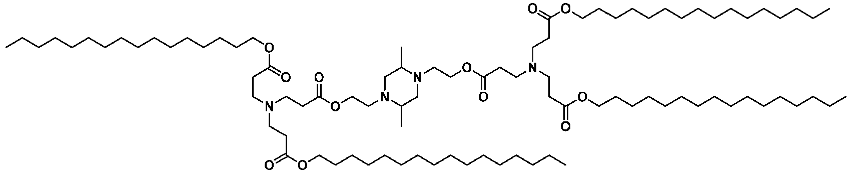 |
| **D7** | 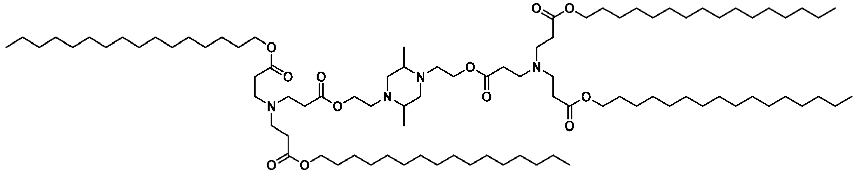 |
| **D8** | 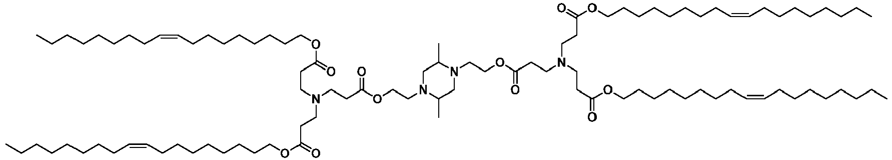 |
| **D9** | 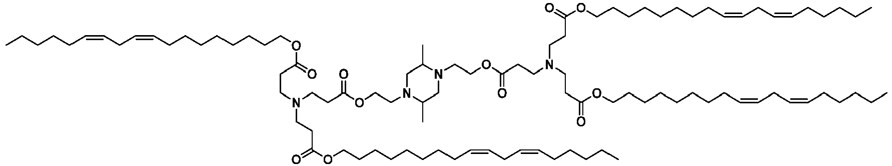 |
| **D10** | 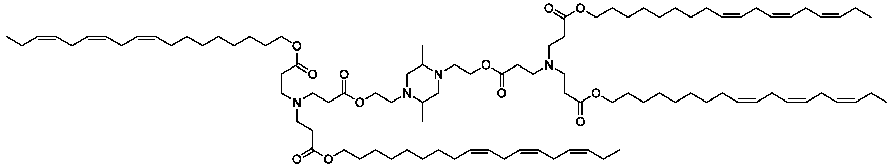 |
| **D11** | 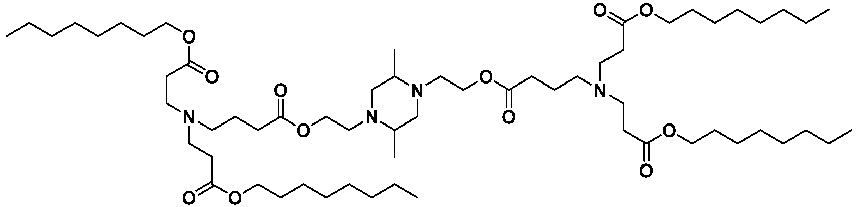 |
| **D12** | 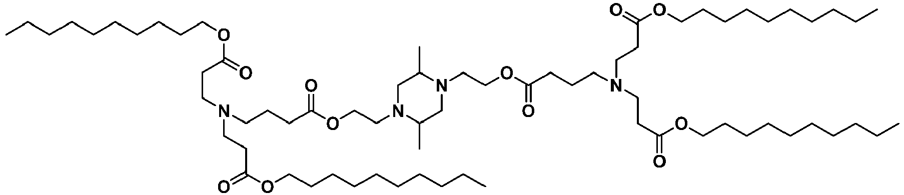 |
| **D13** | 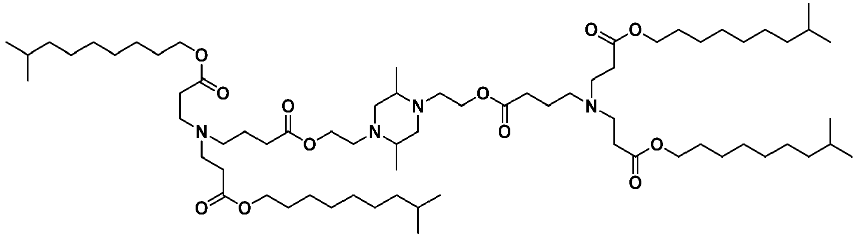 |
| **D14** | 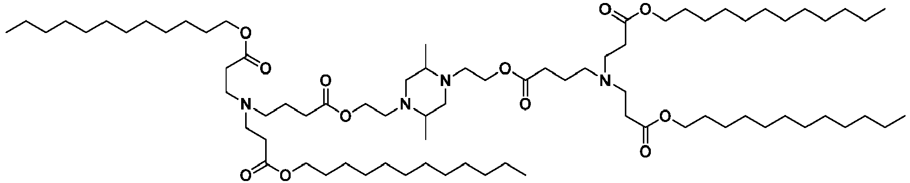 |
| **D15** | 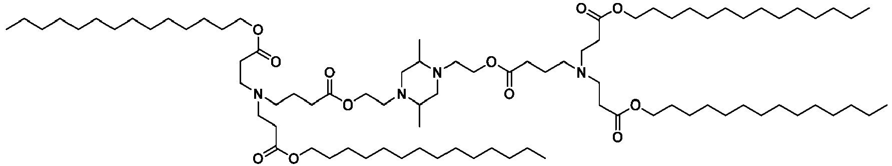 |
| **D16** | 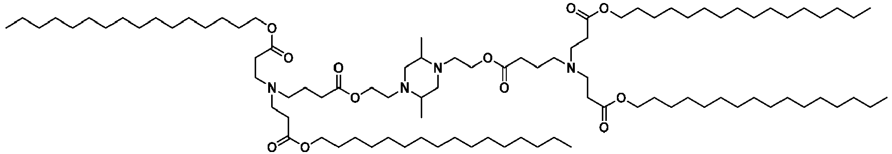 |
| **D17** | 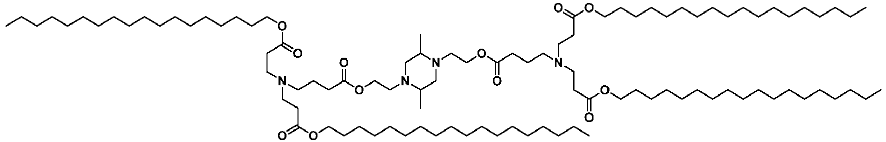 |
| **D18** | 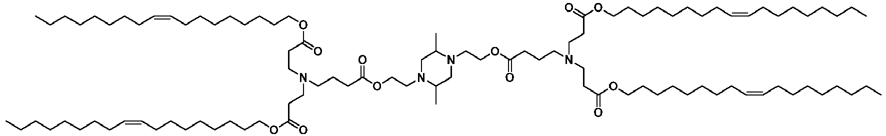 |
| **D19** | 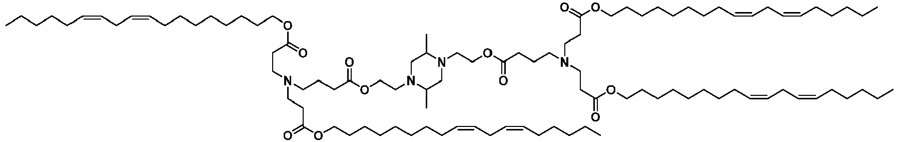 |
| **D20** | 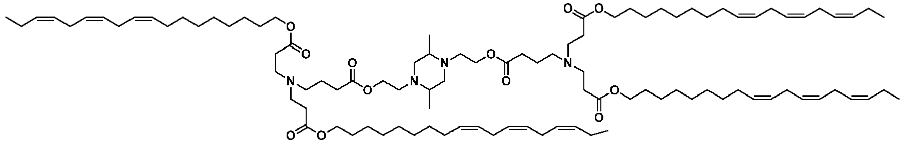 |
| **D21** | 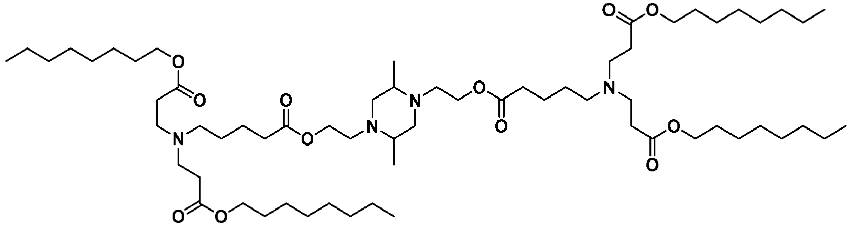 |
| **D22** | 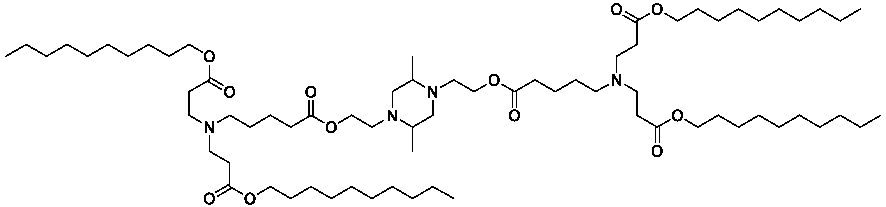 |
| **D23** | 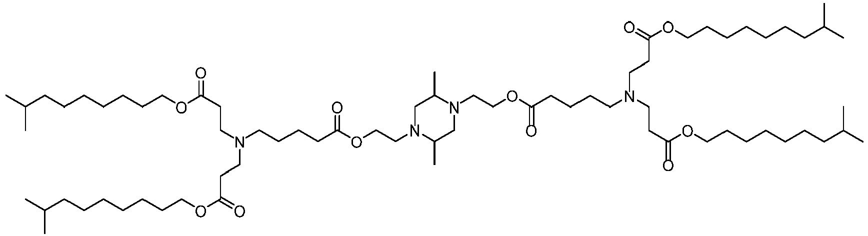 |
| **D24** | 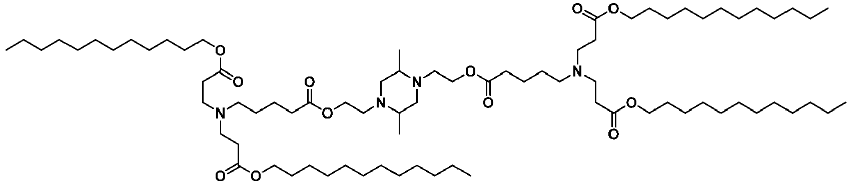 |
| **D25** | 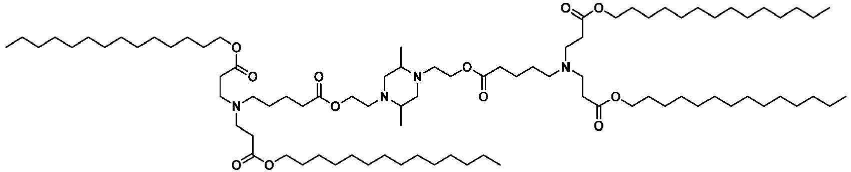 |
| **D26** | 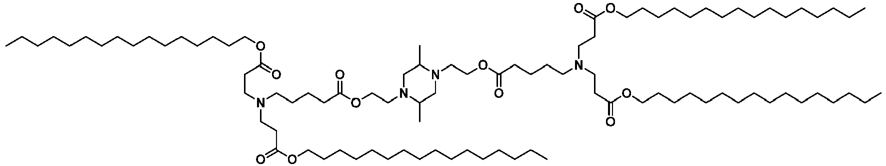 |
| **D27** | 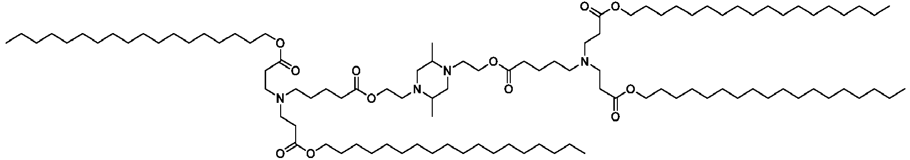 |
| **D28** | 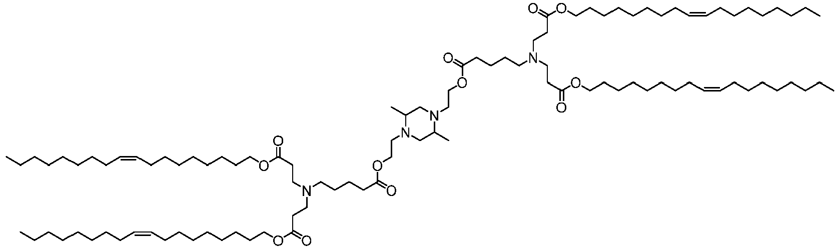 |
| **D29** | 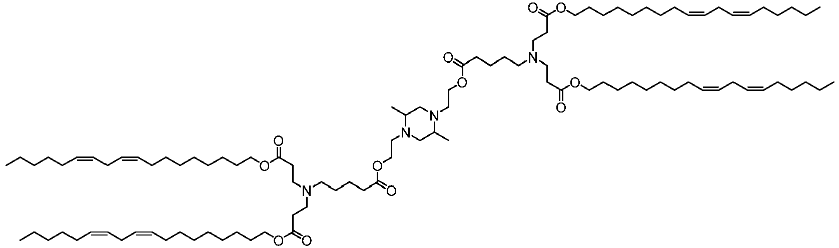 |
| **D30** | 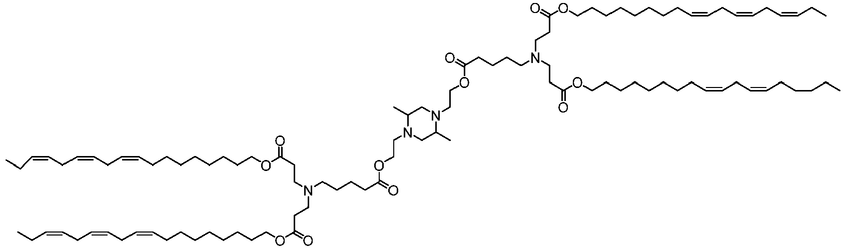 |
| **D31** | 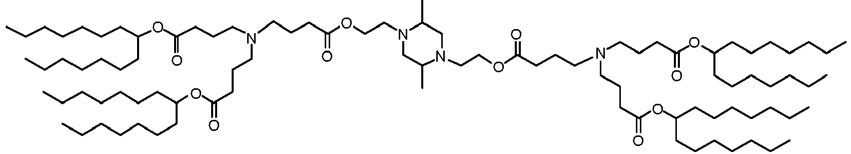 |
| **D32** | 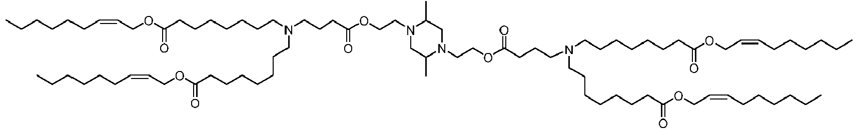 |

Any of the compounds identified in Tables A to D above may be provided in the form of a pharmaceutically acceptable salt and such salts are intended to be encompassed by the present invention.

The compounds of the invention as described herein can be prepared according to methods known in the art, including the exemplary syntheses of the Examples provided herein.

### Nucleic Acids

The compounds of the invention as described herein can be used to prepare compositions useful for the delivery of nucleic acids.

### Synthesis of Nucleic Acids

Nucleic acids according to the present invention may be synthesized according to any known methods. For example, mRNAs according to the present invention may be synthesized via *in vitro* transcription (IVT). Briefly, IVT is typically performed with a linear or circular DNA template containing a promoter, a pool of ribonucleotide triphosphates, a buffer system that may include DTT and magnesium ions, and an appropriate RNA polymerase (e.g., T3, T7, mutated T7 or SP6 RNA polymerase), DNAse I, pyrophosphatase, and/or RNAse inhibitor. The exact conditions will vary according to the specific application.

In some embodiments, for the preparation of mRNA according to the invention, a DNA template is transcribed *in vitro.* A suitable DNA template typically has a promoter, for example a T3, T7, mutated T7 or SP6 promoter, for *in vitro* transcription, followed by desired nucleotide sequence for desired mRNA and a termination signal.

Desired mRNA sequence(s) according to the invention may be determined and incorporated into a DNA template using standard methods. For example, starting from a desired amino acid sequence (e.g., an enzyme sequence), a virtual reverse translation is carried out based on the degenerated genetic code. Optimization algorithms may then be used for selection of suitable codons. Typically, the G/C content can be optimized to achieve the highest possible G/C content on one hand, taking into the best possible account the frequency of the tRNAs according to codon usage on the other hand. The optimized RNA sequence can be established and displayed, for example, with the aid of an appropriate display device and compared with the original (wild-type) sequence. A secondary structure can also be analyzed to calculate stabilizing and destabilizing properties or, respectively, regions of the RNA.

### Modified mRNA

In some embodiments, mRNA according to the present invention may be synthesized as unmodified or modified mRNA. Modified mRNA comprise nucleotide modifications in the RNA. A modified mRNA according to the invention can thus include nucleotide modification that are, for example, backbone modifications, sugar modifications or base modifications. In some embodiments, mRNAs may be synthesized from naturally occurring nucleotides and/or nucleotide analogues (modified nucleotides) including, but not limited to, purines (adenine (A), guanine (G)) or pyrimidines (thymine (T), cytosine (C), uracil (U)), and as modified nucleotides analogues or derivatives of purines and pyrimidines, such as *e.g.,* 1-methyl-adenine, 2-methyl-adenine, 2-methylthio-N-6-isopentenyl-adenine, N6-methyl-adenine, N6-isopentenyl-adenine, 2-thio-cytosine, 3-methyl-cytosine, 4-acetyl-cytosine, 5-methyl-cytosine, 2,6-diaminopurine, 1-methyl-guanine, 2-methyl-guanine, 2,2-dimethyl-guanine, 7-methyl-guanine, inosine, 1-methyl-inosine, pseudouracil (5-uracil), dihydro-uracil, 2-thio-uracil, 4-thio-uracil, 5-carboxymethylaminomethyl-2-thio-uracil, 5-(carboxyhydroxymethyl)-uracil, 5-fluoro-uracil, 5-bromo-uracil, 5-carboxymethylaminomethyl-uracil, 5-methyl-2-thio-uracil, 5-methyl-uracil, N-uracil-5-oxyacetic acid methyl ester, 5-methylaminomethyl-uracil, 5-methoxyaminomethyl-2-thio-uracil, 5'-methoxycarbonylmethyl-uracil, 5-methoxy-uracil, uracil-5-oxyacetic acid methyl ester, uracil-5-oxyacetic acid (v), 1-methyl-pseudouracil, queuosine, beta-D-mannosyl-queuosine, wybutoxosine, and phosphoramidates, phosphorothioates, peptide nucleotides, methylphosphonates, 7-deazaguanosine, 5-methylcytosine and inosine. The preparation of such analogues is known to a person skilled in the art e.g., from the U.S. Pat. No. 4,373,071, U.S. Pat. No. 4,401,796, U.S. Pat. No. 4,415,732, U.S. Pat. No. 4,458,066, U.S. Pat. No. 4,500,707, U.S. Pat. No. 4,668,777, U.S. Pat. No. 4,973,679, U.S. Pat. No. 5,047,524, U.S. Pat. No. 5,132,418, U.S. Pat. No. 5,153,319, U.S. Pat. Nos. 5,262,530 and 5,700,642.

### Pharmaceutical Formulations of Cationic Lipids and Nucleic Acids

In certain embodiments, the compounds of the invention as described herein, as well as pharmaceutical and liposomal compositions comprising such lipids, can be used in formulations to facilitate the delivery of encapsulated materials (*e.g*., one or more polynucleotides such as mRNA) to, and subsequent transfection of one or more target cells. For example, in certain embodiments cationic lipids described herein (and compositions such as liposomal compositions comprising such lipids) are characterized as resulting in one or more of receptor-mediated endocytosis, clathrinmediated and caveolae-mediated endocytosis, phagocytosis and macropinocytosis, fusogenicity, endosomal or lysosomal disruption and/or releasable properties that afford such compounds advantages relative other similarly classified lipids.

According to the present invention, a nucleic acid, *e.g.,* mRNA encoding a protein *(e.g.,* a full length, fragment or portion of a protein) as described herein may be delivered via a delivery vehicle comprising a compound of the invention as described herein.

As used herein, the terms "delivery vehicle," "transfer vehicle," "nanoparticle," or grammatical equivalents thereof, are used interchangeably.

For example, the present invention provides a composition (*e.g*., a pharmaceutical composition) comprising a compound described herein and one or more polynucleotides. A composition (e.g., a pharmaceutical composition) may further comprise one or more cationic lipids, one or more non-cationic lipids, one or more cholesterol-based lipids and/or one or more PEG-modified lipids.

In certain embodiments a composition exhibits an enhanced (*e.g*., increased) ability to transfect one or more target cells. Accordingly, also provided herein are methods of transfecting one or more target cells. Such methods generally comprise the step of contacting the one or more target cells with the cationic lipids and/or pharmaceutical compositions disclosed herein (*e.g*., a liposomal formulation comprising a compound described herein encapsulating one or more polynucleotides) such that the one or more target cells are transfected with the materials encapsulated therein (*e.g*., one or more polynucleotides). As used herein, the terms "transfect" or "transfection" refer to the intracellular introduction of one or more encapsulated materials (*e.g*., nucleic acids and/or polynucleotides) into a cell (*e.g.*, into a target cell). The introduced polynucleotide may be stably or transiently maintained in the target cell. The term "transfection efficiency" refers to the relative amount of such encapsulated material (*e.g*., polynucleotides) uptaken by, introduced into, and/or expressed by the target cell which is subject to transfection. In practice, transfection efficiency may be estimated by the amount of a reporter polynucleotide product produced by the target cells following transfection. In certain embodiments, the compounds and pharmaceutical compositions described herein demonstrate high transfection efficiencies thereby improving the likelihood that appropriate dosages of the encapsulated materials (*e.g.,* one or more polynucleotides) will be delivered to the site of pathology and subsequently expressed, while at the same time minimizing potential systemic adverse effects or toxicity associated with the compound or their encapsulated contents.

Following transfection of one or more target cells by, for example, the polynucleotides encapsulated in the one or more lipid nanoparticles comprising the pharmaceutical or liposomal compositions disclosed herein, the production of the product (*e.g.*, a polypeptide or protein) encoded by such polynucleotide may be stimulated and the capability of such target cells to express the polynucleotide and produce, for example, a polypeptide or protein of interest is enhanced. For example, transfection of a target cell by one or more compounds or pharmaceutical compositions encapsulating mRNA will enhance (*i.e.,* increase) the production of the protein or enzyme encoded by such mRNA.

Further, delivery vehicles described herein (*e.g.*, liposomal delivery vehicles) may be prepared to preferentially distribute to other target tissues, cells or organs, such as the heart, lungs, kidneys, spleen. In embodiments, the lipid nanoparticles of the present invention may be prepared to achieve enhanced delivery to the target cells and tissues. For example, polynucleotides (*e.g.,* mRNA) encapsulated in one or more of the compounds or pharmaceutical and liposomal compositions described herein can be delivered to and/or transfect targeted cells or tissues. In some embodiments, the encapsulated polynucleotides (*e.g.*, mRNA) are capable of being expressed and functional polypeptide products produced (and in some instances excreted) by the target cell, thereby conferring a beneficial property to, for example the target cells or tissues. Such encapsulated polynucleotides (*e.g*., mRNA) may encode, for example, a hormone, enzyme, receptor, polypeptide, peptide or other protein of interest.

### Liposomal Delivery Vehicles

In some embodiments, a composition is a suitable delivery vehicle. In embodiments, a composition is a liposomal delivery vehicle, *e.g.*, a lipid nanoparticle.

The terms "liposomal delivery vehicle" and "liposomal composition" are used interchangeably.

Enriching liposomal compositions with one or more of the cationic lipids disclosed herein may be used as a means of improving (*e.g*., reducing) the toxicity or otherwise conferring one or more desired properties to such enriched liposomal composition (*e.g.*, improved delivery of the encapsulated polynucleotides to one or more target cells and/or reduced in vivo toxicity of a liposomal composition). Accordingly, also contemplated are pharmaceutical compositions, and in particular liposomal compositions, that comprise one or more of the cationic lipids disclosed herein.

Thus, in certain embodiments, the compounds of the invention as described herein may be used as a component of a liposomal composition to facilitate or enhance the delivery and release of encapsulated materials (*e.g.,* one or more therapeutic agents) to one or more target cells (*e.g.,* by permeating or fusing with the lipid membranes of such target cells).

As used herein, liposomal delivery vehicles, *e.g.*, lipid nanoparticles, are usually characterized as microscopic vesicles having an interior aqua space sequestered from an outer medium by a membrane of one or more bilayers. Bilayer membranes of liposomes are typically formed by amphiphilic molecules, such as lipids of synthetic or natural origin that comprise spatially separated hydrophilic and hydrophobic domains (Lasic, Trends Biotechnol., 16: 307-321, 1998). Bilayer membranes of the liposomes can also be formed by amphophilic polymers and surfactants (e.g., polymerosomes, niosomes, *etc*.). In the context of the present invention, a liposomal delivery vehicle typically serves to transport a desired mRNA to a target cell or tissue.

In certain embodiments, such compositions (*e.g.*, liposomal compositions) are loaded with or otherwise encapsulate materials, such as for example, one or more biologically-active polynucleotides (*e.g.*, mRNA).

In embodiments, a composition (*e.g.*, a pharmaceutical composition) comprises an mRNA encoding a protein, encapsulated within a liposome. In embodiments, a liposome comprises one or more cationic lipids, one or more non-cationic lipids, one or more cholesterol-based lipids and one or more PEG-modified lipids, and wherein at least one cationic lipid is a compound of the invention as described herein. In embodiments, a composition comprises an mRNA encoding for a protein (*e.g.*, any protein described herein). In embodiments, a composition comprises an mRNA encoding for cystic fibrosis transmembrane conductance regulator (CFTR) protein. In embodiments, a composition comprises an mRNA encoding for ornithine transcarbamylase (OTC) protein.

In embodiments, a composition (*e.g.*, a pharmaceutical composition) comprises a nucleic acid encapsulated within a liposome, wherein the liposome comprises a compound described herein.

In embodiments, a nucleic acid is an mRNA encoding a peptide or protein. In embodiments, an mRNA encodes a peptide or protein for use in the delivery to or treatment of the lung of a subject or a lung cell (*e.g.,* an mRNA encodes cystic fibrosis transmembrane conductance regulator (CFTR) protein). In embodiments, an mRNA encodes a peptide or protein for use in the delivery to or treatment of the liver of a subject or a liver cell (*e.g.,* an mRNA encodes ornithine transcarbamylase (OTC) protein). Still other exemplary mRNAs are described herein.

In embodiments, a liposomal delivery vehicle (*e.g*., a lipid nanoparticle) can have a net positive charge.

In embodiments, a liposomal delivery vehicle (*e.g.*, a lipid nanoparticle) can have a net negative charge.

In embodiments, a liposomal delivery vehicle (*e.g*., a lipid nanoparticle) can have a net neutral charge.

In embodiments, a lipid nanoparticle that encapsulates a nucleic acid (*e.g.*, mRNA encoding a peptide or protein) comprises one or more compounds of the invention as described herein.

For example, the amount of a compound of the invention as described herein in a composition can be described as a percentage ("wt%") of the combined dry weight of all lipids of a composition (*e.g.*, the combined dry weight of all lipids present in a liposomal composition).

In embodiments of the pharmaceutical compositions described herein, a compound of the invention as described herein is present in an amount that is about 0.5 wt% to about 30 wt% (*e.g.,* about 0.5 wt% to about 20 wt%) of the combined dry weight of all lipids present in a composition (*e.g.,* a liposomal composition).

In embodiments, a compound of the invention as described herein is present in an amount that is about 1 wt% to about 30 wt%, about 1 wt% to about 20 wt%, about 1 wt% to about 15 wt%, about 1 wt% to about 10 wt%, or about 5 wt% to about 25 wt% of the combined dry weight of all lipids present in a composition (*e.g.*, a liposomal composition). In embodiments, a compound of the invention as described herein is present in an amount that is about 0.5 wt% to about 5 wt%, about 1 wt% to about 10 wt%, about 5 wt% to about 20 wt%, or about 10 wt% to about 20 wt% of the combined dry weight of all lipids present in a composition such as a liposomal delivery vehicle.

In embodiments, the amount of a compound of the invention as described herein is present in an amount that is at least about 5 wt%, about 10 wt%, about 15 wt%, about 20 wt%, about 25 wt%, about 30 wt%, about 35 wt%, about 40 wt%, about 45 wt%, about 50 wt%, about 55 wt%, about 60 wt%, about 65 wt%, about 70 wt%, about 75 wt%, about 80 wt%, about 85 wt%, about 90 wt%, about 95 wt%, about 96 wt%, about 97 wt%, about 98 wt%, or about 99 wt% of the combined dry weight of total lipids in a composition (*e.g.*, a liposomal composition).

In embodiments, the amount of a compound of the invention as described herein is present in an amount that is no more than about 5 wt%, about 10 wt%, about 15 wt%, about 20 wt%, about 25 wt%, about 30 wt%, about 35 wt%, about 40 wt%, about 45 wt%, about 50 wt%, about 55 wt%, about 60 wt%, about 65 wt%, about 70 wt%, about 75 wt%, about 80 wt%, about 85 wt%, about 90 wt%, about 95 wt%, about 96 wt%, about 97 wt%, about 98 wt%, or about 99 wt% of the combined dry weight of total lipids in a composition (*e.g*., a liposomal composition).

In embodiments, a composition (*e.g*., a liposomal delivery vehicle such as a lipid nanoparticle) comprises about 0.1 wt% to about 20 wt% (*e.g*., about 0.1 wt% to about 15 wt%) of a compound described herein. In embodiments, a delivery vehicle (*e.g*., a liposomal delivery vehicle such as a lipid nanoparticle) comprises about 0.5 wt%, about 1 wt%, about 3 wt%, about 5 wt%, or about 10 wt% of a compound described herein. In embodiments, a delivery vehicle (*e.g.*, a liposomal delivery vehicle such as a lipid nanoparticle) comprises up to about 0.5 wt%, about 1 wt%, about 3 wt%, about 5 wt%, about 10 wt%, about 15 wt%, or about 20 wt% of a compound described herein. In embodiments, the percentage results in an improved beneficial effect (*e.g.*, improved delivery to targeted tissues such as the liver or the lung).

The amount of a compound of the invention as described herein in a composition also can be described as a percentage ("mol%") of the combined molar amounts of total lipids of a composition (*e.g.*, the combined molar amounts of all lipids present in a liposomal delivery vehicle).

In embodiments of pharmaceutical compositions described herein, a compound of the invention as described herein is present in an amount that is about 0.5 mol% to about 50 mol% (*e.g.,* about 0.5 mol% to about 20 mol%) of the combined molar amounts of all lipids present in a composition such as a liposomal delivery vehicle.

In embodiments, a compound of the invention as described herein is present in an amount that is about 0.5 mol% to about 5 mol%, about 1 mol% to about 10 mol%, about 5 mol% to about 20 mol%, about 10 mol% to about 20 mol%, about 15 mol% to about 30 mol%, about 20 mol% to about 35 mol%, about 25 mol% to about 40 mol%, about 30 mol% to about 45 mol%, about 35 mol% to about 50 mol%, about 40 mol% to about 55 mol %, or about 45 mol% to about 60 mol% of the combined molar amounts of all lipids present in a composition such as a liposomal delivery vehicle. In embodiments, a compound of the invention as described herein is present in an amount that is about 1 mol% to about 60 mol%, 1 mol% to about 50 mol%, 1 mol% to about 40 mol%, 1 mol% to about 30 mol%, about 1 mol% to about 20 mol%, about 1 mol% to about 15 mol%, about 1 mol% to about 10 mol%, about 5 mol% to about 55 mol%, about 5 mol% to about 45 mol%, about 5 mol% to about 35 mol% or about 5 mol% to about 25 mol% of the combined molar amounts of all lipids present in a composition such as a liposomal delivery vehicle

In certain embodiments, a compound of the invention as described herein can comprise from about 0.1 mol% to about 50 mol%, or from 0.5 mol% to about 50 mol%, or from about 1 mol% to about 50 mol%, or from about 5 mol% to about 50 mol%, or from about 10 mol% to about 50 mol%, or from about 15 mol% to about 50 mol%, or from about 20 mol% to about 50 mol%, or from about 25 mol% to about 50 mol%, or from about 30 mol% to about 50 mol%, of the total amount of lipids in a composition (*e.g*., a liposomal delivery vehicle).

In certain embodiments, a compound of the invention as described herein can comprise greater than about 0.1 mol%, or greater than about 0.5 mol%, or greater than about 1 mol%, greater than about 5 mol%, greater than about 10 mol%, greater than about 20 mol%, greater than about 30 mol%, or greater than about 40 mol% of the total amount of lipids in the lipid nanoparticle.

In certain embodiments, a compound as described can comprise less than about 60 mol%, or less than about 55 mol%, or less than about 50 mol%, or less than about 45 mol%, or less than about 40 mol%, or less than about 35 mol %, less than about 30 mol%, or less than about 25 mol%, or less than about 10 mol%, or less than about 5 mol%, or less than about 1 mol% of the total amount of lipids in a composition (*e.g.*, a liposomal delivery vehicle).

In embodiments, the amount of a compound of the invention as described herein is present in an amount that is at least about 5 mol%, about 10 mol%, about 15 mol%, about 20 mol%, about 25 mol%, about 30 mol%, about 35 mol%, about 40 mol%, about 45 mol%, about 50 mol%, about 55 mol%, about 60 mol%, about 65 mol%, about 70 mol%, about 75 mol%, about 80 mol%, about 85 mol%, about 90 mol%, about 95 mol%, about 96 mol%, about 97 mol%, about 98 mol%, or about 99 mol% of the combined molar amounts of total lipids in a composition (*e.g*., a liposomal composition).

In embodiments, the amount of a compound of the invention as described herein is present in an amount that is no more than about 5 mol%, about 10 mol%, about 15 mol%, about 20 mol%, about 25 mol%, about 30 mol%, about 35 mol%, about 40 mol%, about 45 mol%, about 50 mol%, about 55 mol%, about 60 mol%, about 65 mol%, about 70 mol%, about 75 mol%, about 80 mol%, about 85 mol%, about 90 mol%, about 95 mol%, about 96 mol%, about 97 mol%, about 98 mol%, or about 99 mol% of the combined molar amounts of total lipids in a composition (*e.g.*, a liposomal composition).

In embodiments, the percentage results in an improved beneficial effect (*e.g*., improved delivery to targeted tissues such as the liver or the lung).

In a typical embodiment, a composition of the invention (*e.g*., a liposomal composition) comprises one or more cationic lipids, one or more non-cationic lipids, one or more cholesterol-based lipids, and one or more PEG-modified lipids, wherein at least one cationic lipid is a compound of the invention as described herein. For example, a composition suitable for practicing the invention has four lipid components comprising a compound of the invention as described herein as the cationic lipid component, a non-cationic lipid, a cholesterol-based lipid and a PEG-modified lipid. The non-cationic lipid may be DOPE or DEPE. The cholesterol-based lipid may be cholesterol. The PEG-modified lipid may be DMG-PEG2K.

In further embodiments, pharmaceutical (*e.g.*, liposomal) compositions comprise one or more of a PEG-modified lipid, a non-cationic lipid and a cholesterol lipid. In other embodiments, such pharmaceutical (*e.g*., liposomal) compositions comprise: one or more PEG-modified lipids; one or more non-cationic lipids; and one or more cholesterol lipids. In yet further embodiments, such pharmaceutical (*e.g*., liposomal) compositions comprise: one or more PEG-modified lipids and one or more cholesterol lipids.

In embodiments, a composition (*e.g.*, lipid nanoparticle) that encapsulates a nucleic acid (*e.g*., mRNA encoding a peptide or protein) comprises one or more compounds of the invention as described herein and one or more lipids selected from the group consisting of a cationic lipid, a non-cationic lipid, and a PEGylated lipid.

In embodiments, a composition (*e.g*., lipid nanoparticle) that encapsulates a nucleic acid (*e.g*., mRNA encoding a peptide or protein) comprises one or more compound of the invention as described herein; one or more lipids selected from the group consisting of a cationic lipid, a non-cationic lipid, and a PEGylated lipid; and further comprises a cholesterol-based lipid. Typically, such a composition has four lipid components comprising a compound of the invention as described herein as the cationic lipid component, a non-cationic lipid (*e.g.,* DOPE), a cholesterol-based lipid (*e.g.,* cholesterol) and a PEG-modified lipid (*e*.g., DMG-PEG2K).

In embodiments, a lipid nanoparticle that encapsulates a nucleic acid (*e.g*., mRNA encoding a peptide or protein) comprises one or more compounds of the invention as described herein, as well as one or more lipids selected from the group consisting of a cationic lipid, a non-cationic lipid, a PEGylated lipid, and a cholesterol-based lipid.

According to various embodiments, the selection of cationic lipids, non-cationic lipids and/or PEG-modified lipids which comprise the lipid nanoparticle, as well as the relative molar ratio of such lipids to each other, is based upon the characteristics of the selected lipid(s), the nature of the intended target cells, the characteristics of the mRNA to be delivered. Additional considerations include, for example, the saturation of the alkyl chain, as well as the size, charge, pH, pKa, fusogenicity and toxicity of the selected lipid(s). Thus, the molar ratios may be adjusted accordingly.

In some embodiments, the ratio of cationic lipid(s) to non-cationic lipid(s) to cholesterol-based lipid(s) to PEG-modified lipid(s) may be between about 30-60:10-50:10-50:1-10, respectively. In some embodiments, the ratio of cationic lipid(s) to non-cationic lipid(s) to cholesterol-based lipid(s) to PEG-modified lipid(s) may be between about 30-60:20-40:10-30:1-10, respectively.

### Cationic Lipids

In addition to any of the compounds of the invention as described herein, a composition may comprise one or more additional cationic lipids.

In some embodiments, liposomes may comprise one or more additional cationic lipids. As used herein, the phrase "cationic lipid" refers to any of a number of lipid species that have a net positive charge at a selected pH, such as physiological pH. Several cationic lipids have been described in the literature, many of which are commercially available.

Suitable additional cationic lipids for use in the compositions include the cationic lipids as described in the literature.

### Helper Lipids

Compositions (*e.g*., liposomal compositions) may also comprise one or more helper lipids. Such helper lipids include non-cationic lipids. As used herein, the phrase "non-cationic lipid" refers to any neutral, zwitterionic or anionic lipid. As used herein, the phrase "anionic lipid" refers to any of a number of lipid species that carry a net negative charge at a selected pH, such as physiological pH. Non-cationic lipids include, but are not limited to, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), 1,2-Dierucoyl-sn-glycero-3-phosphoethanolamine (DEPE), palmitoyloleoylphosphatidylcholine (POPC), palmitoyloleoyl-phosphatidylethanolamine (POPE), dioleoyl-phosphatidylethanolamine 4-(N-maleimidomethyl)-cyclohexane-1-carboxylate (DOPE-mal), dipalmitoyl phosphatidyl ethanolamine (DPPE), dimyristoylphosphoethanolamine (DMPE), distearoyl-phosphatidyl-ethanolamine (DSPE), 16-O-monomethyl PE, 16-O-dimethyl PE, 18-1-trans PE, 1-stearoyl-2-oleoyl-phosphatidyethanolamine (SOPE), or a mixture thereof. A non-cationic or helper lipid suitable for practicing the invention is dioleoylphosphatidylethanolamine (DOPE). Alternatively, 1,2-Dierucoyl-sn-glycero-3-phosphoethanolamine (DEPE) can be used as a non-cationic or helper lipid.

In some embodiments, a non-cationic lipid is a neutral lipid, *i.e.,* a lipid that does not carry a net charge in the conditions under which the composition is formulated and/or administered.

In some embodiments, a non-cationic lipid may be present in a molar ratio (mol%) of about 5% to about 90%, about 5% to about 70%, about 5% to about 50%, about 5% to about 40%, about 5% to about 30%, about 10 % to about 70%, about 10% to about 50%, or about 10% to about 40% of the total lipids present in a composition. In some embodiments, total non-cationic lipids may be present in a molar ratio (mol%) of about 5% to about 90%, about 5% to about 70%, about 5% to about 50%, about 5% to about 40%, about 5% to about 30%, about 10 % to about 70%, about 10% to about 50%, or about 10% to about 40% of the total lipids present in a composition. In some embodiments, the percentage of non-cationic lipid in a liposome may be greater than about 5 mol%, greater than about 10 mol%, greater than about 20 mol%, greater than about 30 mol%, or greater than about 40 mol%. In some embodiments, the percentage total non-cationic lipids in a liposome may be greater than about 5 mol%, greater than about 10 mol%, greater than about 20 mol%, greater than about 30 mol%, or greater than about 40 mol%. In some embodiments, the percentage of non-cationic lipid in a liposome is no more than about 5 mol%, no more than about 10 mol%, no more than about 20 mol%, no more than about 30 mol%, or no more than about 40 mol%. In some embodiments, the percentage total non-cationic lipids in a liposome may be no more than about 5 mol%, no more than about 10 mol%, no more than about 20 mol%, no more than about 30 mol%, or no more than about 40 mol%.

In some embodiments, a non-cationic lipid may be present in a weight ratio (wt%) of about 5% to about 90%, about 5% to about 70%, about 5% to about 50%, about 5% to about 40%, about 5% to about 30%, about 10 % to about 70%, about 10% to about 50%, or about 10% to about 40% of the total lipids present in a composition. In some embodiments, total non-cationic lipids may be present in a weight ratio (wt%) of about 5% to about 90%, about 5% to about 70%, about 5% to about 50%, about 5% to about 40%, about 5% to about 30%, about 10 % to about 70%, about 10% to about 50%, or about 10% to about 40% of the total lipids present in a composition. In some embodiments, the percentage of non-cationic lipid in a liposome may be greater than about 5 wt%, greater than about 10 wt%, greater than about 20 wt%, greater than about 30 wt%, or greater than about 40 wt%. In some embodiments, the percentage total non-cationic lipids in a liposome may be greater than about 5 wt%, greater than about 10 wt%, greater than about 20 wt%, greater than about 30 wt%, or greater than about 40 wt%. In some embodiments, the percentage of non-cationic lipid in a liposome is no more than about 5 wt%, no more than about 10 wt%, no more than about 20 wt%, no more than about 30 wt%, or no more than about 40 wt%. In some embodiments, the percentage total non-cationic lipids in a liposome may be no more than about 5 wt%, no more than about 10 wt%, no more than about 20 wt%, no more than about 30 wt%, or no more than about 40 wt%.

### Cholesterol-based Lipids

In some embodiments, a composition (*e.g.*, a liposomal composition) comprises one or more cholesterol-based lipids. For example, a suitable cholesterol-based lipid for practicing the invention is cholesterol. Other suitable cholesterol-based lipids include, for example, DC-Chol (N,N-dimethyl-N-ethylcarboxamidocholesterol), 1,4-bis(3-N-oleylamino-propyl)piperazine (Gao, et al. Biochem. Biophys. Res. Comm. 179, 280 (1991); Wolf et al. BioTechniques 23, 139 (1997); U.S. Pat. No. 5,744,335), or imidazole cholesterol ester (ICE), which has the following structure,

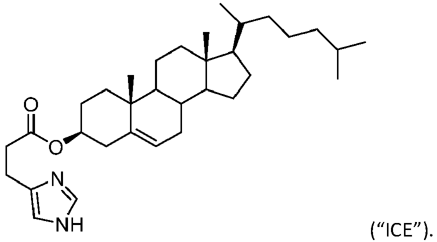

In some embodiments, a cholesterol-based lipid may be present in a molar ratio (mol%) of about 1% to about 30%, or about 5% to about 20% of the total lipids present in a liposome. In some embodiments, the percentage of cholesterol-based lipid in the lipid nanoparticle may be greater than about 5 mol%, greater than about 10 mol%, greater than about 20 mol%, greater than about 30 mol%, or greater than about 40 mol%. In some embodiments, the percentage of cholesterol-based lipid in the lipid nanoparticle may be no more than about 5 mol%, no more than about 10 mol%, no more than about 20 mol%, no more than about 30 mol%, or no more than about 40 mol%.

In some embodiments, a cholesterol-based lipid may be present in a weight ratio (wt%) of about 1% to about 30%, or about 5% to about 20% of the total lipids present in a liposome. In some embodiments, the percentage of cholesterol-based lipid in the lipid nanoparticle may be greater than about 5 wt%, greater than about 10 wt%, greater than about 20 wt%, greater than about 30 wt%, or greater than about 40 wt%. In some embodiments, the percentage of cholesterol-based lipid in the lipid nanoparticle may be no more than about 5 wt%, no more than about 10 wt%, no more than about 20 wt%, no more than about 30 wt%, or no more than about 40 wt%.

### PEGylated Lipids

In some embodiments, a composition (*e.g*., a liposomal composition) comprises one or more further PEGylated lipids. A suitable PEG-modified or PEGylated lipid for practicing the invention is 1,2-dimyristoyl-rac-glycero-3-methoxypolyethylene glycol-2000 (DMG-PEG2K).

For example, the use of polyethylene glycol (PEG)-modified phospholipids and derivatized lipids such as derivatized ceramides (PEG-CER), including N-octanoyl-sphingosine-1-[succinyl(methoxy polyethylene glycol)-2000] (C8 PEG-2000 ceramide) is also contemplated by the present invention in combination with one or more of compounds of the invention as described herein and, in some embodiments, other lipids together which comprise the liposome. In some embodiments, particularly useful exchangeable lipids are PEG-ceramides having shorter acyl chains (e.g., C₁₄ or C₁₈).

Contemplated further PEG-modified lipids (also referred to herein as a PEGylated lipid, which term is interchangeable with PEG-modified lipid) include, but are not limited to, a polyethylene glycol chain of up to 5 kDa in length covalently attached to a lipid with alkyl chain(s) of C₆-C₂₀ length. In some embodiments, a PEG-modified or PEGylated lipid is PEGylated cholesterol or PEG-2K. The addition of such components may prevent complex aggregation and may also provide a means for increasing circulation lifetime and increasing the delivery of the lipid-nucleic acid composition to the target cell, (Klibanov et al. (1990) FEBS Letters, 268 (1): 235-237), or they may be selected to rapidly exchange out of the formulation in vivo (see U.S. Pat. No. 5,885,613).

Further PEG-modified phospholipid and derivatized lipids of the present invention may be present in a molar ratio (mol%) from about 0% to about 10%, about 0.5% to about 10%, about 1% to about 10%, about 2% to about 10%, or about 3% to about 5% of the total lipid present in the composition (*e.g*., a liposomal composition).

### Pharmaceutical Formulations and Therapeutic Uses

Compounds of the invention as described herein may be used in the preparation of compositions (*e.g*., to construct liposomal compositions) that facilitate or enhance the delivery and release of encapsulated materials (*e.g*., one or more therapeutic polynucleotides) to one or more target cells (*e.g*., by permeating or fusing with the lipid membranes of such target cells).

For example, when a liposomal composition (*e.g*., a lipid nanoparticle) comprises or is otherwise enriched with one or more of the compounds disclosed herein, the phase transition in the lipid bilayer of the one or more target cells may facilitate the delivery of the encapsulated materials (*e.g.,* one or more therapeutic polynucleotides encapsulated in a lipid nanoparticle) into the one or more target cells.

Similarly, in certain embodiments compounds of the invention as described herein may be used to prepare liposomal vehicles that are characterized by their reduced toxicity *in vivo.* In certain embodiments, the reduced toxicity is a function of the high transfection efficiencies associated with the compositions disclosed herein, such that a reduced quantity of such composition may administered to the subject to achieve a desired therapeutic response or outcome.

Thus, pharmaceutical formulations comprising a compound described and nucleic acids provided by the present invention may be compositions for use in various therapeutic purposes. To facilitate delivery of nucleic acids *in vivo,* a compound described herein and nucleic acids can be formulated in combination with one or more additional pharmaceutical carriers, targeting ligands or stabilizing reagents. In some embodiments, a compound described herein can be formulated via pre-mixed lipid solution. In other embodiments, a composition comprising a compound described herein can be formulated using post-insertion techniques into the lipid membrane of the nanoparticles. Techniques for formulation and administration of drugs may be found in "Remington's Pharmaceutical Sciences," Mack Publishing Co., Easton, Pa., latest edition.

Suitable routes of administration include, for example, oral, rectal, vaginal, transmucosal, pulmonary including intratracheal or inhaled, or intestinal administration; parenteral delivery, including intradermal, transdermal (topical), intramuscular, subcutaneous, intramedullary injections, as well as intrathecal, direct intraventricular, intravenous, intraperitoneal, or intranasal. In particular embodiments, the intramuscular administration is to a muscle selected from the group consisting of skeletal muscle, smooth muscle and cardiac muscle. In some embodiments the administration results in delivery of the nucleic acids to a muscle cell. In some embodiments the administration results in delivery of the nucleic acids to a hepatocyte (*i.e.,* liver cell).

A common route for administering a liposomal composition of the invention may be intravenous delivery, in particular when treating metabolic disorders, especially those affecting the liver (*e.g*., ornithine transcarbamylase (OTC) deficiency). Alternatively, depending on the disease or disorder to be treated, the liposomal composition may be administered via pulmonary delivery (e.g., for the treatment of cystic fibrosis). For vaccination, a liposomal composition of the invention is typically administered intramuscularly. Diseases or disorders affecting the eye may be treated by administering a liposomal composition of the invention intravitreally.

Alternatively or additionally, pharmaceutical formulations of the invention may be administered in a local rather than systemic manner, for example, via injection of the pharmaceutical formulation directly into a targeted tissue (*e.g*., in a sustained release formulation). Local delivery can be affected in various ways, depending on the tissue to be targeted. Exemplary tissues in which delivered mRNA may be delivered and/or expressed include, but are not limited to the liver, kidney, heart, spleen, serum, brain, skeletal muscle, lymph nodes, skin, and/or cerebrospinal fluid. In embodiments, the tissue to be targeted in the liver. For example, aerosols containing compositions of the present invention can be inhaled (for nasal, tracheal, or bronchial delivery); compositions of the present invention can be injected into the site of injury, disease manifestation, or pain, for example; compositions can be provided in lozenges for oral, tracheal, or esophageal application; can be supplied in liquid, tablet or capsule form for administration to the stomach or intestines, can be supplied in suppository form for rectal or vaginal application; or can even be delivered to the eye by use of creams, drops, or even injection.

Compositions described herein can comprise mRNA encoding peptides including those described herein (*e.g.*, a polypeptide such as a protein).

In embodiments, a mRNA encodes a polypeptide.

In embodiments, a mRNA encodes a protein.

Exemplary peptides encoded by mRNA (*e.g*., exemplary proteins encoded by mRNA) are described herein.

The present invention provides a composition having full-length mRNA molecules encoding a peptide or protein of interest for use in the treatment of a subject, *e.g.*, a human subject or a cell of a human subject or a cell that is treated and delivered to a human subject.

### Delivery Methods

The route of delivery of the invention allows for non-invasive, selfadministration of the compounds of the invention. In some embodiments, the compositions for use in methods involve intratracheal or pulmonary administration by aerosolization, nebulization, or instillation of a compositions comprising mRNA encoding a therapeutic protein in a suitable transfection or lipid carrier vehicles as described above. In some embodiments, the protein is encapsulated with a liposome. In some embodiments, the liposome comprises a lipid, which is a compound of the invention. As used herein below, administration of a compound of the invention includes administration of a composition comprising a compound of the invention.

Although the local cells and tissues of the lung represent a potential target capable of functioning as a biological depot or reservoir for production and secretion of the protein encoded by the mRNA, applicants have discovered that administration of the compounds of the invention to the lung via aerosolization, nebulization, or instillation results in the distribution of even non-secreted proteins outside the lung cells. Without wishing to be bound by any particular theory, it is contemplated that nanoparticle compositions of the invention pass, through the lung airway-blood barrier, resulting in translation of the intact nanoparticle to non-lung cells and tissues, such as, *e.g.,* the heart, the liver, the spleen, where it results in the production of the encoded protein in these non-lung tissues. Thus, the utility of the compounds of the invention and methods of the invention extend beyond production of therapeutic protein in lung cells and tissues of the lung and can be used to delivery to non-lung target cells and/or tissues. They are useful in the management and treatment of a large number of diseases, and in particular peripheral diseases which result from both secreted and non-secreted protein and/or enzyme deficiencies (*e.g*., one or more lysosomal storage disorders). In certain embodiments, the compounds of the invention, used in the methods of the invention result in the distribution of the mRNA encapsulated nanoparticles and production of the encoded protein in the liver, spleen, heart, and/or other non-lung cells. For example, administration of the compounds of the invention, by aerosolization, nebulization, or instillation to the lung will result in the composition itself and its protein product (*e.g*., functional beta galactosidase protein) will be detectable in both the local cells and tissues of the lung, as well as in peripheral target cells, tissues and organs as a result of translocation of the mRNA and delivery vehicle to non-lung cells.

In certain embodiments, the compounds of the invention may be employed in the compositions for use in methods of the invention to specifically target peripheral cells or tissues. Following the pulmonary delivery, it is contemplated the compounds of the invention cross the lung airway-blood barrier and distribute into cells other than the local lung cells. Accordingly, the compounds disclosed herein may be administered to a subject by way of the pulmonary route of administration, using a variety of approach known by those skilled in the art (*e.g*., by inhalation), and distribute to both the local target cells and tissues of the lung, as well as in peripheral non-lung cells and tissues (*e.g*., cells of the liver, spleen, kidneys, heart, skeletal muscle, lymph nodes, brain, cerebrospinal fluid, and plasma). As a result, both the local cells of the lung and the peripheral non-lung cells can serve as biological reservoirs or depots capable of producing and/or secreting a translation product encoded by one or more polynucleotides. Accordingly, the present invention is not limited to the treatment of lung diseases or conditions, but rather can be used as a non-invasive means of facilitating the delivery of polynucleotides, or the production of enzymes and proteins encoded thereby, in peripheral organs, tissues and cells (*e.g*., hepatocytes) which would otherwise be achieved only by systemic administration. Exemplary peripheral non-lung cells include, but are not limited to, hepatocytes, epithelial cells, hematopoietic cells, epithelial cells, endothelial cells, bone cells, stem cells, mesenchymal cells, neural cells, cardiac cells, adipocytes, vascular smooth muscle cells, cardiomyocytes, skeletal muscle cells, beta cells, pituitary cells, synovial lining cells, ovarian cells, testicular cells, fibroblasts, B cells, T cells, reticulocytes, leukocytes, granulocytes and tumor cells.

Following administration of the composition to the subject, the protein product encoded by the mRNA (*e.g*., a functional protein or enzyme) is detectable in the peripheral target tissues for at least about one to seven days or longer following administration of the compound to the subject. The amount of protein product necessary to achieve a therapeutic effect will vary depending on the condition being treated, the protein encoded, and the condition of the patient. For example, the protein product may be detectable in the peripheral target tissues at a concentration (*e.g*., a therapeutic concentration) of at least 0.025-1.5 µg/ml (*e.g.*, at least 0.050 µg/ml, at least 0.075 µg/ml, at least 0.1 µg/ml, at least 0.2 µg/ml, at least 0.3 µg/ml, at least 0.4 µg/ml, at least 0.5 µg/ml, at least 0.6 µg/ml, at least 0.7 µg/ml, at least 0.8 µg/ml, at least 0.9 µg/ml, at least 1.0 µg/ml, at least 1.1 µg/ml, at least 1.2 µg/ml, at least 1.3 µg/ml, at least 1.4 µg/ml, or at least 1.5 µg/ml), for at least about 1, 2, 3, 4, 5, 6, 7, 8, 9, 10, 11,12, 13, 14, 15, 16, 17, 18, 19, 20, 21, 22, 23, 24, 25, 26, 27, 28, 29, 30, 35, 40, 45 days or longer following administration of the compound to the subject.

It has been demonstrated that nucleic acids can be delivered to the lungs by intratracheal administration of a liquid suspension of the compound and inhalation of an aerosol mist produced by a liquid nebulizer or the use of a dry powder apparatus such as that described in U.S. patent 5,780,014.

In certain embodiments, the compounds of the invention may be formulated such that they may be aerosolized or otherwise delivered as a particulate liquid or solid prior to or upon administration to the subject. Such compounds may be administered with the assistance of one or more suitable devices for administering such solid or liquid particulate compositions (such as, *e.g.,* an aerosolized aqueous solution or suspension) to generate particles that are easily respirable or inhalable by the subject. In some embodiments, such devices (*e.g*., a metered dose inhaler, jet-nebulizer, ultrasonic nebulizer, dry-powder-inhalers, propellant-based inhaler or an insufflator) facilitate the administration of a predetermined mass, volume or dose of the compositions (*e.g*., about 0.5 mg/kg of mRNA per dose) to the subject. For example, in certain embodiments, the compounds of the invention are administered to a subject using a metered dose inhaler containing a suspension or solution comprising the compound and a suitable propellant. In certain embodiments, the compounds of the invention may be formulated as a particulate powder (*e.g*., respirable dry particles) intended for inhalation. In certain embodiments, compositions of the invention formulated as respirable particles are appropriately sized such that they may be respirable by the subject or delivered using a suitable device (*e.g*., a mean D50 or D90 particle size less than about 500µm, 400µm, 300µm, 250µm, 200µm, 150µm, 100µm, 75µm, 50µm, 25µm, 20µm, 15µm, 12.5µm, 10µm, 5µm, 2.5µm or smaller). In yet other embodiments, the compounds of the invention are formulated to include one or more pulmonary surfactants (*e.g*., lamellar bodies). In some embodiments, the compounds of the invention are administered to a subject such that a concentration of at least 0.05 mg/kg, at least 0.1 mg/kg, at least 0.5 mg/kg, at least 1.0 mg/kg, at least 2.0 mg/kg, at least 3.0 mg/kg, at least 4.0 mg/kg, at least 5.0 mg/kg, at least 6.0 mg/kg, at least 7.0 mg/kg, at least 8.0 mg/kg, at least 9.0 mg/kg, at least 10 mg/kg, at least 15 mg/kg, at least 20 mg/kg, at least 25 mg/kg, at least 30 mg/kg, at least 35 mg/kg, at least 40 mg/kg, at least 45 mg/kg, at least 50 mg/kg, at least 55 mg/kg, at least 60 mg/kg, at least 65 mg/kg, at least 70 mg/kg, at least 75 mg/kg, at least 80 mg/kg, at least 85 mg/kg, at least 90 mg/kg, at least 95 mg/kg, or at least 100 mg/kg body weight is administered in a single dose. In some embodiments, the compounds of the invention are administered to a subject such that a total amount of at least 0.1 mg, at least 0.5 mg, at least 1.0 mg, at least 2.0 mg, at least 3.0 mg, at least 4.0 mg, at least 5.0 mg, at least 6.0 mg, at least 7.0 mg, at least 8.0 mg, at least 9.0 mg, at least 10 mg, at least 15 mg, at least 20 mg, at least 25 mg, at least 30 mg, at least 35 mg, at least 40 mg, at least 45 mg, at least 50 mg, at least 55 mg, at least 60 mg, at least 65 mg, at least 70 mg, at least 75 mg, at least 80 mg, at least 85 mg, at least 90 mg, at least 95 mg or at least 100 mg mRNA is administered in one or more doses.

### Synthesis of Compounds of the invention

The cationic lipid MC3 is the current gold standard for *in vivo* delivery of *e.g.* siRNA (see WO2010/144740). However, the synthesis of this lipid involves a six-step process and requires handling of a Grignard reagent. In contrast, the present invention provides cationic lipids that can be prepared from readily available starting reagents. The compounds of the invention as described herein can be prepared according to methods known in the art, including the exemplary syntheses of the Examples provided herein.

### EXAMPLES

While certain compounds, compositions and compositions for use in methods of the present invention have beer described with specificity in accordance with certain embodiments, the following examples serve only to illustrate the compounds of the invention and are not intended to limit the same.

### Examples 1-11: Synthesis of HEP-based cationic lipids

HEP-based cationic lipids described herein may be prepared according to **Scheme 1:**

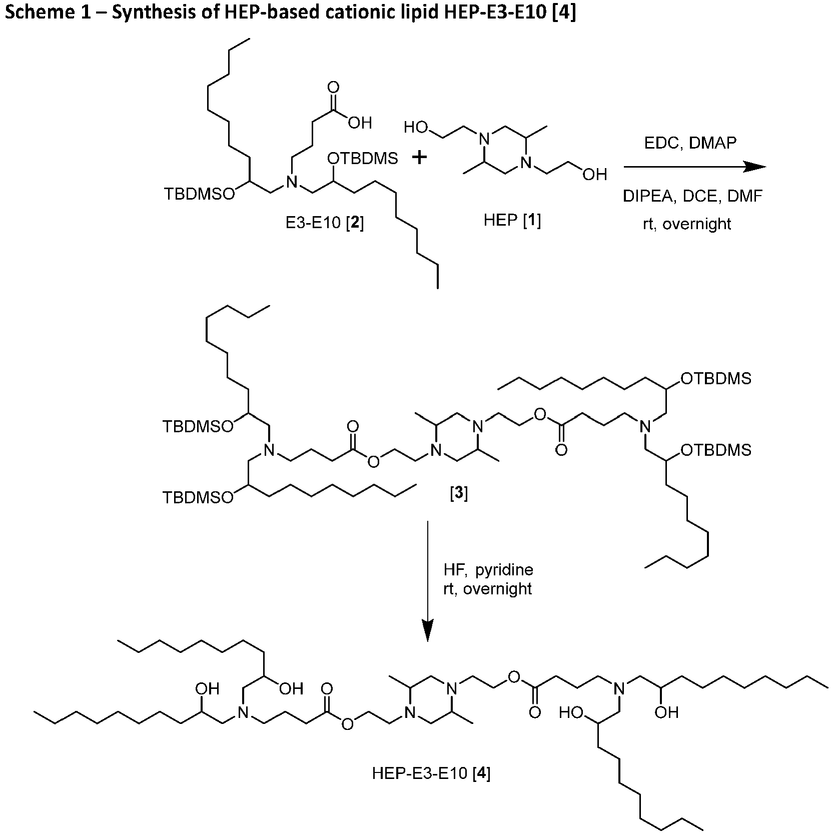

### Synthesis of [3]

As set out in Scheme 1: To a solution containing HEP [1] (0.100 g, 0.494 mmol, 1.0 eq), E3-E10 [2] (0.668 g, 1.038 mmol, 2.1 eq), 1ml of dimethylformamide, 3ml of dichloroethane, diisopropylethylamine (0.344 µL, 1.98 mmol, 4.0 eq), and N,N-Dimethylaminopyridine (0.024 g, 0.198 mmol, 0.4 eq) was added 1-Ethyl-3-(3-dimethylaminopropyl)carbodiimide (0.285 g, 1.48 mmol, 3.0 eq) and allowed to react at room temperature overnight (18hr). Afterwards, the reaction mixture was concentrated using a rotavapor and purified using a Buchi Combi-flash system on 12g, 40µm-sized silica gel columns using hexanes/ethyl acetate as the mobile phase, yielding a colorless oil (70% yield).

### Synthesis of HEP-E3-E10 [4]

As set out in Scheme 1: To a 20 ml Polypropylene scintillation vial equipped with a PTFE stir-bar was added [3] (0.500 g, 0.344 mmol, 1.0 eq) along with 4ml of dry tetrahydrofuran. The vial was cooled to 0-5°C on an ice bath and HF/pyridine (1.76 ml, 67.86 mmol, 197.3 eq) was added dropwise. After addition, the reaction vial was allowed to warm to room temperature and stirred overnight (18hr). Afterwards, the reaction mixture was neutralized with saturated sodium bicarbonate at 0°C. Ethyl acetate was used for extraction (3x). The organic layers were combined, washed with saturated sodium chloride (4x), dried with sodium sulfate, filtered, and rotovaped to yield an off-yellow oil. This oil was further purified using a Buchi Combi-flash system on 12g, 40µm-sized silica gel columns using dichloromethane/methanol (3% methanol) as the mobile phase, yielding a colorless oil (60% yield). 1H NMR (400 MHz, CDCl3) 4.16 (m, 4H), 3.60 (m, 4H), 2.97 (m, 3H), 2.78 (d, 3H), 2.58 (m, 9H), 2.37 (m, 12H), 2.15 (m, 2H), 1.78 (m, 4H), 1.44 (m, 7H), 1.36 (m, 9H), 1.26 (br, 45H), 1.05 (d, 6H), 0.87 (t, 12H). Expected M/Z = 998.59, Observed = 998.0.

HEP-based cationic lipids described herein may be prepared according to **Scheme 2:**

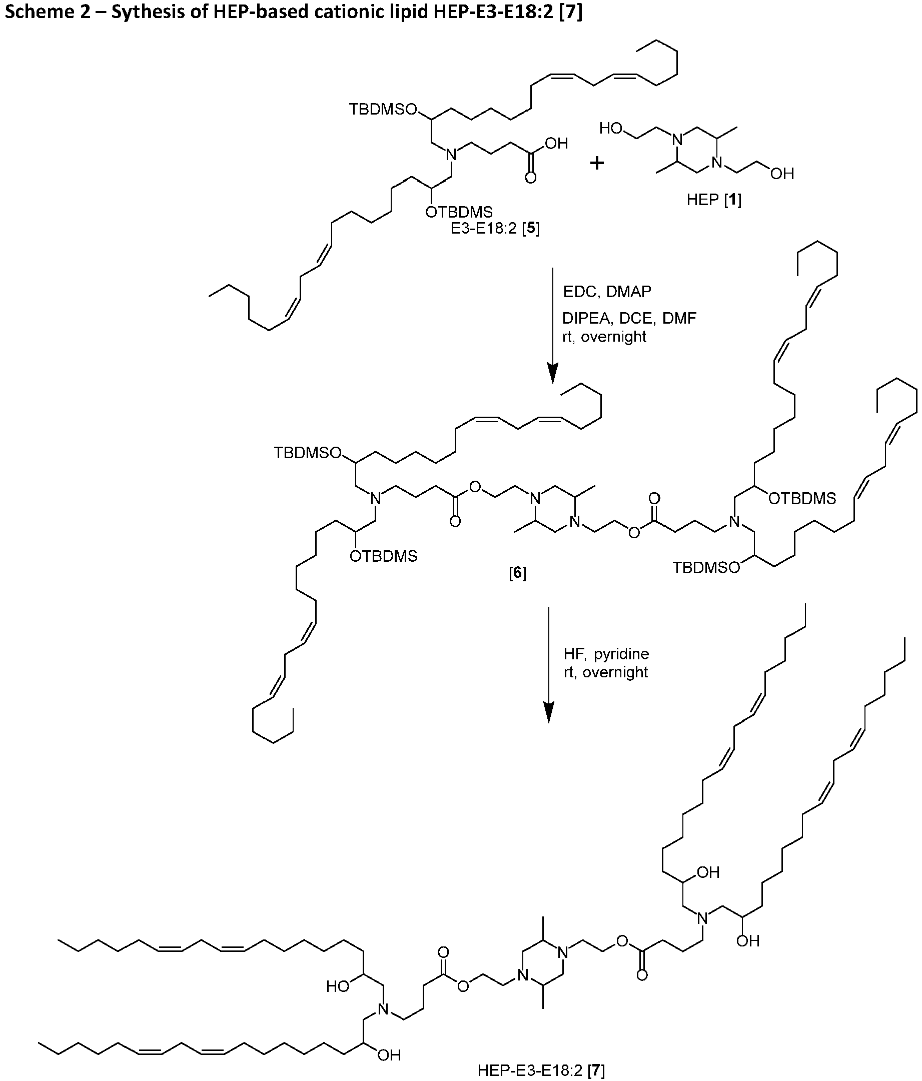

### Synthesis of [6]

As set out in Scheme 2: To a solution containing HEP [1] (0.100 g, 0.494 mmol, 1.0 eq), E3-E18:2 [5] (0.893 g, 1.038 mmol, 2.1 eq), 1ml of dimethylformamide, 3 ml of dichloroethane, diisopropylethylamine (0.344 µL, 1.98 mmol, 4.0 eq), and N,N-Dimethylaminopyridine (0.024 g, 0.198 mmol, 0.4 eq) was added 1-Ethyl-3-(3-dimethylaminopropyl)carbodiimide (0.285 g, 1.48 mmol, 3.0 eq) and allowed to react at room temperature overnight (18hr). Afterwards, the reaction mixture was concentrated using a rotavapor and purified using a Buchi Combi-flash system on 12g, 40µm-sized silica gel columns using hexanes/ethyl acetate as the mobile phase, yielding a colorless oil (45% yield).

### Synthesis of HEP-E3-E18:2 [7]

As set out in Scheme 2: To a 20 ml Polypropylene scintillation vial equipped with a PTFE stir-bar was added [6] (0.418 g, 0.222 mmol, 1.0 eq) along with 4ml of dry tetrahydrofuran. The vial was cooled to 0-5°C on an ice bath and HF/pyridine (1.14 ml, 43.713 mmol, 197.3 eq) was added dropwise. After addition, the reaction vial was allowed to warm to room temperature and stirred overnight (18hr). Afterwards, the reaction mixture was neutralized with saturated sodium bicarbonate at 0°C. Ethyl acetate was used for extraction (3x). The organic layers were combined, washed with saturated sodium chloride (4x), dried with sodium sulfate, filtered, and rotovaped to yield an off-yellow oil. This oil was further purified using a Buchi Combi-flash system on 12g, 40µm-sized silica gel columns using dichloromethane/methanol (3% methanol) as the mobile phase, yielding a colorless oil (47% yield). 1H NMR (400 MHz, CDCl3) 5.35 (m, 16H), 4.16 (br, 4H), 3.62 (br, 4H), 2.96 (m, 2H), 2.77 (t, 12H), 2.55 (m, 9H), 2.37 (m, 14H), 2.15 (m, 2H), 2.04 (m, 16H), 1.79 (br, 4H), 1.44 (m, 6H), 1.30 (br, 64H), 1.05 (d, 6H), 0.89 (t, 12H). Expected M/Z = 1430.40, Observed = 1430.0.

HEP-based cationic lipids described herein may be prepared according to **Scheme 3:**

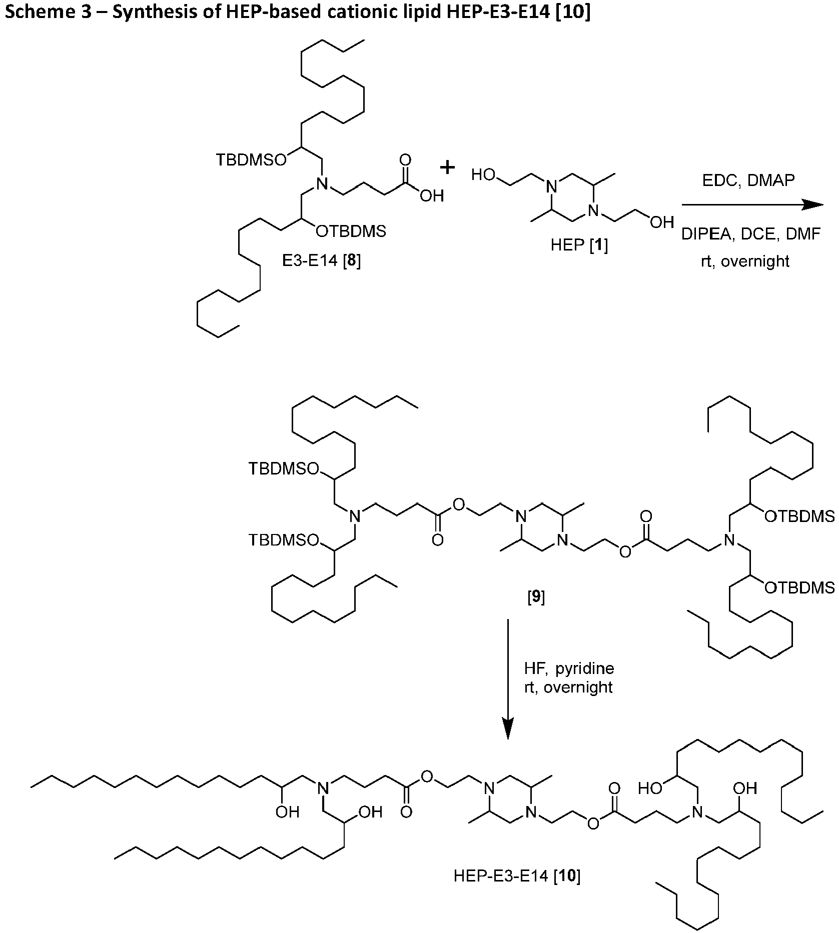

### Synthesis of [9]

As set out in Scheme 3: To a solution containing HEP [1] (0.100 g, 0.494 mmol, 1.0 eq), E3-E14 [8] (0.785 g, 1.038 mmol, 2.1 eq), 1ml of dimethylformamide, 3ml of dichloroethane, diisopropylethylamine (0.344 µL, 1.98 mmol, 4.0 eq), and N,N-Dimethylaminopyridine (0.024 g, 0.198 mmol, 0.4 eq) was added 1-Ethyl-3-(3-dimethylaminopropyl)carbodiimide (0.285 g, 1.48 mmol, 3.0 eq) and allowed to react at room temperature overnight (18hr). Afterwards, the reaction mixture was concentrated using a rotavapor and purified using a Buchi Combi-flash system on 12g, 40µm-sized silica gel columns using hexanes/ethyl acetate as the mobile phase, yielding a colorless oil (60.3% yield).

### Synthesis of HEP-E3-E14 [10]

As set out in Scheme 3: To a 20 ml Polypropylene scintillation vial equipped with a PTFE stir-bar was added [9] (0.500 g, 0.297 mmol, 1.0 eq) along with 4ml of dry tetrahydrofuran. The vial was cooled to 0-5°C on an ice bath and HF/pyridine (1.53 ml, 58.766 mmol, 197.3 eq) was added dropwise. After addition, the reaction vial was allowed to warm to room temperature and stirred overnight (18hr). Afterwards, the reaction mixture was neutralized with saturated sodium bicarbonate at 0°C. Ethyl acetate was used for extraction (3x). The organic layers were combined, washed with saturated sodium chloride (4x), dried with sodium sulfate, filtered, and rotovaped to yield an off-yellow oil. This oil was further purified using a Buchi Combi-flash system on 12g, 40µm-sized silica gel columns using dichloromethane/methanol (3% methanol) as the mobile phase, yielding a colorless oil (55% yield). 1H NMR (400 MHz, CDCl3) 4.17 (m, 4H), 3.62 (m, 4H), 2.97 (m, 3H), 2.76 (d, 2H), 2.55 (m, 8H), 2.37 (m, 14H), 2.15 (m, 2H), 1.79 (m, 4H), 1.45 (m, 6H), 1.37 (m, 6H), 1.25 (br, 80H), 1.04 (d, 6H), 0.89 (t, 12H), Expected M/Z = 1222.02, Observed = 1222.0.

HEP-based cationic lipids described herein may be prepared according to **Scheme 4:**

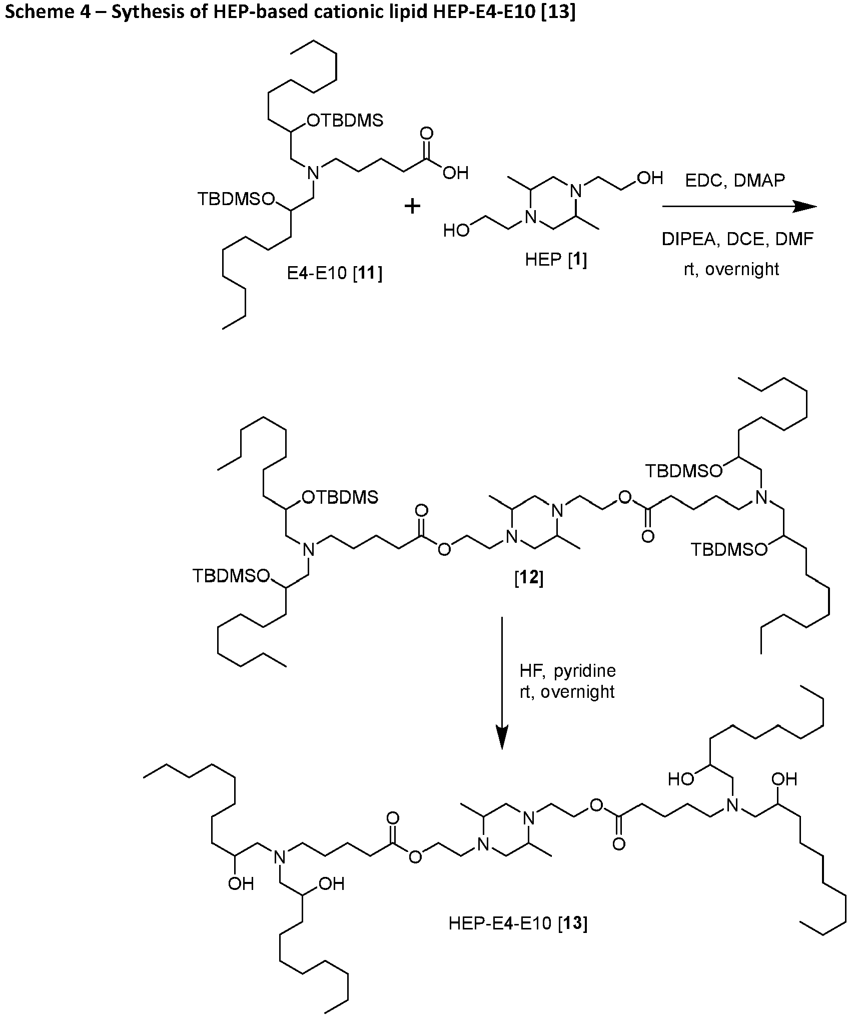

### Synthesis of [12]

As set out in Scheme 4: To a solution of HEP [1] (0.100 g, 0.494 mmol, 1.0 eq), E4-E10 [11] (0.683 g, 1.038 mmol, 2.1 eq), 1ml of dimethylformamide, 3ml of dichloroethane, diisopropylethylamine (0.344 µL, 1.98 mmol, 4.0 eq), and N,N-Dimethylaminopyridine (0.024 g, 0.198 mmol, 0.4 eq) was added 1-Ethyl-3-(3-dimethylaminopropyl)carbodiimide (0.285 g, 1.48 mmol, 3.0 eq) and allowed to react at room temperature overnight (18hr). Afterwards, the reaction mixture was concentrated using a rotavapor and purified using a Buchi Combi-flash system on 12g, 40µm-sized silica gel columns using hexanes/ethyl acetate as the mobile phase, yielding a colorless oil (63.3% yield).

### Synthesis of HEP-E4-E10 [13]

As set out in Scheme 4: To a 20 ml Polypropylene scintillation vial equipped with a PTFE stir-bar was added [12] (0.450 g, 0.303 mmol, 1.0 eq) along with 4ml of dry tetrahydrofuran. The vial was cooled to 0-5°C on an ice bath and HF/pyridine (1.55 ml, 59.920 mmol, 197.3 eq) was added dropwise. After addition, the reaction vial was allowed to warm to room temperature and stirred overnight (18hr). Afterwards, the reaction mixture was neutralized with saturated sodium bicarbonate at 0°C. Ethyl acetate was used for extraction (3x). The organic layers were combined, washed with saturated sodium chloride (4x), dried with sodium sulfate, filtered, and rotovaped to yield an off-yellow oil. This oil was further purified using a Buchi Combi-flash system on 12g, 40µm-sized silica gel columns using dichloromethane/methanol (3%) as the mobile phase, yielding a colorless oil (48.4% yield). 1H NMR (400 MHz, CDCl3) 4.16 (t, 4H), 3.62 (br, 4H), 2.96 (q, 3H), 2.76 (d, 4H), 2.56 (m, 8H), 2.40 (m, 4H), 2.32 (t, 4H), 2.13 (t, 2H), 1.61 (m, 4H), 1.46 (m, 8H), 1.37 (m, 8H), 1.28 (br, 44H), 1.03 (d, 6H), 0.87 (t, 12H), 13C NMR (400 MHz, CDCl3) 173.65 (2C), 69.65 (2C), 68.04 (2C), 62.84 (2C), 61.82 (2C), 61.44 (2C), 60.89 (2C), 55.57 (4C), 51.55 (2C), 35.35 (4C), 34.20 (2C), 32.09 (7C), 30.00 (5C), 29.77 (6C), 29.47 (6C), 26.93 (2C), 25.84 (5C), 22.84 (9C), 17.77 (2C), 14.30 (7C). Expected M/Z = 1025.64, Observed = 1025.8.

HEP-based cationic lipids described herein may be prepared according to **Scheme 5:**

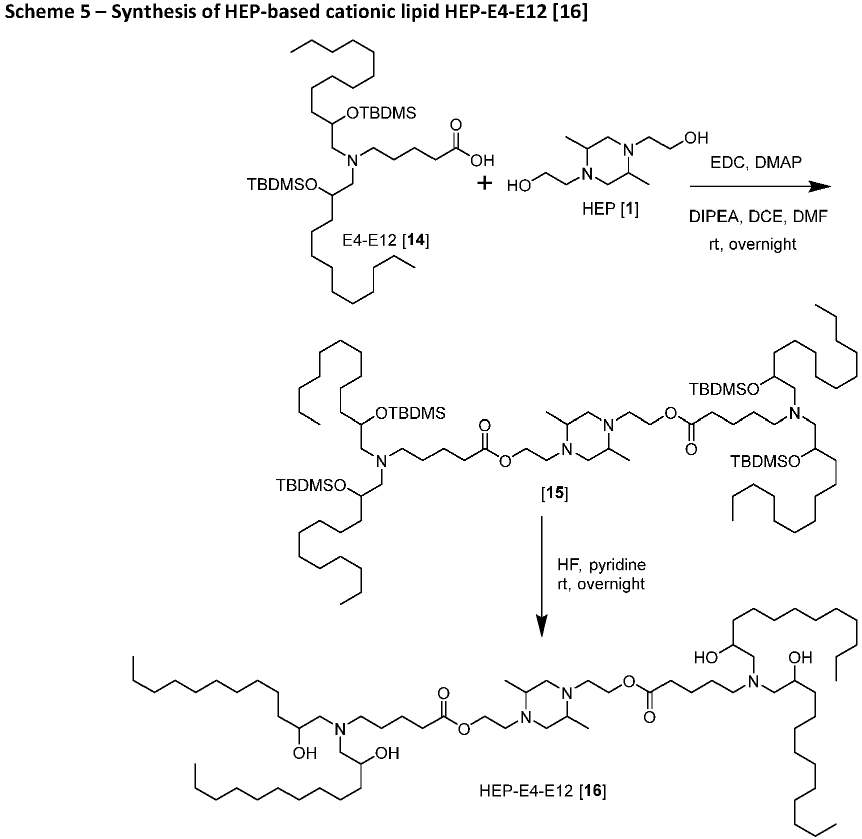

### Synthesis of [15]

As set out in Scheme 5: To a solution of HEP [1] (0.100 g, 0.494 mmol, 1.0 eq), E4-E12 [14] (0.742 g, 1.038 mmol, 2.1 eq), 1ml of dimethylformamide, 3ml of dichloroethane, diisopropylethylamine (0.344 µL, 1.98 mmol, 4.0 eq), and N,N-Dimethylaminopyridine (0.024 g, 0.198 mmol, 0.4 eq) was added 1-Ethyl-3-(3-dimethylaminopropyl)carbodiimide (0.285 g, 1.48 mmol, 3.0 eq) and allowed to react at room temperature overnight (18hr). Afterwards, the reaction mixture was concentrated using a rotavapor and purified using a Buchi Combi-flash system on 12g, 40µm-sized silica gel columns using hexanes/ethyl acetate as the mobile phase, yielding a colorless oil (66% yield).

### Synthesis of HEP-E4-E12 [16]

As set out in Scheme 5: To a 20 ml Polypropylene scintillation vial equipped with a PTFE stir-bar was added [15] (0.520 g, 0.326 mmol, 1.0 eq) along with 4ml of dry tetrahydrofuran. The vial was cooled to 0-5°C on an ice bath and HF/pyridine (1.67 ml, 64.376 mmol, 197.3 eq) was added dropwise. After addition, the reaction vial was allowed to warm to room temperature and stirred overnight (18hr). Afterwards, the reaction mixture was neutralized with saturated sodium bicarbonate at 0°C. Ethyl acetate was used for extraction (3x). The organic layers were combined, washed with saturated sodium chloride (4x), dried with sodium sulfate, filtered, and rotovaped to yield an off-yellow oil. This oil was further purified using a Buchi Combi-flash system on 12g silica gel columns using dichloromethane/methanol (3% methanol) as the mobile phase, yielding a colorless oil (48.4% yield). 1H NMR (400 MHz, CDCl3) 4.17 (m, 4H), 3.63 (m, 4H), 2.95 (m, 3H), 2.76 (d, 4H), 2.56 (m, 8H), 2.39 (m, 9H), 2.32 (t, 4H), 2.13 (t, 2H), 1.61 (m, 4H), 1.46 (m, 8H), 1.37 (m, 12H), 1.25 (br, 61H), 1.04 (d, 6H), 0.87 (t, 12H), 13C NMR (400 MHz, CDCl3) 173.56 (2C), 69.65 (2C), 68.04 (2C), 62.84 (2C), 61.82 (4C), 60.89 (2C), 55.57 (4C), 53.61 (1C), 51.56 (2C), 35.36 (4C), 33.65 (2C), 32.12 (6C), 29.63 (31C), 25.85 (5C), 22.77 (9C), 14.31 (7C), Expected M/Z = 1137.86, Observed = 1138.0.

HEP-based cationic lipids described herein may be prepared according to **Scheme 6:**

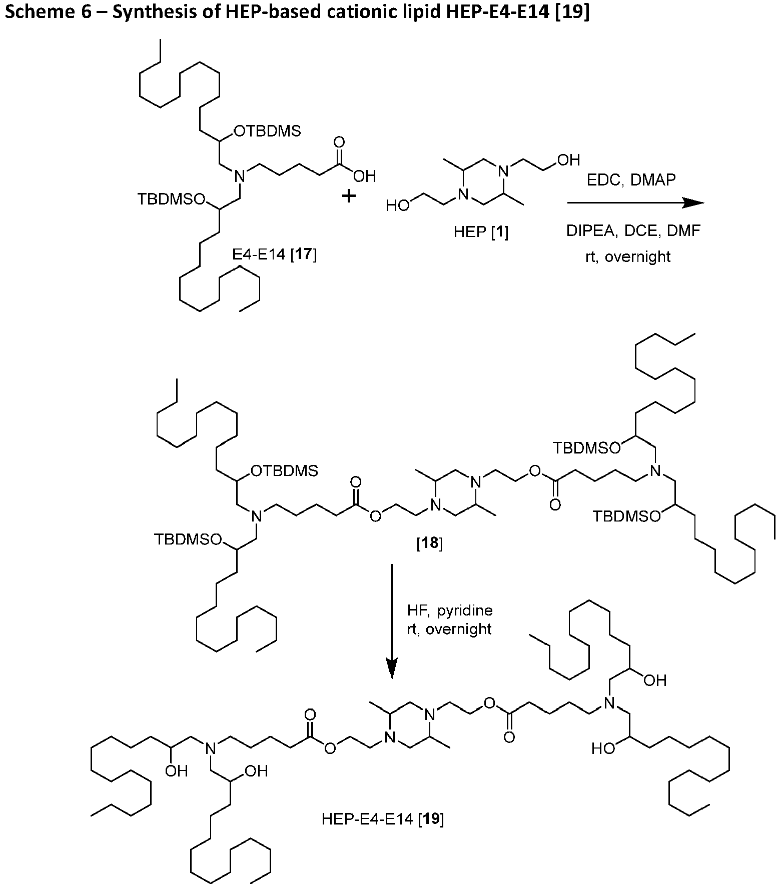

### Synthesis of [18]

As set out in Scheme 6: To a solution of HEP [1] (0.100 g, 0.494 mmol, 1.0 eq), E4-E14 [17] (0.799 g, 1.038 mmol, 2.1 eq), 1ml of dimethylformamide, 3ml of dichloroethane, diisopropylethylamine (0.344 µL, 1.98 mmol, 4.0 eq), and N,N-Dimethylaminopyridine (0.024 g, 0.198 mmol, 0.4 eq) was added 1-Ethyl-3-(3-dimethylaminopropyl)carbodiimide (0.285 g, 1.48 mmol, 3.0 eq) and allowed to react at room temperature overnight (18hr). Afterwards, the reaction mixture was concentrated using a rotavapor and purified using a Buchi Combi-flash system on 12g, 40µm-sized silica gel columns using hexanes/ethyl acetate as the mobile phase, yielding a colorless oil (70.1% yield).

### Synthesis of HEP-E4-E14 [19]

As set out in Scheme 6: To a 20 ml Polypropylene scintillation vial equipped with a PTFE stir-bar was added [18] (0.591 g, 0.346 mmol, 1.0 eq) along with 4ml of dry tetrahydrofuran. The vial was cooled to 0-5°C on an ice bath and HF/pyridine (1.77 ml, 68.322 mmol, 197.3 eq) was added dropwise. After addition, the reaction vial was allowed to warm to room temperature and stirred overnight (18hr). Afterwards, the reaction mixture was neutralized with saturated sodium bicarbonate at 0°C. Ethyl acetate was used for extraction (3x). The organic layers were combined, washed with saturated sodium chloride (4x), dried with sodium sulfate, filtered, and rotovaped to yield an off-yellow oil. This oil was further purified using a Buchi Combi-flash system on 12g, 40µm-sized silica gel columns using dichloromethane/methanol (3% methanol) as the mobile phase, yielding a colorless oil (50% yield). 1H NMR (400 MHz, CDCl3) 4.16 (m, 4H), 3.63 (m, 4H), 2.95 (m, 3H), 2.75 (d, 4H), 2.57 (m, 8H), 2.41 (m, 8H), 2.32 (t, 4H), 2.13 (t, 2H), 1.59 (m, 4H), 1.46 (m, 9H), 1.38 (m, 9H), 1.25 (br, 82H), 1.03 (d, 6H), 0.87 (t, 12H), 13C NMR (400 MHz, CDCl3) 171.77 (2C), 69.66 (3C), 67.25 (2C), 63.12 (3C), 61.42 (2C), 60.90 (2C), 55.90 (2C), 55.57 (2C), 55.14 (1C), 53.61 (1C), 51.15 (2C), 35.36 (5C), 34.21 (8C), 29.89 (48C), 26.93 (2C), 25.85 (5C), 23.28 (10C), 17.77 (3C), 14.31 (8C), Expected M/Z = 1250.07, Observed = 1250.01.

HEP-based cationic lipids described herein may be prepared according to **Scheme 7:**

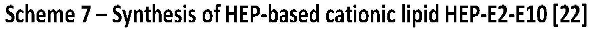

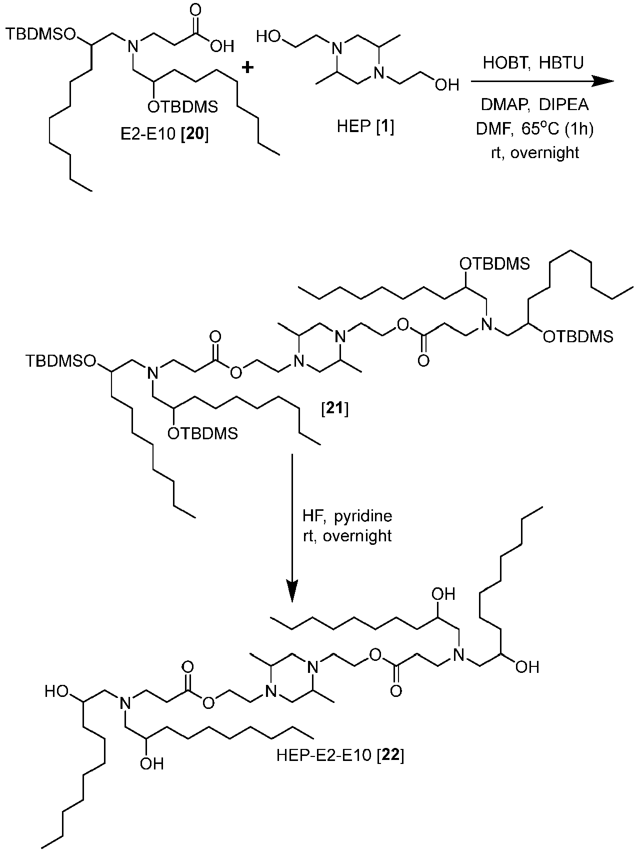

### Synthesis of [21]

As set out in Scheme 7: To a solution of HEP [1] (0.12 g, 0.59 mmol, 1.0 eq), E2-E10 [20] (0.822 g, 1.31 mmol, 2.2 eq), HOBT (0.240 g, 1.78 mmol, 3.0 eq), DMAP (0.022 g, 0.178 mmol, 0.3 eq), DIPEA (1.03 ml, 5.93 mmol, 10.0 eq) and 12ml of dimethylformamide, was added HBTU (0.675 g, 1.78 mmol, 3.0 eq) and allowed to stir at 65°C for 1 hour then at room temperature overnight. Afterwards, the reaction mixture was diluted with ethyl acetate and extracted with saturated sodium chloride (3x), dried with sodium sulfate, filtered, and rotovaped to yield an amber oil. This amber oil was purified using a Buchi Combi-flash system on 12g, 40µm-sized silica gel columns using hexanes/ethyl acetate as the mobile phase, yielding a colorless oil (53.5% yield).

### Synthesis of HEP-E2-E10 [22]

As set out in Scheme 7: To a 20 ml Polypropylene scintillation vial equipped with a PTFE stir-bar was added [21] (0.450 g, 0.315 mmol, 1.0 eq) along with 4ml of dry tetrahydrofuran. The vial was cooled to 0-5°C on an ice bath and HF/pyridine (1.62 ml, 62.25 mmol, 197.3 eq) was added dropwise. After addition, the reaction vial was allowed to warm to room temperature and stirred overnight (18hr). Afterwards, the reaction mixture was neutralized with saturated sodium bicarbonate at 0°C. Ethyl acetate was used for extraction (3x). The organic layers were combined, washed with saturated sodium chloride (4x), dried with sodium sulfate, filtered, and rotovaped to yield an off-yellow oil. This oil was further purified using a Buchi Combi-flash system on 12g, 40µm-sized silica gel columns using dichloromethane/methanol (3% methanol) as the mobile phase, yielding a colorless oil (12.0% yield). Expected M/Z = 969.53, Observed = 969.8

HEP-based cationic lipids described herein may be prepared according to **Scheme 8:**

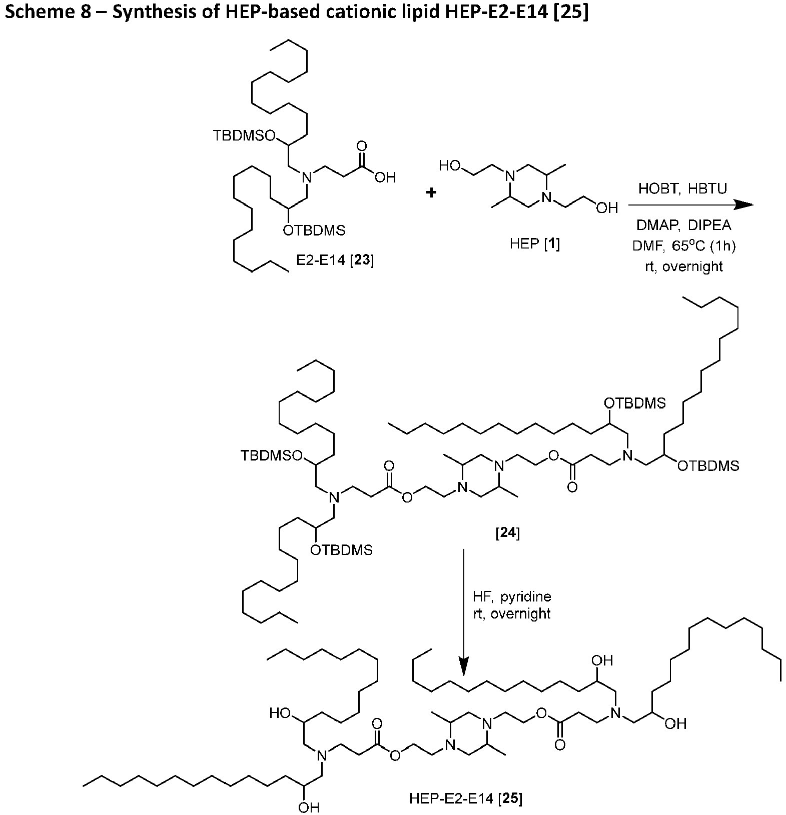

### Synthesis of [24]

As set out in Scheme 8: To a solution of HEP [1] (0.150 g, 0.74 mmol, 1.0 eq), E2-E14 [23] (0.840 g, 1.63 mmol, 2.2 eq), HOBT (0.300 g, 2.22 mmol, 3.0 eq), DMAP (0.027 g, 0.222 mmol, 0.3 eq), DIPEA (1.30 ml, 7.40 mmol, 10.0 eq) and 10 ml of dimethylformamide, was added HBTU (0.840 g, 2.22 mmol, 3.0 eq) and allowed to stir at 65°C for 1 hour then at room temperature overnight. Afterwards, the reaction mixture was diluted with ethyl acetate and extracted with saturated sodium chloride (3x), dried with sodium sulfate, filtered, and rotovaped to yield an amber oil. This amber oil was purified using a Buchi Combi-flash system on 12g, 40µm-sized silica gel columns using hexanes/ethyl acetate as the mobile phase, yielding a colorless oil (18.0% yield).

### Synthesis of HEP-E2-E14 [25]

As set out in Scheme 8: To a 20 ml Polypropylene scintillation vial equipped with a PTFE stir-bar was added [24] (0.150 g, 0.091 mmol, 1.0 eq) along with 4ml of dry tetrahydrofuran. The vial was cooled to 0-5°C on an ice bath and HF/pyridine (0.465 ml, 17.93 mmol, 197.3 eq) was added dropwise. After addition, the reaction vial was allowed to warm to room temperature and stirred overnight (18hr). Afterwards, the reaction mixture was neutralized with saturated sodium bicarbonate at 0°C. Ethyl acetate was used for extraction (3x). The organic layers were combined, washed with saturated sodium chloride (4x), dried with sodium sulfate, filtered, and rotovaped to yield an off-yellow oil. This oil was further purified using a Buchi Combi-flash system on 12g, 40µm-sized silica gel columns using dichloromethane/methanol (3% methanol) as the mobile phase, yielding a colorless oil (10.0% yield). Expected M/Z = 1193.96, Observed = 1193.0

HEP-based cationic lipids described herein may be prepared according to **Scheme 9:**

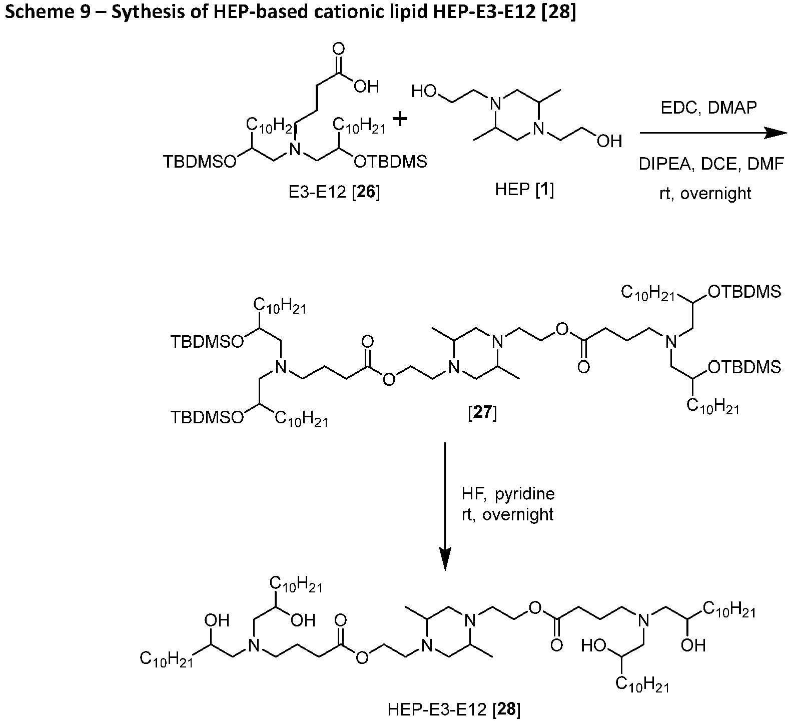

### Synthesis of [27]

As set out in Scheme 9: To a solution containing HEP [**1**] (0.200 g, 0.988 mmol, 1.0 eq), E3-E12 [**26**] (1.6 g, 2.27 mmol, 2.3 eq), 20 ml of dichloroethane, diisopropylethylamine (0.860 mL, 4.94 mmol, 5.0 eq), and N,N-Dimethylaminopyridine (0.036 g, 0.296 mmol, 0.3 eq) was added 1-Ethyl-3-(3-dimethylaminopropyl)carbodiimide (0.568 g, 2.96 mmol, 3.0 eq) and allowed to react at room temperature overnight (18hr). Afterwards, the reaction mixture was concentrated using a rotavapor and purified using a Buchi Combi-flash system on 12g, 40µm-sized silica gel columns using hexanes/ethyl acetate as the mobile phase, yielding a light-yellow oil (0.95 g, 61% yield). Calculated C₉₀H₁₈₉N₄O₈Si₄ [M+H] = 1565.35, Observed [M+H] = 1566.10.

### Synthesis of HEP-E3-E12 [28]

As set out in Scheme 9: To a 20 ml Polypropylene scintillation vial equipped with a PTFE stir-bar was added [**27**] (0.950 g, 0.607 mmol, 1.0 eq) along with 8 ml of dry tetrahydrofuran. The vial was cooled to 0-5°C on an ice bath and HF/pyridine (3.4 ml, 121.4 mmol, 200 eq) was added dropwise. After addition, the reaction vial was allowed to warm to room temperature and stirred overnight (18hr). Afterwards, the reaction mixture was neutralized with saturated sodium bicarbonate at 0°C. Ethyl acetate was used for extraction (3x). The organic layers were combined, washed with saturated sodium chloride (4x), dried with sodium sulfate, filtered, and rotovaped to yield an off-yellow oil. This oil was further purified using a Buchi Combi-flash system on 12g, 40µm-sized silica gel columns using dichloromethane/methanol (3% methanol) as the mobile phase, yielding a colorless oil (381 mg, 56.6% yield). ¹H NMR (400 MHz, CDCl₃): 4.14-4.20 (m, 4H), 3.63-3.66 (m, 4H), 2.95-3.00 (m, 3H), 2.77-2.80 (dd, 2H), 2.33-2.65 (m, 20H), 2.14-2.20 (m, 2H), 1.78-1.83 (m, 4H), 1.25-1.46 (m, 76H), 1.04-1.05 (d, 6H), 0.86-0.89 (m, 12H), Calculated C₆₆H₁₃₃N₄O₈, M/Z= 1109.0, Observed = 1109.8.

HEP-based cationic lipids described herein may be prepared according to **Scheme 10:**

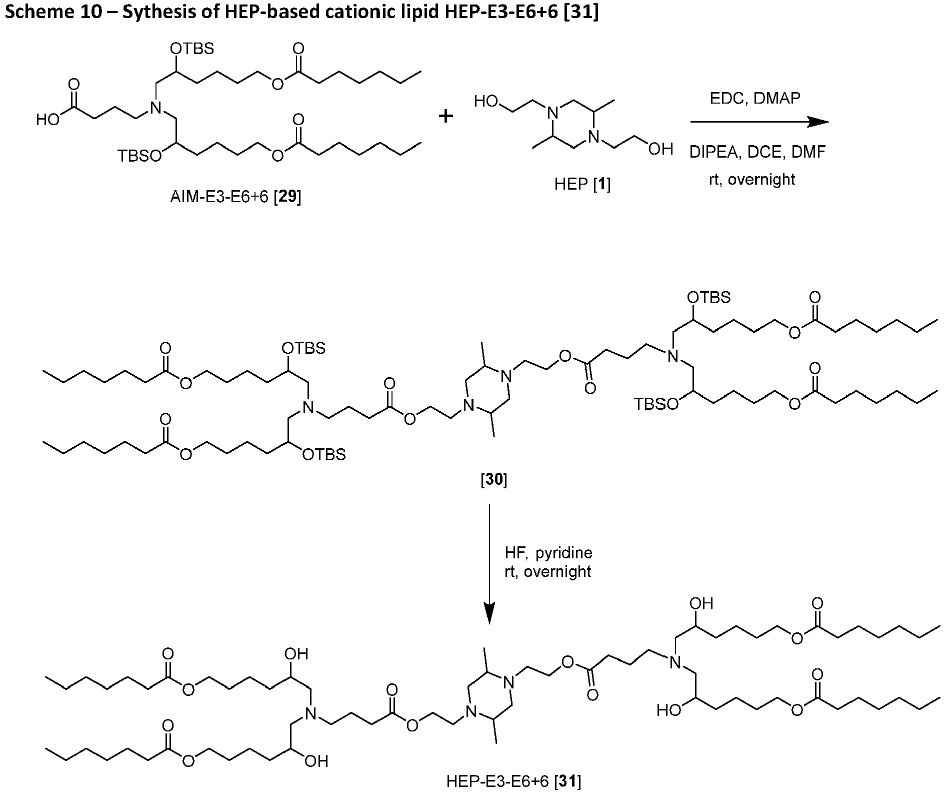

### Synthesis of [30]

As set out in Scheme 10: To a containing HEP [1] (0.100 g, 0.49 mmol, 1.0 eq), AIM-E3-E6+6 [29] (0.86 g, 1.09 mmol, 2.2 eq), 10 ml of dichloroethane, diisopropylethylamine (0.86 mL, 4.94 mmol, 10.0 eq), and N,N-Dimethylaminopyridine (0.06 g, 0.49 mmol, 1.0 eq) was added 1-Ethyl-3-(3-dimethylaminopropyl)carbodiimide (0.237 g, 1.23 mmol, 2.5 eq) and allowed to react at room temperature overnight (18hr). Afterwards, the reaction mixture was concentrated using a rotavapor and purified using a Buchi Combi-flash system on 12g, 40µm-sized silica gel columns using hexanes/ethyl acetate as the mobile phase, yielding a light-yellow oil (0.43 g, 50% yield). Calculated C94H189N4O16Si4 [M+H] = 1743.90, Observed = 1743.10.

### Synthesis of HEP-E3-E6+6 [31]

As set out in Scheme 10: To a 20 ml Polypropylene scintillation vial equipped with a PTFE stir-bar was added [**30**] (0.430 g, 0.247 mmol, 1.0 eq) along with 4 ml of dry tetrahydrofuran. The vial was cooled to 0-5°C on an ice bath and HF/pyridine (2.4 ml, 49.36 mmol, 100 eq) was added dropwise. After addition, the reaction vial was allowed to warm to room temperature and stirred overnight (18hr). Afterwards, the reaction mixture was neutralized with saturated sodium bicarbonate at 0°C. Ethyl acetate was used for extraction (3x). The organic layers were combined, washed with saturated sodium chloride (4x), dried with sodium sulfate, filtered, and roto vaped to yield an off-yellow oil. This oil was further purified using a Buchi Combi-flash system on 12g, 40µm-sized silica gel columns using dichloromethane/methanol (3% methanol) as the mobile phase, yielding a colorless oil (230 mg, 72.5 % yield). ¹H NMR (400 MHz, CDCl₃): 4.15-4.18 (m, 4H), 4.04-4.07 (t, 8H), 3.62-3.66 (m, 4H), 2.94-3.04 (m, 2H), 2.76-2.80 (m, 2H), 2.32-2.64 (m, 20H), 2.26-2.30 (t, 8H), 2.12-2.20 (m, 2H), 1.17-1.83 (m, 4H), 1.53-1.66 (m, 21H), 1.39-1.43 (m, 12H), 1.26-1.32 (m, 26H), 1.03-1.06 (d, 6H), 0.86-0.89 (m, 12H). Expected C₇₀H₁₃₂N₄O₁₆ M/Z = 1285.8, Observed = 1285.9.

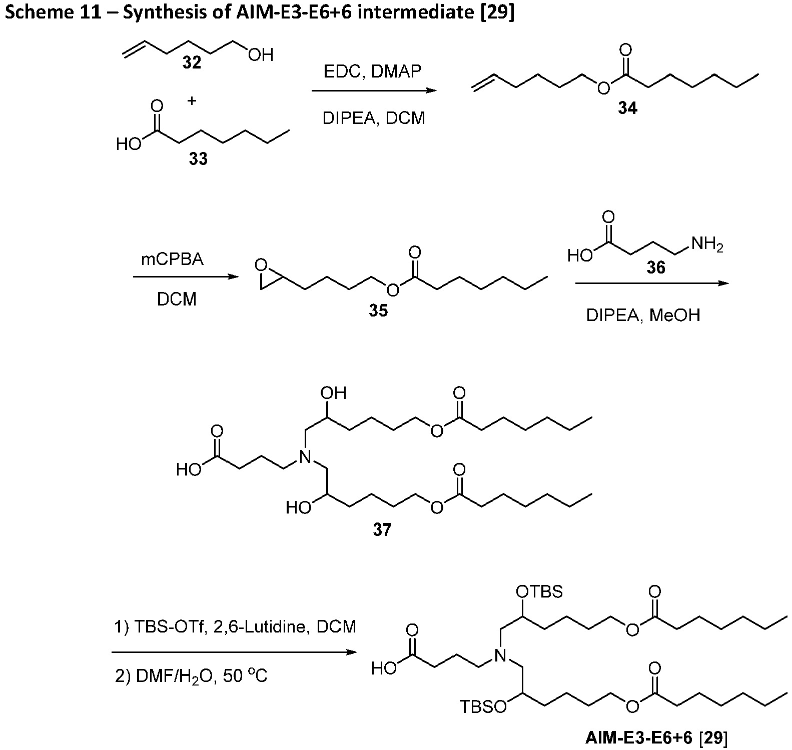

### Synthesis of hex-5-en-1-yl heptanoate (34)

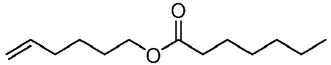

As set out in Scheme 11: To a solution of hex-5-en-1-ol (**32**) (20 g, 199.6 mmol) and heptanoic acid (**33**) (33.9 mL, 239.6 mmol) in 400 mL of dichloromethane were added DMAP (4.9 g, 39.9 mmol), DIPEA (104.3 mL, 599.0 mmol) and EDC (57.4 g, 299.5 mmol). The resulting mixture was stirred at room temperature for overnight. MS and TLC (Rf: 0.6, 10% EtOAc/hexanes) analysis indicated completion of the reaction. Then reaction mixture was diluted with DCM and washed with sat. NaHCO₃ solution, water and brine solution. The organic layer was dried over anhydrous Na₂SO₄ and concentrated. The crude residue was purified (0-1% ethyl acetate in hexane) to get hex-5-en-1-yl heptanoate (**34**) (34.3 g, 81%).

### Results:

ESI-MS analysis: Calculated C13H25O2, [M+H] = 213.19, Observed = 213.3

### Synthesis of 4-(oxiran-2-yl)butyl heptanoate (35)

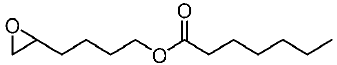

As set out in Scheme 11: To a solution of hex-5-en-1-yl heptanoate (**34**) (5.0 g, 23.5 mmol) in 50 mL of dichloromethane was added 3-chloroperbenzoic acid (6.09 g, 35.3 mmol) at 0 °C. The resultant mixture was warmed to room temperature and stirred for overnight. MS and TLC (Rf: 0.3, 10% EtOAc/hexanes) analysis indicated completion of the reaction. Then reaction mixture was diluted with dichloromethane and washed with 10% sodium hydroxide solution and water. The organic layer was separated and dried over anhydrous sodium sulfate and concentrated. The crude residue was purified (SiO₂: 4-5% ethyl acetate in hexane gradient) to obtain 4-(oxiran-2-yl)butyl heptanoate (**35**) (4.5 g, 84%). It was confirmed by MS analysis.

### Results:

ESI-MS analysis: Calculated C13H25O3, [M+H] = 229.18, Observed = 229.2

### Synthesis of 4-(bis(6-(heptanoyloxy)-2-hydroxyhexyl)amino)butanoic acid (37)

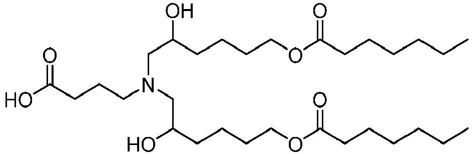

As set out in Scheme 11: To a solution of 4-aminobutanoic acid (**36**) (1.15 g, 11.15 mmol), 4-(oxiran-2-yl)butyl heptanoate (**35**) (5.09 g, 22.3 mmol) and diisopropylethylamine (4.85 mL, 27.88 mmol) in 75 mL methanol was heated at 75 °C for 4 hours. After concentrated to dryness, the oily residue was purified by column chromatography (SiO₂: 7-8% methanol in dichloromethane gradient) to obtain 4-(bis(6-(heptanoyloxy)-2-hydroxyhexyl)amino)butanoic acid (**37**) (3.4 g, 54%). It was confirmed by MS analysis.

### Results:

ESI-MS analysis: Calculated C30H58NO8, [M+H] = 560.42, Observed = 560.3

### Synthesis of AIM-E3-E6+6 (29)

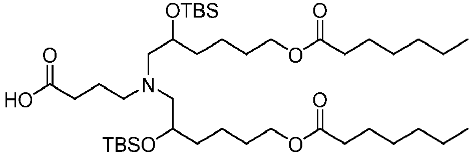

As set out in Scheme 11: To a solution of 4-(bis(6-(heptanoyloxy)-2-hydroxyhexyl)amino)butanoic acid (**37**) (2.0 g, 3.57 mmol) in 40 mL of dichloromethane were added 2,6-lutidine (1.25 mL, 10.71 mmol) and tert-butyldimethylsilyl trifluoromethanesulfonate (1.97 mL, 8.57 mmol) at 0 °C. The resultant mixture was warmed to room temperature and stirred for 1 hour. MS analysis indicated completion of the reaction and formation of di and tri-TBS products. The reaction mixture was diluted with DCM and washed with sat. NaHCO₃ solution, water and brine solution. The organic layer was dried over anhydrous Na₂SO₄. After concentrated to dryness, the oily residue was dissolved in DMF/H₂O (10 mL/1 mL) and the resulting solution was heated at 50 °C for 6 hours. MS analysis indicated completion of the reaction. Then reaction mixture was diluted with DCM and washed with sat. NH₄Cl solution, water and brine solution. The organic layer was dried over anhydrous Na₂SO₄ and concentrated. The crude residue was purified by column chromatography (SiO₂: 36-40% ethyl acetate in hexane gradient) to obtain **AIM-E3-E6+6** (**29,** 1.54 g, 55% for two steps). It was confirmed by MS analysis.

### Results:

ESI-MS analysis: Calculated C42H86NO8Si2, [M+H] = 788.59, Observed = 788.3

### Examples 12-13: Synthesis of TEP thioester based cationic lipids

TEP-based cationic lipids described herein may be prepared according to **Scheme 12:**

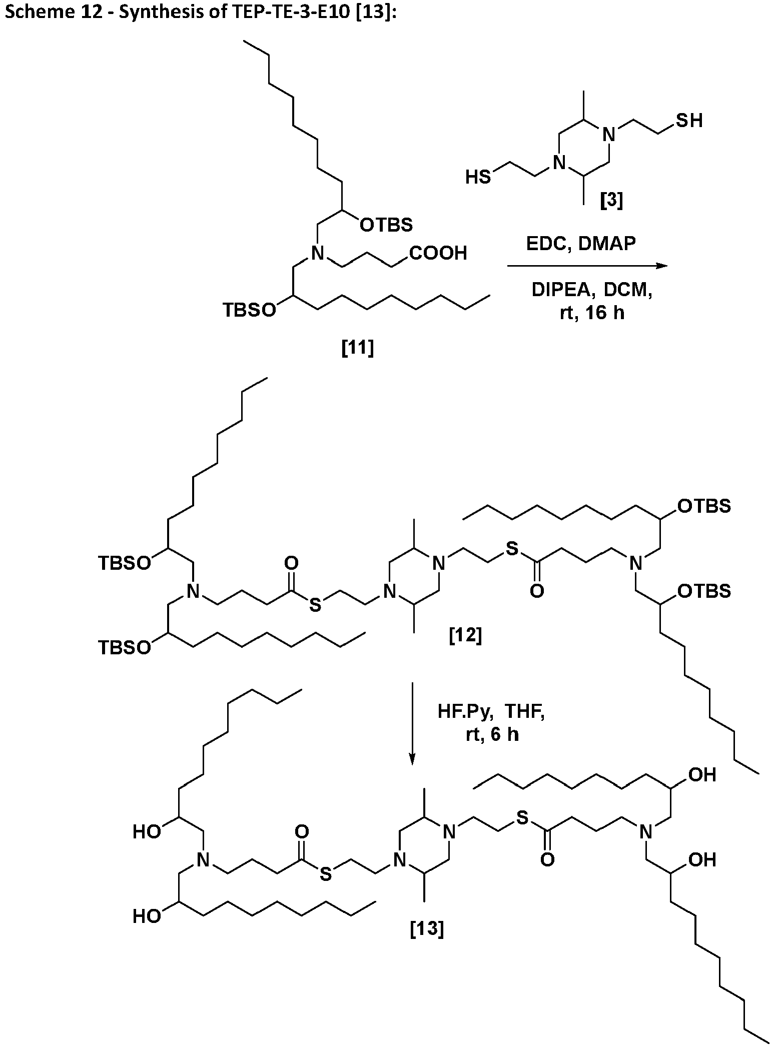

### TEP-TE-3-E10-TBS [12]:

As set out in Scheme 12: To a solution of [3] (0.125 g, 0.534 mmol) in DCM (2 mL) were added [11] (0.722 g, 1.121 mmol) in DCM (2 mL), EDC (0.307 g, 1.602 mmol), DMAP (0.026 g, 0.213 mmol), DIPEA (0.37 mL, 2.135 mmol) and stirred at room temperature for 16 hours. After completion of the reaction as monitored by TLC and MS. The reaction mixture was diluted with DCM (50 mL) washed with NaHCO3 solution, water and brine. The organic layer was dried over anhydrous Na2SO4, concentrated, and the crude compound was purified by silica gel chromatography (eluent: 4% EtOAc in hexanes) to obtain pure compound [12] as a pale-yellow oil (0.44 g, 55%). It was confirmed by MS analysis.

### Results:

ESI-MS analysis: Calculated C82H173N4O6S2Si4, [M+H] = 1486.1, Observed = 1486.0

### TEP-TE-3-E10 [13]:

As set out in Scheme 12: To a solution of [12] (0.435 g, 0.293 mmol) in THF (4 mL) was slowly added HF.Py (70% HF) (1.67 mL, 58.55 mmol) at 0 °C and stirred for 5 minutes. Then reaction mixture was brought to room temperature and stirred for 6 hours. After completion of the reaction as monitored by TLC and MS. The reaction mixture was concentrated, and the obtained residue was dissolved in ethyl acetate, washed with NaHCO3 solution, water and brine. The organic layer was dried over anhydrous Na2SO4, concentrated, and the crude compound was purified by silica gel chromatography (eluent: 3% MeOH in DCM) to obtain pure compound [13] as a colorless oil (0.16 g, 53%). It was confirmed by NMR and MS analysis.

### Results:

1H NMR (500 MHz, CDCl3) δ 3.70 - 3.58 (m, 4H), 3.07 - 2.73 (m, 10H), 2.66 - 2.36 (m, 19H), 2.20 - 2.11 (m, 1H), 1.87 - 1.77 (m, 4H), 1.50 - 1.34 (m, 12H), 1.34 - 1.21 (m, 48H), 1.07 (d, 6H), 0.87 (t, 12H).

13C NMR (100 MHz, CDCl3) δ 199.5, 199.3, 69.7, 68.0, 62.7, 61.2, 59.9, 55.2, 55.0, 54.3, 52.5, 41.7, 41.7, 35.3, 35.2, 32.1, 30.0, 29.8, 29.5, 25.9, 25.8, 25.3, 23.2, 23.0, 22.9, 17.6, 14.3.

ESI-MS analysis: Calculated for C58H117N4O6S2, [M+H] = 1029.8; Observed = 1029.7

TEP-based cationic lipids described herein may be prepared according to **Scheme 13:**

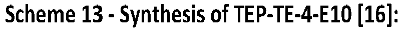

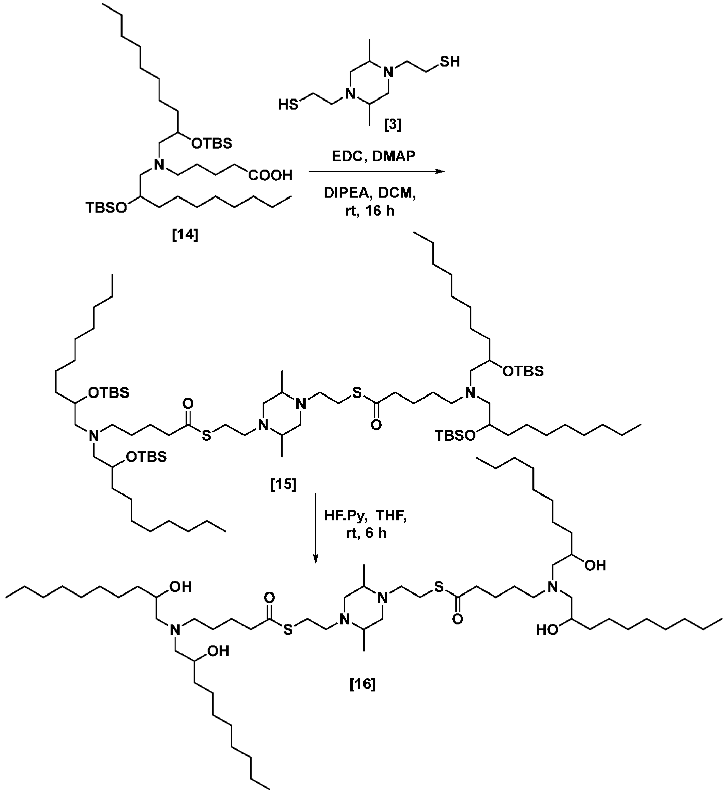

### TEP-TE-4-E10-TBS [15]:

As set out in Scheme 13: To a solution of [3] (0.125 g, 0.534 mmol) in DCM (2 mL) were added [14] (0.737 g, 1.121 mmol) in DCM (2 mL), EDC (0.307 g, 1.602 mmol), DMAP (0.026 g, 0.213 mmol), DIPEA (0.37 mL, 2.135 mmol) and stirred at room temperature for 16 hours. After completion of the reaction as monitored by TLC and MS. The reaction mixture was diluted with DCM (50 mL) washed with NaHCO3 solution, water and brine. The organic layer was dried over anhydrous Na2SO4, concentrated, and the crude compound was purified by silica gel chromatography (eluent: 4% EtOAc in hexanes) to obtain pure compound [15] as a pale-yellow oil (0.495 g, 61%). It was confirmed by MS analysis.

### Results:

ESI-MS analysis: Calculated C84H177N4O6S2Si4, [M+H] = 1514.2, Observed = 1514.0

### TEP-TE-4-E10 [16]:

As set out in Scheme 13: To a solution of [12] (0.49 g, 0.323 mmol) in THF (4 mL) was slowly added HF.Py (70% HF) (1.85 mL, 64.73 mmol) at 0 °C and stirred for 5 minutes. Then reaction mixture was brought to room temperature and stirred for 6 hours. After completion of the reaction as monitored by TLC and MS. The reaction mixture was concentrated, and the obtained residue was dissolved in ethyl acetate, washed with NaHCO3 solution, water and brine. The organic layer was dried over anhydrous Na2SO4, concentrated, and the crude compound was purified by silica gel chromatography (eluent: 3% MeOH in DCM) to obtain pure compound [16] as a colorless oil (0.166 g, 49%). It was confirmed by NMR and MS analysis.

### Results:

1H NMR (500 MHz, CDCl3) δ 3.70 - 3.58 (m, 4H), 3.07 - 2.73 (m, 10H), 2.64 - 2.35 (m, 19H), 2.20 - 2.12 (m, 1H), 1.75 - 1.58 (m, 4H), 1.57 - 1.35 (m, 16H), 1.34 - 1.17 (m, 48H), 1.07 (d, 6H), 0.87 (t, 12H).

13C NMR (100 MHz, CDCl3) δ 199.4, 199.3, 69.6, 68.0, 62.8, 61.4, 59.9, 55.8, 55.1, 55.0, 53.6, 52.6, 43.9, 43.8, 35.3, 35.2, 32.1, 30.0, 29.9, 29.8, 29.5, 26.5, 25.9, 25.8, 25.2, 23.49, 23.4, 22.9, 17.6, 14.3.

ESI-MS analysis: Calculated for C60H121N406S2, [M+H] = 1057.8; Observed = 1057.8

### Examples 14-15: Synthesis of TEP disulfide based cationic lipids

TEP-based cationic lipids described herein may be prepared according to **Scheme 14:**

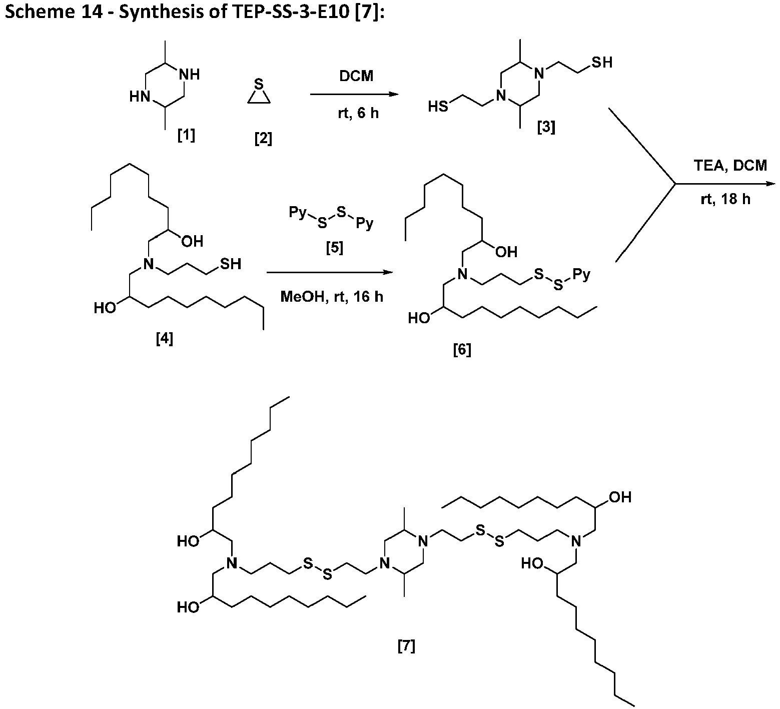

### 2,2'-(2,5-dimethylpiperazine-1,4-diyl)bis(ethane-1-thiol) [3]:

As set out in Scheme 14: To a solution of 2,5-dimethylpiperazine [1] (3.0 g, 26.27 mmol) in DCM (60 mL) was slowly added ethylene sulfide [2] (6.25 mL, 105.08 mmol) in portions. The resulting mixture was stirred at room temperature for 6 hours. After completion of the reaction as monitored by MS. The reaction mixture was concentrated to obtain the compound [3] as a pale-yellow solid (6.1 g, 99%). It was confirmed by MS analysis.

### Results:

ESI-MS analysis: Calculated for C10H23N2S2, [M+H] = 235.1; Observed = 235.2

### 1,1'-((3-(pyridin-2-yldisulfaneyl)propyl)azanediyl)bis(decan-2-ol) [6]:

As set out in Scheme 14: To a solution of [5] (0.027 g, 0.12 mmol) in MeOH (1.5 mL) was added [4] (0.050 g, 0.12 mmol) in MeOH (1.5 mL). The resulting mixture was stirred at room temperature for 18 hours. After completion of the reaction as monitored by TLC and MS. The reaction mixture was concentrated, and the crude compound was purified by silica gel chromatography (eluent: 40% EtOAc in DCM) to obtain pure compound [6] as a pale-yellow oil (0.030 g, 48%). It was confirmed by MS analysis.

### Results:

ESI-MS analysis: Calculated for C28H53N2O2S2, [M+H] = 513.3; Observed = 513.3

### TEP-SS-3-E10 [7]:

As set out in Scheme 14: To a solution of [3] (0.030 g, 0.128 mmol) in DCM (2 mL) was added triethylamine (53 mL, 0.384 mmol) and stirred at room temperature for 3 minutes. To that was added [6] (0.144 g, 0.281 mmol) in DCM (3 mL) and stirred at room temperature for 18 hours. After completion of the reaction as monitored by TLC and MS. The reaction mixture was concentrated, and the crude compound was purified by silica gel chromatography (eluent: 5% to 10% MeOH in DCM) to obtain pure compound [6] as a pale-yellow oil (0.013 g, 59%). It was confirmed by 1H NMR and MS analysis.

1H NMR (400 MHz, Chloroform-d) δ 3.87 - 3.68 (m, 4H), 3.20 - 2.38 (m, 25H), 2.31 - 1.86 (m, 5H), 1.66 - 1.37 (m, 12H), 1.37 - 1.17 (m, 48H), 1.13 (d, 6H), 0.87 (t, 12H).

ESI-MS analysis: Calculated C56H117N4O4S4, [M+H] = 1037.8, Observed = 1037.7

TEP-based cationic lipids described herein may be prepared according to **Scheme 15:**

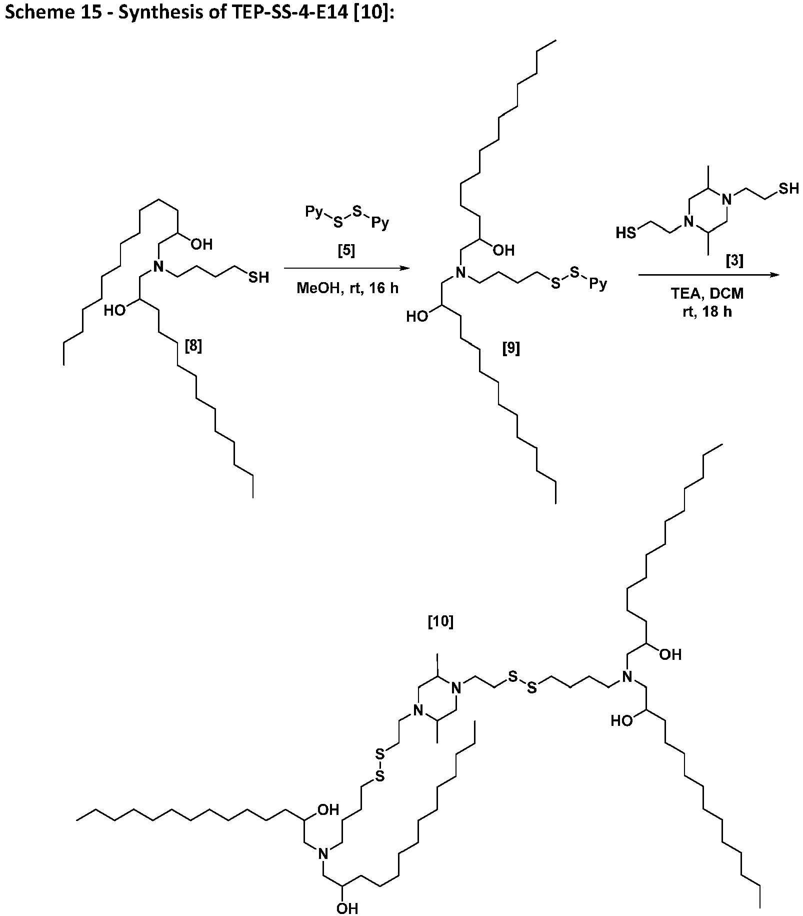

### 1,1'-((4-(pyridin-2-yldisulfaneyl)butyl)azanediyl)bis(tetradecan-2-ol) [9]:

As set out in Scheme 15: To a solution of [5] (0.38 g, 1.75 mmol) in MeOH (15 mL) was added [8] (0.93 g, 1.75 mmol) in MeOH (15 mL). The resulting mixture was stirred at room temperature for 16 hours. After completion of the reaction as monitored by TLC and MS. The reaction mixture was concentrated, and the crude compound was purified by silica gel chromatography (eluent: 30%- 40% EtOAc in DCM) to obtain pure compound [9] as a pale-yellow oil (0.59 g, 53%). It was confirmed by MS analysis.

### Results:

ESI-MS analysis: Calculated for C37H71N2O2S2, [M+H] = 639.5; Observed = 639.5

### TEP-SS-4-E14 [10]:

As set out in Scheme 15: To a solution of [3] (0.1 g, 0.43 mmol) in DCM (6 mL) was added triethylamine (178 mL, 1.28 mmol) and stirred at room temperature for 3 minutes. To that was added [9] (0.6 g, 0.94 mmol) in DCM (9 mL) and stirred at room temperature for 18 hours. After completion of the reaction as monitored by TLC and MS. The reaction mixture was concentrated, and the crude compound was purified by silica gel chromatography (eluent: 4-5% MeOH in DCM) to obtain pure compound [10] as a colorless oil (0.087 g, 16%). It was confirmed by 1H NMR and MS analysis.

1H NMR (400 MHz, Chloroform-d) δ 3.77 - 3.61 (m, 4H), 3.12 - 2.99 (m, 3H), 2.88 - 2.40 (m, 29H), 2.21 - 2.08 (m, 2H), 1.80 - 1.66 (m, 4H), 1.50 - 1.36 (m, 12H), 1.36 - 1.18 (m, 80H), 1.09 (d, 6H), 0.88 (t, 12H).

ESI-MS analysis: Calculated for C74H153N4O4S4, [M+H] = 1290.0; Observed = 1289.9

### Examples 16-23: Synthesis of further HEP based cationic lipids

Intermediates of HEP-based cationic lipids described herein may be prepared according to **Scheme 16:**

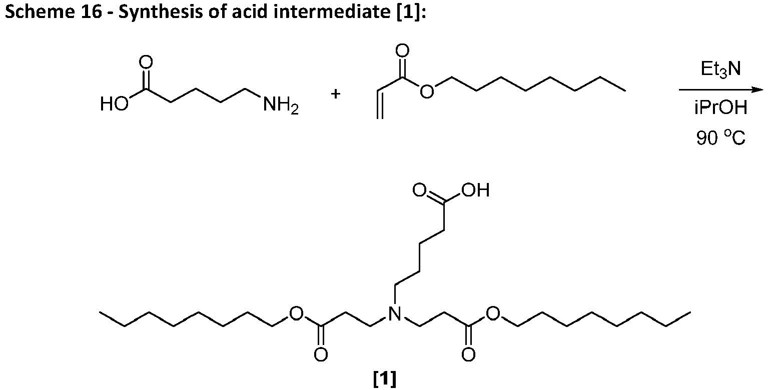

As set out in Scheme 16: To the solution of 5-aminovaleic acid (0.5 g, 4.27 mmol)) dissolved in i-PrOH (5 mL) and Et3N (1.2 mL) was added the n-octyl acrylate (2.7 ml, 12.8 mmol). The reaction was heated to 90°C for 3 h. After the completion of reaction, the crude mixture was evaporated under reduced pressure. Finally, the crude material was purified using silica gel column Chromatography (0-12% MeOH in CH2CI2) to obtain pure compound [1] as a colorless oil (0.56 g, 27%). Expected [M+H] = 486.4 Observed = 486.4

HEP-based cationic lipids described herein may be prepared according to **Scheme 17:**

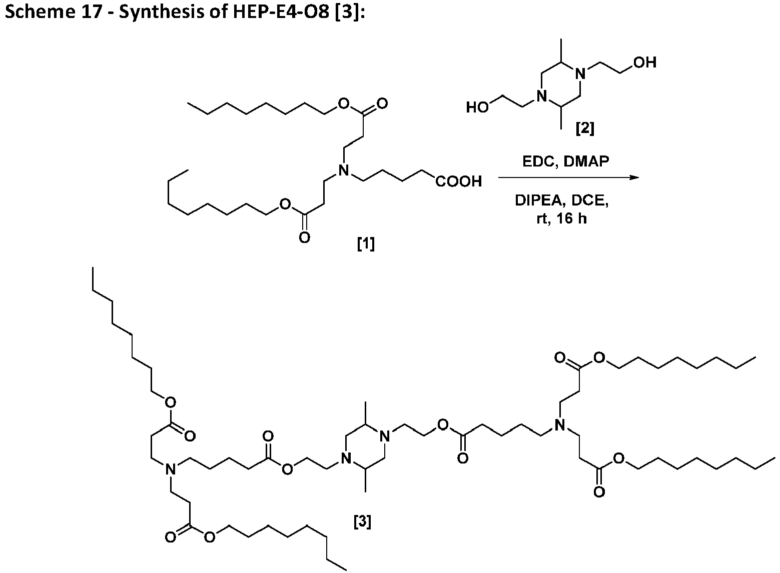

As set out in Scheme 17: To a solution of [2] (0.100 g, 0.494 mmol) in DCE (2 mL) were added [1] (0.530 g, 1.09 mmol) in DCE (8 mL), EDC (0.284 g, 1.48 mmol), DMAP (0.12 g, 0.99 mmol), DIPEA (0.86 mL, 4.94 mmol) and stirred at room temperature for 16 hours. After completion of the reaction as monitored by TLC and MS. The reaction mixture was diluted with DCM (50 mL) washed with NaHCO3 solution, water and brine. The organic layer was dried over anhydrous Na2SO4, concentrated, and the crude compound was purified by silica gel chromatography (eluent: 4% EtOAc in hexanes) to obtain pure compound [3] as a pale-yellow oil (0.15 g, 27%). It was confirmed by MS analysis.

### Results:

ESI-MS analysis: Calculated C64H121N4O12, [M+H] = 1137.90, Observed = 1137.85

Intermediates of HEP-based cationic lipids described herein may be prepared according to **Scheme 18:**

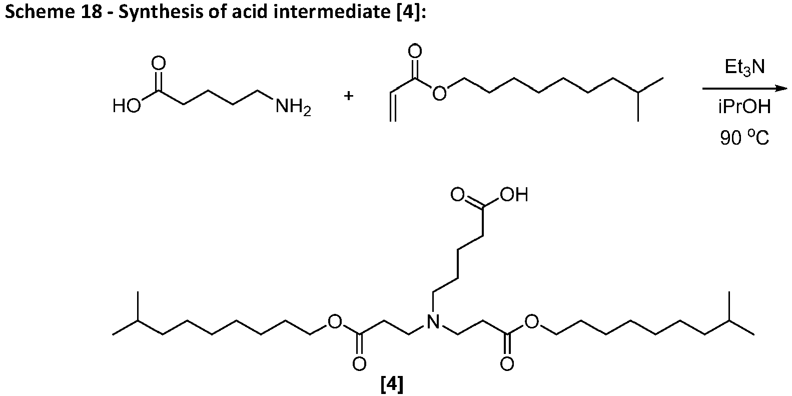

As set out in Scheme 18: To the solution of 5-aminovaleic acid (0.5g, 4.27 mmol)) dissolved in i-PrOH (5 mL) and Et3N (1.2 mL) was added the Isodecyl acrylate (3.12 mL, 12.8 mmol). The reaction was heated to 90°C for 3 h. After the completion of reaction, the crude mixture was evaporated under reduced pressure. Finally, the crude material was purified using silica gel column Chromatography (0-12% MeOH in CH2CI2) to obtain pure compound [4] as a colorless oil (0.420 g, 18%). Expected [M+H] = 542.4 Observed = 542.4

HEP-based cationic lipids described herein may be prepared according to **Scheme 19:**

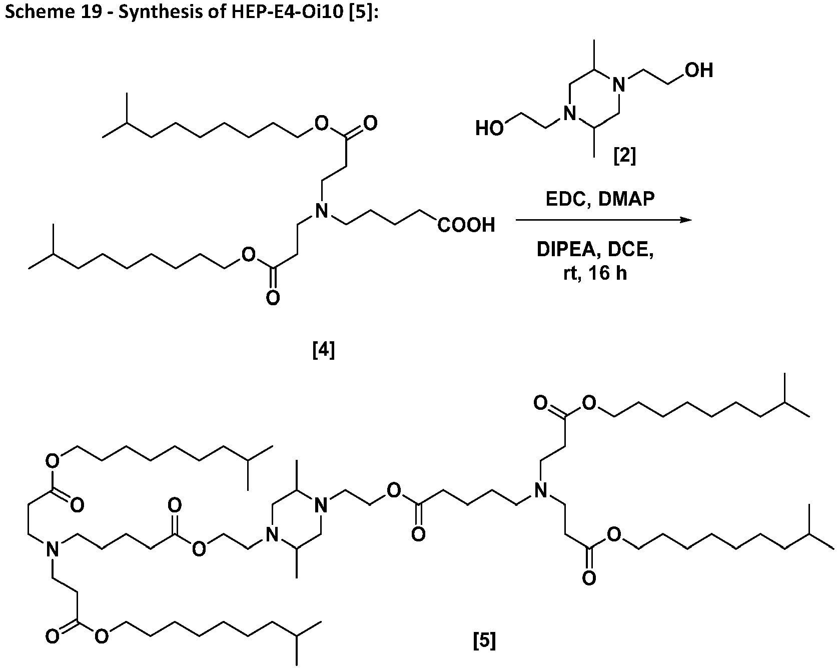

As set out in Scheme 19: To a solution of [2] (0.065 g, 0.321 mmol) in DCE (2 mL) were added [4] (0.382 g, 0.707 mmol) in DCE (6 mL), EDC (0.184 g, 0.964 mmol), DMAP (0.079 g, 0.642 mmol), DIPEA (0.56 mL, 3.21 mmol) and stirred at room temperature for 16 hours. After completion of the reaction as monitored by TLC and MS. The reaction mixture was diluted with DCM (50 mL) washed with NaHCO3 solution, water and brine. The organic layer was dried over anhydrous Na2SO4, concentrated, and the crude compound was purified by silica gel chromatography (eluent: 4% EtOAc in hexanes) to obtain pure compound [5] as a pale-yellow oil (95 mg, 24%). It was confirmed by MS analysis.

### Results:

ESI-MS analysis: Calculated C72H137N4O12, [M+H] = 1250.02, Observed = 1250.0

Intermediates of HEP-based cationic lipids described herein may be prepared according to **Scheme 20:**

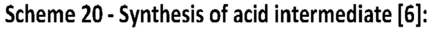

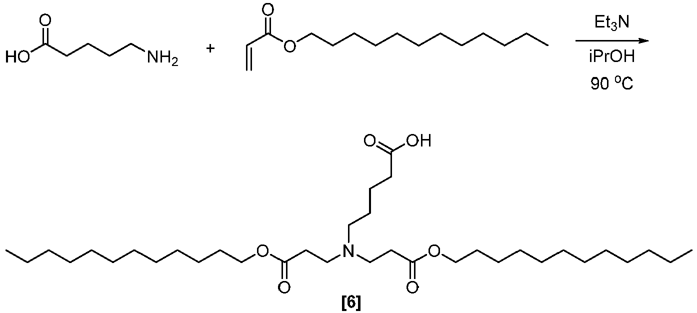

As set out in Scheme 20: To the solution of 5-aminovaleic acid (0.5g, 4.27 mmol)) dissolved in i-PrOH (5 mL) and Et3N (1.2 mL) was added the Tetradecyl acrylate (3.08 g, 12.8 mmol). The reaction was heated to 90°C for 3 h. After the completion of reaction, the crude mixture was evaporated under reduced pressure. Finally, the crude material was purified using silica gel column chromatography (0-12% MeOH in CH2CI2) to obtain pure compound [6] as a colorless oil (0.720 g, 28%). Expected [M+H] = 598.5 Observed = 598.5

HEP-based cationic lipids described herein may be prepared according to **Scheme 21:**

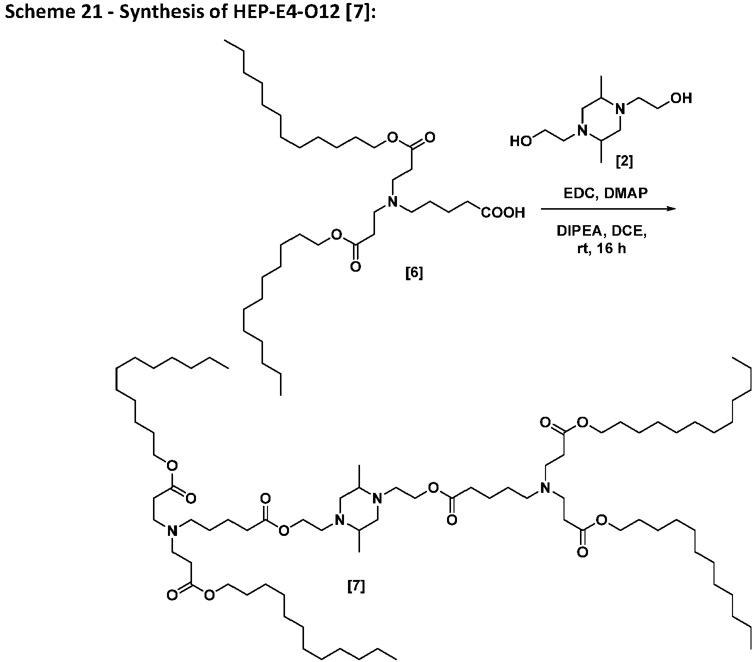

As set out in Scheme 21: To a solution of [2] (0.100 g, 0.494 mmol) in DCE (2 mL) were added [6] (0.650 g, 1.09 mmol) in DCE (8 mL), EDC (0.284 g, 1.48 mmol), DMAP (0.12 g, 0.99 mmol), DIPEA (0.86 mL, 4.94 mmol) and stirred at room temperature for 16 hours. After completion of the reaction as monitored by TLC and MS. The reaction mixture was diluted with DCM (50 mL) washed with NaHCO3 solution, water and brine. The organic layer was dried over anhydrous Na2SO4, concentrated, and the crude compound was purified by silica gel chromatography (eluent: 4% EtOAc in hexanes) to obtain pure compound [7] as a colorless oil (0.20 g, 30%). It was confirmed by MS analysis.

### Results:

ESI-MS analysis: Calculated C80H153N4O12, [M+H] = 1362.15, Observed = 1362.09

Intermediates of HEP-based cationic lipids described herein may be prepared according to **Scheme 22:**

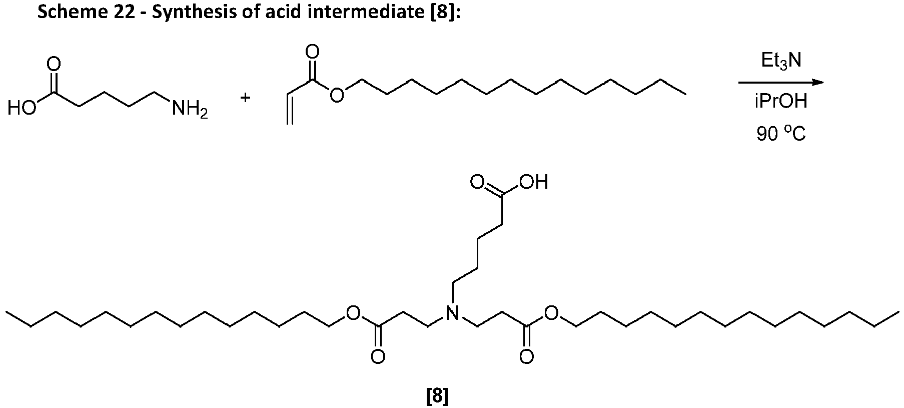

As set out in Scheme 22: To the solution of 5-aminovaleic acid (0.5g, 4.27 mmol)) dissolved in i-PrOH (5 mL) and Et3N (1.2 mL) was added the Tetradecyl acrylate (3.4 g, 12.8 mmol). The reaction was heated to 90°C for 3 h. After the completion of reaction, the crude mixture was evaporated under reduced pressure. Finally, the crude material was purified using silica gel column Chromatography (0-12% MeOH in CH2CI2) to obtain pure compound [8] as a colorless oil (0.680 g, 24%). Expected [M+H] = 654.6 Observed = 654.6

HEP-based cationic lipids described herein may be prepared according to **Scheme 23:**

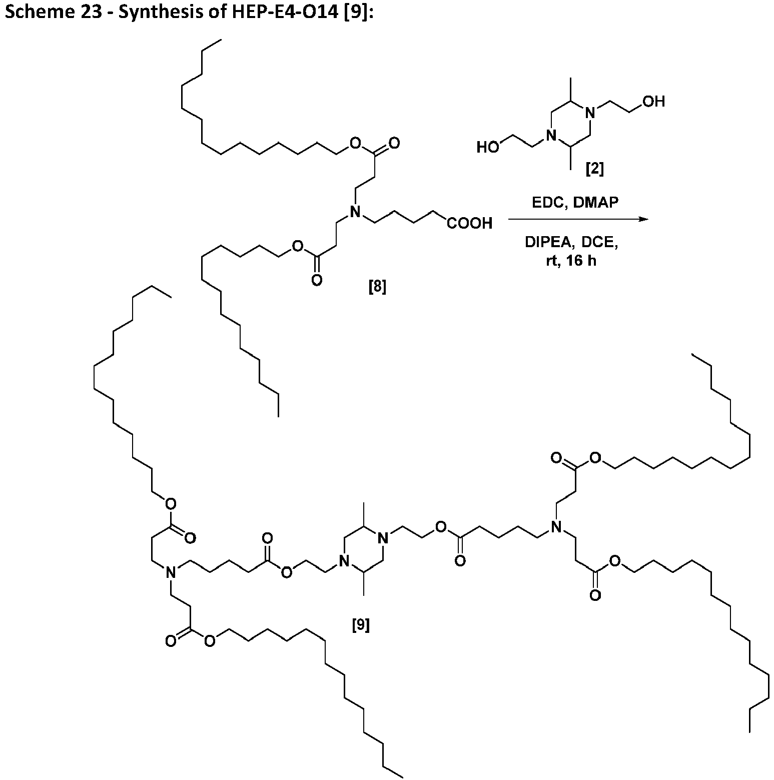

As set out in Scheme 23: To a solution of [2] (0.100 g, 0.494 mmol) in DCE (2 mL) were added [8] (0.711 g, 1.09 mmol) in DCE (8 mL), EDC (0.284 g, 1.48 mmol), DMAP (0.12 g, 0.99 mmol), DIPEA (0.86 mL, 4.94 mmol) and stirred at room temperature for 16 hours. After completion of the reaction as monitored by TLC and MS. The reaction mixture was diluted with DCM (50 mL) washed with NaHCO3 solution, water and brine. The organic layer was dried over anhydrous Na2SO4, concentrated, and the crude compound was purified by silica gel chromatography (eluent: 4% EtOAc in hexanes) to obtain pure compound [9] as a pale-yellow oil (0.22 g, 30%). It was confirmed by MS analysis.

### Results:

ESI-MS analysis: Calculated C88H169N4O12, [M+H] = 1474.27, Observed = 1474.20

### Example 24: Lipid Nanoparticle Formulation

Cationic lipids described herein can be used in the preparation of lipid nanoparticles according to methods known in the art. For example, suitable methods include methods described in International Publication No. WO 2018/089801.

One exemplary process for lipid nanoparticle formulation is *Process A* of WO 2018/089801 (see, e.g., Example 1 and Figure 1 of WO 2018/089801). Process A ("A") relates to a conventional method of encapsulating mRNA by mixing mRNA with a mixture of lipids, without first pre-forming the lipids into lipid nanoparticles. In an exemplary process, an ethanol lipid solution and an aqueous buffered solution of mRNA were prepared separately. A solution of mixture of lipids (cationic lipid, helper lipids, zwitterionic lipids, PEG lipids etc.) was prepared by dissolving lipids in ethanol. The mRNA solution was prepared by dissolving the mRNA in citrate buffer. Then, these two solutions were mixed using a pump system. In some instances, the two solutions were mixed using a gear pump system. In certain embodiments, the two solutions were mixing using a 'T' junction (or "Y" junction). The mixture was then purified by diafiltration with a TFF process. The resultant formulation concentrated and stored at 2-8 °C until further use.

A second exemplary process for lipid nanoparticle formulation is *Process B* of WO 2018/089801 (see, e.g., Example 2 and Figure 2 of WO 2018/089801). Process B ("B") refers to a process of encapsulating messenger RNA (mRNA) by mixing pre-formed lipid nanoparticles with mRNA. A range of different conditions, such as varying temperatures (i.e., heating or not heating the mixture), buffers, and concentrations, may be employed in Process B. In an exemplary process, lipids dissolved in ethanol and citrate buffer were mixed using a pump system. The instantaneous mixing of the two streams resulted in the formation of empty lipid nanoparticles, which was a self-assembly process. The resultant formulation mixture was empty lipid nanoparticles in citrate buffer containing alcohol. The formulation was then subjected to a TFF purification process wherein buffer exchange occurred. The resulting suspension of pre-formed empty lipid nanoparticles was then mixed with mRNA using a pump system. For certain cationic lipids, heating the solution post-mixing resulted in a higher percentage of lipid nanoparticles containing mRNA and a higher total yield of mRNA.

### Example 25: Delivery of Firefly Luciferase (FFL) mRNA by intratracheal administration

Lipid nanoparticle formulations prepared using Process A comprising FFL mRNA, cationic lipid, DMG-PEG2000, cholesterol and DOPE (40:5:25:30 or 45:5:20:30 mol % ratio) were administered to male CD1 mice (6-8 weeks old) by a single intratracheal aerosol administration via a Microsprayer^{®} (50ul/animal) while under anesthesia. At approximately 24 hours post-dose, the animals were dosed with luciferin at 150 mg/kg (60 mg/ml) by intraperitoneal injection at 2.5ml/kg. After 5-15 minutes, all animals were imaged using an IVIS imaging system to measure luciferase production in the lung. **Figure 1** shows that lipid nanoparticles comprising the cationic lipids descried herein are effective in delivering FFL mRNA in vivo based on positive luciferase activity.

## Claims

1. A compound having a structure according to Formula (I'): 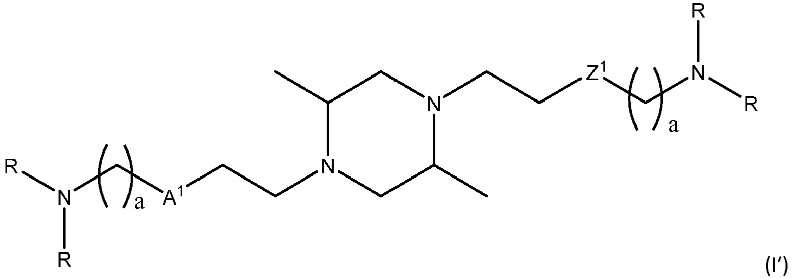 or a pharmaceutically acceptable salt thereof wherein:
**A¹** is selected from 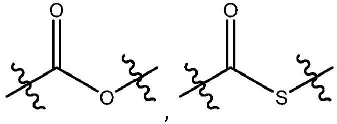 and -S-S-, wherein the left hand side of each depicted structure is bound to the -(CH₂)ₐ-;
**Z¹** is selected from 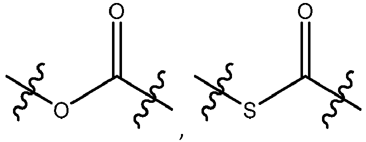 and -S-S-, wherein the right hand side of each depicted structure is bound to the -(CH₂)ₐ-;
each **R** is independently selected from:
(i) 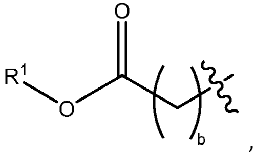 wherein each **R¹** is independently selected from optionally substituted alkyl, optionally substituted alkenyl, optionally substituted alkynyl, -optionally substituted alkyl-(C=O)-O-optionally substituted alkyl, and -optionally substituted alkyl-O-(C=O)-optionally substituted alkyl; and
(ii) 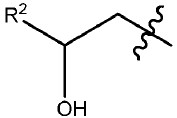 wherein each **R²** is independently selected from optionally substituted alkyl, optionally substituted alkenyl, optionally substituted alkynyl, and optionally substituted acyl;
each **a** is independently selected from 2, 3, 4, and 5; and
each **b** is independently selected from 2, 3, 4, 5, 6 and 7.

2. The compound of claim 1, wherein the compound has a structure according to Formula (I): 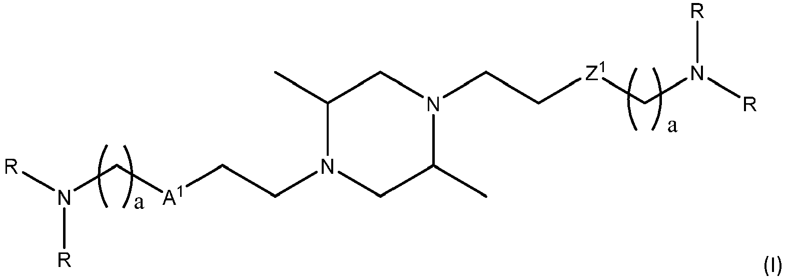 or a pharmaceutically acceptable salt thereof wherein:
**A¹** is selected from 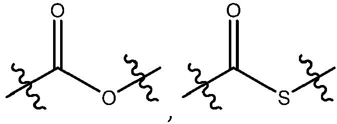 and -S-S-, wherein the left hand side of each depicted structure is bound to the -(CH₂)ₐ-;
**Z¹** is selected from 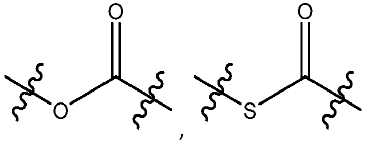 and -S-S-, wherein the right hand side of each depicted structure is bound to the -(CH₂)ₐ-;
each **R** is independently selected from:
(i) 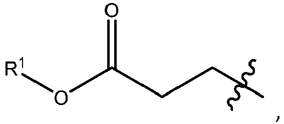 wherein each **R¹** is independently selected from optionally substituted alkyl, optionally substituted alkenyl, optionally substituted alkynyl, -optionally substituted alkyl-(C=O)-O-optionally substituted alkyl, and -optionally substituted alkyl-O-(C=O)-optionally substituted alkyl; and
(ii) 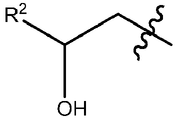 wherein each **R²** is independently selected from optionally substituted alkyl, optionally substituted alkenyl, optionally substituted alkynyl, and optionally substituted acyl; and
each **a** is independently selected from 2, 3, 4, and 5.

3. The compound of claim 1 or 2,
(a) wherein the compound has a structure according to Formula (Ia): 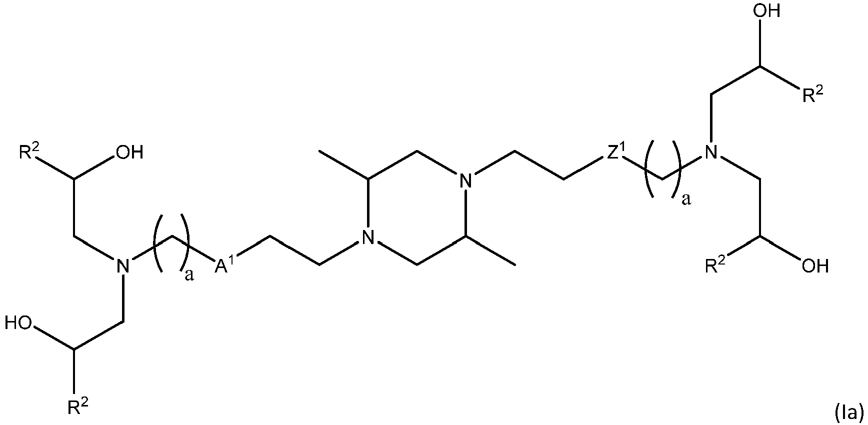 or a pharmaceutically acceptable salt thereof, or
(b) wherein the compound has a structure according to Formula (Ib): 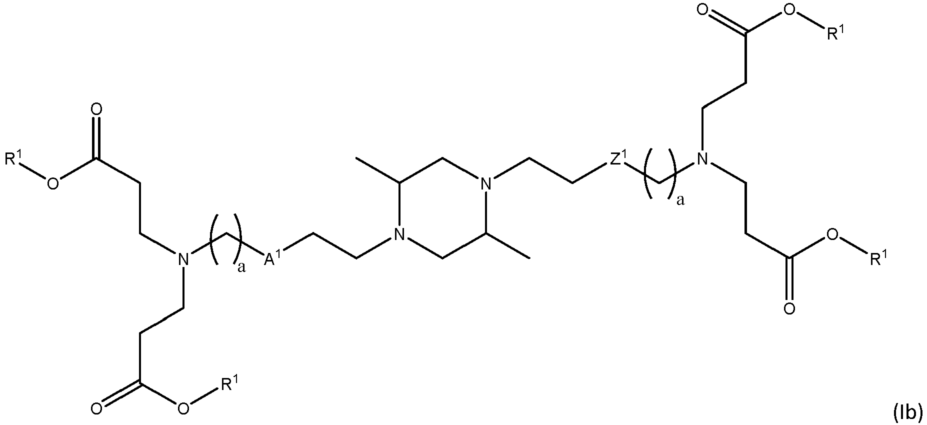
or a pharmaceutically acceptable salt thereof;
or wherein the compound has a structure according to Formula (Ib'): 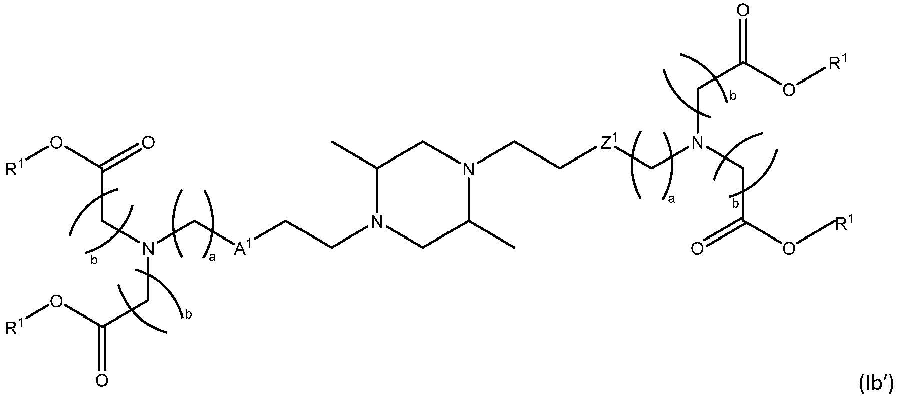
or a pharmaceutically acceptable salt thereof.

4. The compound of any one of claims 1 to 3,
(a) wherein the compound has a structure according to Formula (I'), (I) or (Ia),
(i) wherein the compound has a structure according to Formula (Ic): 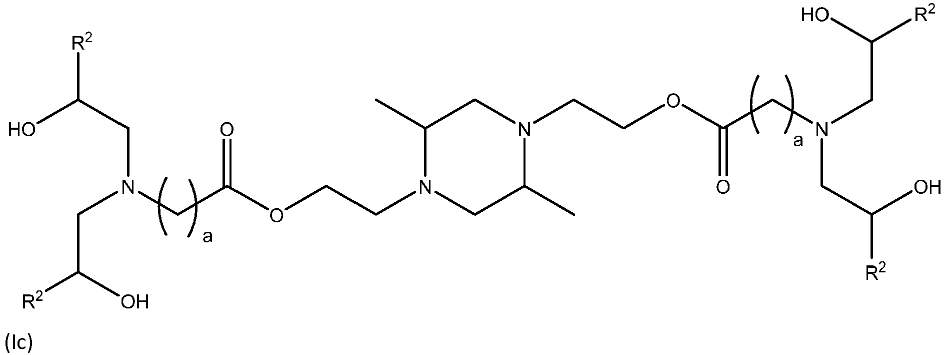 or a pharmaceutically acceptable salt thereof, or
(ii) wherein the compound has a structure according to Formula (Id): 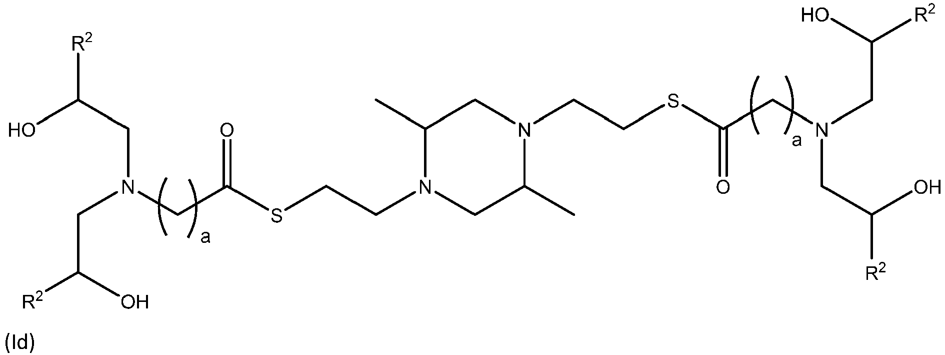 or a pharmaceutically acceptable salt thereof, or
(iii) wherein the compound has a structure according to Formula (le): 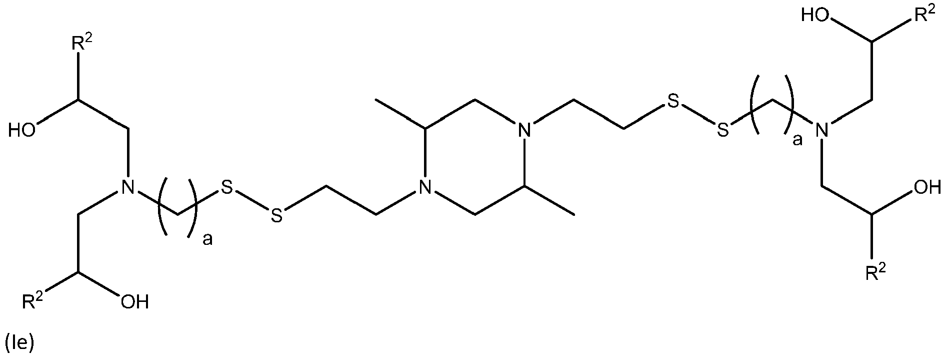 or a pharmaceutically acceptable salt thereof, or
(b) wherein the compound has a structure according to Formula (I'), (I), (lb) or (lb'), wherein the compound has a structure according to Formula (If): 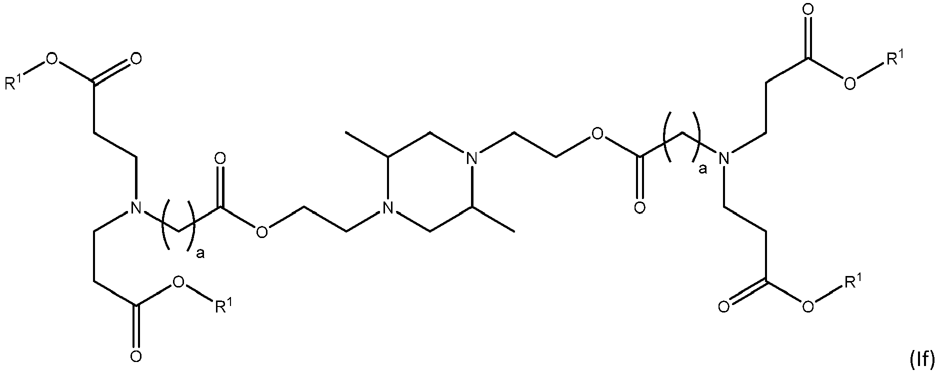
or a pharmaceutically acceptable salt thereof;
or wherein the compound has a structure according to Formula (If'): 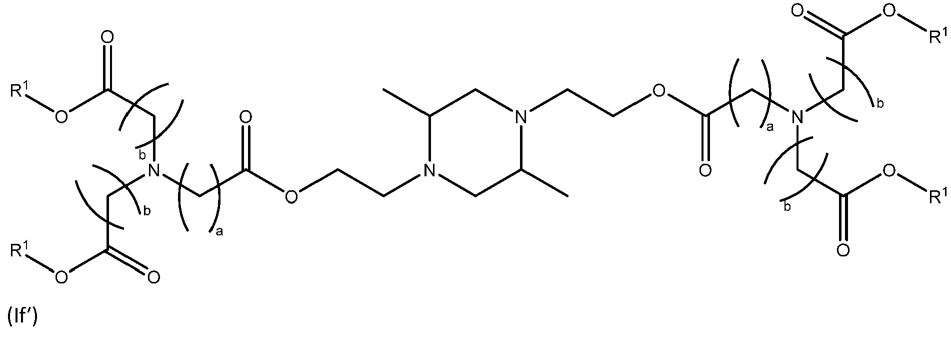
or a pharmaceutically acceptable salt thereof.

5. The compound of any one of the preceding claims, wherein each **a** is independently selected from 2, 3 and 4, optionally
(a) wherein each **a** is 2, or
(b) wherein each **a** is 3, or
(c) wherein each **a** is 4.

6. The compound of any one of claims 1 to 3 and 5 or a pharmaceutically acceptable salt thereof, wherein in the compound of Formula (I'), (I), (Ia), (Ib) or (lb'),
(a)(i) **A¹** is 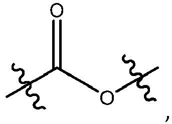 wherein the left hand side of the depicted structure is bound to the -(CH₂)ₐ-, or
(ii) **A¹** is 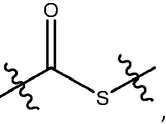 wherein the left hand side of the depicted structure is bound to the -(CH₂)ₐ-, or
(iii) **A¹** is -S-S-, and/or
(b)(i) **Z¹** is 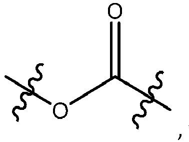 wherein the right hand side of the depicted structure is bound to the -(CH₂)ₐ-, or
(ii) **Z¹** is 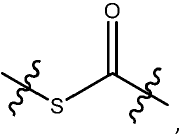 wherein the right hand side of the depicted structure is bound to the -(CH₂)ₐ-, or
(iii) **Z¹** is -S-S-.

7. The compound of any one of the preceding claims or a pharmaceutically acceptable salt thereof, wherein in the compound of Formula (I'), (I), (lb), (lb'), (If) or (If'), each **R¹** is the same, for example,
each **R¹** is optionally substituted alkyl, or
each **R¹** is optionally substituted alkenyl, or
each **R¹** is optionally substituted alkynyl, or
each **R¹** is -optionally substituted alkyl-(C=O)-O-optionally substituted alkyl, or
each **R¹** is -optionally substituted alkyl-O-(C=O)-optionally substituted alkyl, or
each **R¹** is selected from: 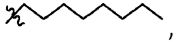 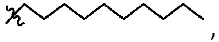 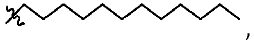 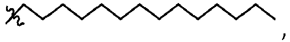 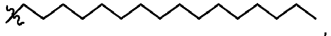 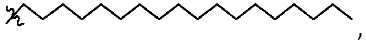 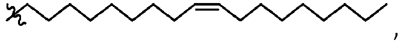 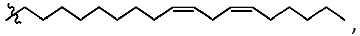 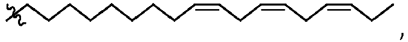 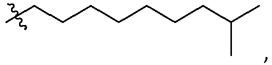 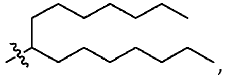 and

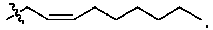

8. The compound of any one of claims 1 to 6 or a pharmaceutically acceptable salt thereof, wherein in the compound of Formula (I'), (I), (Ia), (Ic), (Id), or (Ie), each **R²** is the same, for example,
each **R²** is optionally substituted alkyl, or
each **R²** is optionally substituted alkenyl, or
each **R²** is optionally substituted alkynyl, or
each **R²** is optionally substituted acyl, or
each **R²** is selected from: 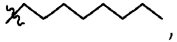 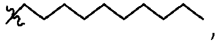 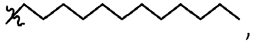 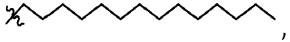 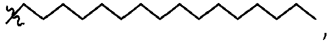 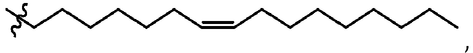 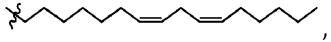 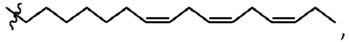 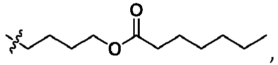 and

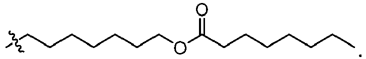

9. A compound selected from those listed in Tables A-D, or a pharmaceutically acceptable salt thereof.

10. A composition comprising the cationic lipid of any one of the preceding claims or a pharmaceutically acceptable salt thereof, one or more non-cationic lipids, one or more cholesterol-based lipids and one or more PEG-modified lipids.

11. The composition of claim 10, wherein the composition is a lipid nanoparticle, optionally a liposome, for example,
wherein the one or more cationic lipid(s) constitute(s) about 30 mol %-60 mol % of the lipid nanoparticle, and/or
wherein the one or more non-cationic lipid(s) constitute(s) 10 mol %-50 mol % of the lipid nanoparticle, and/or
wherein the one or more PEG-modified lipid(s) constitute(s) 1 mol %-10 mol % of the lipid nanoparticle, and/or
wherein the cholesterol-based lipid constitutes 10 mol %-50 mol% of the lipid nanoparticle.

12. The composition of claim 11, wherein the lipid nanoparticle encapsulates a nucleic acid, optionally an mRNA encoding a peptide or protein.

13. The composition of claim 11, wherein the lipid nanoparticle encapsulates an mRNA encoding a peptide or protein, and/or
wherein the lipid nanoparticles have an encapsulation percentage for mRNA of
(i) at least 70%;
(ii) at least 75%;
(iii) at least 80%;
(iv) at least 85%;
(v) at least 90%; or
(vi) at least 95%.

14. The composition of claim 13 for use in therapy.

15. The composition of claim 13 for use in a method of treating or preventing a disease amenable to treatment or prevention by the peptide or protein encoded by the mRNA, optionally wherein the disease is (a) a protein deficiency, optionally wherein the protein deficiency affects the liver, lung, brain or muscle, (b) an autoimmune disease, (c) an infectious disease, or (d) cancer.

16. The composition for use according to claim 14 or 15, wherein the composition is administered intravenously, intrathecally or intramuscularly, or by pulmonary delivery, optionally through nebulization.

## Patentansprüche

1. Verbindung mit einer Struktur gemäß Formel (I'): 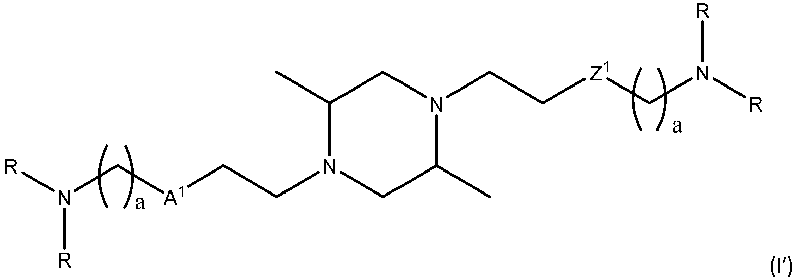 oder ein pharmazeutisch unbedenkliches Salz davon, wobei:
A¹ aus 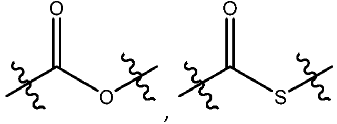 und -S-S- ausgewählt ist, wobei die linke Seite jeder dargestellten Struktur an das -(CH₂)ₐ- gebunden ist;
Z¹ aus 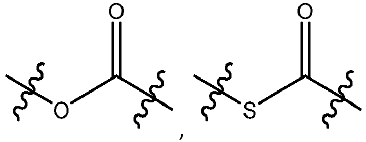 und -S-S- ausgewählt ist, wobei die rechte Seite jeder dargestellten Struktur an das -(CH₂)ₐ- gebunden ist;
R jeweils unabhängig ausgewählt ist aus:
(i) 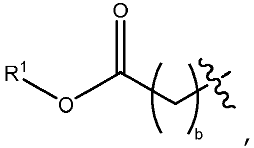 wobei R¹ jeweils unabhängig aus gegebenenfalls substituiertem Alkyl, gegebenenfalls substituiertem Alkenyl, gegebenenfalls substituiertem Alkinyl, -gegebenenfalls substituiertem Alkyl-(C=O)-O-gegebenenfalls substituiertem Alkyl und -gegebenenfalls substituiertem Alkyl-O-(C=O)-gegebenenfalls substituiertem Alkyl ausgewählt ist; und
(ii) 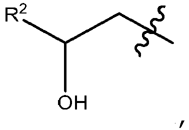 wobei R² jeweils unabhängig aus gegebenenfalls substituiertem Alkyl, gegebenenfalls substituiertem Alkenyl, gegebenenfalls substituiertem Alkinyl und gegebenenfalls substituiertem Acyl ausgewählt ist;
a jeweils unabhängig aus 2, 3, 4 und 5 ausgewählt ist; und
b jeweils unabhängig aus 2, 3, 4, 5, 6 und 7 ausgewählt ist.

2. Verbindung nach Anspruch 1, wobei die Verbindung eine Struktur gemäß Formel (I) aufweist: 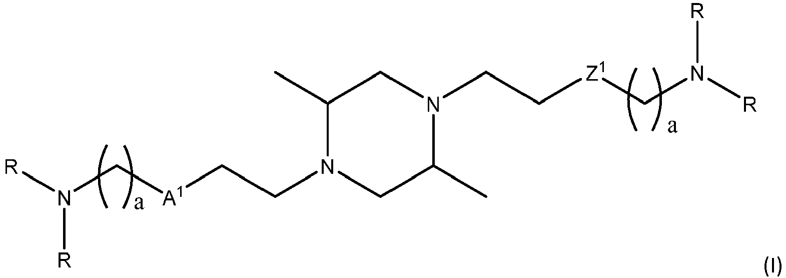 oder ein pharmazeutisch unbedenkliches Salz davon, wobei:
A¹ aus 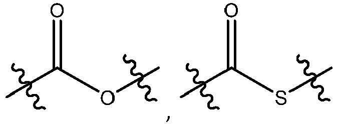 und -S-S- ausgewählt ist, wobei die linke Seite jeder dargestellten Struktur an das -(CH₂)ₐ- gebunden ist;
Z¹ aus 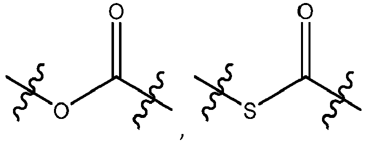 und -S-S- ausgewählt ist, wobei die rechte Seite jeder dargestellten Struktur an das -(CH₂)ₐ- gebunden ist;
R jeweils unabhängig ausgewählt ist aus:
(i) 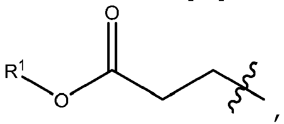 wobei R¹ jeweils unabhängig aus gegebenenfalls substituiertem Alkyl, gegebenenfalls substituiertem Alkenyl, gegebenenfalls substituiertem Alkinyl, -gegebenenfalls substituiertem Alkyl-(C=O)-O-gegebenenfalls substituiertem Alkyl und -gegebenenfalls substituiertem Alkyl-O-(C=O)-gegebenenfalls substituiertem Alkyl ausgewählt ist; und
(ii) 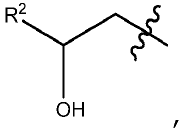 wobei R² jeweils unabhängig aus gegebenenfalls substituiertem Alkyl, gegebenenfalls substituiertem Alkenyl, gegebenenfalls substituiertem Alkinyl und gegebenenfalls substituiertem Acyl ausgewählt ist; und
a jeweils unabhängig aus 2, 3, 4 und 5 ausgewählt ist.

3. Verbindung nach Anspruch 1 oder 2,
(a) wobei die Verbindung eine Struktur gemäß Formel (la) aufweist: 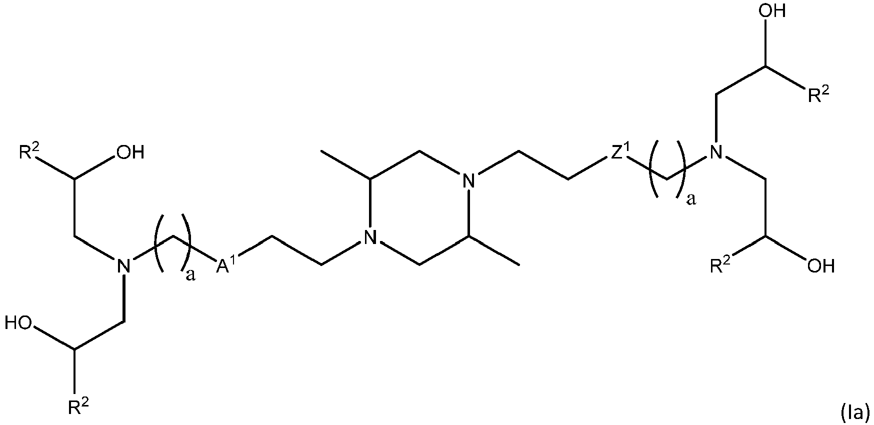 oder ein pharmazeutisch unbedenkliches Salz davon, oder
(b) wobei die Verbindung eine Struktur gemäß Formel (Ib) aufweist: 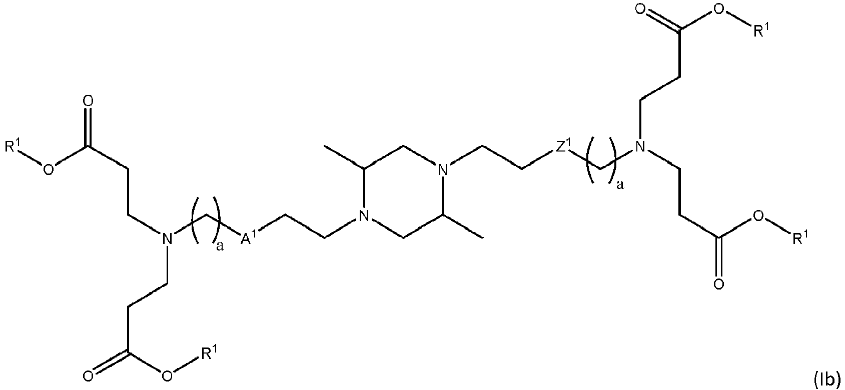
oder ein pharmazeutisch unbedenkliches Salz davon;
oder wobei die Verbindung eine Struktur gemäß Formel (Ib') aufweist: 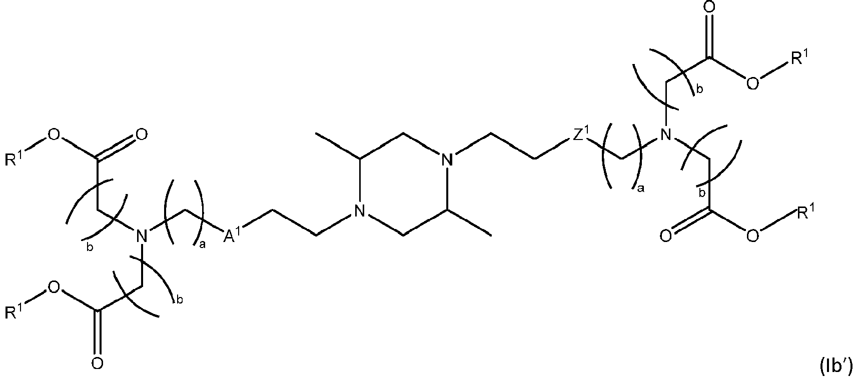
oder ein pharmazeutisch unbedenkliches Salz davon.

4. Verbindung nach einem der Ansprüche 1 bis 3,
(a) wobei die Verbindung eine Struktur gemäß Formel (I'), (I) oder (la) aufweist,
(i) wobei die Verbindung eine Struktur gemäß Formel (Ic) aufweist: 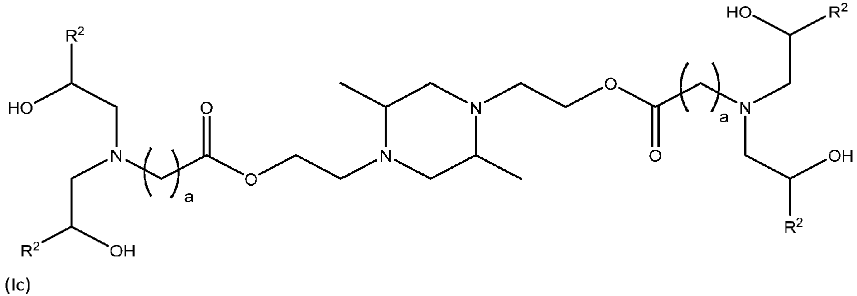 oder ein pharmazeutisch unbedenkliches Salz davon, oder
(ii) wobei die Verbindung eine Struktur gemäß Formel (Id) aufweist: 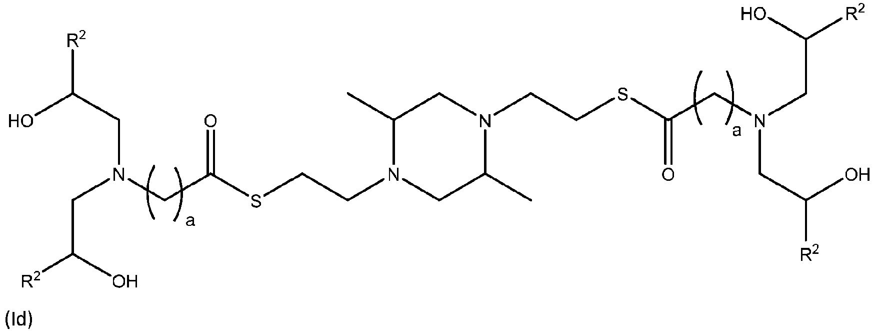 oder ein pharmazeutisch unbedenkliches Salz davon, oder
(iii) wobei die Verbindung eine Struktur gemäß Formel (le) aufweist: 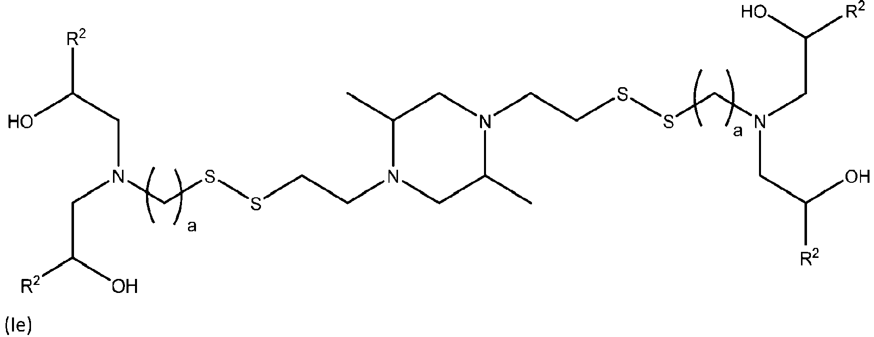 oder ein pharmazeutisch unbedenkliches Salz davon, oder
(b) wobei die Verbindung eine Struktur gemäß Formel (I'), (I), (Ib) oder (Ib') aufweist, wobei die Verbindung eine Struktur gemäß Formel (If) aufweist: 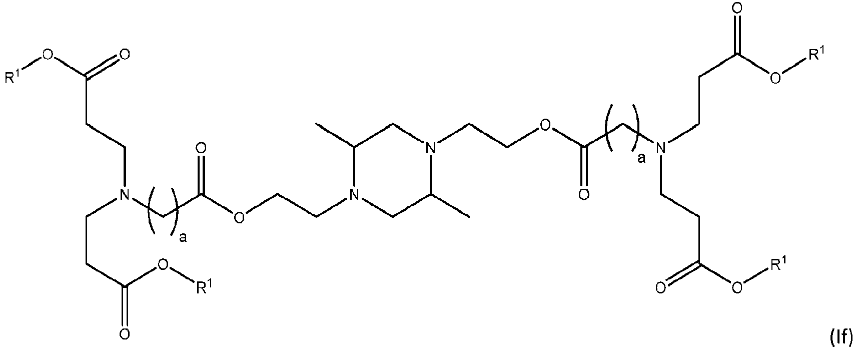
oder ein pharmazeutisch unbedenkliches Salz davon;
oder wobei die Verbindung eine Struktur gemäß Formel (If') aufweist: 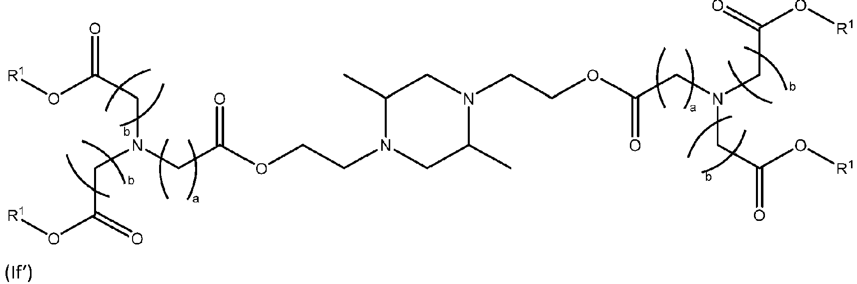
oder ein pharmazeutisch unbedenkliches Salz davon.

5. Verbindung nach einem der vorhergehenden Ansprüche, wobei a jeweils unabhängig aus 2, 3 und 4 ausgewählt ist, gegebenenfalls
(a) wobei a jeweils für 2 steht oder
(b) wobei a jeweils für 3 steht oder
(c) wobei a jeweils für 4 steht.

6. Verbindung nach einem der Ansprüche 1 bis 3 und 5 oder ein pharmazeutisch unbedenkliches Salz davon, wobei in der Verbindung der Formel (I'), (I), (la), (Ib) oder (Ib')
(a) (i) A¹ für 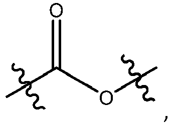 steht, wobei die linke Seite der dargestellten Struktur an das -(CH₂)ₐ- gebunden ist, oder (ii) A¹ für 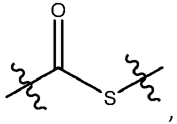 steht, wobei die linke Seite der dargestellten Struktur an das -(CH₂)ₐ- gebunden ist, oder (iii) A¹ für -S-S- steht und/oder
(b) (i) Z¹ für 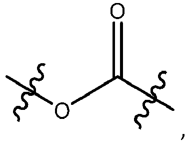 steht, wobei die rechte Seite der dargestellten Struktur an das -(CH₂)ₐ- gebunden ist, oder
(ii) Z¹ für 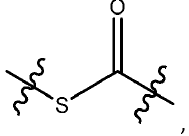 steht, wobei die rechte Seite der dargestellten Struktur an das -(CH₂)ₐ- gebunden ist, oder
(iii) Z¹ für -S-S- steht.

7. Verbindung nach einem der vorhergehenden Ansprüche oder ein pharmazeutisch unbedenkliches Salz davon, wobei in der Verbindung der Formel (I'), (I), (Ib), (Ib'), (If) oder (If') R¹ jeweils gleich ist, beispielsweise
R¹ jeweils für gegebenenfalls substituiertes Alkyl steht oder
R¹ jeweils für gegebenenfalls substituiertes Alkenyl steht oder
R¹ jeweils für gegebenenfalls substituiertes Alkinyl steht oder
R¹ jeweils für -gegebenenfalls substituiertes Alkyl-(C=O)-O-gegebenenfalls substituiertes Alkyl steht oder
R¹ jeweils für -gegebenenfalls substituiertes Alkyl-(C=O)-gegebenenfalls substituiertes Alkyl steht oder
R¹ jeweils ausgewählt ist aus: 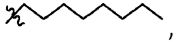 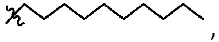 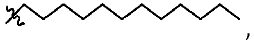 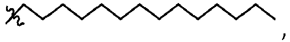 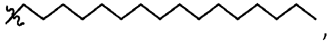 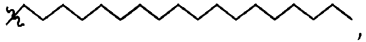 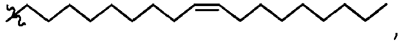 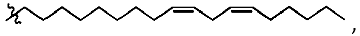 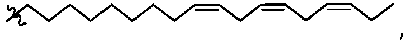 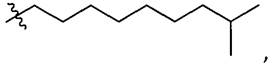 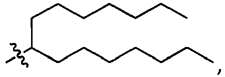 und

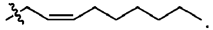

8. Verbindung nach einem der Ansprüche 1 bis 6 oder ein pharmazeutisch unbedenkliches Salz davon, wobei in der Verbindung der Formel (I'), (I), (la), (Ic), (Id) oder (Ie) R² jeweils gleich ist, beispielsweise
R² jeweils für gegebenenfalls substituiertes Alkyl steht oder
R² jeweils für gegebenenfalls substituiertes Alkenyl steht oder
R² jeweils für gegebenenfalls substituiertes Alkinyl steht oder
R² jeweils für gegebenenfalls substituiertes Acyl steht oder
R² jeweils ausgewählt ist aus: 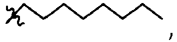 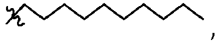 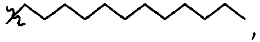 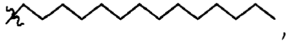 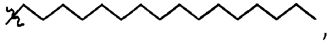 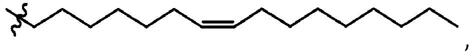 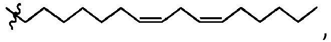 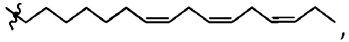 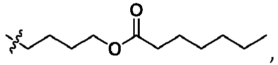 und

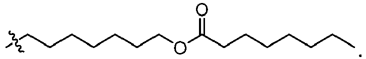

9. Verbindung, ausgewählt aus den in den Tabellen A-D aufgeführten Verbindungen, oder ein pharmazeutisch unbedenkliches Salz davon.

10. Zusammensetzung, umfassend das kationische Lipid nach einem der vorhergehenden Ansprüche oder ein pharmazeutisch unbedenkliches Salz davon, ein oder mehrere nichtkationische Lipide, ein oder mehrere Lipide auf Cholesterinbasis und ein oder mehrere PEGmodifizierte Lipide.

11. Zusammensetzung nach Anspruch 10, wobei es sich bei der Zusammensetzung um ein Lipidnanopartikel, gegebenenfalls ein Liposomen, handelt, beispielsweise
wobei das eine oder die mehreren kationischen Lipide etwa 30 Mol-%-60 Mol-% des Lipidnanopartikels ausmacht bzw. ausmachen und/oder
wobei das eine oder die mehreren nichtkationischen Lipide 10 Mol-%-50 Mol-% des Lipidnanopartikels ausmacht bzw. ausmachen und/oder
wobei das eine oder die mehreren PEG-modifizierten Lipide 1 Mol-%-10 Mol-% des Lipidnanopartikels ausmacht bzw. ausmachen und/oder
Wobei das Lipid auf Cholesterinbasis 10 Mol-%-50 Mol-% des Lipidnanopartikels ausmacht bzw. ausmachen.

12. Zusammensetzung nach Anspruch 11, wobei das Lipidnanopartikel eine Nukleinsäure, gegebenenfalls eine ein Peptid oder Protein codierende mRNA, verkapselt.

13. Zusammensetzung nach Anspruch 11, wobei das Lipidnanopartikel eine ein Peptid oder Protein codierende mRNA verkapselt und/oder
wobei die Lipidnanopartikel einen Verkapselungsprozentsatz für mRNA von
(i) mindestens 70 %;
(ii) mindestens 75 %;
(iii) mindestens 80 %;
(iv) mindestens 85 %;
(v) mindestens 90 %; oder
(vi) mindestens 95 % aufweisen.

14. Zusammensetzung nach Anspruch 13 zur Verwendung bei der Therapie.

15. Zusammensetzung nach Anspruch 13 zur Verwendung bei einem Verfahren zur Behandlung oder Prävention einer Erkrankung, die der Behandlung oder Prävention durch das von der mRNA codierte Peptid oder Protein zugänglich ist, gegebenenfalls wobei es sich bei der Erkrankung um (a) einen Proteinmangel, gegebenenfalls wobei der Proteinmangel die Leber, die Lunge, das Gehirn oder den Muskel betrifft, (b) eine Autoimmunerkrankung, (c) eine Infektionserkrankung oder (d) Krebs handelt.

16. Zusammensetzung zur Verwendung nach Anspruch 14 oder 15, wobei die Zusammensetzung intravenös, intrathekal oder intramuskulär oder durch pulmonale Verabreichung, gegebenenfalls durch Verneblung, verabreicht wird.

## Revendications

1. Composé ayant une structure selon la formule (I') : 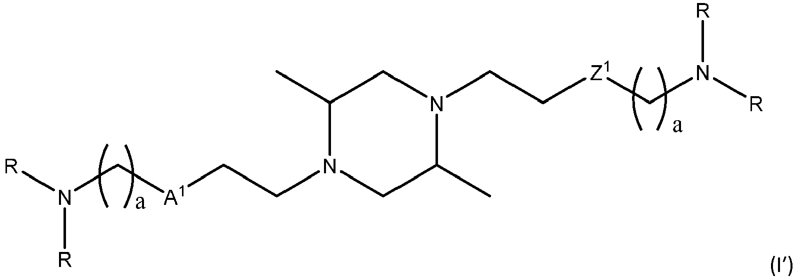 ou un sel pharmaceutiquement acceptable de celui-ci, dans lequel :
A¹ est choisi parmi 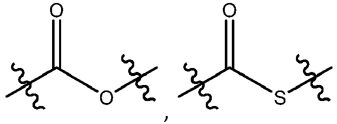 et -S-S-, le côté gauche de chaque structure représentée étant lié au - (CH₂)ₐ- ;
Z¹ est choisi parmi 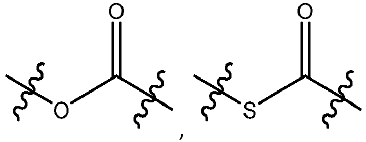 et -S-S-, le côté droit de chaque structure représentée étant lié au -(CH₂)ₐ- ;
chaque R est indépendamment choisi parmi :
(i) 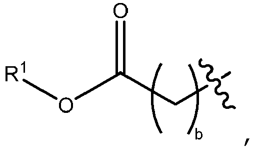 dans lequel chaque R¹ est indépendamment choisi parmi alkyle éventuellement substitué, alcényle éventuellement substitué, alcynyle éventuellement substitué, (alkyle éventuellement substitué)-(C=O)-O-(alkyle éventuellement substitué) et (alkyle éventuellement substitué)-O-(C=O)-(alkyle éventuellement substitué) ; et
(ii) 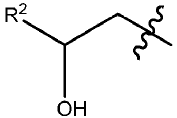 dans lequel chaque R² est indépendamment choisi parmi alkyle éventuellement substitué, alcényle éventuellement substitué, alcynyle éventuellement substitué et acyle éventuellement substitué ;
chaque a est indépendamment choisi parmi 2, 3, 4 et 5 ; et
chaque b est indépendamment choisi parmi 2, 3, 4, 5, 6 et 7.

2. Composé selon la revendication 1, le composé ayant une structure selon la formule (I) : 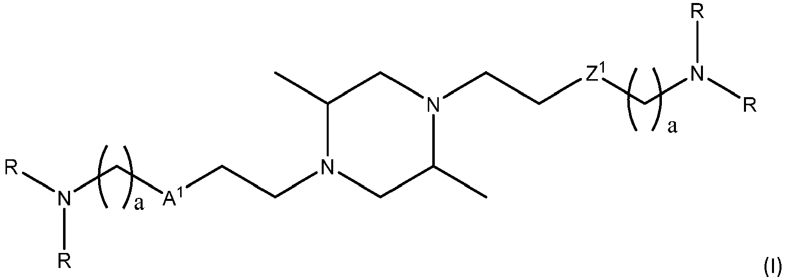 ou un sel pharmaceutiquement acceptable de celui-ci, dans lequel :
A¹est choisi parmi 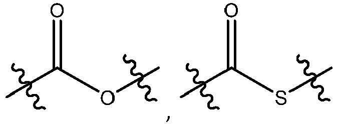 et -S-S-, le côté gauche de chaque structure représentée étant lié au -(CH₂)ₐ- ;
Z¹ est choisi parmi 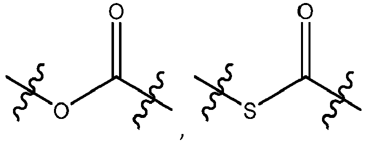 et -S-S-, le côté droit de chaque structure représentée étant lié au -(CH₂)ₐ- ;
chaque R est indépendamment choisi parmi :
(i) 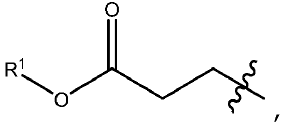 dans lequel chaque R¹ est indépendamment choisi parmi alkyle éventuellement substitué, alcényle éventuellement substitué, alcynyle éventuellement substitué, (alkyle éventuellement substitué)-(C=O)-O-(alkyle éventuellement substitué) et (alkyle éventuellement substitué)-O-(C=O)-(alkyle éventuellement substitué) ; et
(ii) 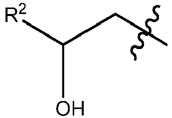 dans lequel chaque R² est indépendamment choisi parmi alkyle éventuellement substitué, alcényle éventuellement substitué, alcynyle éventuellement substitué et acyle éventuellement substitué ; et
chaque a est indépendamment choisi parmi 2, 3, 4 et 5.

3. Composé selon la revendication 1 ou 2,
(a) le composé ayant une structure selon la formule (Ia) : 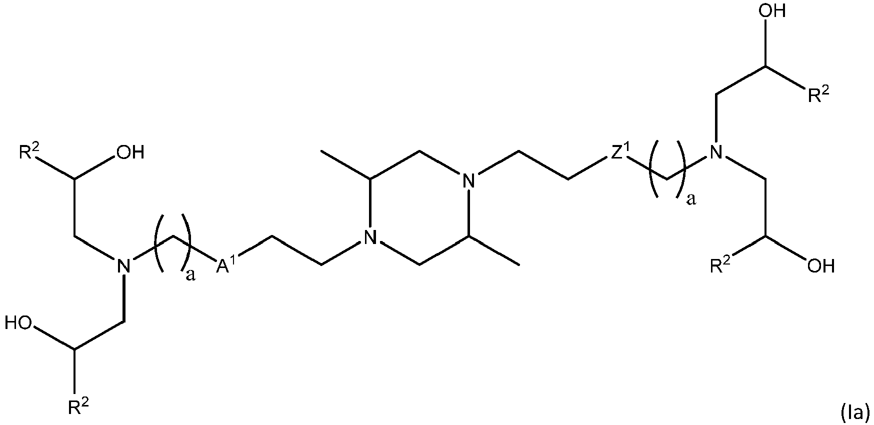 ou un sel pharmaceutiquement acceptable de celui-ci, ou
(b) le composé ayant une structure selon la formule (Ib) : 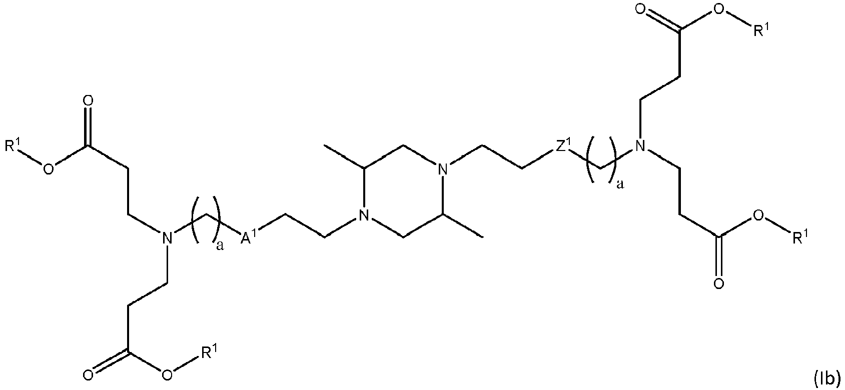
ou un sel pharmaceutiquement acceptable de celui-ci ;
ou le composé ayant une structure selon la formule (Ib') : 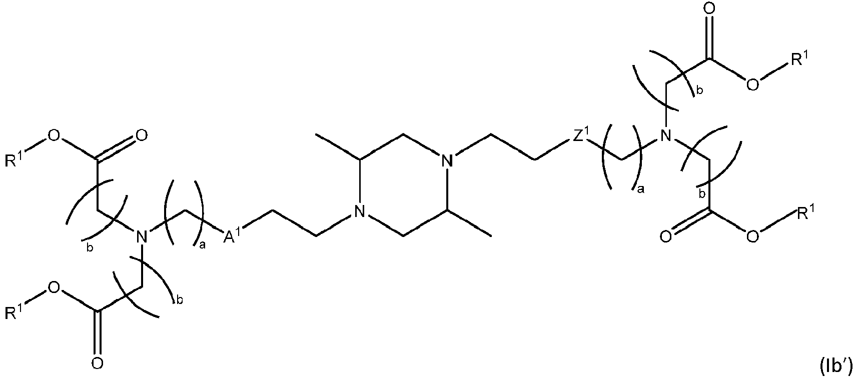
ou un sel pharmaceutiquement acceptable de celui-ci.

4. Composé selon l'une quelconque des revendications 1 à 3,
(a) le composé ayant une structure selon la formule (I'), (I) ou (Ia),
(i) le composé ayant une structure selon la formule (Ic) : 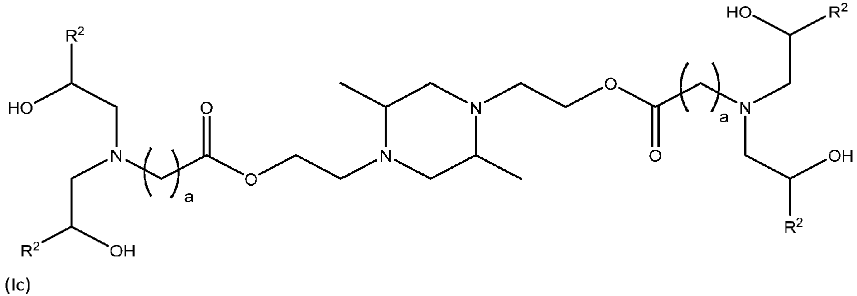 ou un sel pharmaceutiquement acceptable de celui-ci, ou
(ii) le composé ayant une structure selon la formule (Id) : 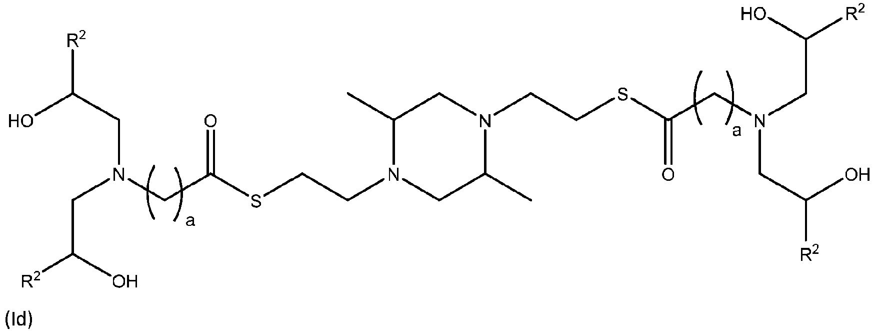 ou un sel pharmaceutiquement acceptable de celui-ci, ou
(iii) le composé ayant une structure selon la formule (Ie) : 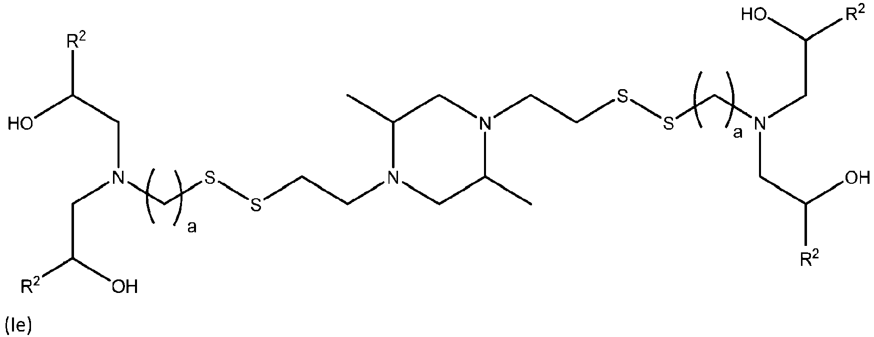 ou un sel pharmaceutiquement acceptable de celui-ci, ou
(b) le composé ayant une structure selon la formule (I'), (I), (Ib) ou (Ib'), ou le composé ayant une structure selon la formule (If) : 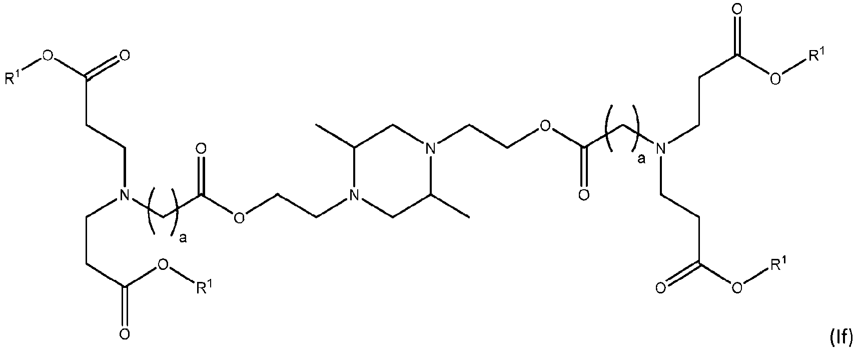
ou un sel pharmaceutiquement acceptable de celui-ci ;
ou le composé ayant une structure selon la formule (If') : 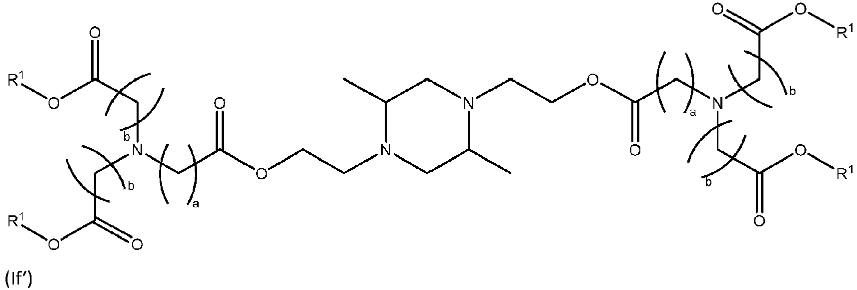
ou un sel pharmaceutiquement acceptable de celui-ci.

5. Composé selon l'une quelconque des revendications précédentes, dans lequel chaque a est indépendamment choisi parmi 2, 3 et 4, facultativement
(a) dans lequel chaque a est 2, ou
(b) dans lequel chaque a est 3, ou
(c) dans lequel chaque a est 4.

6. Composé selon l'une quelconque des revendications 1 à 3 et 5 ou un sel pharmaceutiquement acceptable de celui-ci, dans lequel, dans le composé de formule (I'), (I), (Ia), (Ib) ou (Ib'),
(a) (i) A¹ est 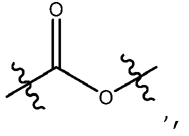 le côté gauche de la structure représentée étant lié au -(CH₂)ₐ-, ou
(ii) A¹ est 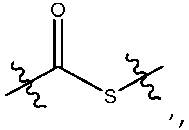 le côté gauche de la structure représentée étant lié au -(CH₂)ₐ-, ou (iii) A¹ est -S-S-, et/ou
(b) (i) Z¹ est 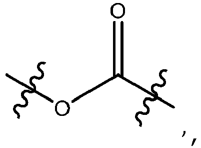 le côté droit de la structure représentée étant lié au -(CH₂)ₐ-, ou
(ii) Z¹ est 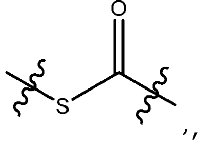 le côté droit de la structure représentée étant lié au -(CH₂)ₐ-, ou
(iii) Z¹ est -S-S-.

7. Composé selon l'une quelconque des revendications précédentes ou un sel pharmaceutiquement acceptable de celui-ci, dans lequel, dans le composé de formule (I'), (I), (Ib), (Ib'), (If) ou (If'), chaque R¹ est identique, par exemple,
chaque R¹ est alkyle éventuellement substitué, ou
chaque R¹ est alcényle éventuellement substitué, ou
chaque R¹ est alcynyle éventuellement substitué, ou
chaque R¹ est (alkyle éventuellement substitué)-(C=O)-O-(alkyle éventuellement substitué), ou
chaque R¹ est (alkyle éventuellement substitué)-O-(C=O)-(alkyle éventuellement substitué), ou
chaque R¹ est choisi parmi : 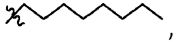 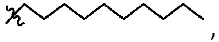 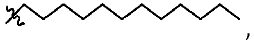 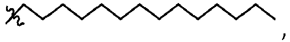 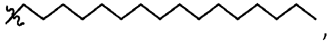 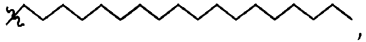 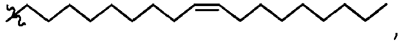 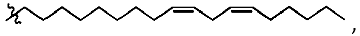 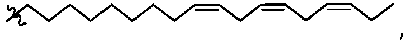 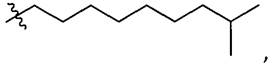 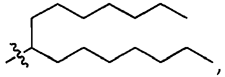 et

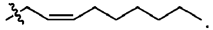

8. Composé selon l'une quelconque des revendications 1 à 6 ou sel pharmaceutiquement acceptable de celui-ci, dans lequel, dans le composé de formule (I'), (I), (Ia), (Ic), (Id) ou (Ie), chaque R² est identique, par exemple,
chaque R² est alkyle éventuellement substitué, ou
chaque R² est alcényle éventuellement substitué, ou
chaque R² est alcynyle éventuellement substitué, ou
chaque R² est acyle éventuellement substitué, ou
chaque R² est choisi parmi : 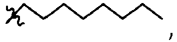 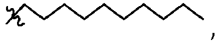 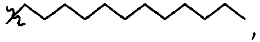 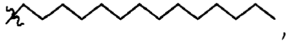 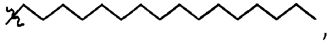 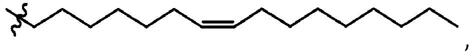 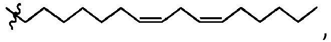 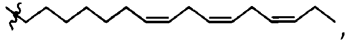 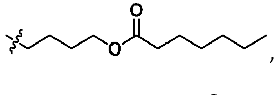 et

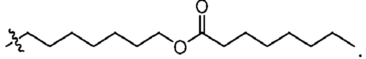

9. Composé choisi parmi les composés répertoriés dans les tableaux A à D, ou un sel pharmaceutiquement acceptable de celui-ci.

10. Composition comprenant le lipide cationique selon l'une quelconque des revendications précédentes ou un sel pharmaceutiquement acceptable de celui-ci, un ou plusieurs lipides non cationiques, un ou plusieurs lipides à base de cholestérol et un ou plusieurs lipides modifiés par PEG.

11. Composition selon la revendication 10, la composition étant une nanoparticule lipidique, éventuellement un liposome, par exemple,
les un ou plusieurs lipides cationiques constituant environ 30 % en moles à 60 % en moles de la nanoparticule lipidique, et/ou
les un ou plusieurs lipides non cationiques constituant 10 % en moles à 50 % en moles de la nanoparticule lipidique, et/ou
les un ou plusieurs lipides modifiés par PEG constituant 1 % en moles à 10 % en moles de la nanoparticule lipidique, et/ou
le lipide à base de cholestérol constituant 10 % en moles à 50 % en moles de la nanoparticule lipidique.

12. Composition selon la revendication 11, la nanoparticule lipidique encapsulant un acide nucléique, éventuellement un ARNm codant pour un peptide ou une protéine.

13. Composition selon la revendication 11, la nanoparticule lipidique encapsulant un ARNm codant pour un peptide ou une protéine, et/ou
les nanoparticules lipidiques ayant un pourcentage d'encapsulation pour l'ARNm de
(i) au moins 70 % ;
(ii) au moins 75 % ;
(iii) au moins 80 % ;
(iv) au moins 85 % ;
(v) au moins 90 % ; ou
(vi) au moins 95 %.

14. Composition selon la revendication 13, destinée à être utilisée en thérapie.

15. Composition selon la revendication 13, destinée à être utilisée dans un procédé de traitement ou de prévention d'une maladie pouvant être traitée ou prévenue par le peptide ou la protéine codé(e) par l'ARNm, la maladie étant éventuellement (a) un déficit en protéine, le déficit en protéine affectant le foie, les poumons, le cerveau ou les muscles, (b) une maladie auto-immune, (c) une maladie infectieuse ou (d) un cancer.

16. Composition pour une utilisation selon la revendication 14 ou 15, la composition étant administrée par voie intraveineuse, intrathécale ou intramusculaire, ou par administration pulmonaire, éventuellement par nébulisation.
